# EUROPEAN PATENT SPECIFICATION

(11) **EP 1 658 263 B1**
(45) Date of publication and mention of the grant of the patent: **02.06.2010**
(21) Application number: 04738987.9
(22) Date of filing: 09.07.2004
(51) Int. Cl.: C07C 225/22, C07C 275/40, A61K 31/136, A61P 27/02, A61P 29/00, A61P 35/00

(54) **NOVEL AMINOBENZOPHENONE COMPOUNDS**
AMINOBENZOPHENONVERBINDUNGEN
NOUVEAUX COMPOSES D'AMINOBENZOPHENONE

(30) Priority: 24.07.2003 US 489488 P
(43) Date of publication of application: 24.05.2006
(73) Proprietor: LEO PHARMA A/S, 2750 Ballerup (DK)
(72) Inventor: OTTOSEN, Erik, Rytter, DK-3650 Olstykke (DK); HORNEMAN, Anne Marie, DK-3050 Humlebaek (DK); LIANG, Xifu, DK-2600 Glostrup (DK); SCHOU, Soren, Christian, DK-2400 Kobenhavn NV (DK); HAVEZ, Sophie, Elisabeth, DK-2500 Valbyhavn v (DK); SABROE, Thomas, Peter, DK-2830 Virum (DK)
(86) International application number: PCT/DK2004/000490
(87) International publication number: WO 2005/009940

(56) References cited:
- WO-A-01/05744
- WO-A-01/05745
- WO-A-01/05746
- WO-A-01/05749
- WO-A-01/05751
- WO-A-01/42189
- WO-A-02/45752
- WO-A-98/32730
- WO-A-02/076447

## Description

### FIELD OF THE INVENTION

The present invention relates to a novel type of aminobenzophenones and to their use in therapy.

### BACKGROUND OF THE INVENTION

Aminobenzophenones are well known from the scientific as well as the patent literature. Thus, WO 98/32730, WO 01/05746, WO 01/05749, WO 01/05751, WO 01/05744, WO 02/45752 and WO 01/05745 all disclose compounds with the common core structure 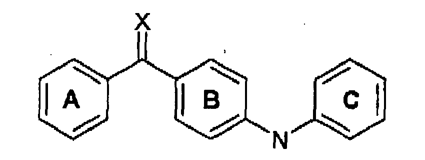 wherein the phenyl ring C is substituted by amine derivatives. Moreover, WO 01/42189 and WO 02/076447 disclose compounds with a similar structure, but with different substituents in phenyl ring A. Finally, WO 01/90074 and WO 02/083622 disclose compounds where the phenyl rings A and C respectively are replaced by heterocycles, The compounds disclosed in these patent applications are indicated to be inhibitors of interleukin 1β (IL-1β) and tumour necrosis factor α (TNF-α) secretion *in vitro,* which makes the compounds potentially useful in the treatment of inflammatory diseases where the production of cytokines is involved in the pathogenesis. Allegedly, aminobenzophenones exert their effect by inhibiting the p38 MAP kinase, which in turn inhibits the production of IL-1β and TNF-α.

The preparation of structurally related aminobenzophenones useful as dyes for textiles is disclosed in Man-Made Text. India (1987), 30(6), 275-6, Man-Made Text. India (1986), 29(5), 224-30, and Man-Made Text. India (1985), 28(11), 425, 427-9, 431.

### SUMMARY OF THE INVENTION

It has surprisingly been found that novel aminobenzophenone derivatives are potent inhibitors of interleukin 1β (IL-1β) and tumour necrosis factor α (TNF-α) secretion *in vitro* and *in vivo,* suggesting their utility in the treatment and/or prevention of inflammatory diseases and other conditions where the secretion and modulation of proinflammatory cytokines is involved in the pathogenesis.
It has been found that aminobenzophenone derivatives of the present invention exert their-anti-inflammatory effect by inhibiting or downregulating MAP kinases, more specifically the p38 MAP kinase, a stress-activated protein which is an important element of the signal transduction pathway leading to the production of proinflammatory cytokines.
The aminobenzophenone derivatives of the present invention may furthermore be useful in the treatment of cancer or ophthalmic diseases or conditions.

Accordingly, the present invention relates to a compound of general formula I 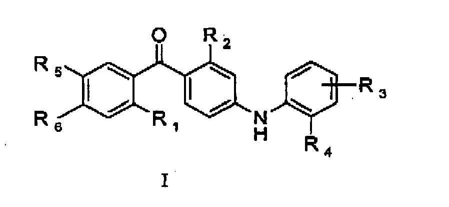 wherein
R₁ is halogen, hydroxy, mercapto, trifluoromethyl, amino, C₁₋₄alkyl, C₂₋₄alkenyl, C₂₋₄alkynyl, C₁₋₄alkoxy, C₁₋₄ alkylthio, C₁₋₆alkylamino, C₁₋₄alkoxycarbonyl, cyano,-CONH₂ or nitro;
R₂ is halogen, amino, C₁₋₄alkyl or nitro;
R₃ represents one or more same or different substituents selected from the group consisting of hydrogen, halogen, C₁₋₄alkyl or C₁₋₄alkoxy;
R₄ is fluoro;
one of R₅ and R₆ is -COOH, -C(O)NHOH, -C(O)NHNH₂, Y₂R₉, Y₂R₉Y₃R₁₀, C₁₋₆alkyl-Y₂R₉, C₁₋₆alkyl-Y₂R₉Y₃R₁₀, C₂₋₆alkenyl-Y₂R₉, C₂₋₆alkenyl-Y₂R₉Y₃R₁₀, Y₂R₉-C₁₋₆-alkyl-Y₃R₁₀, Y₂R₉-C₂₋₆-alkenyl-Y₃R₁₀, C₃₋₁₂carbocyclyl-Y₂R₉, C₃₋₁₂carbocyclyl-Y₂R₉Y₃R₁₀, C₁₋₁₂heterocyclyl-Y₂R₉, C₁₋₁₂heterocyclyl-Y₂R₉Y₃R₁₀, C₃₋₁₂carbocyclyl-C₁₋₆-alkyl-Y₂R₉, C₃₋₁₂carbocyclyl-C₁₋₆-alkyl-Y₂R₉Y₃R₁₀, C₁₋₁₂heterocyclyl-C-₁₋₆-alkyl-Y₂R₉, C₁₋₁₂ heterocyclyl-C₁₋₆-alkyl-Y₂R₉Y₃R₁₀, C₃₋₁₂carbocyclyl- C₁₋₆-alkyl-Y₃R₁₀, C₁₋₁₂ heterocyclyl-C₁₋₆-alkyl-Y₃R₁₀, C₁₋₂₂heterocyclyl-C₁₋₁₀alkyl, C₃₋₁₂carbocyclyl-C₁₋₁₀ alkyl, C₂₋₁₀alkyl-C₁₋₁₂heterocyclyl, C₁₋₁₀alkyl-C₃₋₁₂carbocyclyl, C₁₋₁₀alkyl, C₂₋₁₀ alkenyl, C₂₋₁₀ alkynyl, C₃₋₁₂carbocyclyl or C₁₋₁₂heterocyclyl, each of which being optionally substituted by one or more, same or different substituents represented by R₇, and the other is hydrogen, halogen, hydroxy, mercapto, trifluoromethyl, amino, C₁₋₄alkyl, C₂₋₄alkenyl, C₂₋₄alkynyl, C₁₋₄alkoxy, C₁₋₄ alkylthio, C₁₋₆alkylamino, C₁₋₄alkoxycarbonyl, cyano, -CONH₂ or nitro;
with the proviso that when R₅ or R₆ is phenyl, C₁₋₅alkyl or C₂₋₃alkenyl, said R₅ or R₆ is substituted by one or more, same or different substituents represented by R₇ (except three fluorine when R₅ or R₆ is methyl),
R₇ is halogen, hydroxy, mercapto, trifluoromethyl, amino, C₁₋₄alkyl, C₁₋₆ hydroxyalkyl, C₁₋₄alkoxy, C₁₋₄alkylthio, C₁₋₆ alkylamino, C₁₋₄alkoxycarbonyl, C₁₋₉ trialkylammonium in association with an anion, cyano, azido, nitro, -S(O)₂NH₂,-S(O)₂NRₐR_{b}, -S(O)₂R, -COOH, -CONH₂, -NRₐC(O)R', -CONHR' or -CONRR', wherein R and R' are same or different, each representing hydrogen or C₁₋₃alkyl;
Rₐ, R_{b} and R_{c} are the same or different, each representing hydrogen, C₁₋₄alkyl, C₂₋₄ alkenyl, C₂₋₄ alkynyl, C₃₋₈carbocyclyl, C₁₋₁₂heterocyclyl or aryl, each of C₁₋₄alkyl, C₂₋₄alkenyl, C₂₋₄ alkynyl, C₃₋₈carbocyclyl, C₁₋₁₂heterocyclyl or aryl being optionally substituted by one or more, same or different substituents represented by R₇;
Y₂ is -O-, -S-, -S(O)-, -S(O)₂-_{,} -NRₐ-, -NRₐC(O)NR_{b}-, -NRₐC(O)-, -C(O)NRₐ-,-C(O)NRₐO-, -C(O)-, -NRₐC(O)O-, -NRₐS(O)₂-, -OC(O)-, -C(O)O-,-C(O)NRₐNR_{b}C(S)NR_{c}-, -C(O)NRₐNR_{b}-, or -S(O)₂NRₐ-;
R₉ is C₁₋₁₀alkyl-C₁₋₁₂heterocyclyl, C₁₋₁₀alkyl-C₃₋₁₂carbocyclyl, C₁₋₁₀alkyl, C₂₋₁₀ alkenyl, C₂₋₁₀ alkynyl, C₃₋₁₂carbocyclyl, C₁₋₁₂heterocyclyl, C₃₋₁₂carbocyclyl-C₁₋₁₀ alkyl, or C₁₋₁₂heterocyclyl-C₁₋₁₀alkyl, C₃₋₆carbocyclyl-C₁₋₆alkenyl, C₃₋₆carbocyclyl-C₂₋₆alkynyl, each being optionally substituted by one or more, same or different substituents represented by R₇,
with the proviso that when Y₂ is -O-, -NRₐ-, -S- or -C(O)O-, and R₉ is C₁₋₆alkyl, said C₁₋₆alkyl is substituted by one or more, same or different substituents represented by R₇;
Y₃ is -O-, -S-, -S(O)-, -S(O)₂-, -NRₐ-, -NRₐC(O)NR_{b}-, -NRₐC(O)-, -C(O)NRₐ-,-C(O)NRₐO-, -C(O)-, -NRₐC(O)O-, -NRₐS(O)₂-, -OC(O)- or -C(O)O-;
R₁₀ is C₁₋₁₀alkyl-C₁₋₁₂heterocyclyl, C₁₋₁₀alkyl-C₃₋₁₂carbocyclyl, C₁₋₁₀alkyl, C₂₋₁₀ alkenyl, C₂₋₁₀ alkynyl, C₃₋₁₂carbocyclyl or C₁₋₁₂heterocyclyl, each being optionally substituted by one or more, same or different substituents represented by R₇;
or, when one of R₅ or R₆ is the group -C(O)IVRₐR₉, Rₐ and R₉ together with the nitrogen atom to which they are attached form a C₁₋₁₂heterocyclic ring optionally comprising one or more additional heteroatoms selected from the group consisting of O, S and N, optionally substituted with one or more substituents represented by R₇;
or a pharmaceutically acceptable salt, solvate, or ester thereof.

In another aspect, the invention relates to a pharmaceutical composition comprising a compound of formula I or a pharmaceutically acceptable salt, solvate, or ester thereof together with a pharmaceutically acceptable excipient or vehicle.

In a still further aspect, the invention relates to the use of a compound of formula I for the manufacture of a medicament for the prophylaxis, treatment or amelioration of inflammatory diseases or conditions, or ophthalmic diseases or conditions.

In a still further aspect, the invention relates to the use of a compound of formula I for the manufacture of a medicament for the treatment or amelioration of cancer.

In a still further aspect, the invention relates to a method for producing a compound of general structure I, 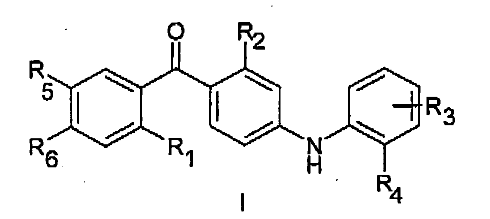 wherein R₁, R₂, R₃, R₄, R₅, and R₆ are as defined above, comprising the steps of
a) transforming a compound general structure VI, 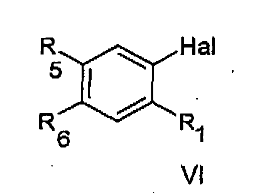 wherein Hal is a halogen, and R₁, R₅ and R₆ are as defined above, each of which are independently protected or unprotected, into an organometallic intermediate;
b) transmetalating said organometallic intermediate to an organozinc intermediate;
c) coupling said organozinc intermediate with an acid halide of general structure V, 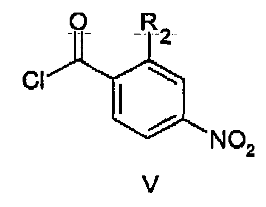 wherein R₂ is as defined above 1, protected or unprotected, in the presence of a catalyst to give a compound of general structure IV, 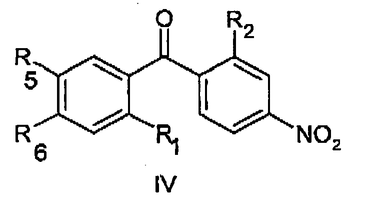 wherein R₁, R₂, R₅, and R₆ are defined as above, each of which are independently protected or unprotected;
d) optionally transforming, protecting or deprotecting one or more substituents or functional groups of R₁, R₂, R₅, and R₆ of the compound of general structure IV to give another compound of general structure IV;
e) reducing the compound of general structure IV from step c) or d) to an amine of general structure III, 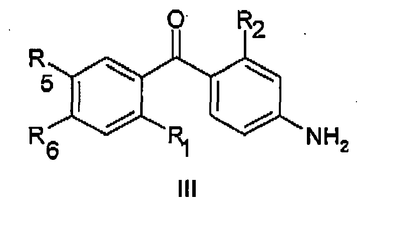 wherein R₁, R₂, R₅, and R₆ are defined as above, each of which are independently protected or unprotected;
f) optionally transforming, protecting or deprotecting one or more substituents or functional groups of R₁, R₂, R₅, and R₆ of the compound of general structure III to give another compound of general structure III;
g) coupling of the amine of general structure III from step e) or f) with a compound of general structure II, 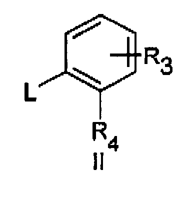 wherein L is triflate or halogen, R₃ and R₄ are as defined above, each of which are independently protected or unprotected, to give a compound of general structure I, wherein R₁, R₂, R₃, R₄, R₅, and R₆ are defined as above, each of which are independently protected or unprotected;
h) optionally transforming, protecting or deprotecting one or more substituents or functional groups of R₁, R₂, R₃, R₄, R₅, or R₆ of the compound of general structure I from step g) to give a another compound of general structure I.

In a still further aspect, the invention relates to a method for producing a compound of general structure I, 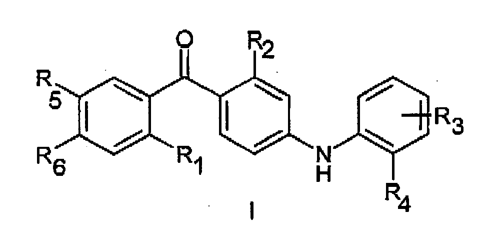 wherein R₁, R₂, R₃, R₄, R₅, and R₆ are as defined above, comprising the steps of
a) transforming a compound general structure VIIa, 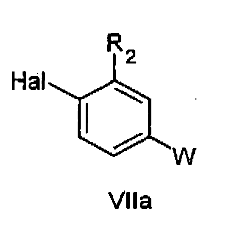 wherein Hal is halogen, W is halogen or triflate, and R₂ is as defined above, protected or unprotected, into an organometallic intermediate;
b) transmetalating said organometallic intermediate to an organozinc intermediate;
c) coupling said organozinc intermediate with an acid halide of general structure VIII, 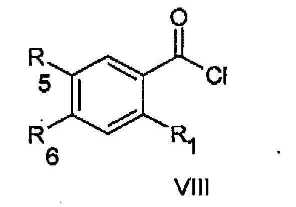 wherein R₁, R₅, and R₆ are as defined above, each of which are independently protected or unprotected, in the presence of a catalyst to give a compound of general structure IIIa, 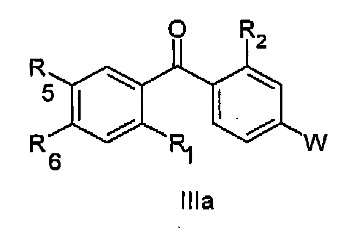 wherein W, R₁, R₂, R₅, and R₆ are defined as above, each of which are independently protected or unprotected;
d) optionally transforming, protecting or deprotecting one or more substituents or functional groups of W, R₁, R₂, R₅, and R₆ of the compound of general structure IIIa to give another compound of general structure IIIa;
e) coupling of the compound of general structure IIIa from step c) or d) with an amine of general structure IIa, 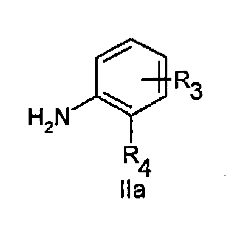 wherein R₃ and R₄ are as defined above, each of which are independently protected or unprotected, to give a compound of general structure I,
   wherein R₁, R₂, R₃, R₄, R₅, and R₆ are defined as above, each of which are independently protected or unprotected;
f) optionally transforming, protecting or deprotecting one or more substituents or functional groups of R₁, R₂, R₃, R₄, R₅, or R₆ of the compound of general structure I from step e) to give another compound of general structure I.

### DETAILED DESCRIPTION OF THE INVENTION

### Definitions

In the present context, the term "alkyl" is intended to indicate a univalent radical derived from straight or branched alkane by removing a hydrogen atom from any carbon atom. The alkyl chain typically comprises 1-10 carbon atoms, in particular 1-6 carbon atoms. The term includes the subclasses normal alkyl (n-alkyl), secondary and tertiary alkyl, such as methyl, ethyl, n-propyl, isopropyl, n-butyl, isobutyl, sec.-butyl, tert.-butyl, pentyl, isopentyl, hexyl and isohexyl.

The term "alkoxy" is intended to indicate a radical of formula OR', wherein R' is alkyl as defined above, e.g. methoxy, ethoxy, propoxy, butoxy, etc.

The term "hydroxyalkyl" is intended to indicate an alkyl radical as defined above, wherein one or more hydrogen atoms are replaced by hydroxy.

The term "alkenyl" is intended to indicate a mono-, di-, tri-, tetra- or pentaunsaturated hydrocarbon radical typically comprising 2-10 carbon atoms, in particular 2-6 carbon atoms, e.g. ethenyl, propenyl, butenyl, pentenyl or hexenyl.

The term "alkynyl" is intended to indicate an hydrocarbon radical comprising 1-5 triple C-C bonds, the alkyne chain typically comprising 2-10 carbon atoms, in particular 2-6 carbon atoms, such as ethynyl, propynyl, butynyl, pentynyl or hexynyl.

The term "alkoxycarbonyl" is intended to indicate a radical of formula -COOR' wherein R' is alkyl as defined above, e,g. methoxycarbonyl, ethoxycarbonyl, n-propoxycarbonyl, isopropoxycarbonyl, etc.

The term "aryl" is intended to include radicals of carbocyclic aromatic rings, in particular 5- or 6-membered rings, optionally fused bicyclic rings, e.g. phenyl or naphthyl,

The term "heteroaryl" is intended to include radicals of heterocyclic aromatic rings, in particular 5- or 6-membered rings with 1-4 heteroatoms selected from O, S and N, or optionally fused bicyclic rings with 1-4 heteroatoms, e.g. pyridyl, tetrazolyl, thiazolyl, imidazolyl, pyrazolyl, oxazolyl, isoxazolyl, thienyl, pyrazinyl, isothiazolyl, benzimidazolyl, and benzofuranyl.

The term "carbocyclyl" includes saturated and unsaturated, optionally fused bicyclic, hydrocarbon rings typically comprising 3-12 carbon atoms, in particular 3-8 carbon atoms, such as cyclopropyl, cyclopentyl, cyclohexyl and cyclooctyl; or a C₃₋₁₂ cycloalkene group, such as cycloprop-2-enyl, cyclobut-2-enyl, cyclopent-2-enyl, cyclohex-3-enyl, cycloocta-4-enyl, cyclohex-3,5-dienyl, indanyl, indeneyl, 1,4-dihydronaphtyl, phenyl and naphtyl. The term "carbocyclyl" also includes cyclic hydrocarbons wherein one or more ring -CH₂- fragments have been replaced by a -C(O)- fragment and /or an exo-cyclic carbon-carbon double bond, such as oxocyclohexyl, oxocyclopentyl, 4-oxo-1,2,3,4-tetrahydronaphtalen-1-yl, 1-oxo-1,2,3,4-tetrahydronaphtalen-1-yl, 2-oxocyclohex-3-en-1-yl and 2-oxocyclohex-1-en-1-yl, and

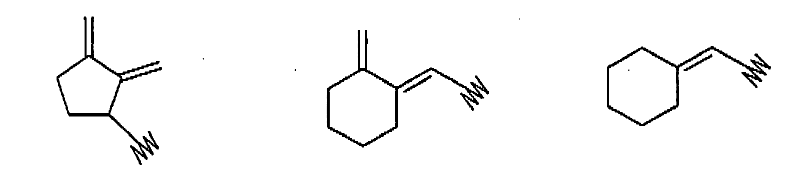

The term "heterocyclyl" is intended to indicate saturated or unsaturated, optionally fused carbocyclic rings comprising 1-12 carbon atoms, such as 1-12 carbon atoms, in particular 1-8 carbon atoms, and comprising one or more heteroatoms selected from the group consisting of O, N and S, such as tetrazolyl, triazolyl, pyrrolyl, furanyl, morpholyl, piperazyl, thiophenyl, imidazolyl, oxazolyl, thiazolyl, pyrazolyl, pyrrolidinyl, pyridinyl, pyrimidinyl, tetrahydrotiophenyl, tetrahydrofuranyl, tetrahydropyranyl, piperidinyl, putinyl, morpholinyl, furanyl, dioxolanyl, thiophenyl, quinolinyl, isoquinolinyl, 1,2-dihydroquinolinyl, etc. The term "heterocyclyl" thus includes "heteroaryl" as defined above and also includes heterocyclic groups wherein one or more ring -CH₂- fragments have been replaced by a -C(O)- fragment and/or an exo-cyclic carbon-carbon double bond, such as dioxopiperidinyl, dioxoimidazolidine, dioxohexahydropyrimidine, oxopyrrolidine, 1-oxo-3,4-dihydroisoquinolin-2(1H)-yl and

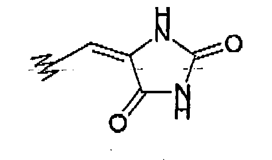

The term "alkylthio" is intended to indicate a radical of the formula -SR, where R is alkyl as defined above, for example C₁₋₁₀alkylthio, C₁₋₄alkylthio, methylthio, ethylthio, *n*-propylthio, 2-propylthio, etc.

The term "alkylamino" is intended to indicate a radical of the formula -NHR or-NR₂, wherein R is alkyl as defined above and includes, for example, methylamino, dimethylamino, di-(n-propyl)amino, n-butyl(ethyl)amino, etc.

The term "halogen" is intended to indicate fluoro, chloro, bromo or iodo.

The term "pharmaceutically acceptable salt" is intended to indicate salts prepared by reacting a compound of formula I with a suitable inorganic or organic acid, such as hydrochloric, hydrobromic, hydroiodic, sulfuric, nitric, phosphoric, formic, acetic, 2,2-dichloroaetic, adipic, ascorbic, L-aspartic, L-glutamic, galactaric, lactic, maleic, L-malic, phthalic, citric, propionic, benzoic, glutaric, gluconic, D-glucuronic, methanesulfonic, salicylic, succinic, malonic, tartaric, benzenesulfonic, ethane-1,2-disulfonic, 2-hydroxy ethanesulfonic acid, toluenesulfonic, sulfamic or fumaric acid. Pharmaceutically acceptable salts of compounds of formula I may also be prepared by reaction with a suitable base such as sodium hydroxide, potassium hydroxide, magnesium hydroxide, calcium hydroxide, ammonia, or suitable non-toxic amines, such as lower alkylamines, for example triethylamine,

hydroxy-lower alkylamines, for example 2-hydroxyethylamine, bis-(2-hydroxyethyl)-amine, cycloalkylamines, for example dicyclohexylamine, or benzylamines, for example N,N'-dibenzylethylenediamine, and dibenzylamine, or L-arginine or L-lysine.

The term "solvate" is intended to indicate a species formed by interaction between a compound, e.g. a compound of formula I, and a solvent, e.g. alcohol, glycerol or water, wherein said species are in a solid form. When water is the solvent, said species is referred to as a hydrate.

The term "pharmaceutically acceptable ester" is intended to indicate easily hydrolysable esters such as alkanoyloxyalkyl, aralkanoyloxyalkyl, aroyloxyalkyl, e.g. acetoxymethyl, pivaloyoxymethyl, benzoyloxymethyl esters and the corresponding 1'-oxyethyl derivatives, or alkoxycarbonyloxyalkyl esters, e.g. methoxycarbonyloxymethyl esters and ethoxycarbonyloxymethyl esters and the corresponding 1'-oxyethyl derivatives, or lactonyl esters, e.g. phthalidyl esters, or dialkylaminoalkyl esters, e.g. dimethylaminoethyl esters. Easily hydrolysable esters include *in vivo* hydrolysable esters of the compounds of formula I. Such esters may be prepared by conventional methods known to persons skilled in the art, such as method disclosed in GB patent No. 1 490 852 incorporated herein by reference.

"p38 MAP kinase" is a stress-activated protein kinase existing in several isoforms (p38α, p38β, p38β2, p38γ and p38δ). The p38 MAP kinase is activated by different stimuli including heat, chemical, osmotic, pH and oxidative stress, growth factor withdrawal, high or low glucose and ultraviolet radiation. p38 is also stimulated by agents that mediate the initial physiological response to injury, infection and inflammation, such as LPS and pro-inflammatory cytokines IL-1β, THF-α, FasL, CD40L and TGF-β. Like other MAP kinases, p38 is phosphorylated by kinases, including MKK3, MEK6 and MKK6, on a threonine and tyrosine in an activation loop (Thr-Xaa-Tyr) close to the ATP and substrate binding site. In turn, p38 phosphorylates and activates the serine-threonine protein kinases MAPKAP kinase-2, MAPKAP kinase-3, MAPKAP kinase-5, MNK-1 and MSK-1. It has been established that activation of p38 regulates cytokine biosynthesis in many cell types either directly by phosphorylating and activating transcription factors involved in the expression of cytokines or indirectly, e.g. by phosphorylating MSK-1 which, when activated, activates the transcription factor CREB. It has also been shown that certain pyridinyl imidazoles, e.g. SB203580, which inhibit p38, inhibit the production of IL-1β and TNF-α from LPS-treated human monocytes. It has therefore been concluded that p38 constitutes a potentially highly interesting target for the development of anti-inflammatory compounds (cf. JC Lee et al., Immunopharmacology 47, 2000, pp, 185-201 and references reviewed therein; PR Young, "Specific Inhibitors of p38 MAP kinase" in Signaling Networks and Cell Cycle Control: The Molecular Basis of Cancer and Other Diseases, JS Gutkind (Ed.), Humana Press, Inc., Totowa, NJ, and references reviewed therein). There are several reports on p38 MAP kinase and inflammatory cytokines in relation to cell growth and apoptosis, such as tumor proliferation and metastasis. Though the exact mechanism of p38 MAP kinase mediated cell growth regulation is not known, it is believed that p38MAP kinase constitutes a potentially highly interesting target for the development of anti cancer drugs (S Nakada et al., Anticancer Research 21(1A), 2001, pp.167-171 and references cited therein; C Denkert et al., Cancer Letters 195(1), 2003 p.p. 101-109 and references cited therein).

Compounds of formula I may comprise asymmetrically substituted (chiral) carbon atoms and carbon-carbon double bonds which may give rise to the existence of isomeric forms, e.g. enantiomers, diastereomers and geometric isomers. The present invention relates to all such isomers, either in pure form or as mixtures thereof. Pure stereoisomeric forms of the compounds and the intermediates of this invention may be obtained by the application of procedures known in the art. Diastereomers may be separated by physical separation methods such as selective crystallization and chromatographic techniques, e. g. liquid chromatography using chiral stationary phases. Enantiomers may be separated from each other by the selective crystallization of their diastereomeric salts with optically active acids. Alternatively, enantiomers may be separated by chromatographic techniques using chiral stationary phases. Said pure stereoisomeric forms may also be derived from the corresponding pure stereoisomeric forms of the appropriate starting materials, provided that the reaction occurs stereoselectively or stereospecifically. Preferably, if a specific stereoisomer is desired, said compound will be synthesized by stereoselective or stereospecific methods of preparation. These methods will advantageously employ chiral pure starting materials. Likewise, pure geometric isomers may be obtained from the corresponding pure geometric isomers of the appropriate starting materials. A mixture of geometric isomers will typically exhibit different physical properties, and they may thus be separated by standard chromatographic techniques well-known in the art.

### Preferred embodiments of the compound of formula I

In a currently preferred embodiment of the compounds of formula I, R₁ may be halogen, trifluoromethyl, C₁₋₄alkyl, C₁₋₄alkoxy or nitro. In particular, R₁ may be methyl, ethyl, methoxy, ethoxy, bromo, fluoro or chloro.

In a further preferred embodiment of the compounds of formula I, R₂ may be methyl, ethyl, nitro, bromo, fluoro or chloro.

In a further preferred embodiment of the compounds of formula I, R₃ may be hydrogen, methyl, ethyl, methoxy, ethoxy, bromo, fluoro or chloro.

In a further preferred embodiment of the compounds of formula I, R₃ represents one substituent. In particular, R₃ may be in the meta position with respect to R₄ and para with respect to -NH, or R₃ may be in the meta position with respect to R₄ and ortho with respect to -NH, or R₃ may be in the ortho position with respect to R₄ and meta with respect to -NH.

In a further preferred embodiment of the compounds of formula I, R₃ may be fluorine.

In a further preferred embodiment of the compounds of formula I, R₇ may be halogen, hydroxy, amino, -S(O)₂CH₃, trifluoromethyl, cyano, C₁₋₄hydroxyalkyl, C₁₋₄ alkoxy, C₁₋₄alkyl, C₁₋₄alkylthio, C₁₋₄alkylamino, C₁₋₄alkoxycarbonyl, -COOH,-CONH₂, -S(O)₂NH₂, azido, -CONHR' or -CONRR', wherein R and R' are as defined above. In particular, R₇ may be methyl, ethyl, methoxy, ethoxy, hydroxy, methoxycarbonyl, ethoxycarbonyl, dimethylamino, ethylamino, amino, -COOH, fluoro, chloro, bromo, -CONH₂, -S(O)₂NH₂, azido, methylthio, -S(O)₂CH₃, trifluoromethyl, cyano or hydroxymethyl,

In a further preferred embodiment of the compounds of formula I, one of R₅ and R₆ may be Y₂R₉, C₁₋₄alkyl-Y₂R₉, Y₂R₉Y₃R₁₀, C₁₋₄alkyl-Y₂R₉Y₃R₁₀, C₂₋₄alkenyl-Y₂R₉, C₂₋₄ alkenyl-Y₂R₉Y₃R₁₀, Y₂R₉-C₁₋₄-alkyl-Y₃R₁₀, Y₂R₉-C₂₋₄-alkenyl-Y₃R₁₀, C₁₋₆ heterocyclyl-C₁₋₄-alkyl-Y₂R₉, C₁₋₄alkyl-C₁₋₆heterocyclyl, C₁₋₄alkyl-C₃₋₆carbocyclyl, C₃₋₆carbocyclyl-C₁₋₄alkyl, C₁₋₄alkyl substituted by R₇, C₂₋₄alkenyl, C₂₋₄ alkynyl, C₃₋₆ carbocyclyl, C₁₋₆heterocyclyl, -COOH, -C(O)NHOH, or C(O)NHNH₂, and the other is hydrogen, halogen, C₁₋₄alkyl or C₁₋₄alkoxy, In particular, R₅ may be Y₂R₉, C₁₋₄ alkyl-Y₂R₉, Y₂R₉Y₃R₁₀, C₁₋₄alkyl-Y₂R₉Y₃R₁₀, C₂₋₄alkenyl-Y₂R₉, C₂₋₄alkenyl-Y₂R₉Y₃R₁₀, Y₂R₉-C₁₋₄-alkyl-Y₃R₁₀, Y₂R₉-C₂₋₄-alkenyl-Y₃R₁₀, C₁₋₆heterocyclyl-C₁₋₄alkyl-Y₂R₉, C₁₋₄ alkyl-C₁₋₆heterocyclyl, C₁₋₄alkyl-C₃₋₆carbocyclyl, C₃₋₆carbocyclyl-C₁₋₄alkyl, C₁₋₄alkyl substituted by R₇, C₂₋₄alkenyl, C₂₋₄ alkynyl, C₃₋₆carbocyclyl, C₁₋₆heterocyclyl,-COOH, -C(O)NHOH, or C(O)NHNH₂, and R₆ is hydrogen, halogen, C₁₋₄alkyl or C₁₋₄ alkoxy. In particular one of R₅ and R₆ is Y₂R₉, Y₂R₉Y₃R₁₀, phenyl, methylphenyl, propenyl, methyl-Y₂R₉, tetrazole, ethynyl, triazole, thiadiazole, dihydrooxazole,-COOH, -C(O)NHOH, or C(O)NHNH₂, and the other is hydrogen, fluoro, chloro, methyl or methoxy.

In a further preferred embodiment of the compounds of formula I, R₅ is hydrogen.

In a further preferred embodiment of the compounds of formula I, R₆ is hydrogen.

In another embodiment of the present invention, when R₂ is hydrogen and one of R₅ or R₆ is not hydrogen or optionally substituted (C₃-C₁₈ heterocyclyl, C₁₋₇alkyl, C₂₋₇alkenyl, C₂₋₇alkynyl or C₁₋₇alkoxy), said optionally substituted (C₃-C₁₈ heterocyclyl, C₁₋₇alkyl, C₂₋₇alkenyl, C₂₋₇alkynyl or C₁₋₇alkoxy) is C₃-C₁₈ heterocyclyl, C₁₋₇alkyl, C₂₋₇alkenyl, C₂₋₇alkynyl or C₁₋₇alkoxy independently substituted by one or more, halogen, hydroxy, cyano, C₁₋₇alkyl, C₁₋₇alkoxy, C₃-C₁₈ heterocyclyl or -NRₓR_{y}, where Rₓ and Ry are independently hydrogen or C₁₋₇alkyl, wherein the latter C₁₋₇alkyl, C₁₋₇alkoxy, C₃-C₁₈ heterocyclyl or -NRₓR_{y} substituents may be further substituted by one or more substituents independently selected from halogen, hydroxy, cyano, C₁₋₇alkyl, C₁₋₇alkoxy, C₃-C₁₈ heterocyclyl or-NRₓR_{y}, where Rₓ and R_{y} are as defined above;

In a further preferred embodiment of the compounds of formula I, Y₂ may be -O--NRₐ-, -NRₐC(O)NR_{b}-, -NRₐC(O)-, -C(O)NRₐ-, -C(O)NRₐO-, -C(O)-, -NRₐC(O)O-,-NRₐS(O)₂-, -C(O)NRₐNR_{b}- or -S(O)₂NRₐ-.

In a further preferred embodiment of the compounds of formula I, Y₃ may be -O-, -NRₐC(O)-, -C(O)NR,-, -C(O)-, -C(O)O- or -NRₐC(O)O-.

In a further preferred embodiment of the compounds of formula I, R₉ may be C₁₋₄ alkyl-C₁₋₆ heterocyclyl, C₁₋₄alkyl-C₃₋₆carbocyclyl, C₁₋₆alkyl, C₂₋₄alkenyl, C₂₋₄ alkynyl, C₃₋₁₀carbocyclyl, C₁₋₆heterocyclyl, C₃₋₆carbocyclyl-C₁₋₆alkyl, C₁₋₆ heterocyclyl-C₁₋₆alkyl, C₃₋₆-carbocyclyl-C₂₋₄alkenyl or C₃₋₆carbocyclyl-C₂₋₄alkynyl.

In particular, R₉ may be C₁₋₄heterocyclyl, C₁₋₆alkyl, C₁₋₃alkyl-C₁₋₅heterocyclyl, C₆₋₁₀ carbocyclyl, C₁₋₃alkyl-C₆carbocyclyl, C₃alkenyl, C₆carbocyclyl-C₁alkyl, C₆carbocyclyl-C₃alkenyl or C₆carbocyclyl-C₂alkynyl. More specifically, R₉ may be morpholinyl, propylmorpholinyl, piperazinyl, methyl, ethyl, n-propyl, n-butyl, *tert-*butyl, isobutyl, hexyl, isopropyl, dimethylpropyl, methyltetrahydrofuranyl, methylpyridinyl, ethylpiperazinyl, cyclohexyl, propyloxopyrrolidinyl, benzyl, methylcyclohexyl, propylphenyl, ethylmorpholinyl, allyl, ethylfuranyl, phenyl, methyldioxoimidazolidinyl, dioxohexahydropyrimidinyl, thiazolyl, methylphenyl, ethylphenyl, methyldioxolanyl, methylthiazolyl, propenylphenyl, methylfuranyl, thiophenyl, tetrahydropyranyl or ethynylphenyl.

In a further preferred embodiment of the compounds of formula I, R₁₀ may be C₁₋₄ alkyl, C₂₋₄alkenyl, C₃₆carbocyclyl or C₁₋₆heterocyclyl. In particular, R₁₀ may be methyl, ethyl, methacryl, tert-butyl, tetrahydropyranyl or ethenyl,

In a further preferred embodiment of the compounds of formula I, said heterocycle or heterocyclyl as above may contain one or two oxygen atoms or one sulphur atom, and/or up to two nitrogen atoms, or three or four nitrogen atoms, wherein optionally one or two CH₂ ring fragments is/are replaced by one or two -C(O)- fragments respectively.

In a further preferred embodiment of the compounds of formula I, Ra, Rb, or Rc independently represent hydrogen, methyl, ethyl, 2-hydroxyethyl or 2-methoxyethyl.

Specific examples of compounds of formula I may be selected from the group consisting of
3-[2-Chloro-4-(2,4-diftuorophenylamino)benzoyl]-*N*-(2-hydroxyethyl)-4-methylbenzamide (Compound 115),
3-[2-Chloro-4-(4-chloro-2-fluoro-phenylamino)-benzoyl]-*N*-(2-hydroxy-ethyl)-4-methyl-benzamide (Compound 120),
3-[2-Chloro-4-(4-chloro-2-fluoro-phenylamino)-benzoyl]-4,*N*-dimethyl-benzamide (Compound 121),
(2-{3-[2-Chloro-4-(4-chloro-2-fluoro-phenylamino)-benzoyl]-4-methyl-benzoylamino}-acetylamino)-acetic acid ethyl ester (Compound 122),
{3-[2-Chloro-4-(4-chloro-2-fluoro-phenylamino)-benzoyl]-4-methyl-benzoylamino}-acetic acid ethyl ester (Compound 123),
3-[2-Chloro-4-(4-chloro-2-fluoro-phenylamino)-benzoyl]-*N*-(2-methoxy-ethyl)-4-methyl-benzamide (Compound 124),
3-[2-Chloro-4-(4-chloro-Z-fluoro-phenylamino)-benzoyl]-*N*-cyclohexyl-4-methyl-benzamide (Compound 125),
3-[2-Chloro-4-(4-chloro-2-fluoro-phenylamino)-benzoyl]-*N*-ethyl-4-methyl-benzamide (Compound 126),
3-[2-Chloro-4-(4-chloro-2-fluoro-phenylamino)-benzoyl]-*N*-(6-hydroxy-hexyl)-4-methyl-benzamide (Compound 127),
3-[2-Chloro-4-(4-chloro-2-fluoro-phenylamino)-benzoyl]-*N*-isopropyl-4-methyl-benzamide (Compound 128),
3-[2-Chloro-4-(4-chloro-2-fluoro-phenylamino)-benzoyl]-*N*-isobutyl-4-methyl-benzamide (Compound 129),
3-[2-Chloro-4-(4-chloro-2-fluoro-phenylamino)-benzoyl]-*N*-(2,2-dimethyl-propyl)-4-methyl-benzamide (Compound 130),
3-[2-Chloro-4-(4-chloro-2-fluoro-phenylamino)-benzoyl]-*N*-(3-methoxy-propyl)-4-methyl-benzamide (Compound 131),
3-[2-Chloro-4-(4-chloro-2-fluoro-phenylamino)-benzoyl]-4-methyl-*N*-[3-(2-oxo-pyrrolidin-1-yl)-propyl]-benzamide (Compound 132),
3-[2-Chloro-4-(4-chloro-2-fluoro-phenylamino)-benzoyl]-*N*-(2-dimethylamino-ethyl)-4-methyl-benzamide (Compound 133),
2-Methyl-acrylic acid 2-{3-[2-chloro-4-(4-chloro-2-fluoro-phenylamino)-benzoyl]-4-methyl-benzoylamino}-ethyl ester (Compound 134),
3-[2-Chloro-4-(4-chloro-2-fluoro-phenylamino)-benzoyl]-*N*-cis-(4-hydroxy-cyclohexyl)-4-methyl-benzamide (Compound 135),
3-[2-Chloro-4-(4-chloro-2-fluoro-phenylamino)-benzoyl]-*N*-trans-(4-hydroxy-cyclohexyl)-4-methyl-benzamide (Compound 136),
(2-{3-[2-Chloro-4-(4-chloro-2-fluoro-phenylamino)-benzoyl]-4-methyl-benzoylamino}-ethyl)-carbamic acid tert-butyl ester (Compound 137),
*N*-(2-Amino-ethyl)-3-[2-chloro-4-(4-chloro-2-fluoro-phenylamino)-benzoyl]-4-methyl-benzamide (Compound 138),
(2-{3-[2-Chloro-4-(4-chloro-2-fluoro-phenylamino)-benzoyl]-4-methyl-benzoylamino}-acetylamino)-acetic acid (Compound 139),
3-[2-Chloro-4-(2,4-difluoro-phenylamino)-benzoyl]-*N*-(2-hydroxy-ethyl)-4-methoxy-benzamide (compound 140),
3-[2-Chloro-4-(2,4-difluoro-phenylamino)-benzoyl]-*N*-(2,2-difluoro-ethyl)-4-methoxy-benzamide (compound 141),
3-[2-Chloro-4-(2,4-difluoro-phenylamino)-benzoyl]-*N*-(2-fluoro-ethyl)-4-methoxy-benzamide (compound 142),
3-[2-Chloro-4-(2,4-difluoro-phenylamino)-benzoyl]-*N*-(2,3-dihydroxy-propyl)-4-methoxy-benzamide (compound 143),
*N*-Carbamoylmethyl-3-[2-chloro-4-(2,4-difluoro-phenylamino)-benzoyl]-4-methoxy-benzamide (compound 144),
*N*-Carbamoylmethyl-3-[2.chloro-4-(2,4.difluoro-phenylamino).benzoyl]-4. methyl-benzamide (Compound 145),
*N*-Benzyl-3-[2-chloro-4-(2,4-difluoro-phenylamino)-benzoyl]-4-methyl-benzamide (compound 146),
3-[2-Chloro-4-(2,4-difluoro-phenylamino)-benzoyl]-*N*-(2-fluoro-ethyl)-4-methyl-benzamide (compound 147),
3-[2-Chloro-4-(2,4-difluoro-phenylamino)-benzoyl]-4-methyl-*N*-(2,2,2-trifluoro-ethyl)-benzamide (compound 148),
3-[2-Chloro-4-(2,4-difluoro-phenylamino)-benzoyl]-*N*-ethyl-4-methyl-benzamide (compound 149),
3-[2-Chloro-4-(2,4-difluoro-phenylamino)-benzoyl]-*N*-cyclohexylmethyl-4-methyl-benzamide (compound 150),
3-[2-Chloro-4-(2,4-difluoro-phenylamino)-benzoyl]-*N*-(2-hydroxy-propyl)-4-methyl-benzamide (compound 151),
3-[2-Chloro-4-(2,4-difluoro-phenylamino)-benzoyl]-*N*-(2,3-dihydroxy-propyl)-4-methyl-benzamide (compound 152),
3-[2-Chloro-4-(2,4-difluoro-phenylamino)-benzoyl]-*N*-(1-hydroxymethyl-propyl)-4-methyl-benzamide (compound 153),
3-[2-Chloro-4-(2,4-difluoro-phenylamino)-benzoyl]-4-methyl-*N*-(2,2,3,3,3-pentafluoro-propyl)-benzamide (compound 154),
3-[2-Chloro-4-(2,4-difluoro-phenylamino)-benzoyl]-*N*-(3-hydroxy-propyl)-4-methyl-benzamide (compound 155),
3-[2-Chloro-4-(2,4-difluoro-phenylamino)-benzoyl]-*N*-(2-hydroxy-1,1-dimethyl-ethyl)-4-methyl-benzamide (compound 156),
3-[2-Chloro-4-(2,4-difluoro-phenylamino)-benzoyl]-*N*-(2-hydroxy-1-hydroxymethyl-1-methyl-ethyl)-4-methyl-benzamide (compound 157),
{3-[2-Chloro-4-(2,4-difluoro-phenylamino)-benzoyl]-4-methyl-benzoylamino}-acetic acid ethyl ester (compound 158),
3-[2-Chloro-4-(2,4-difluoro-phenylamino)-benzoyl]-*N*-(4-hydroxy-butyl)-4-methyl-benzamide (compound 159),
3-[2-Chloro-4-(2,4-difluoro-phenylamino)-benzoyl]-*N*-(3-hydroxy-1,1-dimethyl-butyl)-4-methyl-benzamide (compound 160),
3-[2-Chloro-4-(2,4-difluoro-phenylamino)-benzoyl]-4-methyl-*N*-(3-phenyl-propyl)-benzamide (compound 161),
(R)-3-[2-Chloro-4-(2,4-difluoro-phenylamino)-benzoyl]-*N*-(1-hydroxymethyl-3-methyl-butyl)-4-methyl-benzamide (compound 162),
3-[2-Chloro-4-(2,4-difluoro-phenylamino)-benzoyl]-*N*-isopropyl-4-methyl-benzamide (compound 164),
3-[2-Chloro-4-(2,4-difluoro-phenylamino)-benzoyl]-*N*-cyclohexyl-4-methyl-benzamide (compound 165),
3-[2-Chloro-4-(2,4-difluoro-phenylamino)-benzoyl]-*N*-(2,2-difluoro-ethyl)-4-methyl-benzamide (compound 166),
5-{3-[2-Chloro-4-(2,4-difluoro-phenylamino)-benzoyl]-4-methyl-benzoylamino}-4-oxo-pentanoic acid methyl ester (compound 167),
*N*-[(2-Carbamoyl-ethylcarbamoyl)-methyl]-3-[2-chloro-4-(2,4-difluoro-phenylamino)-benzoyl]-4-methyl-benzamide (compound 168),
(2-{3-[2-Chloro-4-(2,4-difluoro-phenylamino)-benzoyl]-4-methyl-benzoylamino}-acetylamino)-acetic acid ethyl ester (compound 169),
*N*-Allyl-3-[2-chloro-4-(2,4-difluoro-phenylamino)-benzoyl]-4-methyl-benzamide (compound 170),
3-[2-Chloro-4-(2,4-difluoro-phenylamino)-benzoyl]-4-methyl-*N-*(2-sulfamoyl-ethyl)-benzamide (compound 171),
*N*-(2-Acetylamino-ethyl)-3-[2-chloro-4-(2,4-difluoro-phenylamino)-benzoyl]-4-methyl-benzamide (compound 172),
3-[2-Chloro-4-(2,6-difluoro-phenylamino)-benzoyl]-*N*-(2-hydroxy-ethyl)-4-methoxy-benzamide (compound 173),
3-[2-Chloro-4-(2,6-difluoro-phenylamino)-benzoyl]-*N*-(2-fluoro-ethyl)-4-methoxy-benzamide (compound 174),
3-[2-Chloro-4-(2,6-difluoro-phenylamino)-benzoyl]-*N*-(2,3-dihydroxy-propyl)-4-methoxy-benzamide (compound 175),
3-[2-Chloro-4-(2,6-difluoro-phenylamino)-benzoyl]-*N*-(3-hydroxy-propyl)-4-methoxy-benzamide (compound 176),
3-[2-Chloro-4-(2,6-difluoro-phenylamino)-benzoyl]-4-methoxy-*N*-phenethyl-benzamide (compound 177),
3-[2-Chloro-4-(2,6-difluoro-phenylamino)-benzoyl]-*N*-(2-hydroxy-1,1-dimethyl-ethyl)-4-methoxy-benzamide (compound 178),
3-[2-Chloro-4-(2,6-difluoro-phenylamino)-benzoyl]-4-methoxy-*N*-(2-morpholin-4-yl-ethyl)-benzamide (compound 179),
3-[2-Chloro-4-(2,6-difluoro-phenylamino)-benzoyl]-*N*-(2-hydroxy-1-hydroxymethyl-1-methyl-ethyl)-4-methoxy-benzamide (compound 180),
3-[2-Chloro-4-(2,6-difluoro-phenylamino)-benzoyl]-*N*-(Z-hydroxy-ethyl)-4-methoxy-*N*-methyl-benzamide (compound 181),
{3-[2-Chloro-4-(2,6-difluoro-phenylamino)-benzoyl]-4-methoxy-benzoylamino}-acetic acid ethyl ester (compound 182),
(2-{3-[2-Chloro-4-(2,6-difluoro-phenylamino)-benzoyl]-4-methoxy-benzoylamino}-acetylamino)-acetic acid ethyl ester (compound 183),
3-[2-Chloro-4-(2,6-difluoro-phenylamino)-benzoyl]-*N*,*N*-bis-(2-hydroxy-ethyl)-4-methoxy-benzamide (compound 184),
3-[2-Chloro-4-(2,6-difluoro-phenylamino)-benzoyl]-4-methoxy-*N*,*N*-bis-(2-methoxy-ethyl)-benzamide (compound 185),
4-Chloro-3-[2-chloro-4-(2,4-difluoro-phenylamino)-benzoyl]-*N*-(2-hydroxy-ethyl)-benzamide (compound 204),
*N*-{3-[2-Chloro-4-(2,4-difluoro-phenylamino)-benzoyl]-4-methyl-phenyl}-3-methoxy-propionamide (compound 241),
*N*-{3-[2-Chloro-4-(2,4-difluoro-phenylamino)-benzoyl]-4-methyl-phenyl}-propionamide (compound 242),
*N*-{3-[2-Chloro-4-(2,4-difluoro-phenylamino)-benzoyl]-4-methyl-phenyl}-2-(2-methoxy-ethoxy)-acetamide (compound 243),
*N*-{3-[2-Chloro-4-(2,4-difluoro-phenylamino)-benzoyl]-4-methyl-phenyl}-3-morpholin-4-yl-propionamide (compound 244),
*N*-{3-[2-Chloro-4-(2,4-difluoro-phenylamino)-benzoyl]-4-methyl-phenyl}-3-hydroxy-propionamide (compound 245),
*N*-{3-[2-Chloro-4-(2,4-difluoro-phenylamino)-benzoyl]-4-methyl-phenyl}-3-furan-2-yl-propionamide (compound 246),
*N*-{3-[2-Chloro-4-(2,4-difluoro-phenylamino)-benzoyl]-4-methyl-phenyl}-2-hydroxy-benzamide (compound 247),
*N*-{3-[2-Chloro-4-(2,4-difluoro-phenylamino)-benzoyl]-4-methyl-phenyl}-2-(2,5-dioxo-imidazolidin-4-yl)-acetamide (compound 248),
2,6-Dioxo-hexahydro-pyrimidine-4-carboxylic acid {3-[2-chloro-4-(2,4-difluoro-phenylamino)-benzoyl]-4-methyl-phenyl}-amide (compound 249),
Acrylic acid 2-{3-[2-chloro-4-(2,4-difluoro-phenylamino)-benzoyl]-4-methyl-phenylcarbamoyl}-ethyl ester (compound 250),
*N*-{3-[2-Chloro-4-(2,4-difluoro-phenylamino)-benzoyl]-4-methyl-phenyl}-3-methylsulfanyl-propionamide (compound 251),
*N*-{3-[2-Chloro-4-(2,4-difluoro-phenylamino)-benzoyl]-4-methyl-phenyl}-3-methanesulfonyl-propionamide (compound 252),
Ethanesulfonic acid {3-[2-chloro-4-(2,4-difluoro-phenylamino)-benzoyl]-4-methyl-phenyl}-amide (compound 253),
*N*-{3-[2-Chloro-4-(2,4-difluoro-phenylamino)-benzoyl]-4-methyl-phenyl}-4-methoxy-benzenesulfonamide (compound 254),
*N*-(5-{3-[2-Chloro-4-(2,4-difluoro-phenylamino)-benzoyl]-4-methyl-phenylsulfamoyl}-4-methyl-thiazol-2-yl)-acetamide (compound 255),
5-Acetyl-2-chloro-*N*-{3-[2-chloro-4-(2,4-difluoro-phenylamino)-benzoyl]-4-methyl-phenyl}-benzenesulfonamide (compound 256),
Naphthalene-2-sulfonic acid {3-[2-chloro-4-(2,4-difluoro-phenylamino)-benzoyl]-4-methyl-phenyl}-amide (compound 257),
*N*-{3-[2-Chloro-4-(2,4-difluoro-phenylamino)-benzoyl]-4-methyl-phenyl}-C-phenyl-methanesulfonamide (compound 258),
2-Methyl-acrylic acid 2-(3-{3-[2-chloro-4-(2,4-difluoro-phenylamino)-benzoyl]-4-methyl-phenyl}-ureido)-ethyl ester (compound 259),
1-{3-[2-Chloro-4-(2,4-difluoro-phenylamino)-benzoyl]-4-methyl-phenyl}-3-(2-hydroxy-ethyl)-urea (compound 260),
(3-{3-[2-Chloro-4-(2,4-difluoro-phenylamino)-benzoyl]-4-methyl-phenyl}-ureido)-acetic acid ethyl ester (compound 261),
1-{3-[2-Chloro-4-(2,4-difluoro-phenylamino)-benzoyl]-4-methyl-phenyl}-3-(3-methoxy-phenyl)-urea (compound 262),
1-{3-[2-Chloro-4-(2,4-difluoro-phenylamino)-benzoyl]-4-methyl-phenyl}-3-(3-trifluoromethyl-phenyl)-urea (compound 263),
1-{3-[2-Chloro-4-(2,4-difluoro-phenylamino)-benzoyl]-4-methyl-phenyl}-3-propyl-urea (compound 264),
3-(3-{3-[2-Chloro-4-(2,4-difluoro-phenylamino)-benzoyl]-4-methyl-phenyl}-ureido)-propionic acid ethyl ester (compound 265),
1-{3-[2-Chloro-4-(2,4-difluoro-phenylamino)-benzoyl]-4-methyl-phenyl}-3-cyclohexyl-urea (compound 266),
1-Allyl-3-{3-[2-chloro-4-(2,4-difluoro-phenylamino)-benzoyl]-4-methyl-phenyl}-urea (compound 267),
1-Benzyl-3-{3-[2-chloro-4-(2,4-difluoro-phenylamino)-benzoyl]-4-methyl-phenyl}-urea (compound 268),
1-{3-[2-Chloro-4-(2,4-difluoro-phenylamino)-benzoyl]-4-methyl-phenyl}-3-ethyl-urea (compound 269),
1-{3-[2-Chloro-4-(2,4-difluoro-phenylamino)-benzoyl]-4-methyl-phenyl}-3-phenyl-urea (compound 270),
1-Butyl-3-{3-[2-chloro-4-(2,4-difluoro-phenylamino)-benzoyl]-4-methyl-phenyl}-urea (compound 271),
1-{3-[2-Chloro-4-(2,4-difluoro-phenylamino)-benzoyl]-4-methyl-phenyl}-3-phenethyl-urea (compound 272),
2-(3-{3-[2-Chloro-4-(2,4-difluoro-phenylamino)-benzoyl]-4-methyl-phenyl}-ureido)-benzoic acid methyl ester (compound 273),
1-{3-[2-Chloro-4-(2,4-difluoro-phenylamino)-benzoyl]-4-methyl-phenyl}-3-(3-cyano-phenyl)-urea (compound 274),
1-{3-[2-Chloro-4-(2,4-difluoro-phenylamino)-benzoyl]-4-methyl-phenyl}-3-isopropyl-urea (compound 275),
1-{3-[2-Chloro-4-(2,4-difluoro.phenylamlno)-benzoyl]-4-methyl-phenyl}-3-(4-methoxy-phenyl)-urea (compound 276),
{3-[2-Chloro-4-(2,4-difluoro-phenylamino)-benzoyl]-4-methyl-phenyl}-carbamic acid benzyl ester (compound 277),
{3-[2-Chloro-4-(2,4-difluoro-phenylamino)-benzoyl]-4-methyl-phenyl}-carbamic acid allyl ester (compound 278),
{3-[2-Chloro-4-(2,4-difluoro-phenylamino)-benzoyl]-4-methyl-phenyl}-carbamic acid ethyl ester (compound 279),
[2-Chloro-4-(2,4-difluoro-phenylamino)-phenyl]-[5-(3-hydroxy-butylamino)-2-methyl-phenyl]-methanone (compound 281),
[2-Chloro-4-(2,4-difluoro-phenylamino)-phenyl]-(3'-hydroxymethyl-4-methyl-biphenyl-3-yl)-methanone (compound 282),
[2-Chloro-4-(2,4-difluoro-phenylamino)-phenyl]-(3'-hydroxy-4-methyl-biphenyl-3-yl)-methanone (compound 283),
[2-Chloro-4-(2,4-difluoro-phenylamino)-phenyl]-(4'-methoxy-4-methyl-biphenyl-3-yl)-methanone (compound 284),
*N*-{3'-[2-Chloro-4-(2,4-difluoro-phenylamino)-benzoyl]-4'-methyl-biphenyl-3-yl}-acetamide (compound 285),
[2-Chloro-4-(2,4-difluoro-phenylamino)-phenyl]-(4-methyl-3'-trifluoromethoxy-biphenyl-3-yl)-methanone (compound 286),
[2-Chloro-4-(2,4-difluoro-phenylamino)-phenyl]-(3',4',5'-trifluoro-4-methyl-biphenyl-3-yl)-methanone (compound 288),
[2-Chloro-4-(2,4-difluoro-phenylamino)-phenyl]-(3',4'-dimethoxy-4-methyl-biphenyl-3-yl)-methanone (289),
3'-[2-Chloro-4-(2,4-difluoro-phenylamino)-benzoyl]-4'-methyl-biphenyl-3-carbonitrile (compound 290),
3-[2-Chloro-4-(2,4-difluoro-phenylamino)-benzoyl]-*N*-(2-hydroxy-ethyl)-4-methyl-benzenesulfonamide (compound 291),
3-[2-Chloro-4-(2,4-difluoro-phenylamino)-benzoyl]-4-methyl-*N*-(2-morpholin-4-yl-ethyl)-benzenesulfonamide (compound 292),
*N*-Allyl-3-[2-chloro-4-(2,4-difluoro-phenylamino)-benzoyl]-4-methyl-benzenesulfonamide (compound 293),
*N*-(2-{3-[2-Chloro-4-(2,4-difluoro-phenylamino)-benzoyl]-4-methyl-benzenesulfonylamino}-ethyl)-acetamide (compound 294),
3-[2-Chloro-4-(2,4-difluoro-phenylamino)-benzoyl]-4-methyl-*N*-propyl-benzenesulfonamide (compound 295),
3-[2-Chloro-4-(2,4-difluoro-phenylamino)-benzoyl]-*N*-(2,3-dihydroxy-propyl)-4-methyl-benzenesulfonamide (compound 296),
3-[2-Chloro-4-(2,4-difluoro-phenylamino)-benzoyl]-*N*-(2-methoxy-ethyl)-4-methyl-benzenesulfonamide (compound 297),
[4-(2,4-Difluoro-phenylamino)-2-nitro-phenyl]-[5-(4-methoxy-benzyloxy)-2-methyl-phenyl]-methanone (Compound 306),
[4-(2,4-Difluoro-phenylamino)-2-nitro-phenyl]-[5-(3-hydroxy-propoxy)-2-methyl-phenyl]-methanone (Compound 307),
[2-Amino-4-(2,4-difluoro-phenylamino)-phenyl]-[5-(3-hydroxy-propoxy)-2-methyl-phenyl]-methanone (compound 308),
[4-(2,4-Difluoro-phenylamino)-2-nitro-phenyl]-[2-methyl-5-(2-morpholin-4-yl-ethoxy)-phenyl]-methanone (compound 309),
[2-Amino-4-(2,4-difluoro-phenylamino)-phenyl]-[2-methyl-5-(2-morpholin-4-yl-ethoxy)-phenyl]-methanone (compound 310),
[4-(2,4-Difluoro-phenylamino)-2-nitro-phenyl]-[5-(2,2-dimethyl-[1,3]dioxolan-4-ylmethoxy)-2-methyl-phenyl]-methanone (compound 311),
[4-(2,4-Difluoro-phenylamino)-2-nitro-phenyl]-[5-(2,3-dihydroxy-propoxy)-2-methyl-phenyl]-methanone (compound 312),
[2-Amino-4-(2,4-difluoro-phenylamino)-phenyl]-[5-(2,3-dihydroxy-propoxy)-2-methyl-phenyl]-methanone (compound 313),
[2-Chloro-4-(2,4-difluoro-phenylamino)-phenyl]-[2-fluoro-5-(3-hydroxy-propoxy)-phenyl]-methanone (compound 314),
[2-Chloro-4-(2,4-difluoro-phenylamino)-phenyl]-[5-(2,2-dimethyl-[1,3]dioxolan-4-ylmethoxy)-2-fluoro-phenyl]-methanone (compound 315),
[2-Chloro-4-(2,4-difluoro-phenylamino)-phenyl]-[5-(2,3-dihydroxy-propoxy)-2-fluoro-phenyl]-methanone (Compound 316),
[2-Chloro-4-(2,6-difluoro-phenylamino)-phenyl]-[2-chloro-5-(2-morpholin-4-yl-ethoxy)-phenyl]-methanone (compound 328),
(±)-[2-Chloro-4-(2,6-difluoro-phenylamino)-phenyl]-[2-chloro-5-(2,3-dihydroxy-propoxy)-phenyl]-methanone (compound 329),
[5-(3-Bromo-propoxy)-2-chloro-phenyl]-[2-chloro-4-(2,6-difluoro-phenylamino)-phenyl]-methanone (compound 330),
[2-Chloro-4-(2,4-difluoro-phenylamino)-phenyl]-(5-hydroxymethyl-2-methyl-phenyl)-methanone (compound 331),
[2-Chloro-4-(2,4-difluoro-phenylamino)-phenyl]-(5-chloromethyl-2-methyl-phenyl)-methanone (compound 332),
(5-Azidomethyl-2-methyl-phenyl)-[2-chloro-4-(2,4-difluoro-phenylamino)-phenyl]-methanone (compound 333),
(5-Aminomethyl-2-methyl-phenyl)-[2-chloro-4-(2,4-difluoro-phenylamino)-phenyl]-methanone (compound 334),
[2-Chloro-4-(2,4-difluoro-phenylamino)-phenyl]-(5-hydroxymethyl-2-methoxy-phenyl)-methanone (compound 335),
Acetic acid 3-[2-chloro-4-(2,4-difluoro-phenylamino)-benzoyl]-4-methoxy-benzyl ester (compound 336),
*N*-*tert*-Butoxy-3-[2-chloro-4-(2,4-difluoro-phenylamino)-benzoyl]-4-methoxy-benzamide (compound 337),
3-[2-Chloro-4-(2,4-difluoro-phenylamino)-benzoyl]-*N*-methoxy-4-methyl-benzamide (compound 338),
N-Butoxy-3-[2-chloro-4-(2,4-difluoro-phenylamino)-benzoyl]-4-methyl-benzamide (compound 339),
3-[2-Chloro-4-(2,4-difluoro-phenylamino)-benzoyl]-*N*-cyclohexylmethoxy-4-methyl-benzamide (compound 340),
3-[2-Chloro-4-(2,4-difluoro-phenylamino)-benzoyl]-4-methyl-*N*-(2-methyl-thiazol-4-ylmethoxy)-benzamide (compound 341),
N-benzyloxy-3-[2-chloro-4-(2,4-difluoro-phenylamino)-benzoyl]-4-methyl-benzamide (compound 342),
3-[2-Chloro-4-(2,4-difluoro-phenylamino)-benzoyl]-*N*-(4-methoxy-benzyloxy)-4-methyl-benzamide (compound 343),
3-[2-Chloro-4-(2,4-difluoro-phenylamino)-benzoyl]-4-methyl-benzoic acid *N*',*N*'-dimethyl-hydrazide (compound 344),
3-[2-Chloro-4-(2,4-difluoro-phenylamino)-benzoyl]-4-methyl-*N*-morpholin-4-yl-benzamide (compound 345),
3-[2-Chloro-4-(2,4-difluoro-phenylamino)-benzoyl]-*N*-hydroxy-4-methyl-benzamide (compound 346),
4-[2-Chloro-4-(2,4-difluoro-phenylamino)-benzoyl]-*N*-(2-hydroxy-ethyl)-3-methyl-benzamide (compound 347),
[2-Chloro-4-(2,4-difluoro-phenylamino)-phenyl]-[5-(3-hydroxy-propenyl)-2-methyl-phenyl]-methanone (compound 348),
4-{3-[2-Chloro-4-(2,4-difluoro-phenylamino)-benzoyl]-4-methyl-benzoylamino}-thiophene-3-carboxylic acid methyl ester (compound 349),
3-[2-Chloro-4-(2,4-difluoro-phenylamino)-benzoyl]-*N*-furan-2-ylmethyl-4-methyl-benzamide (compound 350),
3-[2-Chloro-4-(2,4-difluoro-phenylamino)-benzoyl]-*N*-(3-methoxy-phenyl)-4-methyl-benzamide (compound 351),
2-{3-[2-Chloro-4-(2,4-difluoro-phenylamino)-benzoyl]-4-methyl-benzoylamino}-benzoic acid methyl ester (compound 352),
3-{3-[2-Chloro-4-(2,4-difluoro-phenylamino)-benzoyl]-4-methyl-benzoylamino}-thiophene-2-carboxylic acid methyl ester (compound 353),
4-{3-[2-Chloro-4-(2,4-difluoro-phenylamino)-benzoyl]-4-methyl-benzoylamino}-thiophene-3-carboxylic acid (compound 354),
2-{3-[2-Chloro-4-(2,4-difluoro-phenylamino)-benzoyl]-4-methyl-benzoylamino}-benzoic acid (compound 355),
3-[2-Chloro-4-(2,4-difluoro-phenylamino)-benzoyl]-*N*-[2-(2-hydroxy-ethylcarbamoyl)-phenyl]-4-methyl-benzamide (compound 356),
3-{3-[2-Chloro-4-(2,4-difluoro-phenylamino)-benzoyl]-4-methyl-benzoylamino}-thiophene-2-carboxylic acid (2-hydroxy-ethyl)-amide (compound 357),
[2-Chloro-4-(2,4-difluoro-phenylamino)-phenyl]-(5-ethynyl-2-methyl-phenyl)-methanone (compound 362),
3-[2-Chloro-4-(2,4-difluoro-phenylamino)-benzoyl]-4-methyl-benzoic acid hydrazide (compound 364),
1-{3-[2-Chloro-4-(2,4-difluoro-phenylamino)-benzoyl]-4-methyl-benzoyl}-4-ethyl-3-thio semicarbazide (compound 365),
[2-Chloro-4-(2,4-difluoro-phenylamino)-phenyl]-[5-(5-ethylamino-[1,3,4]thiadiazol-2-yl)-2-methyl-phenyl]-methanone (compound 366),
[2-Chloro-4-(2,4-difluoro-phenylamino)-phenyl]-[2-methyl-5-(1H-tetrazol-5-yl)-phenyl]-methanone (compound 367),
3-{3-[2-Chloro-4-(2,4-difluoro-phenylamino)-benzoyl]-4-methyl-phenyl}-3-oxo-propionic acid ethyl ester (compound 368),
[2-Chloro-4-(2,4-difluoro-phenylamino)-phenyl]-[5-(4,5-dihydro-oxazol-2-yl)-2-methyl-phenyl]-methanone (compound 369),
3-[2-Chloro-4-(2,4-difluorophenylamino)benzoyl]-4-methylbenzoic acid (Compound 424),
2-Methylacrylic acid 2-{3-[2-chloro-4-(2,4-difluorophenylamino)benzoyl]-4-methylbenzoylamino}ethyl ester (Compound 425),
3-[2-Chloro-4-(4-chloro-2-fluoro-phenylamino)-benzoyl]-4-methyl-benzoic acid (Compound 432),
3-[2-Chloro-4-(2,4-difluoro-phenylamino)-benzoyl]-4-methoxy-benzoic acid (compound 437),
3-[2-Chloro-4-(2,6-difluoro-phenylamino)-benzoyl]-4-methoxy-benzoic acid (compound 443).

In addition to the definition of R₄ indicated above, it is envisaged that R₄ may include substituents included in the definition of R₆ in WO 03/018535, the content of which is hereby incorporated by reference in its entirety.

It is further envisaged that the compounds of formula I may be *N*-substituted at the amino group between rings B and C of the core structure, using substituents substantially as disclosed in US Provisional Application No. 60/434,798, the content of which is hereby incorporated by reference in its entirety.

### Methods of preparation

The compounds of the present invention may be prepared in a number of ways well known to those skilled in the art of organic synthesis. The compounds of the present invention can be synthesised using the methods outlined below, together with methods known in the art of synthetic organic chemistry, or variations thereof as appreciated by those skilled in the art, Preferred methods include, but are not limited to, those described below.
The compounds of formula I may be prepared using the reactions and techniques described in this section. The reactions are performed in solvents that are appropriate with respect to the reagents and materials employed and that are suitable for the transformations being effected. Also, in the synthetic methods described below, it is to be understood that all proposed reaction conditions, including choice of solvent, reaction atmosphere, reaction temperature, duration of experiment and work-up procedures, are chosen to be conditions of standard for that reaction, which should be readily recognised by one skilled in the art. It is understood by one skilled in the art of organic synthesis, that the functionality present on various positions of the molecules used as the starting compounds or intermediates in the syntheses, must be compatible with the reagents and reactions proposed. Not all compounds of formula I falling into a given class may be compatible with some of the reaction conditions required in some of the methods described. Such restrictions of substituents or functional groups which are compatible with the reaction conditions will be readily apparent to one skilled in the art and alternative methods can be used.

Compounds according to the present invention may be prepared by a process comprising coupling of an amine of formula III with a triflate or halide, such as a bromide, iodide, fluoride, chloride, of formula II, as shown in Scheme 1; or alternatively by a process comprising coupling of an amine of formula IIa with a triflate or halide, such as a bromide or iodide, of formula IIIa, as shown in Scheme 1; where R₁, R₂, R₃, R₄, R₅, and R₆ are as defined above; except that any substituents or functional groups which are potentially reactive in the coupling reaction may be protected before the coupling reaction is performed, and the protective groups removed subsequently.

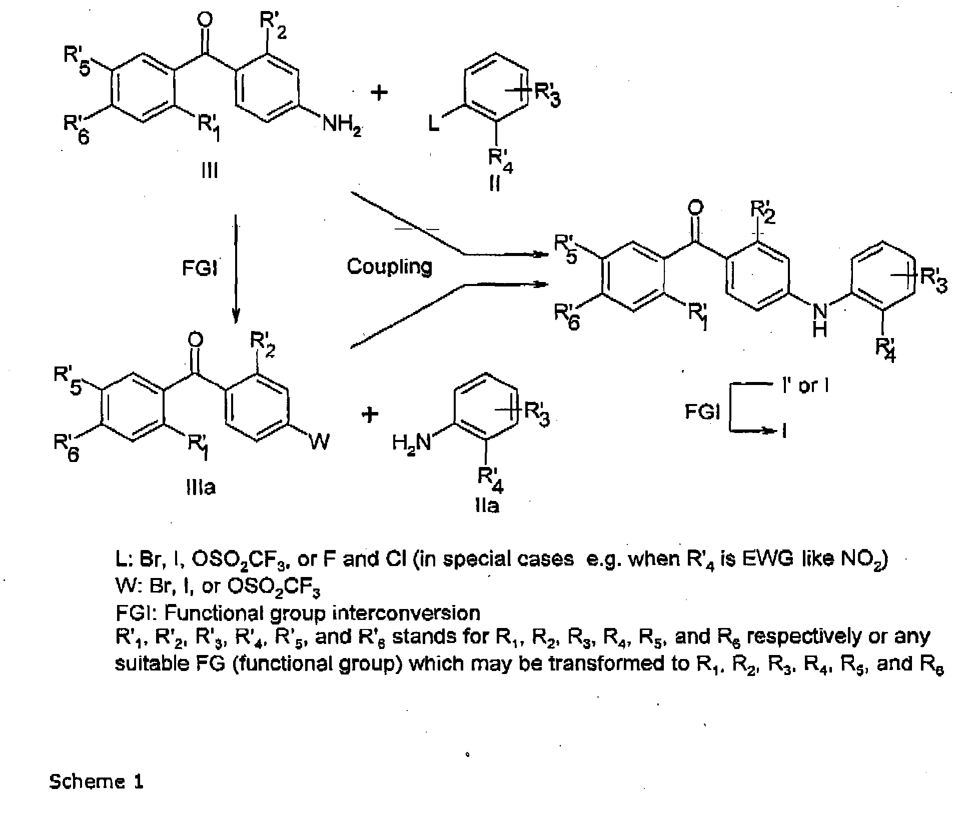

The coupling reaction is carried out by using a method for the formation of diphenylamines well known to one skilled in the art of organic synthesis. The preferred method is the palladium catalysed amination method which comprises coupling of an amine with an arylhalogenide (or aryltriflate) in the presence of a base, a suitable Pd source, and a suitable phosphine ligand in an inert solvent. Different palladium compounds may be used in the process, non-limiting examples of which are palladium(II) acetate, palladium(II) chloride, palladium(II) bromide, dichlorobis(triphenylphosphine)palladium(II), tetrakis(triphenylphosphine)palladium(0), tris(dibenzylideneacetone)dipalladium(0). The preferred phosphine ligands include, but are not limited to, racemic or non-racemic 2,2'-bis(diphenylphosphino)-1,1'-binaphthyl (hereinafter refered to as BINAP), tri-o-tolylphosphine, tri-*tert*-butylphosphine, 1,1'-bis(diphenylphosphino)-ferrocene, bis[(2-diphenylphosphino)phenyl]ether (DPEphos), 2-dicyclohexylphosphanyl-2'-dimethylaminobiphenyl, 2-(di-*tert*-butylphosphino)biphenyl and 9,9-dimethyl-4,6-bis(diphenylphosphino)xanthene (Xantphos). The amount of palladium and ligand used in this catalytic process may be typically in the range 0.1 to 10 % by mole relative to the amount of the aromatic halide (or triflate) used. Especially sodium-*tert*-butoxide (NaOt-Bu) and caesium carbonate (Cs₂CO₃) have proven to be the preferred bases in this process, but other bases may be used as well. The reaction is typically performed at elevated temperatures (80-120 °C) in inert solvents such as 1,4-dioxane, toluene, benzene, and tetrahydrofuran under an inert atmosphere, e.g. argon or nitrogen.

When R'₄ is an electron withdrawing group (EWG) such as nitro or cyano the above coupling may also be performed non-catalytically in the presence of strong bases like potassium- or sodium-*tert*-butoxide. The reaction is typically performed at room temperature or higher (20-200 °C) in aprotic solvents like dimethylsulfoxide (DMSO), *N*,*N*-dimethylformamide (DMF) or *N-*methylpyrrolidinone (NMP) under an inert atmosphere, e.g. argon or nitrogen.

Compounds according to the present invention of the general formula III may be prepared by several methods known to those skilled in the art of organic synthesis, One useful sequence is shown in Scheme 2. The key step comprises the coupling of a halide, preferably an iodide or bromide of general formula VI with an acid chloride of general formula V to afford a benzophenone of general formula IV. The benzophenone IV may then be reduced to the corresponding amine of general formula III by treatment with standard reducing agents. Examples of such reducing agents include, but are not limited to, stannous chloride dihydrate, hydrogen, ammonium formiate or hydrazine hydrate and a catalytic amount of palladium on carbon, The coupling reaction may be achieved by transforming the halide (VI) into a reactive organometallic intermediate, such as by treatment with iso-propyl magnesium chloride to afford the corresponding magnesium derivative, or by treatment with n-butyllithium to afford the corresponding lithium derivative.

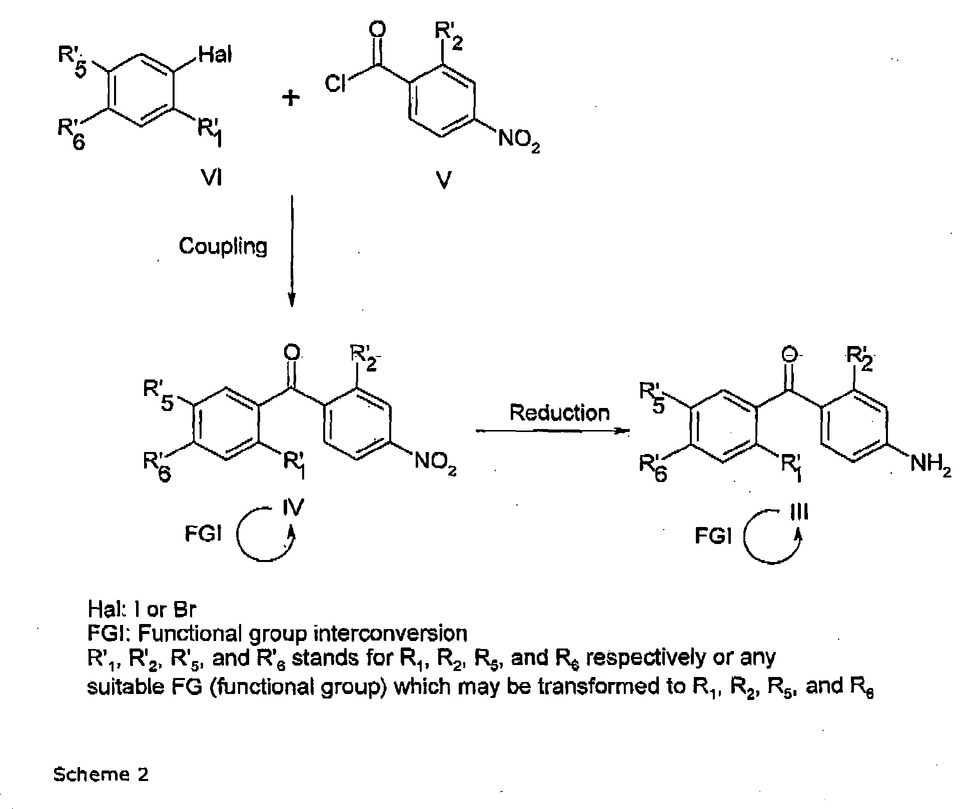

The reactivity of this organometallic intermediate is then modulated by transmetalation to e.g. zinc, by treatment with ZnCl₂, ZnBr₂, or ZnI₂. This organozinc compound is then coupled with the acid halide, such as an acid chloride of general formula V, in the presence or meditated by a catalytic amount of a palladium(0) complex. Examples of such palladium catalysts include but are not limited to tetrakis(triphenylphosphine)palladium(0), tetrakis(triphenylarsine)palladium(0), dichlorobis(triphenylphosphine)palladium(II), or benzylchlorobis(triphenylphosphine)palladium(II). Alternatively, the organozinc compound is coupled with the acid halide, such as the acid chloride, of general formula V, in the presence or mediated by an equimolar or substoichiometric or catalytic amount (with regard to V), such as 0.1- 99 mol%, e.g. 0.5- 10 mol%, e.g. 1-5 mol%, e.g. 2-3 mol% of copper (I) or (II) salts, such as copper(II)acetate or the soluble complex CuCN·2LiCl or CuCN·2LiBr. The coupling reaction is typically performed at room temperature in inert solvents such as 1,4-dioxane, toluene, benzene, and tetrahydrofuran under an inert atmosphere, e.g. argon or nitrogen.

Compounds accordingly to the present invention of general formula IIIa may be prepared by analogous zinc mediated cross-coupling procedures as shown in Scheme 3.

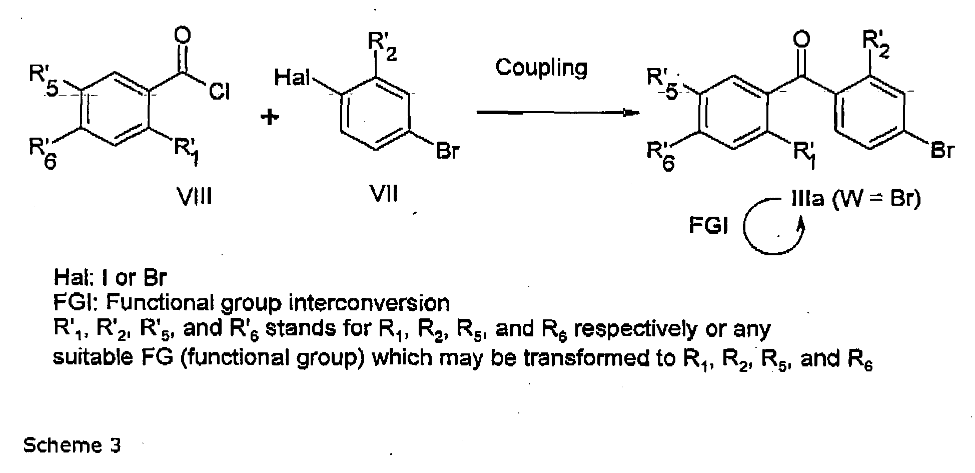

Compounds according to the present invention may in special cases be prepared by a simple functional group interconversion (FGI), meaning a standard process, known to those skilled in the art of organic synthesis, where a functional group in compounds with the general formula I or I' is transformed into a different functional group in one or more synthetic steps, leading to a new compound with the general formula I. Examples of such processes include, but are not limited to, hydrolysis of an ester to give an acid under basic conditions, deprotection of a methylether to give a phenol by treatment with e.g. borontribromide (BBr₃), and catalytic hydrogenation of an olefin to give a saturated hydrocarbon. Non limiting examples of such transformations are described in "Comprehensive Organic Transformations", by R. C. Larock, VCH 1989, which is hereby incorporated as reference and in general procedures. Especially, the use of general protective groups in one or more synthetic steps may be convenient in the synthesis of compounds with the general formula I. Examples of such general protective groups include, but are not limited to, methyl, ethyl, *tert*-butyl or benzyl esters as protective groups of an hydroxy group; tetrahydropyranyl- or silyl-ethers as protective groups of an hydroxy group.

As shown in Scheme 2 and 3 each intermediate compound may be transformed by an FGI process as described above to give new compounds with the same general formula (e.g. an hydroxy group may be protected as an *tert*-butyl-dimethyl-silyl ether). This is only to illustrate the flexibility in the synthesis, and in general the described sequence of processes is only one of many possible strategies for the synthesis of compounds of the present invention. That is, it may be more advantageous in some cases to alter the sequence of the processes described above. The described sequence of processes is not considered as being limiting the preparation of compounds of the present invention of general formula I, and alteration of the reaction sequence may be an obvious alternative for those skilled in the art of organic synthesis. This aspect of the invention may especially be advantageous in the synthesis of compounds with different substituents in the R₄, R₅, and R₆ groups. Readily available intermediates may serve as starting point for the synthesis of various series of compounds covered by the general formula I.

### Pharmaceutical compositions

In another aspect, the invention relates to a pharmaceutical composition comprising, as an active component, a compound of formula I together with a pharmaceutically acceptable excipient, carrier or vehicle, Furthermore, the invention relates to the use of a compound of formula I for the preparation of a medicament for the prevention, treatment or amelioration of inflammatory diseases or conditions.

Pharmaceutical compositions of the invention may be in unit dosage form such as tablets, pills, capsules, powders, granules, elixirs, syrups, emulsions, ampoules, suppositories or parenteral solutions or suspensions; for oral, parenteral, opthalmic, transdermal, intra-articular, topical, pulmonal, nasal, buccal or rectal administration or in any other manner appropriate for the formulation of anti-inflammatory compounds and in accordance with accepted practices such as those disclosed in Remington: The Science and Practice of Pharmacy. 19th Ed., Mack Publishing Company, 1995. In the composition of the invention, the active component may be present in an amount of from about 0.01 to about 99%, such as 0.1% to about 10 % by weight of the composition.

For oral administration in the form of a tablet or capsule, a compound of formula I may suitably be combined with an oral, non-toxic, pharmaceutically acceptable carrier such as ethanol, glycerol, water or the like. Furthermore, suitable binders, lubricants, disintegrating agents, flavouring agents and colourants may be added to the mixture, as appropriate. Suitable binders include, e.g., lactose, glucose, starch, gelatin, acacia gum, tragacanth gum, sodium alginate, carboxymethylcellulose, polyethylene glycol, waxes or the like. Lubricants include, e.g., sodium oleate, sodium stearate, magnesium stearate, sodium benzoate, sodium acetate, sodium chloride or the like. Disintegrating agents include, e.g., starch, methyl cellulose, agar, bentonite, xanthan gum or the like. Additional excipients for capsules include macrogols or lipids.

For the preparation of solid compositions such as tablets, the active compound of formula I is mixed with one or more excipients, such as the ones described above, and other pharmaceutical diluents such as water to make a solid preformulation composition containing a homogenous mixture of a compound of formula I. The term "homogenous" is understood to mean that the compound of formula I is dispersed evenly throughout the composition so that the composition may readily be subdivided into equally effective unit dosage forms such as tablets or capsules. The preformulation composition may then be subdivided into unit dosage forms containing from about 0.05 to about 1000 mg, in particular from about 0.1 to about 500 mg, of the active compound of the invention.

Liquid formulations for either oral or parenteral administration of the compound of the invention include, e.g., aqueous solutions, syrups, aqueous or oil suspensions and emulsion with edible oils such as cottonseed oil, sesame oil, coconut oil or peanut oil. Suitable dispersing or suspending agents for aqueous suspensions include synthetic or natural gums such as tragaca nth, alginate, acacia, dextran, sodium carboxymethylcellulose, gelatin, methylcellulose or polyvinylpyrolidone.

For parenteral administration, e.g. intramuscular, intraperitoneal, subcutaneous or intravenous injection or infusion, the pharmaceutical composition preferably comprises a compound of formula I dissolved or solubilised in an appropriate, pharmaceutically acceptable solvent. For parenteral administration, the composition of the invention may include a sterile aqueous or non-aqueous solvent, in particular water, isotonic saline, isotonic glucose solution, buffer solution or other solvent conventionally used for parenteral administration of therapeutically active substances. The composition may be sterilised by, for instance, filtration through a bacteria-retaining filter, addition of a sterilising agent to the composition, irradiation of the composition, or heating the composition. Alternatively, the compound of the invention may be provided as a sterile, solid preparation, e.g. a freeze-dried powder, which is dissolved in sterile solvent immediately prior to use.

The composition intended for parenteral administration may additionally comprise conventional additives such as stabilisers, buffers or preservatives, e.g. antioxidants such as methylhydroxybenzoate or the like.

Compositions for rectal administration may be in the form of a suppository incorporating the active ingredient and a carrier such as cocoa butter, or in the form of an enema.

Compositions suitable for intra-articular administration may be in the form of a sterile aqueous preparation of the active ingredient which may be in microcrystalline form, for example, in the form of an aqueous microcrystalline suspension. Liposomal formulations or biodegradable polymer systems may also be used to present the active ingredient for both intra-articular and ophthalmic administration.

Compositions suitable for topical administration, including ophthalmic treatment, include liquid or semi-liquid preparations such as liniments, lotions, gels, applicants, oil-in-water or water-in-oil emulsions such as creams, ointments or pastes; or solutions or suspensions such as drops, For topical administration, the compound of formula I may typically be present in an amount of from 0.01 to 20% by weight of the composition, such as 0.1% to about 10 %, but may also be present in an amount of up to about 50% of the composition.

Compositions for ophthalmic treatment may preferably additionally contain a cyclodextrin.

Compositions suitable for administration to the nasal or buccal cavity or for inhalation include powder, self-propelling and spray formulations, such as aerosols and atomizers. Such compositions may comprise a compound of formula I in an amount of 0.01-20%, e.g. 2%, by weight of the composition.

The composition may additionally comprise one or more other active components conventionally used in the treatment of various inflammatory diseases and conditions. Examples of such additional active components may be selected from the group consisting of glucocorticoids, vitamin D and vitamin D analogues, antihistamines, platelet activating factor (PAF) antagonists, anticholinergic agents, methylxanthines, β-adrenergic agents, COX-2 inhibitors, salicylates, indomethacin, flufenamate, naproxen, timegadine, gold salts, penicillamine, serum cholesterol lowering agents, retinoids, zinc salts and salicylazosulfapyridine.

The compounds of the invention may be used in a method of treating inflammatory diseases or conditions, or ophthalmic diseases or conditions, or cancer, the method comprising administering, to a patient in need thereof, an effective amount of a compound of formula I.

A suitable dosage of the compound of the invention will depend, inter alia, on the age and condition of the patient, the severity of the disease to be treated and other factors well known to the practising physician. The compound may be administered either orally, parenterally or topically according to different dosing schedules, e.g. daily or with weekly intervals. In general a single dose will be in the range from 0.01 to 400 mg/kg body weight. The compound may be administered as a bolus (i.e. the entire daily dosis is administered at once) or in divided doses two or more times a day.

Inflammatory diseases or conditions contemplated for treatment with the present compounds are inflammatory diseases where modulation of cytokine expression and secretion may be mediated by MAP kinases such as the p38 MAP kinase as discussed above. Examples of inflammatory diseases or conditions believed to be mediated by the p38 MAP kinase are selected from the group consisting of asthma, arthritis, including rheumatoid arthritis and spondyloarthritis, gout, atherosclerosis, inflammatory bowel diease, Crohn's disease, neurological inflammations, inflammatory eye diseases, proliferative and inflammatory skin disorders, such as psoriasis, atopic dermatitis and acne vulgaris, uveitis, sepsis, septic shock and osteoporosis.

The treatment may additionally involve administration of one or more other anti-inflammatory active components such as glucocorticoids, vitamin D and vitamin D analogues, antihistamines, platelet activating factor (PAF) antagonists, anticholinergic agents, methylxanthines, β-adrenergic agents, COX-2 inhibitors, salicylates, indomethacin, flufenamate, naproxen, timegadine, gold salts, penicillamine, serum cholesterol lowering agents, retinoids, zinc salts and salicylazosulfapyridine. The administration of a compound of the present invention and another anti-inflammatory component may be either concomitantly or sequentially.

Ophthalmic diseases or conditions contemplated for treatment with the present compounds include the ophthalmic disease or condition is non-infectious (e.g. allergic) conjunctivitis, iritis, keratitis, uveitis, scleritis, episcleritis, sympathetic ophthalmitis, blepharitis or keratoconjunctivitis sicca.

### Pharmacological methods

To study the effect of compounds of the present invention *in vitro,* the inhibition of IL-1β and TNF-α secretion was determined using the following procedure: Cytokine production was measured in the media from lipopolysaccharide (LPS) stimulated peripheral blood mononuclear cells. The mononuclear cells were isolated from human peripheral blood by Lymphoprep^{®} (Nycomed, Norway) fractionation and suspended in RPMI 1640 (growth medium) with foetal calf serum (FCS, 2%), at a concentration of 5 x 10⁵ cells/ml. The cells were incubated in 24-well tissue culture plates in 1 ml aliquots. Test compounds were dissolved in dimethylsulfoxide (DMSO, 10 mM) and were diluted with the medium. Compounds were added to the cells for 30 minutes, then LPS (1 mg/ml final concentration) was added. The plates were incubated for 18 hours, and the concentration of IL-1β and TNF-α in the medium was determined by enzyme-linked immunosorbent assays. The median inhibitory concentrations (IC₅₀) of the compounds were calculated. The results are shown in Table 1.

**Table 1. Inhibition of cytokines production in vitro by compounds of the present invention.**

| **The median inhibition concentration (IC₅₀, nM) of** | | |
|---|---|---|
| **Compound No.** | **IL-1β** | **TNF-α** |
| Compound 115 | 1.3 | 0.3 |
| Compound 120 | 2.7 | 2.0 |
| Compound 121 | 2.8 | 0.6 |
| Compound 122 | 0.5 | 0.2 |
| Compound 123 | 0.5 | 0.3 |
| Compound 126 | 2.8 | 0.6 |
| Compound 129 | 2.5 | 1.3 |
| Compound 131 | 3.2 | 1.3 |
| Compound 135 | 4.0 | 2.0 |
| Compound 136 | 4.0 | 2.0 |
| Compound 140 | 1.4 | 1.0 |
| Compound 141 | 0.9 | 0.6 |
| Compound 143 | 2.5 | 1.6 |
| Compound 144 | 1.0 | 0.6 |
| Compound 145 | 1.3 | 0.3 |
| Compound 147 | 2.5 | 0.8 |
| Compound 148 | 0.6 | 0.2 |
| Compound 149 | 1.6 | 0.5 |
| Compound 151 | 1.8 | 0.4 |
| Compound 152 | 2.8 | 0.6 |
| Compound 153 | 8.9 | 1.6 |
| Compound 154 | 3.2 | 2.5 |
| Compound 155 | 0.6 | 0.2 |
| Compound 156 | 4.5 | 0.9 |
| Compound 157 | 2.2 | 0.9 |
| Compound 158 | 0.6 | 0.2 |
| Compound 159 | 7.9 | 1.3 |
| Compound 164 | 1.3 | 2.0 |
| Compound 166 | 0.5 | 0.4 |
| Compound 1.67 | 1.7 | 2.0 |
| Compound 168 | 2.5 | 2.5 |
| Compound 169 | 0.4 | 0.4 |
| Compound 170 | 3.2 | 2.5 |
| Compound 173 | 2.5 | 2.0 |
| Compound 174 | 3.2 | 2.0 |
| Compound 176 | 3.2 | 2.0 |
| Compound 178 | 6.3 | 1.6 |
| Compound 180 | 2.5 | 3.2 |
| Compound 182 | 8.9 | 2.2 |
| Compound 183 | 2.0 | 0.7 |
| Compound 241 | 1.1 | 0.3 |
| Compound 242 | 1.4 | 0.5 |
| Compound 243 | 2.8 | 1.3 |
| Compound 244 | 1.3 | 0.5 |
| Compound 245 | 3.5 | 0.9 |
| Compound 246 | | 3.2 |
| Compound 247 | 5.6 | 1.4 |
| Compound 248 | 1.0 | 0.7 |
| Compound 249 | 1.6 | 1.1 |
| Compound 251 | 1.1 | 0.4 |
| Compound 252 | 0.6 | 0.6 |
| Compound 260 | 1.8 | 0.5 |
| Compound 261 | 2.2 | 1.0 |
| Compound 262 | 2.0 | 0.6 |
| Compound 263 | | 1.6 |
| Compound 264 | 1.0 | 0.3 |
| Compound 265 | 1.0 | 0.3 |
| Compound 266 | 0.9 | 0.4 |
| Compound 267 | 0.4 | 0.2 |
| Compound 269 | 0.8 | 0.6 |
| Compound 271 | 1.0 | 0.4 |
| Compound 272 | 3.2 | 1.4 |
| Compound 275 | 5.0 | 0.3 |
| Compound 277 | 2.5 | 0.8 |
| Compound 278 | 1.3 | 0.6 |
| Compound 279 | 0.6 | 0.5 |
| Compound 282 | 5.6 | 0.6 |
| Compound 285 | 1.4 | 0.6 |
| Compound 314 | | 2.8 |
| Compound 335 | 2.7 | 2.5 |
| Compound 336 | 5.0 | 2.0 |
| Ref. comp. a | 13 | 7.1 |
| | | |
| Ref. comp. b | 6.3 | 6.3 |
| | | |
| Ref. comp. c | 32 | 6.3 |
| | | |
| Ref. comp. d | 7.9 | 3.2 |
| | | |
| Ref. comp. e | 6.3 | 3.2 |
| | | |
| Ref. comp. f | 13 | 4.0 |

| | | |
|---|---|---|
| Ref. comp. a: (4-(2-aminophenylamino)-2-Chloro-2'-methylbenzophenone, Compound 156 disclosed in WO 98/32730. Ref. comp. b: 2'-[3-Chloro-4-(2-methylbenzoyl)phenylamino]octananilide, Compound 102 disclosed in WO 01/05746. Ref. comp. c: 1-Acetoxymethyl *N*-[2-[3-chloro-4-(2-methylbenzoyl)phenylamino]phenyl]carbamate, Compound 109 disclosed in WO 01/05749. Ref. comp. d: 1-Ethyl-3-[2-[3-chloro-4-(2-methylbenzoyl)phenylamino]-5-fluorophenyl]urea, Compound 114 disclosed in WO 01/05751. Ref. comp. e: 2,2,2-Trifluoro-*N*-[2-[3-chloro-4-(2-methylbenzoyl)phenylamino]-5-fluoro-phenyl]acetamide, Compound 102 disclosed in WO 01/05745. Ref. comp. f: 2-Chloro-4-(3-fluoro-2-methyl-phenylamino)-2'-methylbenzophenone, Compound 131 disclosed in WO 01/42189. | | |

These results show that compounds of the present invention are highly potent inhibitors of the production of IL-1β, TNF-α and show a surprisingly higher cytokine inhibitory activity than the reference compounds, thus making them potentially useful in the treatment of inflammatory diseases.

Furthermore, the novel aminobenzophenone derivatives have surprisingly favourable pharmacokinetic properties such as absorption and metabolic stability.

### p38α MAP kinase assay

### Cell culture

COS-1 cells (derived from African green monkey kidney fibroblast-like cell containing wild type T antigen under control of the SV40 promotor) were obtained from ATCC (ATCC no. CRL-1650) and grown in growth medium (DMEM without phenolred, 10% FCS, 2 mM L-glutamine, 100U penicillin and 100 µg streptomycin/ml) at 37°C with 5% CO₂. The cells were passaged twice a week by trypsination (0.25% trypsin, 1 mM EDTA in PBS) and were split 1:10. The medium was changed every second or third day. The cell line was regularly tested with the Mycoplasma PCR Primer Set (Stratagene) and found to be free of *Mycoplasma.* Tissue culture media, FCS, L-glutamine and penicilin and streptomycin are from Bribco BRL, Gaithersburg, MD, USA.

### Transient expression of COS-1 cells

On day one COS-1 cells were seeded in 143 cm² petridish with a density of 2×10⁴celler/cm² in growth medium. At day 2 the cells were co-transfected with 5 µg (total) of experimental plasmid DNA, expressing the FLAG-p38α and FLAG-MKK6(EE). The plasmids were introduced into the COS-1 cells in serum-free medium using DOTAP™ (Boehringer-Mannheim, Mannheim, Germany). Plasmid DNA was prepared and purified using the QIAGEN EndoToxin-free Maxiprep-500 kit (Hilden, Germany). Briefly, DNA and DOTAP™ were mixed for exactly 15 min. at 37°C in the CO₂ incubator. The transfection-mixture was hereafter transferred to a 15-ml falcon-tube and transfection-medium (DMEM with L-Glutamine and Pen./Strep. but without serum) was added to the transfection-mixture, followed by addition to the cell-monolayer. After 4 hours of incubation with DOTAP™ and plasmids, the medium containing double amount of serum was added to the cells bringing the final concentration of serum up to 10%. The cells were then incubated for 24 hours before kinase reaction.

### Immunoprecipitation

After 24 hrs of incubation the reaction was stopped by putting the petri dish on an ice-bath. The medium was aspirated, and the cell monolayer was washed once in ice-cold PBS (137 mM NaCl, 1.5 mM KH₂PO₄, 2.7 mM KCl, 8.1 mM Na₂HPO₄.2H₂O), and hereafter solubillsed for 10 min. in 1.5 ml lysis buffer (50 mM HEPES, pH 7.5, 150 mM NaCl, 10 mM EDTA, 10 mM Na₄P₂O₇, 100 mM NaF, 2 mM Na₃VO₄, 1% Triton-X-100, Pefabloc 500 µM, Leupeptin 10 µg/µl, Aprotinin 10 µg/µl) was added. The cell-monolayer was scraped by a rubber-policeman, and transferred to an Eppendorf tube. The solubilised cells were clarified by centrifugation at 10.000xg for 10 min. at 4°C. The supernatant was transferred to 50 µl prewashed Protein G Sepharose beads in HNT-buffer (30 mM HEPES, pH 7.5, 30 mM NaCl, 0.1% Triton X-100) and were incubated with 2 µg/sample of monoclonal anti-FLAG™ M2 antibody (raised against the FLAG-epitope, NH₂-Asp-Tyr-Lys-Asp-ASp-Asp-Asp-Lys-COOH) for 1 hour at room temperature. The anti-FLAG M2 monoclonal antibody was obtained from Sigma (cat. no. F-3165). Approx. 60 µg protein of clarified cell lysate were added to the preadsorbed anti-FLAG™ antibodies on Protein G Sepharose beads and incubated for 90 min. at 4°C in a blood sample mixer. After the immunoprecipitation period the Sepharose beads were washed twice in lysis buffer and twice in a kinase reaction buffer (25 mM HEPES pH 7.5, 10 mM magnesium acetate, 50µM ATP).

### Incubation of the compounds with purified p38α kinase

The pre-washed immunoprecipitated anti-FLAG-p38 adsorbed on Protein G Sepharose beads was washed twice in 1×kinase-buffer (25 mM HEPES pH 7.5, 10 mM magnesium acetate, 50µM ATP), and the supernatant was aspirated. The compounds were diluted in 1× kinase buffer at the appropriate concentration. The compounds were added to the washed immunoprecipitated and activated FLAG-p38 adsorbed on the Protein G Sepharose beads for 30 min. at 30°C in a volume of 100 µl. Every 10 min. the Eppendorf tubes were tapped to ensure that the beads and the compounds were in the solution. After 30 min. incubation, the beads were spun down and the supernatant was aspirated.

### p38α MAP Kinase Redaction

The kinase reaction was started by add ing 1 µg GST-ATF-2 substrate (Santa Cruz, LaJolla, CA, USA, cat. no. sc-4114) together with 2 µCi γ-³²P-ATP in 1× kinase-buffer per sample. The reaction was allowed to proceed for 30 min. at 30°C, and it was stopped by adding 40 µl of 2×SDS-sample buffer to the kinase reaction. The samples were boiled, spinned down, and resolved on a 15% SDS-PAGE. The dried SDS-PAGE gel was exposed to a Phospho-Imager screen and the radioactive PHAS-1 bands were quantified by the STORM860 Phospho-Imager (Molecular Dynamics, Sunnyvale, CA, USA) using the ImageQuaNT software.

In this assay, Compound 112 was found to be a potent p38 MAP kinase inhibitor with an IC₅₀ of 2 nM.

### In vivo screening model of LPS induced TNF-α response in mice

To study the effect of compounds of the present invention *in vivo,* an *in vivo* screening model of LPS induced TNF-α response in mice was set up as follows: Groups of 6 mice (C3H/HeN, female, about 8 weeks (20 g), Bomholtgaard) were dosed with test compounds in suspension vehicle 1 hour prior to LPS administration (LPS from *E. coli* 055:B5, L-4005, Sigma). At time 0, the mice were dosed ip with 1.0 mg LPS/kg. After anesthetization with Hypnorm/Dormicum, the mice were bled from the periorbital venous plexus 80-90 minutes after LPS administration. The blood samples were sampled in EDTA stabilised tubes and centrifuged at 4000 rpm for 10 minutes at 4°C. The plasma level of TNF-α was analysed by ELISA. Compound 156 of WO 98/32730 was used as reference compound. The plasma level of TNF-α was determined using a sandwich ELISA. Microtiter plates were coated with a monoclonal antibody against mouse TNF-α, washed and blocked with a casein buffer. Samples of mouse TNF-α recombinant standards were added to the wells of the microtiter plates and incubated. All standards were tested in triplicate, all plasma samples in single. After sample and standard incubation, the plates were washed and incubated with biotinylated polyclonal secondary antibody against mouse TNF-α and washed. Enzyme conjugate was added to all wells and incubated. Substrate was added and the enzyme/substrate reaction stopped after 15 minutes at room temperature with 1M H₂SO₄. The colour development (OD) was measured at 450 nm on an ELISA reader and the background OD at 620 nm was subtracted. Experiments were approved if the group treated with the reference compound showed a significant inhibition (p<0.05) of the TNF-α response compared to the LPS treated control group. The results of the tested compounds are indicated as a percentage inhibition compared to an LPS treated control group. Compounds were tested at 10 mg/kg. The Mann-Whitney test was used to compare drug treated groups to the LPS treated control group (p<0.05). The results are shown in Table 3.

**Table 3. In vivo inhibition of LPS induced TNF-α production (in %)**

| **Compound No.** | |
|---|---|
| Compound 115 | 96 |
| Compound 120 | 86 |
| Compound 123 | 47 |
| Compound 126 | 79 |
| Compound 129 | 73 |
| Compound 131 | 70 |
| Compound 140 | 76 |
| Compound 141 | 46 |
| Compound 145 | 94 |
| Compound 147 | 89 |
| Compound 148 | 71 |
| Compound 149 | 89 |
| Compound 151 | 99 |
| Compound 152 | 99 |
| Compound 153 | 86 |
| Compound 155 | 91 |
| Compound 156 | 73 |
| Compound 157 | 83 |
| Compound 164 | 76 |
| Compound 166 | 83 |
| Compound 170 | 87 |
| Compound 173 | 90 |
| Compound 174 | 64 |
| Compound 176 | 52 |
| Compound 178 | 67 |
| Compound 251 | 49 |
| Compound 260 | 95 |
| Compound 264 | 87 |
| Compound 266 | 54 |
| Compound 278 | 65 |
| Compound 424 | 44 |
| Ref. comp. a | 23 |

| | |
|---|---|
| Ref. comp. a: (4-(2-aminophenylamino)-2-chloro-2'-methylbenzophenone, Compound 156 disclosed in WO 98/32730. | |

The results show that compounds of the present invention surprisingly show an improved biological activity *in vivo* with respect to inhibition of LPS induced TNF-α production in mice compared to the reference compound, thus making them potentially useful in the treatment of inflammatory diseases.

The invention is described in further detail in the following examples which are not in any way intended to limit the scope of the invention as claimed.

### EXAMPLES

### General procedures

All melting points are uncorrected. For ¹H nuclear magnetic resonance (NMR) spectra (300 MHz) and ¹³C NMR (75.6 MHz) chemical shift values (δ) (in ppm) are quoted, unless otherwise specified; for deuteriochloroform solutions relative to internal tetramethylsilane (δ = 0.00) or chloroform (δ = 7.26) or deuteriochloroform (δ = 76.81 for ¹³C NMR) standard. The value of a multiplet, either defined (doublet (d), triplet (t), quartet (q)) or not (m) at the approximate mid point is given unless a range is quoted, All organic solvents used were anhydrous. Chromatography was performed on silica gel using the flash technique. Appropriate mixtures of ethyl acetate, dichloromethane, methanol, and petroleum ether (40-60) were used as eluents unless otherwise noted.
The following abbreviations have been used:

| | |
|---|---|
| aq | aqueous |
| dba | Dibenzylideneacetone |
| DCM | Dichloromethane |
| DMAP | 4-Dimethylaminopyridine |
| DMF | *N*,*N*-Dimethylformamide |
| DIEA | Ethyl diisopropyl amine |
| EDCI | 1-(3-dimethylaminopropyl)-3-ethylcarbodiimide hydrochloride |
| EtOAc | Ethyl acetate |
| FDPP | Diphenyl-phosphinic acid pentafluorophenyl ester |
| h | Hour(s) |
| HATU | *O*-(7-Azabenzotriazol-1-yl)-*N*,*N*,*N*',*N*'-tetramethyluronium-hexafluorophosphate |
| min | Minutes |
| NMP | *N*-methylmorpholine |
| NMR | Nuclear magnetic resonance |
| rac-BINAP | Racemic 2,2'-bis(diphenylphosphino)-1,1'-binaphtyl |
| RT | Room temperature |
| TBAF | Tetra-*n*-butylammonium fluoride |
| TFA | Trifluoroacetic acid |
| THF | Tetrahydrofuran |
| THP | Tetra hydropyra n |
| TIPSCI | Triisopropylsilyl chloride |
| v | Volume |

**Table 4. Exemplified compounds of general formula I**

| Compound | Example no. | Structure |
|---|---|---|
| 115 | 15 | 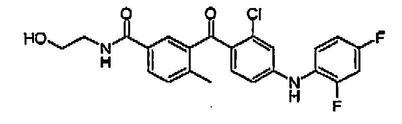 |
| 120 | 20 | 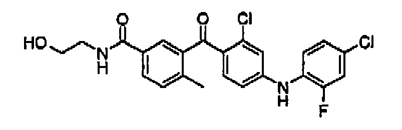 |
| 121 | 21 | 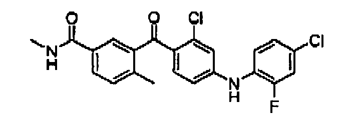 |
| 122 | 22 | 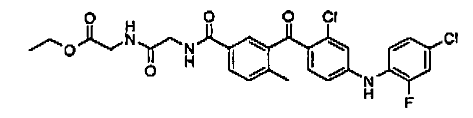 |
| 123 | 23 | 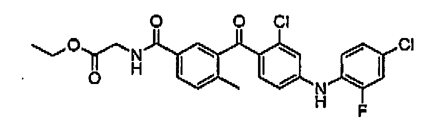 |
| 124 | 24 | 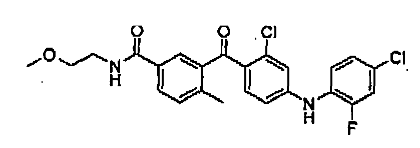 |
| 125 | 25 | 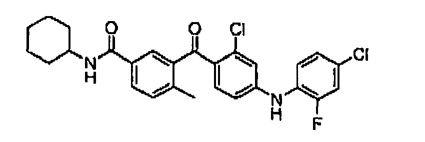 |
| 126 | 26 | 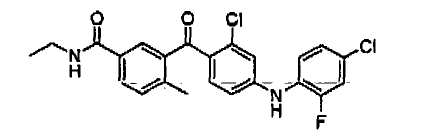 |
| 127 | 27 | 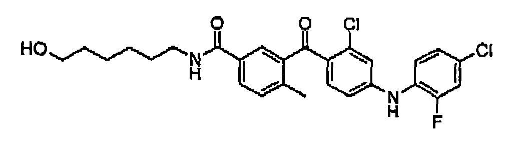 |
| 128 | 28 | 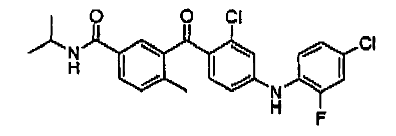 |
| 129 | 29 | 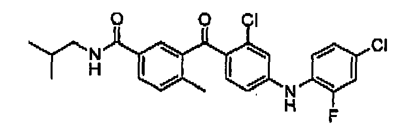 |
| 130 | 30 | 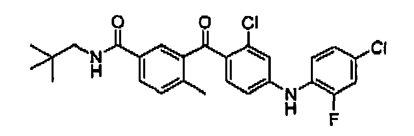 |
| 131 | 31 | 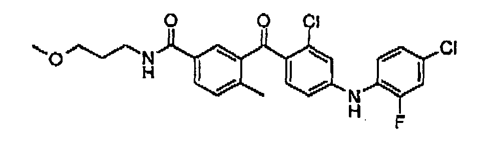 |
| 132 | 32 | 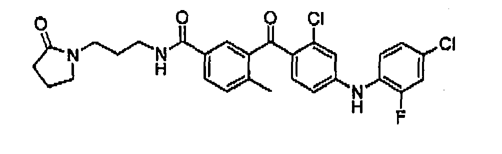 |
| 133 | 33 | 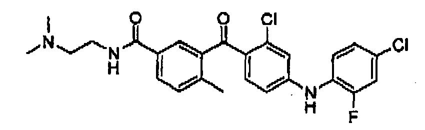 |
| 134 | 34 | 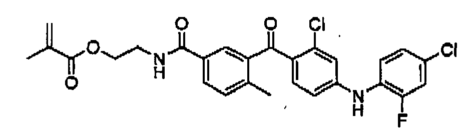 |
| 135 | 35 | 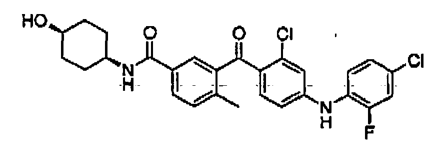 |
| 136 | 36 | 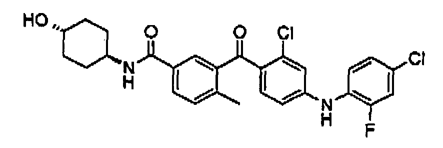 |
| 137 | 37 | 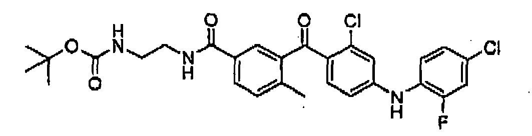 |
| 138 | 38 | 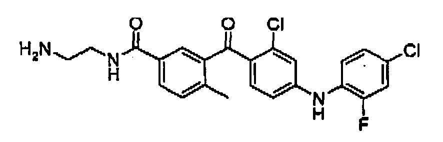 |
| 139 | 39 | 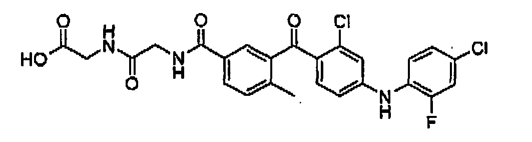 |
| 140 | 40 | 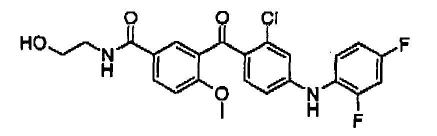 |
| 141 | 41 | 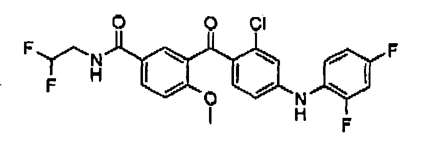 |
| 142 | 42 | 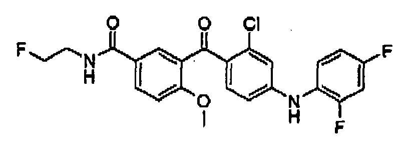 |
| 143 | 43 | 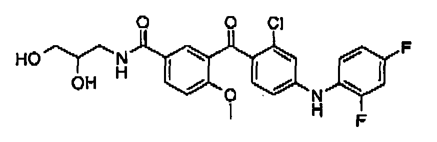 |
| 144 | 44 | 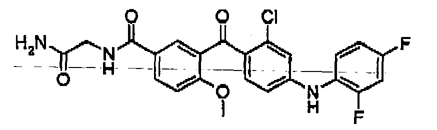 |
| 145 | 45 | 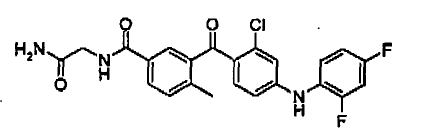 |
| 146 | 46 | 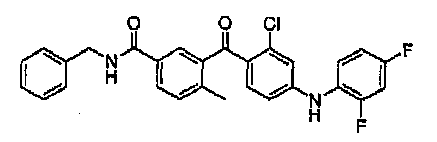 |
| 147 | 47 | 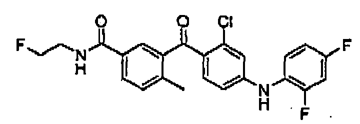 |
| 148 | 48 | 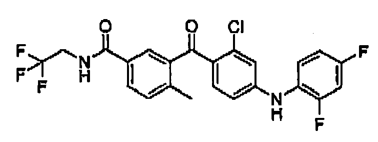 |
| 149 | 49 | 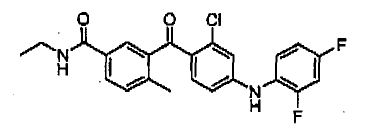 |
| 150 | 50 | 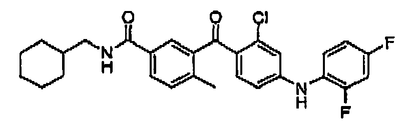 |
| 151 | 51 | 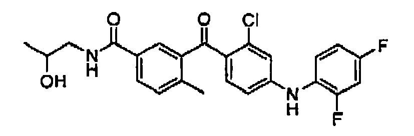 |
| 152 | 52 | 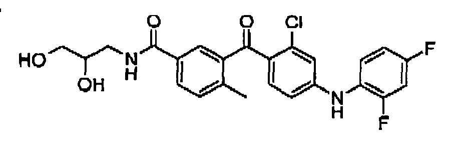 |
| 153 | 53 | 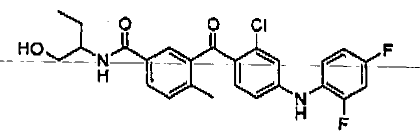 |
| 154 | 54 | 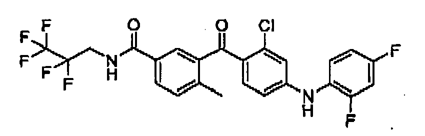 |
| 155 | 55 | 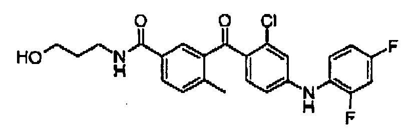 |
| 156 | 56 | 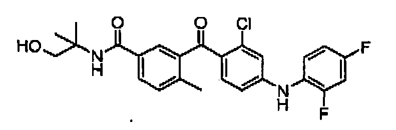 |
| 157 | 57 | 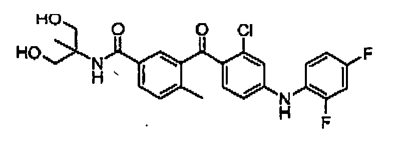 |
| 158 | 58 | 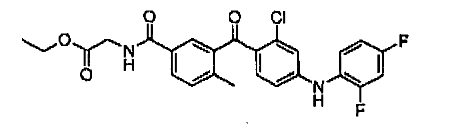 |
| 159 | 59 | 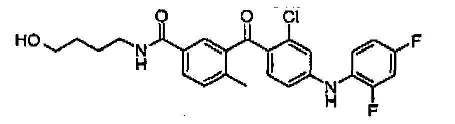 |
| 160 | 60 | 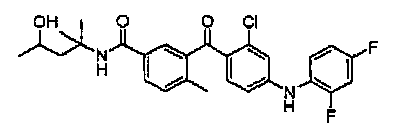 |
| 161 | 61 | 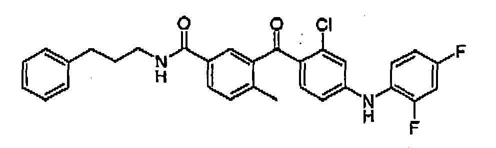 |
| 162 | 62 | 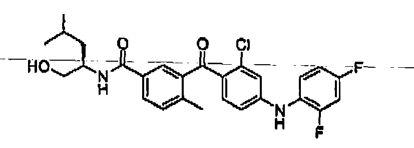 |
| 164 | 64 | 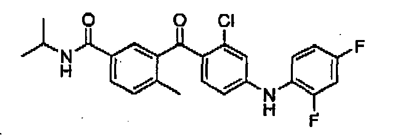 |
| 165 | 65 | 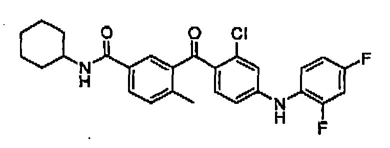 |
| 166 | 66 | 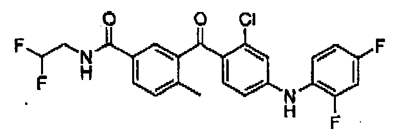 |
| 167 | 67 | 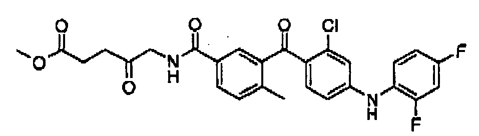 |
| 168 | 68 | 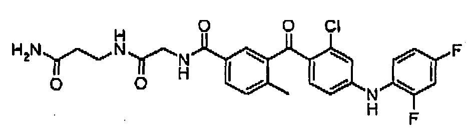 |
| 169 | 69 | 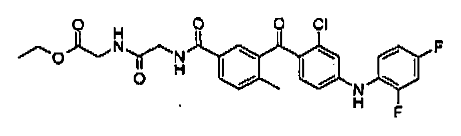 |
| 170 | 70 | 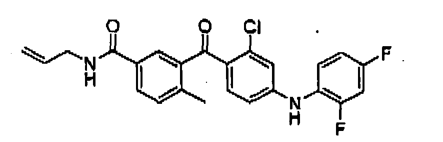 |
| 171 | 71 | 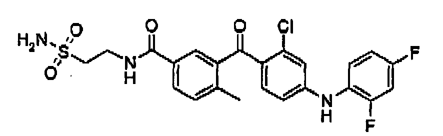 |
| 172 | 72 | 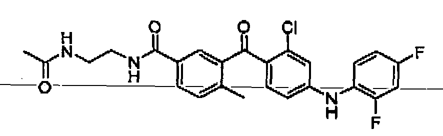 |
| 173 | 73 | 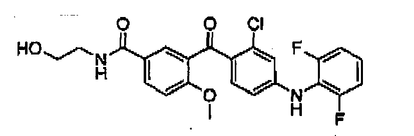 |
| 174 | 74 | 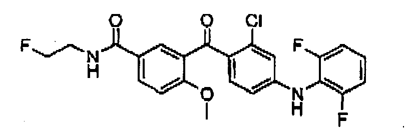 |
| 175 | 75 | 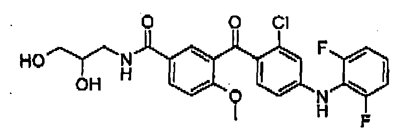 |
| 176 | 76 | 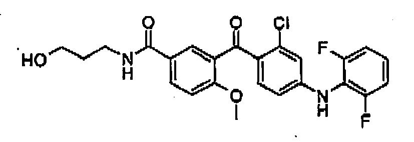 |
| 177 | 77 | 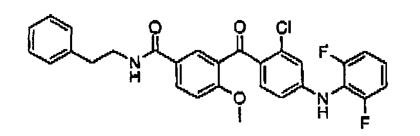 |
| 178 | 78 | 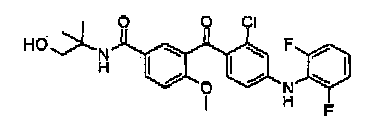 |
| 179 | 79 | 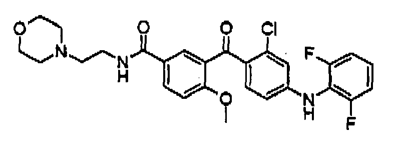 |
| 180 | 80 | 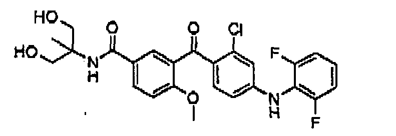 |
| 181 | 81 | 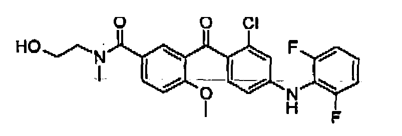 |
| 182 | 82 | 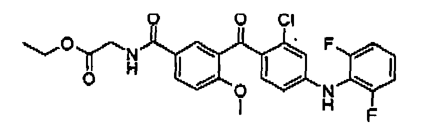 |
| 183 | 83 | 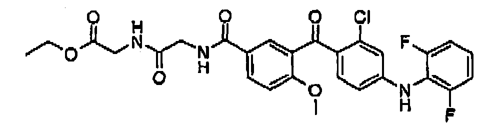 |
| 184 | 84 | 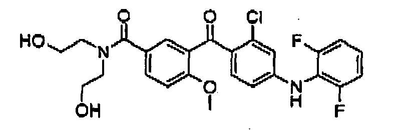 |
| 185 | 85 | 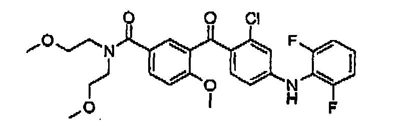 |
| 204 | 104 | 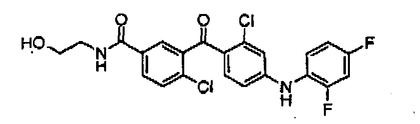 |
| 241 | 141 | 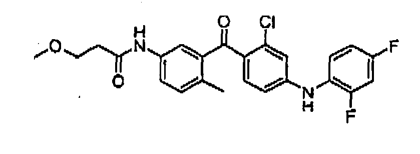 |
| 242 | 142 | 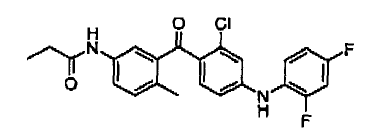 |
| 243 | 143 | 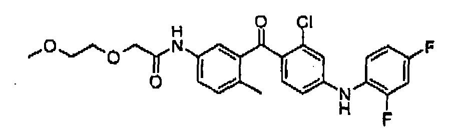 |
| 244 | 144 | 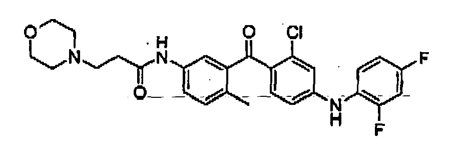 |
| 245 | 145 | 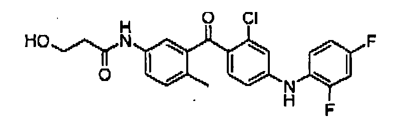 |
| 246 | 146 | 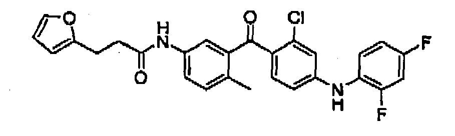 |
| 247 | 147 | 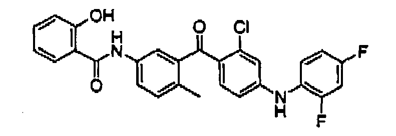 |
| 248 | 148 | 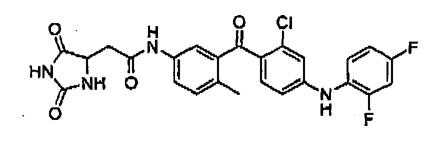 |
| 249 | 149 | 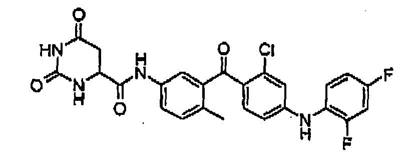 |
| 250 | 150 | 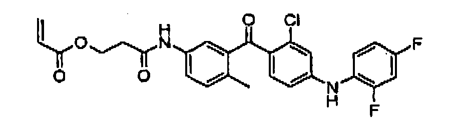 |
| 251 | 151 | 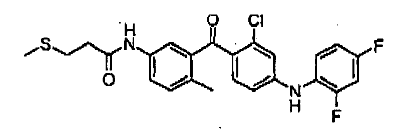 |
| 252 | 152 | 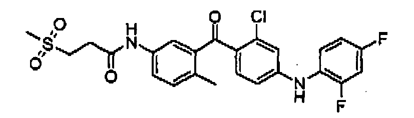 |
| 253 | 153 | 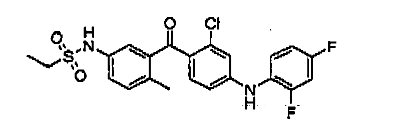 |
| 254 | 154 | 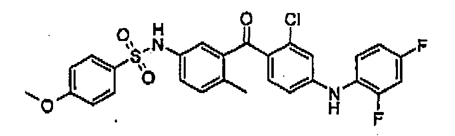 |
| 255 | 155 | 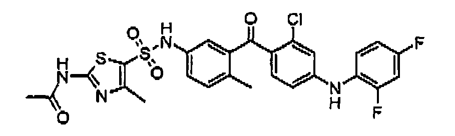 |
| 256 | 156 | 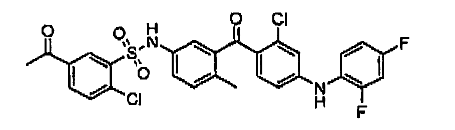 |
| 257 | 157 | 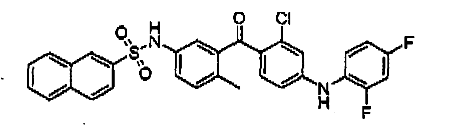 |
| 258 | 158 | 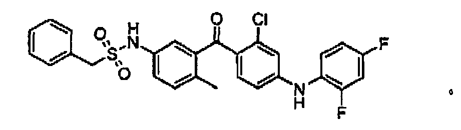 |
| 259 | 159 | 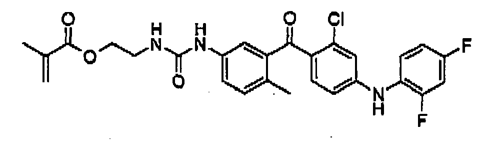 |
| 260 | 160 | 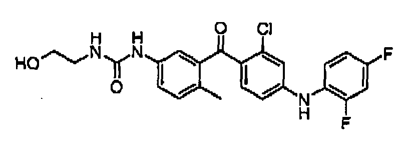 |
| 261 | 161 | 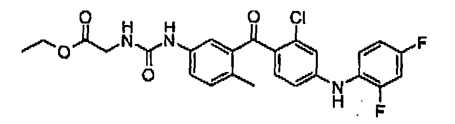 |
| 262 | 162 | 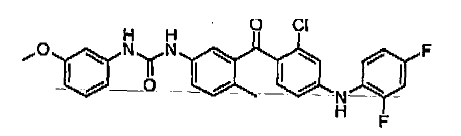 |
| 263 | 163 | 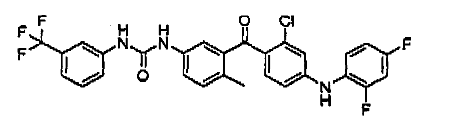 |
| 264 | 164 | 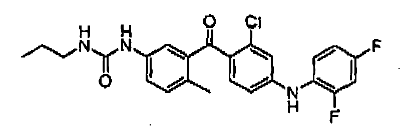 |
| 265 | 165 | 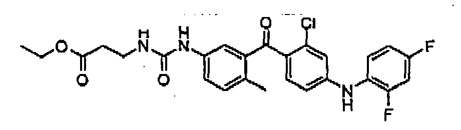 |
| 266 | 166 | 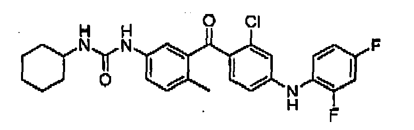 |
| 267 | 167 | 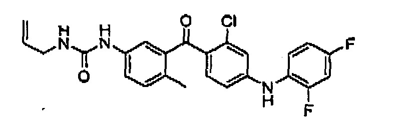 |
| 268 | 168 | 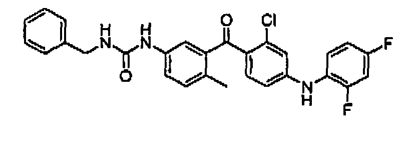 |
| 269 | 169 | 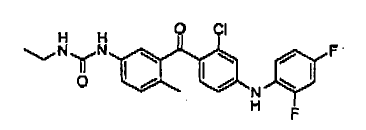 |
| 270 | 170 | 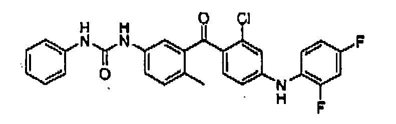 |
| 271 | 171 | 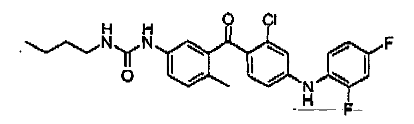 |
| 272 | 172 | 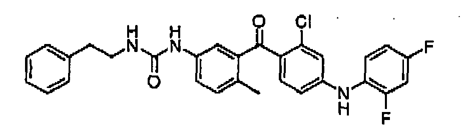 |
| 273 | 173 | 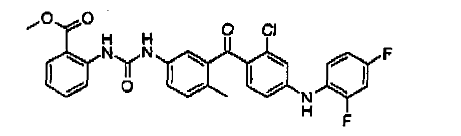 |
| 274 | 174 | 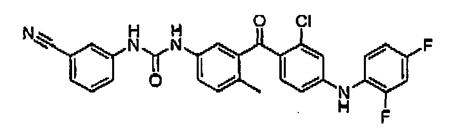 |
| 275 | 175 | 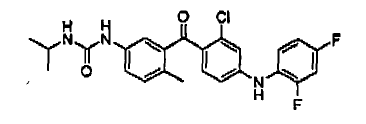 |
| 276 | 176 | 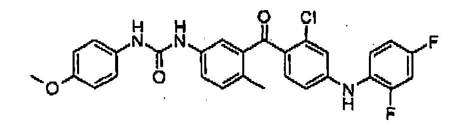 |
| 277 | 177 | 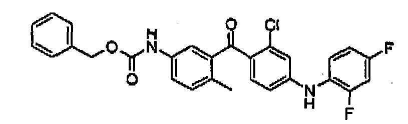 |
| 278 | 178 | 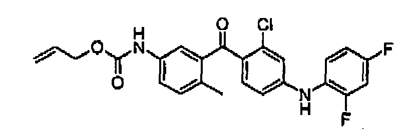 |
| 279 | 179 | 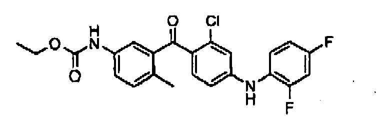 |
| 281 | 181 | 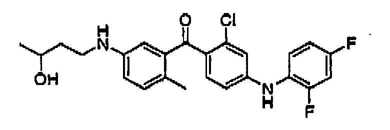 |
| 282 | 182 | 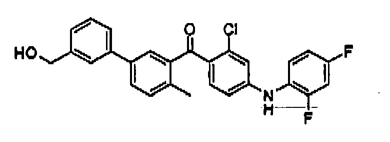 |
| 283 | 183 | 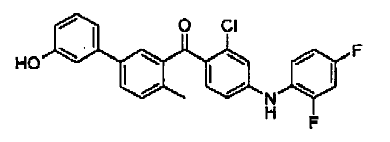 |
| 284 | 184 | 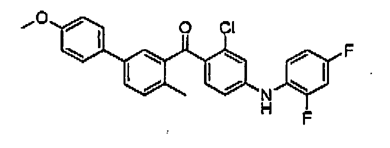 |
| 285 | 185 | 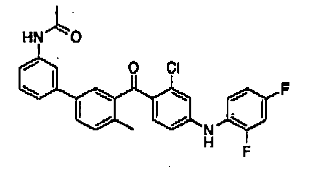 |
| 286 | 186 | 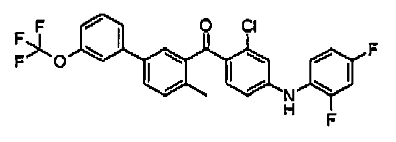 |
| 288 | 188 | 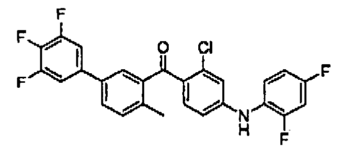 |
| 289 | 189 | 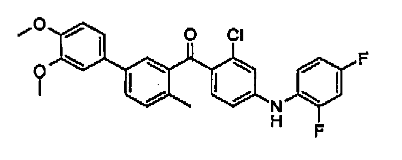 |
| 290 | 190 | 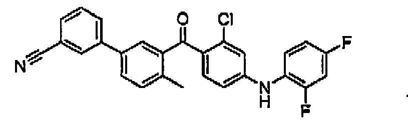 |
| 291 | 191 | 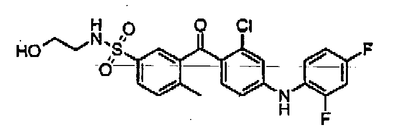 |
| 292 | 192 | 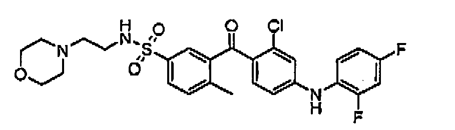 |
| 293 | 193 | 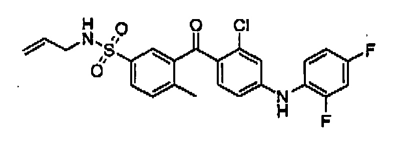 |
| 294 | 194 | 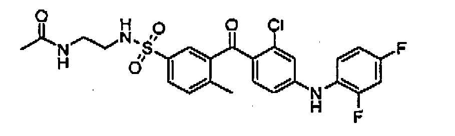 |
| 295 | 195 | 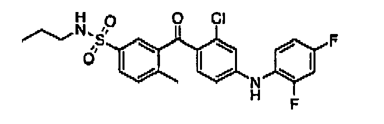 |
| 296 | 196 | 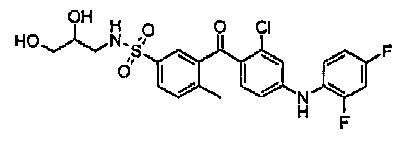 |
| 297 | 197 | 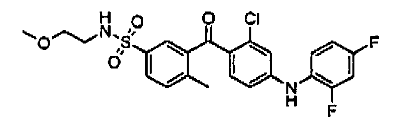 |
| 306 | 206 | 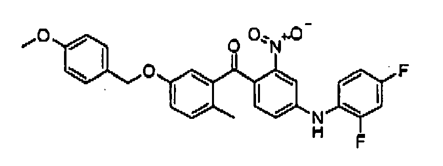 |
| 307 | 207 | 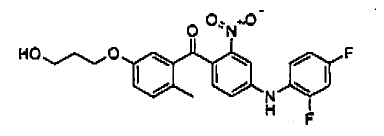 |
| 308 | 208 | 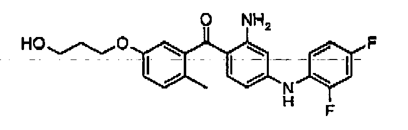 |
| 309 | 209 | 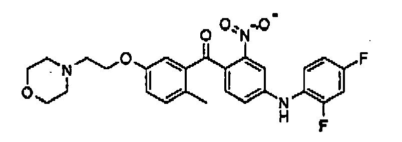 |
| 310 | 210 | 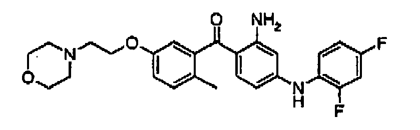 |
| 311 | 211 | 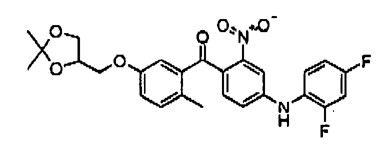 |
| 312 | 212 | 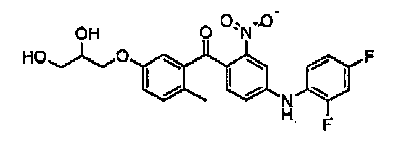 |
| 313 | 213 | 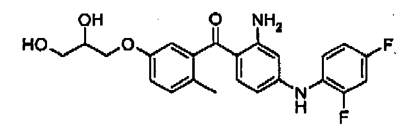 |
| 314 | 214 | 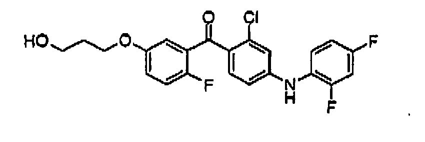 |
| 315 | 215 | 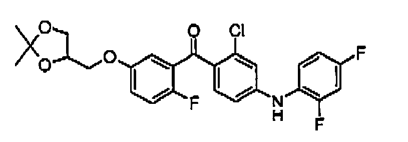 |
| 316 | 216 | 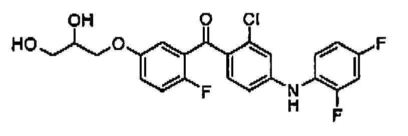 |
| 328 | 228 | 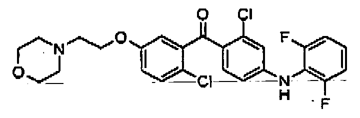 |
| 329 | 229 | 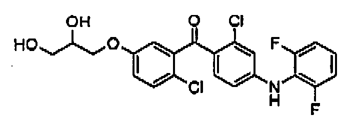 |
| 330 | 230 | 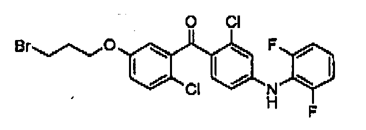 |
| 331 | 231 | 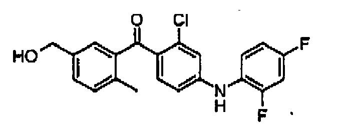 |
| 332 | 232 | 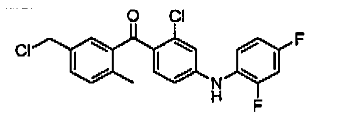 |
| 333 | 233 | 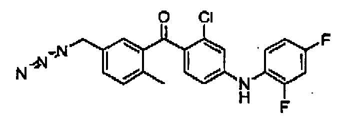 |
| 334 | 234 | 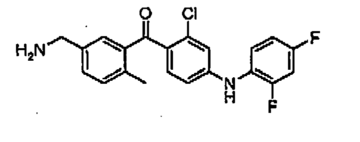 |
| 335 | 235 | 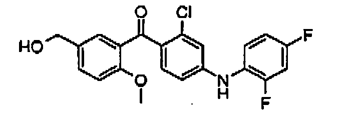 |
| 336 | 236 | 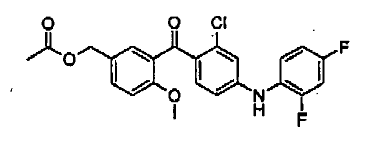 |
| 337 | 237 | 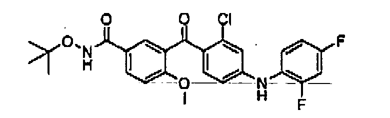 |
| 338 | 238 | 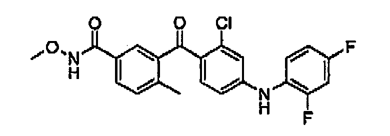 |
| 339 | 239 | 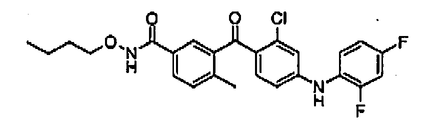 |
| 340 | 240 | 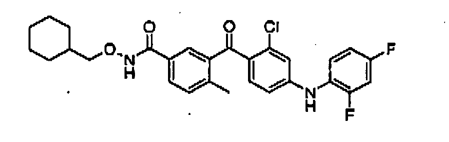 |
| 341 | 241 | 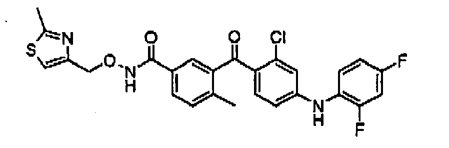 |
| 342 | 242 | 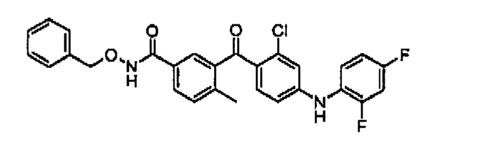 |
| 343 | 243 | 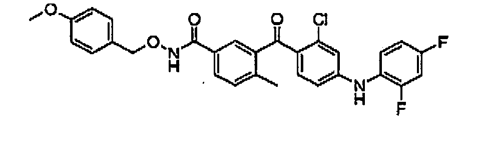 |
| 344 | 244 | 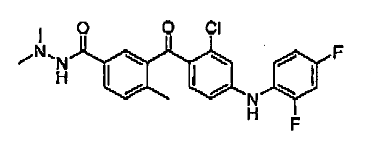 |
| 345 | 245 | 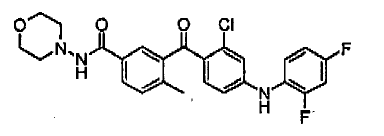 |
| 346 | 246 | 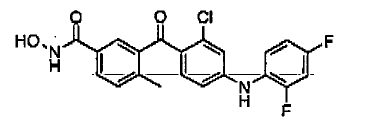 |
| 347 | 247 | 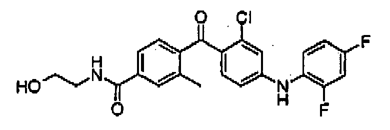 |
| 348 | 248 | 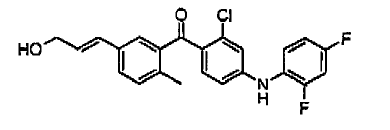 |
| 349 | 249 | 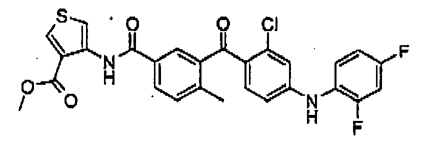 |
| 350 | 250 | 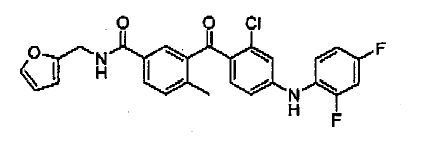 |
| 351 | 251 | 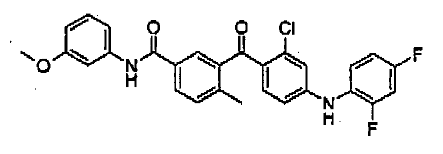 |
| 352 | 252 | 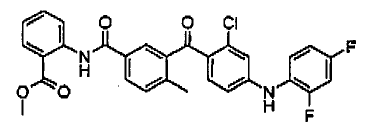 |
| 353 | 253 | 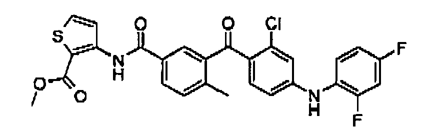 |
| 354 | 254 | 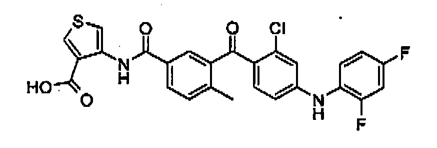 |
| 355 | 255 | 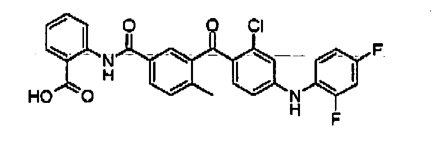 |
| 356 | 256 | 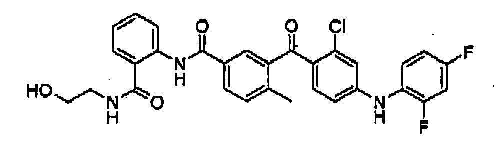 |
| 357 | 257 | 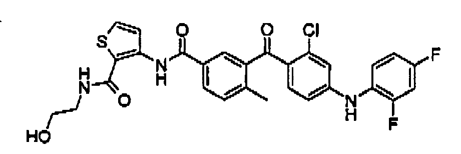 |
| 362 | 262 | 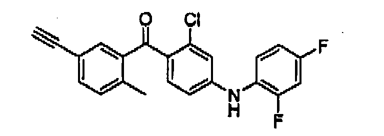 |
| 364 | 264 | 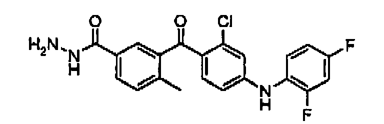 |
| 365 | 265 | 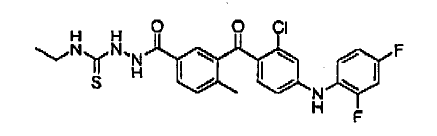 |
| 366 | 266 | 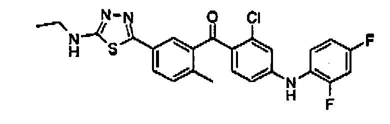 |
| 367 | 267 | 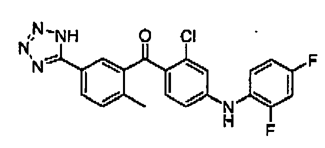 |
| 368 | 268 | 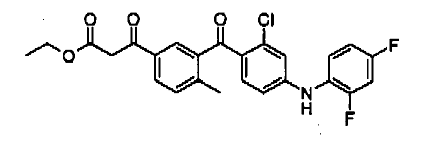 |
| 369 | 269 | 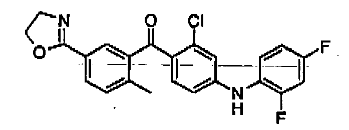 |
| 424 | 275 | 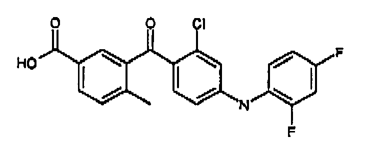 |
| 425 | 276 | 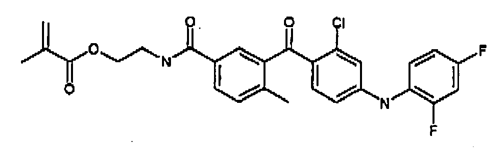 |
| 432 | 278 | 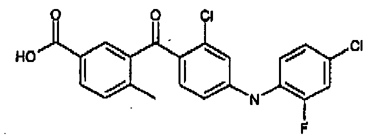 |
| 437 | 279 | 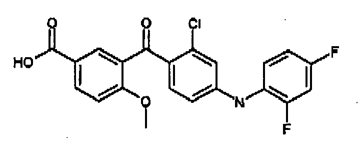 |
| 443 | 280 | 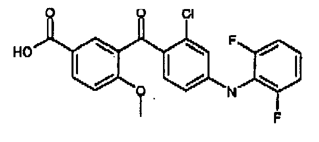 |

### Preparation 1:

### 3-(2-Chloro-4-nitrobenzoyl)-4-methylbenzoic acid methyl ester (Compound 401)

A dry flask was charged with 3-iodo-4-methylbenzoic acid methyl ester (21.6 g, 78.2 mmol) and the flask was evaporated and then filled with argon and this process repeated twice. Dry THF (140 mL) was added, and the solution cooled to -50 °C; then isopropylmagnesium chloride (41 mL, 2.0 M in diethyl ether, 82 mmol) was added slowly over 15 min keeping the temperature below -40 °C. On completion of the addition the reaction mixture was stirred at -40 °C for 45 min. A THF solution of ZnCl₂ (10.78 g, 79.1 mmol, 0.8 M) was added dropwise over 20 min. The reaction mixture was stirred ,at 0 °C for 65 min; then 2-chloro-4-nitro-benzoyl chloride (17.2 g, 78.2 mmol) and tetrakis(triphenylphosphine)palladium(0) (4.03 g, 3.49 mmol) were added and the reaction mixture was allowed to warm to room temperature. After 4 h the reaction mixture was poured into a mixture of toluene/EtOAc/water, then shaken and separated. The aqueous phase was extracted with two more portions of EtOAc, The organic phases were combined, dried (MgSO₄), filtered, and concentrated *in vacuo* to afford the crude product. Crystallization from mixtures of EtOAc/petroleum ether (40-60) gave the title compound as yellow solid. The mother liquid was concentrated *in vacuo* and purified by chromatography using DCM as the eluent to give a second crop of the title compound.

### Preparation 2:

### 3-(4-Amino-2-chlorobenzoyl)-4-methylbenzoic acid methyl ester (Compound 402)

To a solution of compound 401 (7.83 g, 23.5 mmol) in methanol (100 mL) was added zinc dust (15.3 g, 235 mmol) and ammonium chloride (6.27 g, 117 mmol) in one portion under stirring. A CaCl₂ tube was mounted on the flask and the flask was placed in an oil bath with a temperature of 90 °C. After 2 h the reaction mixture was cooled to RT, filtered, and then poured into a mixture of EtOAc/water. The aqueous phase was extracted with more EtOAc. The organic phases were combined, dried (MgSO4), filtered, and concentrated *in vacuo* to afford the crude product. Crystallization from a mixture of EtOAc/petroleum ether (40-60) (2:3) gave the title compound as light yellow solid.

### Preparation 3:

### 3-(4-Amino-2-chlorobenzoyl)-4-methylbenzoic acid (Compound 403)

To a solution of compound 402 (1.61 g, 5.3 mmol) in ethanol (50 mL) was added a solution of sodium hydroxide (2 M, 30 mL) and then stirred under reflux for 90 min. The reaction mixture was made weakly acidic (pH = 5) by slowly addition of hydrochloric acid (4N), and then poured into a mixture of EtOAc/water. The aqueous phase was extracted with more EtOAc. The organic phases were combined, dried (MgSO₄), filtered, and concentrated *in vacuo* to afford the title compound as orange solid. It was used without any further purification.

### Preparation 4:

### (4-Amino-2-chlorophenyl)-[2-methyl-5-(morpholine-4-carbonyl)phenyl]-methanone (Compound 404)

To a solution of compound 403 (150 mg, 0.47 mmol) in DMF (2.00 mL) in a reaction vial (8 mL) was added morpholine (41 µL, 0.47 mmol), FDPP (253 mg, 0.66 mmol) and DIEA (402 µL, 2.35 mmol). The vial was flushed with argon, closed, and then shaken at RT for 24 h.

The reaction mixture was concentrated *in vacuo* at 40 °C and then purified by chromatography using EtOAc/petroleum ether (40-60) 4:1 followed by EtOAc as the eluent to give the title compound as orange syrup.

### Preparation 15:

### 3-[2-Chloro-4-(2-nitrophenylamino)benzoyl]-4-methylbenzoic acid methyl ester (Compound 415)

A Schlenk tube was charged with compound 402 (4.00 g, 13.1 mmol) in 1,4-dioxane (40 mL), 1-iodo-2-nitro-benzene (3.91 g, 15.7 mmol), Cs₂CO₃ (5.98 g, 18.3 mmol), Pd₂(dba)₃ (302 mg, 0.33 mmol), and rac-BINAP (308 mg, 0.49 mmol). The tube was capped with a rubber septum, flushed with argon for 5 min, and then stirred at 100 °C for 2 h. The reaction mixture was allowed to cool to room temperature, and then poured into a mixture of water and EtOAc. The aqueous phase was extracted twice with more EtOAc. The combined organic phases were washed with brine, dried (MgSO₄), filtered and concentrated *in vacuo.* The crude product was purified by chromatography eluting with petroleum ether (40-60)/EtOAc 4:1 to afford the title compound as yellow solid.

### Preparation 16:

### 3-[2-Chloro-4-(2-nitrophenylamino)benzoyl]-4-methylbenzoic acid (Compound 416)

To a suspension of compound 415 (3.00 g, 7.06 mmol) in methanol (20 mL) was added water (4.0 mL) followed by lithium hydroxide (845 mg, 35 mmol), The mixture was then stirred at reflux for 30 min. The reaction mixture was made acidic (pH = 5) by slowly addition of H₂SO₄ (1N), and then poured into a mixture of EtOAc/water. The aqueous phase was extracted with more EtOAc. The organic phases were combined, dried (MgSO₄), filtered, and concentrated *in vacuo.* The crude product was triturated in EtOAc/pentane 1:1 (20 mL) to afford the title compound as yellow solid.

### Preparation 18:

### 3-[4-(4-Bromo-2-nitrophenylamino)-2-chlorobenzoyl]-4-methylbenzoic acid methyl ester (Compound 418)

To a solution of compound 402 (1.00 g, 3.29 mmol) and 4-bromo-1-fluoro-2-nitrobenzene (0.4 mL, 3.29 mmol) in DMSO (7.0 mL) was slowly added potassium *tert*-butoxide (816 mg, 7.27 mmol) under stirring. After 4 h at RT the reaction mixture was poured into a mixture of EtOAc/water. The aqueous phase was extracted with more EtOAc. The organic phases were combined, dried (MgSO₄), filtered, and concentrated *in vacuo* to afford the crude product. The crude product was purified by chromatography eluting with petroleum ether (40-60)/EtOAc 9:1 to afford the title compound as orange solid.

### Preparation 19:

### 3-[4-(4-Bromo-2-nitrophenylamino)-2-chlorobenzoyl]-4-methylbenzoic acid (Compound 419)

To a suspension of compound 418 (540 mg, 1.07 mmol) in methanol (5 mL) was added water (0.5 mL) and lithium hydroxide (128 mg, 5.35 mmol). The mixture was then stirred at reflux for 3 h. The reaction mixture was made acidic (pH = 2) by slowly addition of HCl (aq.) (1N), and then poured into a mixture of EtOAc/water. The aqueous phase was extracted with more EtOAc. The organic phases were combined, dried (MgSO₄), filtered, and concentrated *in vacuo* to afford the title product as orange solid. It was used without any further purification.

### Preparation 23:

### 3-[2-Chloro-4-(2,4-difluorophenylamino)benzoyl]-4-methylbenzoic acid ester (Compound 423)

A reaction vial was charged with compound 402 (750 mg, 2,47 mmol) in toluene (7.5 mL), 1-bromo-2,4-difluorobenzene (0.33 mL, 2.96 mmol), Cs₂CO₃ (1.13 g, 3.46 mmol), Pd₂(dba)₃ (114 mg, 0.12 mmol), and rac-BINAP (116 g, 0.18 mmol). The tube was flushed with argon for 5 min, closed and then warmed slowly to 200 °C. The reaction vial was shaken at 200 °C for 4 h. The reaction mixture was allowed to cool to room temperature, and then poured into EtOAc. Filtration and concentration *in vacuo* gave the crude product. The crude product was purified by continuous gradient flash chromatography using EtOAc/petroleum ether (40-60) (v:v = 2:98 to 20:80) as the eluent to afford the title compound as brown syrup.

### Preparation 24/Example 275:

### 3-[2-Chloro-4-(2,4-difluorophenylamino)-benzoyl]-4-methylbenzoic acid (Compound 424)

The reaction was carried out as described in the preparation of compound 419, using compound 423 (360 mg, 0.87 mmol) as the ester. Purification was done by flash chromatography to afford the title compound as orange solid. ¹³C NMR (DMSO-d₆) δ 194.4, 166.5, 158.8 (dd), 155.8 (dd), 149.5, 141.8, 139.3, 133.6, 131.9, 131.5; 131.2, 129.4, 128.3, 126.5 (m), 126.3, 124.1 (dd), 114.7, 112.0 (dd), 111.8, 105.0 (dd), 19.8

### Preparation 25/Example 276:

### 2-Methylacrylic acid 2-{3-[2-chloro-4-(2,4-difluorophenylamino)benzoyl]-4-methylbenzoylamino}-ethyl ester (Compound 425)

The reaction was carried out as described in the preparation of compound 404, using 2-methylacrylic acid 2-aminoethyl ester (54 mg, 0.33 mmol) as the amine and compound 424 (120 mg, 0.30 mmol) as the carboxylic acid. Purification was done by flash chromatography to afford the title compound as orange foam. ¹³C NMR (CDCl₃) δ 195.5, 167.8, 166.8, 159.2 (dd), 155.6. (dd), 148.1, 141.6, 139.7, 135.9, 135.3, 133.7, 131.7, 131.6, 128.9, 128.8, 127.8, 126.3, 124.5 (dd), 124.2 (dd), 116.2, 112.8, 111.6 (dd), 105.0 (dd), 63.4, 39.7, 20.4, 18.3

### Example 15:

### 3-[2-Chloro-4-(2,4 difluorophenylamino)benzoyl]-N-(2-hydroxyethyl)-4-methylbenzamide (Compound 115)

To a suspension of compound 425 (95 mg, 0.19 mmol) in methanol (1.0 mL) was added water (0.1 mL) and lithium hydroxide (23 mg, 0.95 mmol). The mixture was then stirred at reflux for 45 min. The reaction mixture was made acidic (pH = 2) by slowly addition of HCl (aq.) (1N), and then poured into a mixture of EtOAc/water, The aqueous phase was extracted with more EtOAc. The organic phases were combined, dried (MgSO₄), filtered, and concentrated *in vacuo.* The crude product was purified by flash chromatography using EtOAc/petroleum ether (40-60) (v:v = 4:1 and 6:1) as the eluent to afford the title compound as yellow oil. ¹³C NMR (CDCl₃) δ 195.6, 167.7, 159.4, 155.7, 148.3, 141.5, 139.8, 135.4, 133.8, 131.6, 131.5, 129.0, 128.6, 127.7, 1244.6, 124.2, 116.2, 112.8, 111.7, 105.0, 62.3, 42.9, 20.3.

### Preparation 31:

### 3-[2-Chloro-4-(4-chloro-2-fluoro-phenylamino)-benzoyl]-4-methyl-benzoic acid methyl ester (Compound 431)

1-Bromo-4-chloro-2-fluorobenzene (820 µL, 6.58 mmol) was dissolved in 20 mL dry 1,4-dioxane under an argon atmosphere. Compound 402 (2.00 g, 6.58 mmol) was added and dissolved in the solvent. Dicyclohexyl-(2',4',6'-triisopropyl-biphenyl-2-yl)-phosphane (125 mg, 0.26 mmol), Pd(OAc)₂ (30 mg, 0.13 mmol) and Cs₂CO₃ (2,68 g, 8.22 mmol) were added, and the reaction mixture was stirred under an argon atmosphere at 120 °C for 48 h. The reaction mixture was filtered and then purified by flash chromatography using EtOAc/petroleum ether (40-60) 1:9 as the eluent to afford the title compound as a solid.

### Preparation 32/Example 278:

### 3-[2-Chloro-4-(4-chloro-2-fluoro-phenylamino)-benzoyl]-4-methyl-benzoic acid (Compound 432)

The reaction was carried out as described in the preparation of compound 416, using compound 431 (1.71 g, 3.96 mmol) as the ester. The title compound was used without any further purification, ¹³C NMR (DMSO-d₆) δ 194.6, 166.7, 154.8 (d), 148.3, 141.7, 139.0, 133.3, 131.5, 131.4, 129.5, 128.8, 127.7 (d), 127.2 (d), 127.2, 125.2 (d), 124.8 (d), 116.9 (d), 115.6, 112.7, 19.9

### Preparation 33:

### 3-[2-Chloro-4-(4-chloro-2-fluoro-phenylamino)-benzoyl]-4-methyl-benzoyl chloride (Compound 433)

To a suspension of compound 432 (100 mg, 0.24 mmol) in toluene (2 mL) was added thionyl chloride (35 µL, 0.48 mmol) and then refluxed for 2 h. The reaction mixture was concentrated *in vacuo* to afford the title compound without any further purification.

### Example 20.

### 3-[2-Chloro-4-(4-chloro-2-fluoro-phenylamino)-benzoyl]-N-(2-hydroxy-ethyl)-4-methyl-benzamide (Compound 120)

A solution of compound 433 (80 mg, 0.18 mmol), DIEA (31 µL, 0,18 mmol), and 2-amino-ethanol (22 µL, 0.37 mmol) in dry DCM (2 mL) was stirred until completion of the reaction as judged by TLC (1 h). The reaction mixture was poured into a mixture of EtOAc/water. The aqueous phase was extracted with more EtOAc. The organic phases were combined, dried (MgSO₄) filtered, and concentrated *in vacuo* to afford the crude product. Purification by continuous gradient flash chromatography using EtOAc/petroleum ether (40-60) (v:v= 40:60 to 100:0) as the eluent afforded the title compound as brown oil, ¹³C NMR (CDCl₃) δ 195.8, 167.7, 154.4 (d,J=248Hz), 147.2, 141.5, 139.6, 135.2, 133.7, 131.6, 131.5, 129.1, 129.1, 128.7 (d,J=9.5Hz), 127.8, 127.2 (d,J=11.7Hz), 124.9 (d,J=3.6Hz), 122.4 (d), 117.1, 117.0 (d,J=22.8Hz), 113.6, 62.1, 42.9, 20.4.

### Example 21:

### 3-[2-Chloro-4-(4-chloro-2-fluoro-phenylamino)-benzoyl]-4,N-dimethyl-benzamide (Compound 121)

The reaction was carried out similarly as described in the preparation of compound 120, using methylamine (0.13 mmol) as the amine. ¹³C NMR (CDCl₃) δ 195.7, 167.4, 154.4 (d), 147.1, 141.4, 139.4, 135.1, 133.5, 132.0, 131.6, 129.3, 129.0, 128.6 (d), 127.8, 127.3 (d), 124.9 (d), 122.4 (d), 117.1, 117.0 (d), 113.6, 26.8, 20.4.

### Example 22:

### (2-{3-[2-Chloro-4-(4-chloro-2-fluoro-phenylamino)-benzoyl]-4-methyl-benzoylamino}-acetylamino)-acetic acid ethyl ester (Compound 122)

The reaction was carried out similarly as described in the preparation of compound 120, using (2-amino-acetylamino)-acetic acid ethyl ester (0.13 mmol) as the amine. ¹³C NMR (DMSO-d₆) δ 194.8, 169.6, 169.4, 165.4, 154.8 (d), 148.3, 139.9, 139.1, 133.6, 133.5, 131.3, 131.0, 129.4, 127.7 (d), 1.27.5, 127.2 (d), 127.1, 125.2 (d), 124.8 (d), 117.0 (d), 115.7, 112.6, 60.3, 42.3, 40,6, 19.6, 14.0.

### Example 23:

### {3-[2-Chloro-4-(4-chloro-2-fluoro-phenylamino)-benzoyl]-4-methyl-benzoylamino}-acetic acid ethyl ester (Compound 123)

The reaction was carried out similarly as described in the preparation of compound 120, using amino-acetic acid ethyl ester (0.13 mmol) as the amine. ¹³C NMR (CDCl₃) δ 195.6, 170.0, 166.7, 154.4 (d), 147.0, 141.9, 139.5, 135.2, 133.6, 131.7, 131.1, 129.3, 129.1, 128.5 (d), 128.1, 127.3 (d), 124.9 (d), 122.3 (d), 117.1, 117,0 (d), 113.7, 61.7, 41.9, 20.4, 14.1.

### Example 24:

### 3-[2-Chloro-4-(4-chloro-2-fluoro-phenylamino)-benzoyl]-N-(2-methoxy-ethyl)-4-methyl-benzamide (Compound 124)

The reaction was carried out similarly as described in the preparation of compound 120, using 2-methoxy-ethylamine (0.13 mmol) as the amine. ¹³C NMR (CDCl₃) δ 195.7, 166.7, 154.4 (d), 147.0, 141,5, 139.5, 135.2, 133.6, 131.9, 131.5, 129.4, 128.9, 128.5 (d), 128.0, 127.3 (d), 124.9 (d), 122.3 (d), 117.1, 117.0 (d), 113.7, 71.1, 58.8, 39.8, 20.4

### Example 25:

### 3-[2-Chloro-4-(4-chloro-2-fluoro-phenylamino)-benzoyl]-N-cyclohexyl-4-methyl-benzamide (Compound 125)

The reaction was carried out similarly as described in the preparation of compound 120, using cyclohexylamine (0.13 mmol) as the amine. ¹³C NMR (CDCl₃) δ 195.7, 165.8, 154.4 (d), 147.1, 141.1, 139.6, 135.2, 133.6, 132.5, 131.4, 129.3, 128.8, 128.6 (d), 127.8, 127.3 (d), 124.9 (d), 122.4 (d), 117.1, 117.0 (d), 113.6, 48.9, 33.2, 25.5, 24.9, 20.4

### Example 26:

### 3-[2-Chloro-4-(4-chloro-2-fluoro-phenylamino)benzoyl]-N-ethyl-4-methyl-benzamide (Compound 126)

The reaction was carried out similarly as described in the preparation of compound 120, using ethylamine (0.13 mmol) as the amine. ¹³C NMR (CDCl₃) δ 195.7, 166.6, 154.4 (d), 147.1, 141.3, 139.5, 135.2, 133.6, 132.1, 131.5, 129.3, 128.9, 128.6 (d), 127.8, 127.3 (d), 124.8 (d), 122.4 (d), 117.1, 117.0 (d), 213.6, 35.0, 20.4, 14.8

### Example 27:

### 3-[2-Chloro-4-(4-chloro-2-fluoro-phenylamino)-N-(6-hydroxy-hexyl)-4-methyl-benzamide (Compound 127)

The reaction was carried out similarly as described in the preparation of compound 120, using 6-aminohexanol (0.13 mmol) as the amine. ¹³C NMR (CDCl₃) δ 195.8, 166.9, 154.5 (d), 147.3, 141.2, 139.5, 135.1, 133.6, 132.1, 131.5, 129.0, 129.0, 128.6 (d), 127.8, 127.3 (d), 124.8 (d), 122.6 (d), 117.1, 117.0 (d), 113.5, 62.6, 40.0, 32.5, 29.5, 26.6, 25.3, 20.3

### Example 28:

### 3-[2-Chloro-4-(4-chloro-2-fluoro-phenylamino)-benzoyl]-N-isopropyl-4-methyl-benzamide (Compound 128)

The reaction was carried out similarly as described in the preparation of compound 120, using isopropylamine (0.13 mmol) as the amine, ¹³C NMR (CDCl₃) δ 195.7, 165.9, 154.5 (d), 147.2, 141.1, 139.6, 135.2, 133.6, 132.3, 131.4, 129.2, 128.8, 128.6 (d), 127.7, 127.3 (d), 124.8 (d), 122.5 (d), 117.1, 117.0 (d), 113.6, 42.1, 22.8, 20.3

### Example 29:

### 3-[2-Chloro-4-(4-chloro-2-fluoro-phenylamino)-benzoyl]-N-isobutyl-4-methyl-benzamide (Compound 129)

The reaction was carried out similarly as described in the preparation of compound 120, using isobutylamine (0.13 mmol) as the amine, ¹³C NMR (CDCl₃) δ 195.7, 166.8, 154.4 (d), 147.1, 141.3, 139.5, 135.1, 133.6, 132.3, 131.5, 129.3, 128.8, 128.6 (d), 127.9, 127,3 (d), 124.8 (d), 122.4 (d), 117.1, 117.0 (d), 113.6, 47,4, 28.6, 20,4, 20.2

### Example 30:

### 3-[2-Chloro-4-(4-chloro-2-fluoro-phenylamino)-benzoyl]-N-(2,2-dimethyl-propyl)-4-methyl-benzamide (Compound 130)

The reaction was carried out similarly as described in the preparation of compound 120, using 2,2-dimethyl-propylamine (0.13 mmol) as the amine. ¹³C NMR (CDCl₃) δ 195.7, 166.9, 154,5 (d), 147.2, 141.3, 139.6, 135.1, 133.6, 132.4, 131.5, 129.2, 128.7, 128.6 (d), 128,0, 127.3 (d), 124.8 (d), 122.5 (d), 117.1, 117.0 (d), 113.6, 51.0, 32.2, 27.3, 20,4

### Example 31:

### 3-[2-Chloro-4-(4-chloro-2-fluoro-phenylamino)-benzoyl]-N-methoxy-propyl)-4-methyl-benzamide (Compound 131)

The reaction was carried out similarly as described in the preparation of compound 120, using 3-methoxy-propylamine (0.13 mmol) as the amine. ¹³C NMR (CDCl₃) δ 195.7, 166.4, 154.4 (d), 147.0, 141.4, 139.4, 135.1, 133.5, 132.1, 131.6, 129.4, 129.0, 128.5 (d), 127.9, 127.3 (d), 124.8 (d), 122.4 (d), 117.1, 117.0 (d), 113.6, 72.4, 58.8, 39.2, 28.7, 20.4

### Example 32:

### 3-[2-Chloro-4-(4-chloro-2-fluoro-phenylamino)-benzoyl]-4-methyl-N-[3-(2-oxo-pyrrolidin-1-yl)-propyl]-benzamide (Compound 132)

The reaction was carried out similarly as described in the preparation of compound 120, using 1-(3-amino-propyl)-pyrrolidin-2-one (0.13 mmol) as the amine. ¹³C NMR (CDCl₃) δ 195.9, 176.3, 166.3, 154.3 (d), 146.8, 141.5, 139.2, 135.0, 133.4, 131.8, 131.6, 129.7, 128.9, 128.7, 128.1 (d), 127.6 (d), 124.8 (d), 122.0 (d), 117.3, 116.9 (d), 113.8, 47.5, 39.6, 35.7, 30.9, 26.2, 20.5, 18.0

### Example 33:

### 3-[2-Chloro-4-(4-chloro-2-fluoro-phenylamino)-benzoyl]-N-(2-dimethylamino-ethyl)-4-methyl-benzamide (Compound 133)

The reaction was carried out similarly as described in the preparation of compound 120, using 2-dimethylamino-ethylamine (0.13 mmol) as the amine. ¹³C NMR (CDCl₃) δ 195.8, 166.7, 154.5 (d), 147.2, 141.4, 139.4, 135.1, 133.6, 131.8, 131.5, 129.3, 129.0, 128.5 (d), 128.3, 127.4 (d), 124.8 (d), 122.6 (d), 117.1, 117.0 (d), 113.6, 57.8, 44.9, 37.0, 20.4

### Example 34:

### 2-Methyl-acrylic acid 2-{3-[2-chloro-4-(4-phenylamino)-benzoyl]-4-methyl-benzoylamino}-ethyl ester (Compound 134)

The reaction was carried out similarly as described in the preparation of compound 404, using 2-methylacrylic acid 2-aminoethyl ester (61 mg, 0.37 mmol) as the amine and compound 432 as the acid (140 mg, 0.33 mmol). Purification was done by flash chromatography to afford the title compound. ¹³C NMR (CDCl₃) δ 195.6, 167.8, 166.7, 154.5 (d), 146,9, 141.8, 139.5, 135.9, 135.2, 133.5, 131.7, 131.6, 129.6, 129.0, 128.6 (d), 128.0, 127.3 (d), 126.3, 124.9 (d), 122.1 (d), 117.2, 117.0 (d), 113.8, 63.4, 39.7, 20.4, 18.3

### Example 35:

### 3-[2-Chloro-4-(4-chloro-2-fluoro-phenylamino)-benzoyl]-N-cis(4-hydroxy-cyclohexyl)-4-methyl-benzamide (Compound 135)

The reaction was carried out similarly as described in the preparation of compound 120, using 4-amino-cyclohexanol (0.13 mmol) as the amine. ¹³C NMR (CDCl₃) δ 195.6, 165.9, 154.3 (d), 146.9, 141.4, 139.5, 135.2, 133.6, 132.3, 131.6, 129.6, 128.9, 128.5 (d), 127.8, 127.3 (d), 124.9 (d), 122.1 (d), 117.3, 117.0 (d), 113.8, 66.1, 47.4, 31.4, 27.2, 20.4

### Example 36:

### 3-[2-Chloro-4-(4-chloro-2-fluoro-phenylamino)-benzoyl]-N-trans-(4-hydroxy-cyclohexyl)-4-methyl-benzamide (Compound 136)

The reaction was carried out similarly as described in the preparation of compound 120, using 4-Amino-cyclohexanol (0.13 mmol) as the amine. ¹³C NMR (CDCl₃) δ 195.6, 166.0, 154.4 (d), 146.9, 141.2, 139.6, 135.2, 133.6, 132.2, 131.5, 129.5, 128.9, 128.7 (d), 127.7, 127.2 (d), 124.9 (d), 122.2 (d), 117.2, 117.1 (d), 113.7, 69.8, 48.3, 34.0, 30.9, 20.4

### Example 37:

### (2-{3-[2-Chloro-4-(4-chloro-2-fluoro-phenylamino)-benzoyl]-4-methyl-benzoylamino}-ethyl)-carbamic acid tert-butyl ester (Compound 137)

The reaction was carried out similarly as described in the preparation of compound 120, using (2-amino-ethyl)-carbamic acid tert-butyl ester (2.4 mmol) as the amine, ¹³C NMR (DMSO-d₆) δ 194.8, 165.3, 155.6, 154.8 (d), 148.4, 139.6, 139.2, 133.6, 133.6, 131.8, 131.0, 129.2, 127.8 (d), 127.2, 127.2 (d), 127.1, 125.2 (d), 124.8 (d), 117.0 (d), 115.8, 112.6, 77.6, 39.7, 39.5, 28.1, 19.6

### Example 38:

### N-(2-Amino-ethyl)-3-[2-chloro-4-(4-chloro-2-fluoro-phenylamino)-benzoyl]-4-methyl-benzamide (Compound 138)

A solution of compound 137 (100 mg, 0.8 mmol) in a mixture of EtOAc (5 mL), methanol (5 mL), and 4M HCl (aq, 1.5 mL) was stirred at 70 °C for 2 h. The crude mixture was concentrated *in vacuo* and purified by flash chromatography using MeOH/DCM/triethyl amine 20:80:1 as the eluent to afford the title compound. ¹³C NMR (DMSO-d₆) δ 194.8, 165.4, 154.8 (d), 148.4, 139.6, 139.2, 133.6, 133.5, 131.7, 131.0, 129.2, 127.7 (d), 127.3, 127.1 (d), 127.0, 125.2 (d), 124.8 (d), 117.0 (d), 115.7, 112.6, 40.3, 40.0, 19.6

### Example 39:

### (2-{3-[2-Chloro-4-(4-chloro-2-fluoro-phenylamino)-benzoyl]-4-methyl-benzoylamino}-acetylamino)-acetic acid (Compound 139)

To a suspension of compound 122 (100 mg, 0.28 mmol) in methanol (2 mL) was added water (0.2 mL) followed by lithium hydroxide (21 mg, 0.89 mmol). The mixture was then stirred at reflux for 90 min. The reaction mixture was made acidic (pH = 5) by slowly addition of H₂SO₄ (1N), and then poured into a mixture of EtOAc/water. The aqueous phase was extracted with more EtOAc. The organic phases were combined, dried (MgSO₄), filtered, and concentrated *in vacuo.* The crude product was purified by flash chromatography using EtOAc with 0 to 2% acetic acid as the eluent to afford the title compound as yellow solid. ¹³C NMR (DMSO-d₆) δ 194.8, 171.0, 169.1, 165.4, 154.8 (d), 148.3, 139.9, 139.2, 133.5, 131.3, 131.0, 129.4, 127.7 (d), 127.5, 127.2 (d), 127.1, 125.2 (d), 124.8, 116.9 (d), 115.8, 112.6, 42.3, 40.7, 19.6

### Preparation 34:

### 3-(2-Chloro-4-nitro-benzoyl)-4-methoxy-benzoic acid methyl ester (compound 434)

A dry flask was charged with 3-iodo-4-methoxy-benzoic acid methyl ester (8.9 g, 30.5 mmol) and the flask was evaporated and then filled with argon and this process repeated twice. Dry THF (50 mL) was added, and the solution cooled to-50 °C; then isopropylmagnesium chloride (15.2 mL, 2.0 M in diethyl ether, 30.5 mmol) was added slowly over 20 min keeping the temperature below -40 °C. On completion of the addition the reaction mixture was stirred at -40 °C for 45 min.
A THF solution of ZnCl₂ (5.19 g, 38.1 mmol, 1.0 M) was added d ropwise over 20 min. The reaction mixture was stirred at 0 °C for 20 min; then 2-chloro-4-nitro-benzoyl chloride (7.04 g, 32.0 mmol) and Cu(OAc)₂ (122 mg, 0.61 mmol) were added and the reaction mixture was allowed to warm to room temperature. After 16 h the reaction mixture was poured into a mixture of EtOAc/water, then shaken and separated. The aqueous phase was extracted with more EtOAc. The organic phases were combined, dried (MgSO₄), filtered, and concentrated *in vacuo* to afford the crude product. The crude product was purified by flash chromatography using EtOAc/petroleum ether (40-60) 1:6 followed by 1:3 as the eluent to give the title compound as yellow solid.

### Preparation 35:

### 3-(4-Amino-2-chloro-benzoyl)-4-methoxy-benzoic acid methyl ester (compound 435)

The reaction was carried out similarly as described in the preparation of compound 402, using compound 434 (22.9 mmol) as the nitro compound. Purification was done by flash chromatography to afford the title compound as yellow solid.

### Preparation 36:

### 3-[2-Chloro-4-(2,4-difluoro-phenylamino)-benzoyl]-4-methoxy-benzoic acid methyl ester (compound 436)

The reaction was carried out similarly as described in the preparation of compound 431, using 1-bromo-2,4-difluorobenzene (3.94 mmol) and compound 435 (3.28 mmol). Purification was done by flash chromatography to afford the title compound as yellow foam.

### Preparation 37/Example 279:

### 3-[2-Chloro-4-(2,4-difluoro-phenylamino)-benzoyl]-4-methoxy-benzoic acid (compound 437)

The reaction was carried out as described in the preparation of compound 416, using compound 436 (0.92 g, 2.13 mmol) as the ester. The title compound was used without any further purification. ¹³C NMR (DMSO-d₆) δ 191.4, 166.4, 160.6, 158.7 (dd), 155.7 (dd), 149.2, 133.8, 133.7, 133.5, 130.6, 129.2, 126.5, 126.4 (dd), 124.2 (dd), 122.8, 114.7, 112.0, 111.7, 105.0 (dd), 56.1

### Preparation 38:

### 3-[2-Chloro-4-(2,4-difluoro-phenylamino)-benzoyl]-4-methoxy-benzoyl chloride (compound 438)

To a suspension of compound 437 (292 mg, 0.7 mmol) in toluene (3 mL) was added thionyl chloride (100 µL, 1 mmol) and then refluxed for 2 h. The reaction mixture was concentrated *in vacuo* to afford the title compound without any further purification.

### Example 40:

### 3-[2-Chloro-4-(2,4-difluoro-phenylamino)-benzoyl]-N-(2-hydroxy-ethyl)-4-methoxy-benzamide (compound 140)

The reaction was carried out similarly as described in the preparation of compound 120, using 2-amino-ethanol (0.28 mmol) and compound 438 (0.14 mmol). Purification was done by flash chromatography to afford the title compound as yellow solid, ¹³C NMR (DMSO-d₆) δ 191.7, 165.0, 159.2, 158.8 (dd), 155.6 (dd), 149.2, 133.8, 133.6, 131.6, 129.0, 128.3, 126,6, 126.5, 126.4 (dd), 124.2 (dd), 114.8, 111.9 (dd), 111.7, 111.6, 105.0 (dd), 59.7, 56.0, 42.1

### Example 41:

### 3-[2-Chloro-4-(2,4-difluoro-phenylamino)-benzoyl]-N-(2,2-difluoro-ethyl)-4-methoxy-benzamide (compound 141)

The reaction was carried out similarly as described in the preparation of compound 120, using 2,2-difluoro-ethylamine (0.28 mmol) and compound 438 (0.14 mmol). Purification was done by flash chromatography to afford the title compound as yellow solid. ¹³C NMR (DMSO-d₆) δ 191.6, 165.5, 159.5, 158.8 (dd), 155.6 (dd), 149.2, 133.8, 133.6, 131.8, 129.2, 128.4, 126.5, 126.4 (dd), 125.5, 124.2 (dd), 114.8, 114.5 (t), 112.0 (dd), 111.8, 111.7, 105.0 (dd), 56.1, 41.5

### Example 42:

### 2-[2-Chloro-4-(2,4-difluoro-phenylamino)-benzoyl]-N-(2-fluoro-ethyl)-4-methoxy-benzamide (compound 142)

The reaction was carried out similarly as described in the preparation of compound 120, using 2-fluoro-ethylamine (0.28 mmol) and compound 438 (0.14 mmol). Purification was done by flash chromatography to afford the title compound as yellow foam. ¹³C NMR (CDCl₃) δ 192.7, 166.5, 160.6, 159.1 (dd), 155.5 (dd), 147.7, 134.9, 133.4, 132.4, 129.7, 129.5, 128.7, 126.4, 124.5 (dd), 124.1 (dd), 116.0, 112.9, 111.6 (dd), 111.5, 104.9 (dd), 82.8 (d), 56,1, 40.5 (d)

### Example 43:

### 3-[2-Chloro-4-(2,4-difluoro-phenylamino)benzoyl]-N-(2,3-dihydroxy-propyl)-4-methoxy-benzamide (compound 143).

The reaction was carried out similarly as described in the preparation of compound 120, using 3-amino-propane-1,2-diol (0.28 mmol) and compound 438 (0.14 mmol). Purification was done by flash chromatography to afford the title compound as yellow foam. ¹³C NMR (DMSO-d₆) δ 191.7, 165.3, 159.2, 158.8 (dd), 155.6 (dd), 149.2, 133.8, 133.6, 131.6, 129.0, 128.3, 126.6, 126.4, 126.4 (dd), 124.2 (dd), 114.8, 111.9 (dd), 111.7, 111.6, 105.0 (dd), 70.4, 63.9, 56.0, 42.9

### Example 44:

### N-Carbamoylmethyl-3-[2-chloro-4-(2,4-difluoro-phenylamino)-benzoyl]-4-methoxy-benzamide (compound 144)

The reaction was carried out similarly as described in the preparation of compound 120, using 2-amino-acetamide (0.28 mmol) and compound 438 (0.14 mmol), Purification was done by flash chromatography to afford the title compound as yellow solid. ¹³C NMR (DMSO-d₆) δ 191.8, 171.1, 165.2, 159.4, 158.8 (dd), 155.8 (dd), 149.3, 133.9, 133.7, 131.9, 129.1, 128.6, 126.7, 126.4 (dd), 126.2, 124.3 (dd), 114.9, 112.0 (dd), 111.8, 111.7, 105.1 (dd), 56.1, 42,4

### Preparation 39:

### 3-[2-Chloro-4-(2,4-difluoro-phenylamino)-benzoyl]-4-methyl-benzoyl chloride (compound 439)

To a suspension of compound 424 (1.8 g, 4.5 mmol) in toluene (10 mL) was added thionyl chloride (650 µL, 9.0 mmol) and then refluxed for 2 h. The reaction mixture was concentrated *in vacuo* to afford the title compound without any further purification.

### Example 45:

### N-Carbamoylmethyl-3-[2-chloro-4-(2,4-difluoro-phenylamino)-benzoyl]-4-methyl-benzamide (Compound 145)

The reaction was carried out similarly as described in the preparation of compound 120, using 2-amino-acetamide (4.48 mmol) and compound 439 (2.24 mmol). Purification was done by flash chromatography to afford the title compound as yellow foam. ¹³C NMR (DMSO-d₆) δ 194.7, 170.8, 165.3, 158.9 (dd), 155.6 (dd), 149.5, 139.7, 139.3, 133.8, 133.7, 131.4, 131.0, 129.2, 127.3, 126.5 (dd), 126.3, 124.1 (dd), 114.8, 111.9 (dd), 111.8, 105.0 (dd), 42.3, 19.6

### Example 46:

### N-Benzyl-3-[2-chloro-4-(2,4-difluoro-phenylamino)-benzoyl]-4-methyl-benzamide (compound 146)

The reaction was carried out similarly as described in the preparation of compound 120, using benzylamine (0.13 mmol) and compound 439 (0.11 mmol). Purification was done by flash chromatography to afford the title compound as yellow foam. ¹³C NMR (CDCl₃) δ 195.6, 166.6, 159.2 (dd), 155.7 (dd), 148.4, 141.4, 139.8, 138.1, 135.3, 133.8, 131.7, 131.5, 128.9, 128.7, 128.3, 127,9, 127.8, 127.6, 124.8 (dd), 124.2 (dd), 116.1, 112.6, 111.6 (dd), 104.9 (dd), 44.1, 20.3

### Example 47:

### 3-[2-Chloro-4-(2,4-difluoro-phenylamino)-benzol]-N-(2-fluoro-ethyl)-4-methyl-benzamide (compound 147)

The reaction was carried out similarly as described in the preparation of compound 120, using 2-fluoro-ethylamine (0.13 mmol) and compound 439 (0.11 mmol). Purification was done by flash chromatography to afford the title compound as yellow foam. ¹³C NMR (CDCl₃) δ 195.6, 166.9, 159.3 (dd), 155.7 (dd), 148.4, 141.6, 139.9, 135.3, 1.33,8, 131.6, 131.4, 128.9, 128.4, 127.7, 124.7 (dd), 124.2 (dd), 116.2, 112.7, 111.6 (dd), 104.9 (dd), 82.7 (d), 40.5 (d), 20.3

### Example 48:

### 3-[2-Chloro-4-(2,4-difluoro-phenylamino)-benzoyl]-4-methyl-N-(2,2,2-trifluoro-ethyl)-benzamide (compound 148)

The reaction was carried out similarly as described in the preparation of compound 120, using 2,2,2-trifluoro-ethylamine (0.13 mmol) and compound 439 (0.11 mmol), Purification was done by flash chromatography to afford the title compound as yellow syrup. ¹³C NMR (CDCl₃) δ 195-5, 166.9, 159.4 (dd), 155.8 (dd), 148.5, 142.1, 140.0, 135.4, 133.9, 131.6, 130.6, 129.0, 128.2, 127.8, 124.8 (dd), 124.2 (q), 124.1 (dd), 116.2, 112.7, 111.7 (dd), 105.0 (dd), 41.1 (q), 20.3

### Example 49:

### 3-[2-Chloro-4-(2,4-difluoro-phenylamino)-benzoyl]-N-ethyl-4-methyl-benzamide (compound 149)

The reaction was carried out similarly as described in the preparation of compound 120, using ethylamine (0.13 mmol) and compound 439 (0.11 mmol). Purification was done by flash chromatography to afford the title compound as yellow foam. ¹³C NMR (CDCl₃) δ 195.7, 166.7, 159.3 (dd), 155.7 (dd), 148.4, 141.1, 139.7, 135.3, 133.8, 132.1, 131.5, 128.9, 128.4, 127.6, 124.8 (dd), 124.2 (dd), 116.2, 112.6, 111.6 (dd), 104.9 (dd), 35.0, 20.3, 14.8

### Example 50:

### 3-[2-Chloro-4-(2,4-difluoro-phenylamino)-benzoyl]-N-cyclohexylmethyl-4-methyl-benzamide(compound 150)

The reaction was carried out similarly as described in the preparation of compound 120, using cyclohexyl-methylamine (0.13 mmol) and compound 439 (0,11 mmol). Purification was done by flash chromatography to afford the title compound as yellow foam. ¹³C NMR (CDCl₃) δ 195.6, 166.8, 159.3 (dd), 155.7 (dd), 148.2, 141.2, 139.7, 135.3, 133.8, 132.3, 131.5, 128.8, 128.7, 127.7, 124.5 (dd), 124.2 (dd), 116.2, 112.7, 111.6 (dd), 105.0 (dd), 46.3, 38.0, 30.9, 26.4, 25.8, 20.4

### Example 51:

### 3-[2-Chloro-4-(2,4-difluoro-phenylamino)-benzoyl]-N-(2-hydroxy-propyl)-4-methyl-benzamide (compound 151)

The reaction was carried out similarly as described in the preparation of compound 120, using 1-amino-2-propanol (0,13 mmol) and compound 439 (0.11 mmol). Purification was done by flash chromatography to afford the title compound as yellow foam. ¹³C NMR (CDCl₃) δ 195.7, 167.6, 159.3 (dd), 155.7 (dd), 148.4, 141.4, 139.8, 135.4, 133.9, 131.6, 131.5, 129.1, 128.4, 127.7, 124.7 (dd), 124.2 (dd), 116.2, 112.7, 111.6 (dd), 105.0 (dd), 67.4, 47.6, 21.0, 20.3

### Example 52:

### 3-[2-Chloro-4-(2,4-difluoro-phenylamino)-benzoyl]-N-(2,3-dihydroxy-propyl)-4-methyl-benzamide (compound 152)

The reaction was carried out similarly as described in the preparation of compound 120, using 3-amino-propane-1,2-diol (4.48 mmol) and compound 439 (2.24 mmol). Purification was done by flash, chromatography to afford the title compound as yellow foam. ¹³C NMR (CDCl₃) δ 195.8, 168.3, 159.3 (dd), 155.8 (dd), 148.6, 141.5, 139.9, 135.4, 134.0, 131.5, 130.9, 129.2, 128.0, 127.6, 124.8 (d), 124.1 (dd), 116.2, 112.6, 111.6 (dd), 105.0 (dd), 71.1, 63.8, 42.8, 20.3

### Example 53:

### 3-[2-Chloro-4-(2,4-difluoro-phenylamino)-benzoyl]-N-(1-hydroxymethyl-propyl)-4-methyl-benzamide (compound 153)

The reaction was carried out similarly as described in the preparation of compound 120, using 2-amino-butan-1-ol (0.13 mmol) and compound 439 (0.11 mmol). Purification was done by flash chromatography to afford the title compound as yellow syrup. ¹³C NMR (CDCl₃) δ 195.7, 167.4, 159.3 (dd), 155.7 (dd), 148.5, 141.2, 139.8, 135.4, 133.9, 131.8, 131.4, 128.9, 128.2, 127.7, 124.8 (dd), 124.2 (dd), 116.2, 112.6, 111.6 (dd), 105.0 (dd), 64.9, 53.8, 24.2, 20.3, 10.7

### Example 54:

### 3-[2-Chloro-4-(2,4-difluoro-phenylamino)-benzoyl]-4-methyl-N-(2,2,3,3,3-pentafluoro-propyl)-benzamide (compound 154)

The reaction was carried out similarly as described in the preparation of compound 120, using 2,2,3,3,3-pentafluoro-propylamine (0.13 mmol) and compound 439 (0.11 mmol). Purification was done by flash chromatography to afford the title compound as yellow syrup. ¹³C NMR (CDCl₃) δ 195.4, 166.9, 159.4 (dd), 155.7 (dd), 148.4, 142.2, 140.0, 135.4, 133.8, 131.7, 130.6, 129.0, 128.4, 127.9, 124.8 (dd), 124.1 (dd), 116.2, 112.7, 111.7 (dd), 105.0 (dd), 39.2 (t), 20.4

### Example 55:

### 3-[2-Chloro-4-(2,4-difluoro-phenylamino)-benzoyl]-N-(3-hydroxy-propyl)-4-methyl-benzamide (compound 155)

The reaction was carried out similarly as described in the preparation of compound 120, using 3-amino-propanol (0.13 mmol) and compound 439 (0.11 mmol). Purification was done by flash chromatography to afford the title compound as yellow syrup. ¹³C NMR (CDCl₃) δ 195.7, 167.6, 159.3 (dd), 155.7 (dd), 148.4, 141.4, 139.8, 135.3, 133.8, 131.5, 128.9, 128.4, 127.7, 124.8 (dd), 124.2 (dd), 116.2, 112.7, 111.6 (dd), 105.0 (dd), 60.0, 37.4, 31.9, 20.3

### Example 56:

### 3-[2-Chloro-4-(2,4-difluoro-phenylamino)-benzoyl]-N-(2-hydroxy-1,1-dimethyl-ethyl)-4-methyl-benzamide (compound 156)

The reaction was carried out similarly as described in the preparation of compound 120, using 2-amino-2-methyl-prypane-1-ol (0.28 mmol) and compound, 439 (0.14 mmol). Purification was done by flash chromatography to afford the title compound as yellow foam. ¹³C NMR (CDCl₃) δ 195.5, 167.6, 159.3 (dd), 155.7 (dd), 148.4, 141.3, 140.0, 135.4, 133.9, 132.2, 131.5, 128.7, 128.4, 127.6, 124.7 (dd), 124.1 (dd), 116.2, 112.7, 111.6 (dd), 105.0 (dd), 70.6, 56.6, 24.7, 20.3

### Example 57:

### 3-2-Chloro-4-(2,4-difluoro-phenylamino)-benzoyl]-N-(2-hydroxy-1-hydroxymethyl-1-methyl-ethyl)-4-Methyl-benzamide (compound 157)

The reaction was carried out similarly as described in the preparation of compound 120, using 2-amino-2-methyl-propane-1,3-diol (0.28 mmol) and compound 439 (0.14 mmol). Purification was done by flash chromatography to afford the title compound as yellow foam. ¹³C NMR (CDCl₃) δ 195.6, 167.7, 159.3 (dd), 155.7 (dd), 148.4, 141.5, 140.0, 135.4, 133.9, 132.0, 131.5, 128.7, 128.4, 127.7, 124.7 (dd), 124.1 (dd), 116.2, 112.8, 111.7 (dd), 105.0 (dd), 67.7, 59.2, 20.3, 20.0

### Example 58:

### {3-[2-Chloro-4-(2,4-difluoro-phenylamino)-benzoy]-4-methyl-benzoylamino}-acetic acid methyl ester (compound 158)

The reaction was carried out similarly as described in the preparation of compound 120, using amino-acetic acid ethyl ester (0.28 mmol) and compound 439 (0.14 mmol). Purification was done by flash chromatography to afford the title compound as yellow foam. ¹³C NMR (CDCl₃) δ 195.5, 170.0, 166.6, 159.2 (dd), 155.6 (dd), 148.2, 141.9, 139.7, 135.3, 133.7, 131.6, 131.1, 129.0, 128.7, 128.0, 124.5 (dd), 124.3 (dd), 116.2, 112.8, 111.6 (dd), 105.0 (dd), 61.7, 41.9, 20.4, 14.1

### Example 59:

### 3-[-Chloro-4-(2,4-difluoro-phenylamino)-benzoyl]-N-(4-hydroxy-butyl)-4-methyl-benzamide (compound 159)

The reaction was carried out similarly as described in the preparation of compound 120, using 4-amino-butanol (0.28 mmol) and compound 439 (0.14 mmol). Purification was done by flash chromatography to afford the title compound as yellow foam. ¹³C NMR (CDCl₃) δ 195.7, 166.8, 159.3 (dd), 155.7 (dd), 148.2, 141.2, 139.6, 135.3, 133.7, 132.1, 131.5, 129.0, 128.7, 127.7, 124.6 (dd), 124.2 (dd), 116.2, 112.7, 111.6 (dd), 105.0 (dd), 62.4, 39.9, 29.8, 26.3, 20.4

### Example 60:

### 3-[2-Chloro-4-(2,4-difluoro-phenylamino)-benzoyl]-N-(3-hydroxy-1,1-dimethyl-butyl)-4-methyl-benzamide (compound 160)

The reaction was carried out similarly as described in the preparation of compound 120, using 4-amino-4-methyl-pentan-2-ol (0.28 mmol) and compound 439 (0.14 mmol). Purification was done by flash chromatography to afford the title compound as yellow foam. ¹³C NMR (CDCl₃) δ 196.0, 165.8, 159.2 (dd), 155.6 (dd), 147.9, 141.0, 139.2, 135.1, 133.5, 133.3, 131.4, 129.1, 128.9, 128.2, 124.6 (dd), 124.3 (dd), 116.1, 112.7, 111.6 (dd), 105.0 (dd), 65.7, 53.8, 50.5, 28.4, 25.7, 24.6, 20.4

### Example 61:

### 3-[2-Chloro-4-(2,4-difluoro-phenylamino)-benzoyl]-4-methyl-N-(3-phenyl-propyl)-benzamide (compound 161)

The reaction was carried out similarly as described in the preparation of compound 120, using 3-phenyl-propylamine (0.28 mmol) and compound 439 (0.14 mmol). Purification was done by flash chromatography to afford the title compound as yellow foam. ¹³C NMR (CDCl₃) δ 195.6, 166.6, 159.3 (dd), 155.6 (dd), 148.2, 141.4, 141.2, 139.6, 135.3, 133.8, 132.0, 131.5, 128.8, 128.7, 128.6, 128.4, 127.6, 126.1, 124.6 (dd), 124.2 (dd), 116.2, 112.7, 111.6 (dd), 105.0 (dd), 39.9, 33.5, 31.0, 20.3

### Example 62:

### (R)-3-[2-Chloro-4-(2,4-difluoro-phenylamino)-benzoyl]-N-(1-hydroxymethyl-3-methyl-butyl)-4-methyl-benzamide (compound 162)

The reaction was carried out similarly as described in the preparation of compound 120, using (R)-leucinol (0.28 mmol) and compound 439 (0.14 mmol). Purification was done by flash chromatography to afford the title compound as yellow foam, ¹³C NMR (CDCl₃) δ 195.6, 167.4, 159.3 (dd), 155.7 (dd), 148.3, 141.4, 139.8, 135.4, 133.8, 131.8, 131.5, 128.8, 128.6, 127.8, 124.6 (dd), 124.2 (dd), 116.2, 112.8, 111.6 (dd), 105.0 (dd), 66.2, 50.6, 40.3, 25.1, 23.0, 22.3, 20,4

### Example 64:

### 3-[2-Chloro-4-(2,4-difluoro-phenylamino)-benzoyl]-N-isopropyl-4-methyl-benzamide(compound 164)

The reaction was carried out similarly as described in the preparation of compound 120, using isopropylamine (0.28 mmol) and compound 439 (0.14 mmol). Purification was done by flash chromatography to afford the title compound as yellow foam. ¹³C NMR (CDCl₃) δ 195.7, 165.9, 159.2 (dd), 155.6 (dd), 148.2, 141.0, 139.8, 135.4, 133.8, 132.3, 131.4, 128.7, 128.7, 127.6, 124.6 (dd), 124.2 (dd), 116.2, 112.7, 111.6 (dd), 105.0 (dd), 42.0, 22.8, 20.3

### Example 65:

### 3-[2-Chloro-4-(2,4-difluoro-phenylamino)-benzoyl]-N-cyclohexyl-4-methyl-benzamide (compound 165)

The reaction was carried out similarly as described in the preparation of compound 120, using cyclohexylamine (0.28 mmol) and compound 439 (0.14 mmol). Purification was done by flash chromatography to afford the title compound as yellow foam. ¹³C NMR (CDCl₃) δ 195.7, 165.8, 159.3 (dd), 155.7 (dd), 148.3, 141.0, 1.39.8, 135.4, 133.8, 132.5, 131.4, 128.8, 128.6, 127.6, 124.7 (dd), 124.3 (dd), 116.2, 112.7, 111.6 (dd), 105.0 (dd), 48.9, 33.2, 25.6, 24.9, 20.3

### Example 66.

### 3-[2-Chloro-4-(2,4-difluoro-phenylamino)-benzoyl]-N-(2,2-difluoro-ethyl)-4-methyl-benzamide (compound 166)

The reaction was carried out similarly as described in the preparation of compound 120, using 2,2-difluoro-ethylamine (0.28 mmol) and compound 439 (0.14 mmol), Purification was done by flash chromatography to afford the title compound as yellow foam. ¹³C NMR (CDCl₃) δ 195.4, 167.1, 159.3 (dd), 155.7 (dd), 148.3, 142.0, 140.0, 135.4, 133.8, 131.7, 130.8, 129.0, 128.6, 127.7, 124.6 (dd), 124.1 (dd), 116.2, 113.6 (t), 112.8, 111.7 (dd), 105.0 (dd), 42.3 (t), 20.4

### Example 67:

### 5-{3-[2-Chloro-4-(2,4-difluoro-phenylamino)-benzoyl]-4-methyl-benzoylamino}-4-oxo-pentanoic acid methyl ester (compound 167)

The reaction was carried out similarly as described in the preparation of compound 120, using 5-amino-4-oxo-pentanoic acid methyl ester (0.28 mmol) and compound 439 (0.14 mmol). Purification was done by flash chromatography to afford the title compound as yellow foam. ¹³C NMR (CDCl₃) δ 203.9, 195.5, 172.9, 166.5, 159.2 (dd), 155.5 (dd), 148.2, 141.9, 139.7, 135.3, 133.8, 131.6, 131.0, 129.0, 128.6, 127.9, 124.4 (dd), 124.3 (dd), 116.2, 112.8, 111.6 (dd), 105.0 (dd), 52.0, 49.6, 34.6, 27.6, 20.4

### Example 68:

### N-[(2-Carbamoyl-ethylcarbamoyl)-methyl]-3-[2-chloro-4-(2,4-difluoro-phenylamino)-benzoyl]-4-methyl-benzamide (compound 168)

The reaction was carried out similarly as described in the preparation of compound 120, using 3-(2-amino-acetylamino)-propionamide (0.28 mmol) and compound 439 (0.14 mmol). Purification was done by flash chromatography to afford the title compound as yellow solid. ¹³C NMR (CD₃OD) δ 197.6, 176.6, 171.8, 169.4, 161.2 (dd), 158.0 (dd), 151.6, 142.5, 141.5, 136.4, 135.2, 132.5, 130.5, 129.0, 128.3, 127.7 (dd), 125.8 (dd), 116.6, 113.0, 112.7 (dd), 105.8 (dd), 44.2, 37.0, 35.9, 20.3

### Example 69:

### (2-{3-[2-Chloro-4-(2,4-difluoro-phenylamino)-benzoyl]-4-methyl-benzoylamino}-acetylamino)-acetic acid ethyl ester (compound 169)

The reaction was carried out similarly as described in the preparation of compound 120, using (2-amino-acetylamino)-acetic acid ethyl ester (0.28 mmol) and compound 439 (0.14 mmol). Purification was done by flash chromatography to afford the title compound as yellow foam. ¹³C NMR (DMSO-d₆) δ 194.7, 169.7, 169.4, 165.4, 158.9 (dd), 155.7 (dd), 149.4, 139.9, 139.4, 133.8, 133.8, 131.2, 131.0, 129.3, 127.4, 126,5 (dd), 126.4, 124.1 (dd), 114.9, 112.0 (dd), 111.8, 105.0 (dd), 60.3, 42.2, 40.5, 19.6, 14.0

### Example 70:

### N-Allyl-3-[2-chloro-4-(difluoro-phenylamino)-benzoy]-4-methyl-benzamide (compound 170)

The reaction was carried out similarly as described in the preparation of compound 120, using allylamine (0.50 mmol) and compound 439 (0.25 mmol). Purification was done by flash chromatography to afford the title compound. ¹³C NMR (CDCl₃) δ 195.6, 166.5, 159.3 (dd), 155.6 (dd), 148.1, 141.5, 139.7, 135.3, 134.0, 133.7, 131.8, 131.6, 128.9, 128.8, 127.7, 124.5 (dd), 124.2 (dd), 116.9, 116.2, 112.8, 111.6 (dd), 105.0 (dd), 42.5, 20.4

### Example 71:

### 3-[2-Chloro-4-(2,4-difluoro-phenylamino)-benzoyl]-4-methyl-N-(2-sulfamoyl-ethyl)-benzamide (compound 171)

The reaction was carried out similarly as described in the preparation of compound 120, using 2-amino-ethanesulfonic acid amide (0.50 mmol) and compound 439 (0.25 mmol). Purification was done by flash chromatography to afford the title compound. ¹³C NMR (DMSO-d₆) δ 194.6, 165.3, 158.8 (dd), 155.8 (dd), 149.5, 139.8, 139.5, 133.8, 131.4, 131.1, 129.0, 127.1, 126.5 (dd), 126.2, 124.1 (dd), 114.9, 112.0 (dd), 111.8, 105.0 (dd), 53.5, 34.7, 19.6

### Example 72:

### N-(2-Acetylamino-ethyl)-3-[2-chloro-4-(2,4-difluoro-phenylamino)-benzoyl]-4-methyl-benzamide (compound 172)

The reaction was carried out similarly as described in the preparation of compound 120, using *N*-(2-amino-ethyl)-acetamide (0.50 mmol) and compound 439 (0.25 mmol). Purification was done by flash chromatography to afford the title compound. ¹³C NMR (CDCl₃) δ 195.9, 172.1, 167.6, 159.3 (dd), 155.8 (dd), 148.6, 141.3, 139.8, 135.3, 133.8, 131.5, 131.3, 129.0, 128.1, 127.9, 124.9 (dd), 124.2 (dd), 116.2, 112.5, 111.6 (dd), 104.9 (dd), 40.9, 39.8, 23.0, 20.3

### Preparation 40:

### 4-Bromo-2-chloro-thiobenzoic acid S-pyridin-2-yl ester (compound 440)

A mixture of 4-bromo-3-chlorobenzoic acid (5.00 g, 21.24 mmol), 2,2'-dithiodipyridine (4.68 g, 21.24 mmol) and triphenylphosphine (5.57 g, 21.24 mmol) in acetonitrile (150 ml) was stirred at room temperature for 0.5 h. The crystals were filtered and washed with petroleum ether to give the title compound.

### Preparation 41 :

### 3-(4-Bromo-2-chloro-benzoyl)-4-methoxy-benzoic acid methyl ester (compound 441)

To a solution of 3-iodo-4-methoxy-benzoic acid methyl ester (6.31 g, 21.6 mmol) in THF (20 ml) was added 2 M solution of isopropylmagnesium chloride in THF (11.0 ml, 22.00 mmol) at - 50°C. The reaction mixture was stirred at the same temperature for 30 min. Then compound 440 (5.91 g, 18 mmol) was added, The solution was warmed to room temperature and stirred for 2 h at the same temperature. Afterwards, the solution was quenched with saturated aqueous solution of NH₄Cl. The aqueous phase was extracted twice with diethyl ether. The combined organic phases were dried over MgSO₄ and concentrated *in vacuo.* The crude material was dissolved in ethyl acetate and silica gel added. The mixture was concentrated *in vacuo.* The residue was purified by chromatography (petroleum ether/ethyl acetate 5:1) to provide title compound as oil, which was solidified to a white solid.

### Preparation 42:

### 3-[2-Chloro-4-(2,6-difluoro-phenylamino)-benzoyl]-4-methoxy-benzoic acid methyl ester (compound 442)

A mixture of compound 441 (4.65 g, 12.1 mmol), 2,6-difluoroaniline (1.87 g, 14.5 mmol), dicyclohexyl-(2',4',6'-triisopropyl-biphenyl-2-yl)-phosphane (231 mg, 0.48 mmol), Pd(OAc)₂ (54 mg, 0.24 mmol), Cs₂CO₃ (8.3 g, 16.9 mmol) and celite (4.0 g) in 1,4-dioxane (30 ml) was stirred at 130°C for 18 h. The mixture was concentrated together with silica gel. The residue was purified by chromatography (petroleum ether/ethyl acetate 3:1) to give the title compound as a yellow solid.

### Preparation 43/Example 280 :

### 3-[2-Chloro-4-(2,6-difluoro-phenylamino)-benzoyl]-4-mehtoxy-benzoic acid (compound 443)

The reaction was carried out as described in the preparation of compound 416, using compound 442 (1.00 g, 2.40 mmol) as the ester. The title compound was used without any further purification. ¹³C NMR (DMSO-d₆) δ 191.5, 166.4, 160.6, 157.5 (dd), 149.0, 133.9, 133.5, 133.3, 130.6, 129.1, 126.7, 126.5 (t), 122.9, 116.4 (t), 114.6, 112.5 (m), 112.0, 111.7, 56.1.

### Preparation 44:

### 3-[-Chloro-4-(2,6-difluoro-phenylamino)-benzoyl]-4-methoxy-benzoyl chloride (compound 444)

To a suspension of compound 443 (590 mg, 1.4 mmol) in toluene (7 mL) was added thionyl chloride (206 µL, 2.8 mmol) and then refluxed for 2 h. The reaction mixture was concentrated *in vacuo* to afford the title compound without any further purification.

### Examples 73:

### 3-[2-Chloro-4-(2,6-difluoro-phenylamino)-benzoyl]-N-(2-hydroxy-ethyl)-4-methoxy-benzamide (compound 173)

The reaction was carried out similarly as described in the preparation of compound 120, using 2-amino-ethanol (0.28 mmol) and compound 444 (0.14 mmol). Purification was done by flash chromatography to afford the title compound. ¹³C NMR (CD₃CN) δ 193.6, 167.5, 161.1, 159.0 (dd), 149.9, 135.0, 134.4, 132.8, 130.5, 129.7, 129.4, 127.9, 127.6 (t), 117.8 (t), 116.3, 113.3 (m), 113.2, 112.8, 62.0, 56.9, 43.5

### Example 74:

### 3-[2-Cloro-4-(2,6-difluoro-phenylamino)-benzoyl]-N-(2-fluoro-ethyl)-4-methoxy-benzamide (compound 174)

The reaction was carried out similarly as described in the preparation of compound 120, using 2-fluoro-ethylamine (0.28 mmol) and compound 444 (0.14 mmol). Purification was done by flash chromatography to afford the title compound. ¹³C NMR (CDCl₃) δ 192.9, 166.6, 160.6, 157.5 (dd), 147.7, 134.6, 133.0, 132.4, 129.5, 129.4, 128.7, 126.4, 125.7 (t), 117.1 (t), 116.0, 112.8, 112.2 (m), 111.5, 82.7 (d), 56.0, 40.5 (d)

### Example 75:

### 3-[2-Chloro-4-(2,6-difluoro-phenylamino)-benzoyl]-N-(2,3-dihydroxy-propyl)-4-methoxy-benzamide (compound 175)

The reaction was carried out similarly as described in the preparation of compound 120, using 3-amino-propane-1,2-diol (0.28 mmol) and compound 444 (0.14 mmol). Purification was done by flash chromatography to afford the title compound. ¹³C NMR (acetone-D₆) δ 192.8, 167.7, 161.1, 159.0 (dd), 149.8, 134.8, 134.1, 132.8, 130.6, 129.6, 129.5, 127.6, 127.4 (t), 118.1 (t), 116.1, 113.1 (m), 113.0, 112.4, 72.2, 64.6, 56.5, 43.9

### Example 76:

### 3-[2-Chloro-4-(2,6-difluoro-phenylamino)-benzoyl]-N-(3-hydroxy-propyl)-4-methoxy-benzamide (compound 176)

The reaction was carried out similarly as described in the preparation of compound 120, using 3-amino-propan-1-ol (0.28 mmol) and compound 444 (0.14 mmol). Purification was done by flash chromatography to afford the title compound. ¹³C NMR (CDCl₃) δ 193.1, 167.4, 160.5, 157.5 (dd), 147.9, 134.6, 133.2, 132.4, 129.3, 129.1, 128.7, 126.4, 125.7 (t), 117.1 (t), 116.0, 112.7, 112.2 (m), 111.5, 59.7, 56.0, 37.1, 32.1

### Examples 77:

### 3-[2-Chloro-4-(2,6-difluoro-phenylamino)-benzoyl]-4-methoxy-N-phenethyl-benzamide (compound 177)

The reaction was carried out similarly as described in the preparation of compound 120, using phenylethylamine (0.28 mmol) and compound 444 (0.14 mmol). Purification was done by flash chromatography to afford the title compound. ¹³C NMR (CDCl₃) δ 192.8, 166.3, 160.5, 157.4 (dd), 147.5, 138.9, 134.5, 132.9, 132.5, 129.8, 129.2, 128.8, 128.7, 128.4, 126.9, 126.6, 125.6 (t), 117.1 (t), 116.0, 112.8, 112.2 (m), 111.6, 56.0, 41.3, 35.8

### Example 78:

### 3-[2-Chloro-4-(2,6-difluoro-phenylamino)-benzoyl]-N-(2-hydroxy-1,1-dimethyl-ethyl)-4-methoxy-benzamide (compound 178)

The reaction was carried out similarly as described in the preparation of compound 120, using 2-amino-2-methyl-propan-1-ol (0.28 mmol) and compound 444 (0.14 mmol). Purification was done by flash chromatography to afford the title compound. ¹³C NMR (CDCl₃) δ 192.9, 167.3, 160.5, 157.5 (dd), 147.8, 134.6, 133.1, 132.5, 129.3, 129.3, 128.5, 127.0, 125.7 (t), 117.1 (t), 116.0, 112.7, 112.2 (m), 111.5, 70.7, 56.6, 56.0, 24.7

### Examples 79:

### 3-[2-Chloro-4-(2,6-difluoro-phenylamino)-benzoyl]-4-methoxy-N-(2-morpholin-4-yl-ethyl)-benzamide (compound 179)

The reaction was carried out similarly as described in the preparation of compound 120, using 2-morpholin-4-yl-ethylamine (0.28 mmol) and compound 444 (0.14 mmol). Purification was done by flash chromatography to afford the title compound. ¹³C NMR (CDCl₃) δ 192.9, 166.3, 160.5, 157.5 (dd), 147.7, 134.5, 133.0, 132.5, 129.5, 129.3, 128.6, 126.9, 125.7 (t), 117.1 (t), 116.0, 112.7, 112.2 (m), 111.6, 66.9, 57.0, 56.0, 53.3, 36.2

### Example 80:

### 3-[2-Chloro-4-(2,6-difluoro-phenylamino)-benzoyl]-N-(2-hydroxy-1-hydroxymethyl-1-methyl-ethyl)-4-methoxy-benzamide (compound 180)

The reaction was carried out similarly as described in the preparation of compound 120, using 2-amino-2-methyl-propane-1,3-diol (0.28 mmol) and compound 444 (0.14 mmol). Purification was done by flash chromatography to afford the title compound. ¹³C NMR (CDCl₃) δ 193.0, 167.6, 160.6, 157.4 (dd), 147.7, 134.7, 133.2, 132.4, 129.3, 129.3, 128.8, 126.8, 125.7 (t), 117.1 (t), 116.0, 112.8, 112.2 (m), 111.4, 67.6, 59.3, 56.0, 20.0

### Example 81:

### 3-[2-Chloro-4-(2,6-difluoro-phenylamino)-benzoyl]-N-(2-hydroxy-ethyl)-4-methoxy-N-methyl-benzamide (compound 181)

The reaction was carried out similarly as described in the preparation of compound 120, using 2-methylamino-ethanol (0.28 mmol) and compound 444 (0.14 mmol). Purification was done by flash chromatography to afford the title compound. ¹³C NMR (CDCl₃) δ 192.8, 159.4, 157.4 (dd), 147.5, 134.5, 133.0, 132.7, 129.7, 129.7, 129.1, 125.6 (t), 117.1 (t), 116.0, 112.8, 112.2 (m), 111.6, 61.2, 56.0

### Example 82:

### {3-[2-Chloro-4-(2,6-difluoro-phenylamino)-benzoyl]-4-methoxy-benzoylamino}-acetic acid ethyl ester (compound 182)

The reaction was carried out similarly as described in the preparation of compound 120, using amino-acetic acid ethyl ester (0.84 mmol) and compound 444 (0.42 mmol). Purification was done by flash chromatography to afford the title compound. ¹³C NMR (CDCl₃) δ 192.8, 170.1, 166.4, 160.7, 157.5 (dd), 147.6, 134.6, 133.1, 132.4, 129.5, 129.4, 128.9, 126.0, 125.6 (t), 117.1 (t), 116.0, 112.8, 112.2 (m), 111.5, 61.6, 56.0, 41.9, 14.2

### Example 83:

### (2-{3-[2-Chloro-4-(2,6-difluoro-phenylamino)-benzoyl]-4-ethoxy-benzoylamino}-acetylamino)-acetic acid ethyl ester (compound 183)

The reaction was carried out similarly as described in the preparation of compound 120, using (2-amino-acetylamino)-acetic acid ethyl ester (0.84 mmol) and compound 444 (0.42 mmol). Purification was done by flash chromatography to afford the title compound. ¹³C NMR (CDCl₃) δ 192.9, 169.9, 169.7, 166.9, 160.7, 157.5 (dd), 147.8, 134.6, 133.2, 132.4, 129.4, 129.3, 129.2, 125.7, 125.6 (t), 117.1 (t), 116.0, 112.8, 112.2 (m), 111.4, 61.5, 56.0, 43.7, 41.4, 14,1

### Example 84:

### 3-[2-Chloro-4-(2,6-difluoro-phenylamino)-benzoyl]-N,N-bis-(2-hydroxy-ethyl)-4-methoxy-benzamide compound 184)

The reaction was carried out similarly as described in the preparation of compound 120, using 2-(2-hydroxy-ethylamino)-ethanol (0.28 mmol) and compound 444 (0.14 mmol). Purification was done by flash chromatography to afford the title compound. ¹³C NMR (CDCl₃) δ 193.0, 172.8, 159.1, 157.4 (dd), 147.6, 134.5, 133.1, 132.5, 129.8, 129.5, 129.2, 128.3, 125.6 (t), 117.1 (t), 116.0, 112.8, 112.2 (m), 111.6, 60.7 (bs), 53.5 (bs), 49.9 (bs)

### Example 85:

### 3-[2-Chloro-4-(2,6-difluoro-phenylamino)-benzoyl]-4-methoxy-N,N-bis-(2-methoxy-ethyl)-benzamide (compound 185)

The reaction was carried out similarly as described in the preparation of compound 120, using bis-(2-methoxy-ethyl)-amine (0.28 mmol) and compound 444 (0.14 mmol). Purification was done by flash chromatography to afford the title compound. ¹³C NMR (CDCl₃) δ 192.9, 171.4, 158.8, 157.5 (dd), 147.7, 134.5, 133.1, 132.2, 129.5, 129.5, 129.1, 128.8, 125.6 (t), 117.2 (t), 115.9, 112.6, 112.1 (m), 111.5, 70.6, 58.8, 56.0, 49.9 (bs), 45.5 (bs)

### Preparation 45:

### 3-[2-Chloro-4-(3-fluoro-2-methyl-phenylamino)-benzoyl]-4-methyl-benzoic acid methyl ester (compound 445)

1-Bromo-3-fluoro-2-methyl-benzene (189 mg, 1.0 mmol) was dissolved in 4 mL dry 1,4-dioxane under an argon atmosphere. Compound 402 (304 mg, 1.00 mmol) was added and dissolved in the solvent. Dicyclohexyl-(2',4',6'-triisopropyl-biphenyl-2-yl)-phosphane (19 mg, 0.04 mmol), Pd(OAc)₂ (5 mg, 0.02 mmol) and Cs₂CO₃ (407 mg, 1.25 mmol) were added, and the reaction mixture was stirred under an argon atmosphere at 120 °C for 60 h. The reaction mixture was filtered and then purified by flash chromatography using EtOAc/petroleum ether (40-60) 1:4, as the eluent to afford the title compound as a solid. ¹³C NMR (CDCl₃) δ

### Preparation 51:

### 3-[2-Chloro-4-(4-fluoro-phenylamino)-benzoyl]-4-methyl-benzoic acid methyl ester (Compound 451)

The reaction was carried out similarly as described in the preparation of compound 445, using 1-bromo-4-fluoro-benzene (110 µL, 1.0 mmol) as the bromide. Purification was done by flash chromatography to afford the title compound.

### Example 90:

### 3-[2-Chloro-4-(4-fluoro-phenylamino)-benzoyl]-N-(2-hydroxy-ethyl)-4-methyl-benzamide (compound 190)

To a solution of compound 451 (96 mg, 0.24 mmol) in acetonitrile (0.7 mL) and 2-amino-ethanol (0.50 mL) was added K₂CO₃ (50 mg, 0.36 mmol) and stirred for 18 h at RT. The reaction mixture was poured into a mixture of EtOAc/water. The aqueous phase was extracted with more EtOAc. The organic phases were combined and concentrated on silica gel *in vacuo*. Purification was done by flash chromatography eluting with mixtures of MeOH/DCM to afford the title compound as yellow syrup. ¹³C NMR (CDCl₃) δ 195.9, 167.9, 159.5 (d), 149.5, 141.0, 140.0, 135.8 (d), 135.5, 134.2, 131.4, 128.9, 127.4, 126.8, 123.9 (d), 116.3 (d), 115.7, 112.1, 61.7, 42.9, 20.2

### Preparation 61:

### 4-Chloro-3-(2-chloro-4-nitro-benzoyl)-benzoic acid methyl ester (compound 461)

A dry flask was charged with 4-chloro-3-iodo-benzoic acid methyl ester (5.0 g, 16.9 mmol) and the flask was evaporated and then filled with argon and this process repeated twice. Dry THF (35 mL) was added, and the solution cooled to-40 °C; then isopropylmagnesium chloride (8.85 mL, 2.0 M in diethyl ether, 17.7 mmol) was added slowly over 20 min keeping the temperature below -40 °C. On completion of the addition the reaction mixture was stirred at -40 °C for 45 min. A THF solution of ZnCl₂ (2.32 g, 17.0 mmol, 0.9 M) was added dropwise over 20 min. The reaction mixture was stirred at 0 °C for 20 min; then 2-chloro-4-nitro-benzoyl chloride (3.7 g, 17 mmol) and Cu(OAc)₂ (68 mg, 0.34 mmol) were added and the reaction mixture was allowed to warm to room temperature. After 16 h the reaction mixture was poured into a mixture of EtOAc/water, then shaken and separated. The aqueous phase was extracted with more EtOAc. The organic phases were combined, dried (MgSO₄), filtered, and concentrated *in vacuo* to afford the crude product. The crude product was purified by flash chromatography using EtOAc/petroleum ether 1:6 as the eluent to give the title compound as yellow solid.

### Preparation 62:

### 3-(4-Amino-2-chloro-benzoyl)-4-chloro-benzoic acid methyl ester (compound 462)

To a solution of compound 461 (3.31 g, 9.35 mmol) in methanol (125 mL) was added zinc dust (6.1 g, 94 mmol) and ammonium chloride (2.5 g, 47 mmol) in one portion under stirring. A CaCl₂ tube was mounted on the flask and the flask was placed in an oil bath with a temperature of 90 °C. After 18 h the reaction mixture was cooled to RT, filtered, and then poured into a mixture of EtOAc/water. The aqueous phase was extracted with more EtOAc. The organic phases were combined, dried (MgSO₄), filtered, and concentrated *in vacuo* to afford the crude product. The crude product was purified by flash chromatography using EtOAc/pentane 1:1 as the eluent, The product was triturated in DCM and then filtered and dried to give the title compound as light yellow solid.

### Preparation 63:

### 4-Chloro-3-[2-chloro-4-(2,4-difluoro-phenylamino)-benzoyl]-benzoic acid methyl ester (compound 463)

1-Bromo-2,4-difluorobenzene (87 µL, 0.77 mmol) was dissolved in 2.5 mL dry toluene under an argon atmosphere. Compound 462 (250 mg, 0.77 mmol) was added and dissolved in the solvent. 4,5-bis-diphenylphosphanyl-9,9-dimethyl-9H-xanthene (13 mg, 0.023 mmol), Pd(OAc)₂ (3.5 mg, 0.015 mmol) and Cs₂CO₃ (352 mg, 1.08 mmol) were added, and the reaction mixture was stirred under an argon atmosphere at 120 °C for 24 h. The reaction mixture was filtered and then purified by flash chromatography using EtOAc/petroleum ether 1:2 as the eluent to afford the title compound as a solid.

### Example 104:

### 4-Chloro-3-[2-Chloro-4-(2,4-difluoro-phenylamino)-benzoyl]-N-(2-hydroxy-ethyl)-benzamide (compound 204)

The reaction was carried out similarly as described in the preparation of compound 190, using 2-amino-ethanol (0.50 mL) and compound 463 (0.12 mmol), Purification was done by flash chromatography using MeOH/DCM 7:93 to afford the title compound. ¹³C NMR (DMSO-d₆) δ 190.9, 164.6, 159.1 (dd), 156.0 (dd), 150.4, 139.4, 134.9, 134.8, 133.4, 132.9, 130.4, 130.0, 127.8, 127.0 (dd), 124.4, 123.9 (dd), 115.2, 112.1 (dd), 111.9, 105.2 (dd), 59.6, 42.4

### Preparation 91:

### 2-Methyl-5-nitro-thiobenzoic acid S-pyridin-2-yl ester (compound 491)

2-Methyl-5-nitrobenzoic acid (22.5 g, 124 mmol), 2,2'-dithiopyridine (27.5 g, 124 mmol) and triphenylphosphine (32.6 g, 124 mmol) were dissolved in CH₃CN (650 mL). The solution was stirred at room temperature for 18 h. The reaction mixture was filtered and the solid was washed with small amounts of CH₃CN. This afforded the title compound as a colourless solid.

### Preparation 92:

### (4-Bromo-2-chloro-phenyl)-(2-methyl-5-nitro-phenyl)-methanone (compound 492)

The reaction was run under an argon atmosphere using dry glassware. 4-Bromo-2-chloroiodobenzene (25.5 g, 80.9 mmol) was dissolved in dry THF (400 mL) and cooled to -60 °C. Isopropylmagnesium chloride (2 M in THF, 40.4 mL, 80.9 mmol) was added under stirring during 30 minutes. The reaction mixture was allowed to warm up to -40 °C and the mixture was stirred at -40°C for 4 h. Compound 491 (22.2 g, 80.9 mmol) was added and the mixture was stirred at-40 °C for 3 h after which it was allowed to warm to room temperature and stirred for 17 h. A saturated aqueous solution of NH₄Cl (200 mL) was added and the mixture was stirred for 1 h. The phases were separated and the aqueous phase was extracted with Et₂O (4 x 100 mL). The combined organic phases were washed with brine, dried (MgSO₄), filtered and concentrated *in vacuo.* The crude product was purified by flash chromatography using CH₂Cl₂/petroleum ether (40-60) 2:3 as the eluent to afford the title compound as yellow crystalline compound.

### Preparation 93:

### [2-Chloro-4-(2,4-difluoro-phenylamino)-phenyl]-(2-methyl-5-nitro-phenyl)-methanone (compound 493)

Compound 492 (5.4 g, 15.2 mmol) was dissolved in dry 1,4-dioxan (150 mL) in a 200 mL screw cap vessel. 2,4-Difluoroaniline (1.7 mL, 16.7 mmol) was added and argon was blown over the mixture. Cs₂CO₃ (14.9 g, 45.7 mmol), BINAP (0.38 g, 0.6 mmol) and Pd(OAc)₂ (0.14 g, 0.6 mmol) were added and argon was blown through the mixture and the screw cap vessel was closed. The mixture was stirred at 100 °C for 7 h. The reaction mixture was poured into H₂O (100 mL) and EtOAc (200 mL). The water phase was extracted with EtOAc (x 3) and the combined organic phases were washed with brine, dried (MgSO₄), filtered and concentrated *in vacuo*. The crude product was purified by flash chromatography using CH₂Cl₂/petroleum ether (40-60) 2:3 -> 1:1 -> 1:0 followed by EtOAc as the eluent to afford the title compound as a yellow crystalline compound.

### Preparation 94:

### (5-Amino-2-methyl-phenyl)-[2-Chloro-4-(2,4-difluoro-phenylamino)-phenyl]-methanone (compound 494)

Compound 493 (6.0 g, 14.9 mmol) was dissolved in MeOH (350 mL). Zinc-dust (12.69 g, 194 mmol) and NH₄Cl (5.59 g, 104 mmol) were added. The reaction mixture was heated at reflux temperature for 1 h. The mixture was filtered and washed with MeOH. The filtrate was concentrated and the solid was dissolved in EtOAc (150 mL) and saturated aqueous Na₂CO₃ (100 mL). The water phase was extracted with EtOAc and the combined organic phases were dried (MgSO₄), filtered and concentrated *in vacuo.* The crude product was purified by flash chromatography using EtOAc/petroleum ether (40-60) 1:2 as the eluent to afford the title compound as a slightly coloured crystalline compound.

### Preparation 95:

### [2-Chloro-4-(2,4-difluoro-phenylamino)-phenyl]-(5-iodo-2-methyl-phenyl)-methanone (compound 495)

Compound 494 (0.62g, 1.66 mmol) was dissolved in acetone (14 mL). Concentrated HCl (37%, 0.69 mL, 8.3 mmol) was added and the solution was cooled on an icebath. NaNO₂ (0.14 g, 1.99 mmol) was dissolved in H₂O (1 mL) and added to the above solution during 15 minutes. The internal temperature was kept at 0 °C - 2 °C during the addition. The suspension was stirred on an ice bath for 0.5 h, after which a solution of KI (0.41 g, 2.45 mmol) and I₂ (0.31 g, 1.22 mmol) in H₂O (4 mL) was added drop wise during 5 minutes. The mixture was stirred at 0 °C for 2 h. H₂O (20 mL) and EtOAc (20 mL) was added and stirred and the phases were separated. The organic phase was washed with aqueous NaHSO₃, then with aqueous Na₂CO₃, dried (MgSO₄), filtered and concentrated *in vacuo.* The crude product was purified by flash chromatography using EtOAc/petroleum ether (40-60) 1:5 to afford the title compound as a slightly coloured crystalline compound.

### Example 141:

### N-{3-[2-Chloro-4-(2,4-difluoro-phenylamino)-benzoyl]-4-methyl-phenyl}-3-methoxy-propionamide (compound 241)

3-Methoxypropionic acid (0.022mL, 0.23 mmol) was dissolved in dry DMF (5 mL). *O*-(7-Azabenzotriazol-1-yl)-*N*,*N*,*N*',*N*'-tetramethyluronium-hexafluorophosphate (HATU) (0.09 g, 0.23 mmol) was added followed by addition of 2,4,6-trimethylpyridine (0.048 mL, 0.36 mmol). The mixture was stirred for 0.5 h after which compound 494 (0.067 g, 0.18 mmol) was added. The mixture was stirred at room temperature for 18 h. The reaction mixture was concentrated *in vacuo* and the crude product was purified by flash chromatography using a gradient of EtOAc/petroleum ether (40-60) 1:4 -> 4:1 as the eluent. This afforded the title compound as a slightly coloured crystalline compound. ¹³C NMR (CDCl₃) δ 195.8, 169.8, 159.1 (dd), 155.5 (dd), 147.7, 139.6, 135.6, 135.2, 133.7, 133.4, 131.8, 129.2, 124.4 (dd), 124.2 (dd), 122.5, 120.8, 116.3, 112.8, 111.6 (dd), 104.9 (dd), 68.5, 58.9, 37.9, 19.8

### Example 142:

### N-{3-[2-Chloro-4-(2,4-difluoro-phenylamino)-benzoyl]-4-methyl-phenyl}-propionamide (compounds 242)

Propionic acid (0.01mL, 0.13 mmol) was dissolved in dry DMF (5 mL). HATU (0.05 g, 0.13 mmol) was added followed by addition of 2,4,6-trimethylpyridine (0.027 mL, 0.2 mmol). The mixture was stirred for 0.5 h after which compound 494 (0.038 g, 0.10 mmol) was added. The mixture was stirred at room temperature for 18 h. The reaction mixture was concentrated *in vacuo* and the crude product was purified by flash chromatography using a gradient of EtOAc/petroleum ether (40-60) 1:4 -> 4:1 as the eluent. This afforded the title compound as yellow oil. ¹³C NMR (CDCl₃) δ 196.1, 172.4, 159.2 (dd), 155.6 (dd), 148.0, 139.5, 135.7, 135.1, 133.7, 133.2, 131.8, 128.8, 124.6 (dd), 124.3 (dd), 122.5, 120.7, 116.2, 112.6, 111.6 (dd), 104.9 (dd), 30.5, 19.7, 9.6

### Example 143:

### N-{3-[2-Chloro-4-(2,4-difluoro-phenylamino)-benzoyl]-4-methyl-phenyl}-2-(2-methoxy-ethoxy)-acetamide (compound 243)

2-(2-Methoxyethoxy)acetic acid (0.017 mL, 0,15 mmol) was dissolved in dry DMF (5 mL). HATU (0.055 g, 0.15 mmol) was added followed by addition of 2,4,6-trimethylpyridine (0.03 mL, 0.22 mmol). The mixture was stirred for 0.5 h after which compound 494 (0.042 g, 0.11 mmol) was added. The mixture was stirred at room temperature for 18 h. The reaction mixture was concentrated *in vacuo* and the crude product was purified by flash chromatography using a gradient of EtOAc/petroleum ether (40-60) 1:4 -> 4:1 as the eluent. This afforded the title compound as yellow oil. ¹³C NMR (CDCl₃) δ 195.9, 168.4, 159.1 (dd), 155.6 (dd), 147.9, 139.4, 135.1, 135.1, 133.7, 133.5, 131.9, 129.2, 124.5 (dd), 124.4 (dd), 122.3, 120.7, 116.1, 112.7, 111.6 (40), 104.9 (dd), 71.4, 71.3, 70.4, 58.9, 19.8

### Example 144:

### N-{3-[2-Chloro-4-(2,4-difluoro-phenylamino)-benzoyl]-4-methyl-phenyl}-3-morpholin-4-yl-propionamide (compound 244)

3-(4-Morpholino)propionic acid hydrochloride (0,027 g, 0.14 mmol) was dissolved In dry DMF (5 mL). HATU (0.052 g, 0.14 mmol) was added followed by addition of 2,4,6-trimethylpyridine (0.035 mL, 0.26 mmol). The mixture was stirred for 0,5 h after which compound 494 (0.039 g, 0.10 mmol) was added. The mixture was stirred at room temperature for 18 h. The reaction mixture was concentrated *in vacuo* and the crude product was purified by flash chromatography using a gradient of MeOH/dichloromethane 1:50 -> 1:12 as the eluent. This afforded the title compound as yellow oil. ¹³C NMR (COCl₃) δ 195.9, 170.2, 159.1 (dd), 155.5 (dd), 147.6, 138.9, 136.2, 134.8, 133.5, 133.2, 132.1, 129.6, 124.5 (dd), 124.2 (dd), 122.1, 121.0, 116.1, 112.8, 111.6 (dd), 104.9 (dd), 66.9, 54.0, 52.7, 32.1, 20.0

### Example 145:

### N-{3-[2-Chloro-4-(2,4-difluoro-phenylamino)-benzoyl]-4-methyl-phenyl}-3-hydroxy-propionamide (compound 245)

3-Hydroxypropionic acid (30% in H₂O, 0.33 mmol) was dissolved in dry DMF (5 mL), HATU (0.125 g, 0.33 mmol) was added followed by addition of 2,4,6-trimethylpyridine (0.061 mL, 0.5 mmol). The mixture was stirred for 0,5 h after which compound 494 (0.094 g, 0.25 mmol) was added. The mixture was stirred at room temperature for 18 h. The reaction mixture was concentrated *in vacuo* and the crude product was purified by flash chromatography using EtOAc/petroleum ether (40-60) 1:1 as the eluent. This afforded the title compound as a yellow oil, ¹³C NMR (DMSO-d₆) δ 195.1, 169.8, 158.8 (dd), 155.6 (dd), 149.1, 139.0, 136.9, 133.4, 133.3, 131.3, 130.9, 127.0, 126.4 (dd), 124.3 (dd), 121.1, 119.4, 114.7, 111.9 (dd), 111.8, 105.0 (dd), 57.3, 40.0, 19.1

### Example 146,

### N-{3-[2-Chloro-4-(2,4-difluoro-phenylamino)-benzoyl]-4-methyl-phenyl}-3-furan-2-yl-propionamide (compound 246)

3-(2-Furfuryl)propionic acid (0.02 g, 0.14 mmol) was dissolved in dry DMF (4 mL). HATU (0.053 g, 0.14 mmol) was added followed by addition of 2,4,6-trimethylpyridine (0.029 mL, 0,22 mmol). The mixture was stirred for 0.5 h after which compound 494 (0.040 g, 0.11 mmol) was added. The mixture was stirred at room temperature for 18h. The reaction mixture was concentrated *in vacuo* and the crude product was purified by flash chromatography using a gradient of EtOAc/petroleum ether (40-60) 1:9 -> 2:3 as the eluent. This afforded the title compound as yellow oil. ¹³C NMR (CDCl₃) δ 196.0, 170.1, 159.2 (dd), 155.6 (dd), 154.1, 147.9, 141.3, 139.5, 135.4, 135.1, 133.7, 133.5, 131.8, 128.9, 124.4 (dd), 124.3 (dd), 122.6, 120.8, 116.2, 112.7, 111.6 (dd), 110.3, 105.7, 104.9 (dd), 35.8, 23.8, 19.8

### Example 147:

### N-{3-[2-Chloro-4-(2,4-difluoro-phenylamino)-benzyl]-4-methyl-phenyl}-2-hydroxy-benzamide (compound 247)

2-Hydroxybenzoic acid (0.019 g, 0.14 mmol) was dissolved in dry DMF (4 mL). HATU (0.053 g, 0.14 mmol) was added followed by addition of 2,4,6-trimethylpyridine (0.029 mL, 0.22 mmol). The mixture was stirred for 0.5 h after which compound 494 (0.040 g, 0.11 mmol) was added. The mixture was stirred at room temperature for 18 h. The reaction mixture was concentrated *in vacuo* and the crude product was purified by flash chromatography using a gradient of EtOAc/petroleum ether (40-60) 1:9 -> 3:2 as the eluent. This afforded the title compound as yellow oil. ¹³C NMR (CDCl₃) δ 196.0, 168.5, 161.7, 159.2 (dd), 155.6 (dd), 148.1, 139.7, 135.3, 134.7, 134.5, 134.4, 133.8, 132.0, 128.7, 125.9, 124.5 (dd), 124.2 (dd), 124.0, 122.1, 119.0, 118.8, 116.2, 114.6, 112.7, 111.6 (dd), 105.0 (dd), 19.8

### Example 148:

### N-{3-[2-Chloro-4-(2,4-difluoro-phenylamino)-benzoyl]-4-methyl-phenyl}-2-(2,5-dioxo-imidazolidin-4-yl)-acetamide (compound 248)

Hydantoin-5-acetic acid (0.022 g, 0.14 mmol) was dissolved in dry DMF (4 mL). HATU (0.053 g, 0.14 mmol) was added followed by addition of 2,4,6-trimethylpyridine (0.029 mL, 0.22 mmol). The mixture was stirred for 0.5 h after which compound 494 (0.040 g, 0.11 mmol) was added. The mixture was stirred at room temperature for 18 h. The reaction mixture was concentrated *in vacuo* and the crude product was purified by flash chromatography using MeOH/CH₂Cl₂ 3:100 as the eluent. This afforded the title compound as a yellow oil. ¹³C NMR (CD₃OD) δ 198.1, 177.6, 169.6, 161.1 (dd), 160.0, 157.9 (dd), 151.3, 141.1, 137.5, 136.1, 134.9, 134.1, 132.7, 128.8, 127.5 (dd), 126.0 (dd), 123.4, 121.8, 116.5, 113.0, 112.7 (dd), 105.8 (dd), 56.7, 39.2, 19.8

### Example 149:

### 2,6-Dioxo-hexahydro-pyrimidine-4-carboxylic acid {3-[2-chloro-4-(2,4-difluoro-phenylamino)-benzoyl]-4-methyl-phenyl}-amide (compound 249)

D,L-Dihydroorotic acid (0.022 g, 0.14 mmol) was dissolved in dry DMF (4 mL). HATU (0.053 g, 0.14 mmol) was added followed by addition of 2,4,6-trimethylpyridine (0.029 mL, 0.22 mmol). The mixture was stirred for 0.5 h after which compound 494 (0.040 g, 0.11 mmol) was added, The mixture was stirred at room temperature for 18 h. The reaction mixture was concentrated *in vacuo* and the crude product was purified by flash chromatography using EtOAc/acetone 10:1 as the eluent. This afforded the title compound as a colourless solid. ¹³C NMR (DMSO-d₆) δ 194.9, 169.2, 169.0, 158.7 (dd), 155.7 (dd), 153.6, 149.2, 139.2, 136.1, 133,4, 133.3, 131.7, 131.5, 126.8, 126.4 (dd), 124.2 (dd), 121.4, 119.6,114.7, 112.0 (dd), 111.8, 105.0 (dd), 50.3, 38.9, 19.1

### Example 150:

### Acylic acid 2-{3-[2-chloro-4-(2,4-difluoro-phenylamino)-benzoyl]-4-methyl-phenylcarbamoyl}-ethyl ester (compound 250)

2-Carboxyethyl acrylate (0.041 mL, 0,35 mmol) was dissolved in dry DMF (7 mL). HATU (0.13 g, 0.35 mmol) was added followed by addition of 2,4,6-trimethylpyridine (0.07 mL, 0.54 mmol). The mixture was stirred for 0.5 h after which compound 494 (0.10 g, 0.27 mmol) was added. The mixture was stirred at room temperature for 18 h. The reaction mixture was concentrated *in vacuo* and the crude product was purified by flash chromatography using a gradient of EtOAc/petroleum ether (40-60) 15:85 -> 70:30 as the eluent, This afforded the title compound as oil. ¹³C NMR (DMSO-d₆) δ 196.1, 168.3, 166.1, 159.2 (dd), 155.6 (dd), 148.2, 139.6, 135,4, 135.1, 133.8, 133.5, 131.8, 131.4, 128.6, 128.0, 124.6 (dd), 124.3 (dd), 122.6, 120.7, 116.2, 112.6, 111.6 (dd), 104.9 (dd), 60.4, 36.6, 19.7

### Example 151:

### N-{3-[2-Chloro-4-(2,4-difluoro-phenylamino)-benzoyl]-4-methyl-phenyl}-3-methylsulfanyl-propionamide (compound 251)

3-Methylthiopropionic acid (0.067 g, 0.55 mmol) was dissolved in dry DMF (16 mL). HATU (0.21 g, 0.55 mmol) was added followed by addition of 2,4,6-trimethylpyridine (0.11 mL, 0.86 mmol). The mixture was stirred for 0.5 h after which compound 494 (0.16 g, 0.43 mmol) was added. The mixture was stirred at room temperature for 18 h. The reaction mixture was concentrated *in vacuo* and the crude product was purified by flash chromatography using a gradient of EtOAc/petroleum ether (40-60) 1:9 -> 1;1 as the eluent. This afforded the title compound as yellow. oil. ¹³C NMR (CDCl₃) δ 196.1, 169.8, 159.2 (dd), 155.6 (dd), 148.0, 139.6, 135.5, 135.2, 133,8, 133.5, 131.8, 128.7, 124.5 (dd), 124.3 (dd), 122.6, 120.7, 116.2, 112.6, 111.6 (dd), 104,9 (dd), 37.2, 29.7, 19.8, 15.7

### Example 152:

### N-{3-[2-Chloro-4-(2,4-difluoro-phenylamino)-benzyl]-4-methyl-phenyl}-3-methanesulfonyl-propionamide (compound 252)

Compound 251 (0.22 g, 0.45 mmol) was dissolved in CH₂Cl₂ (5 mL). 3-Chloroperoxybenzoic acid (0.3 g, ca, 1.4 mmol) was added slowly and the mixture was stirred at room temperature for 1 h. Na₂S₂O₅ (0.34 g) was added and stirring was continued for 0.5 h. The mixture was filtered and the filtrate was stirred with K₂CO₃ for 0.5 h, MgSO₄ was added and the mixture was filtered. The filtrate was concentrated *in vacuo* and the crude product was purified by flash chromatography using a gradient of EtOAc/petroleum ether (40-60) 30:70 -> 100:0 as the eluent. This afforded the title compound as yellow oil. ¹³C NMR (CDCl₃) δ 196.0, 167.4, 159.2 (dd), 155.6 (dd), 148.0, 139.7, 135.3, 135.2, 133.8, 133.6, 131.9, 128.8, 124.5 (dd), 124.2 (dd), 122.6, 120.6, 116.2, 112.7, 111.6 (dd), 105.0 (dd), 50.1, 41.6, 29.4, 19,8

### Example 153:

### Ethanesulfonic acid {3-[2-chloro-4-(2,4-difluoro-phenylamino)-benzoyl]-4-methyl-phenyl}-amide (compound 253)

Compound 494 (0.045 g, 0.12 mmol) was dissolved in pyridine (0.3 mL), Ethanesulphonyl chloride (0.017 mL, 0.18 mmol) was added and the solution was stirred at room temperature for 1 h. The reaction mixture was concentrated *in vacuo* and the oil was dissolved in EtOAc and washed with H₂O. The water phase was extracted with EtOAc and the combined organic phases were dried (MgSO₄) filtered and concentrated *in vacuo.* The crude product was purified by flash chromatography using a gradient of EtOAc/petroleum ether (40-60) 15:85 -> 40:60 as the eluent. This afforded the title compound as yellow solid, ¹³C NMR (CDCl₃) δ 195.5, 159.3 (dd), 155.7 (dd), 148.3, 140.4, 135.3, 134.5, 134.5, 133.8, 132.5, 128.5, 124.6 (dd), 124.2 (dd), 123.2, 121.6, 116.1, 112.8, 111.6 (dd), 105.0 (dd), 45.9, 19.7, 8.1

### Example 154:

### N-{3-[2-Chloro-4-(2-4-difluoro-phenylamino)-benzoyl]-4-methyl-phenyl}-4-methoxy-benzenesulfonamide (compound 254)

The compound was prepared as described in the preparation of compound 253 using compound 494 (0.04g, 0.11 mmol) and 4-methoxybenzenesulphonyl chloride (0,033 g, 0.16 mmol) in pyridine (0.3 mL). The crude product was purified by flash chromatography using a gradient of EtOAc/petroleum ether (40-60) 10:90 -> 50:50 as the eluent. This afforded the title compound as yellow oil. ¹³C NMR (CDCl₃) δ 195.6, 163.1, 159.2 (dd), 155,6 (dd), 148.0, 139.8, 135.1, 134.9, 134.1, 133.5, 132.2, 130.3, 129.4, 128.7, 124.6, 124.5 (dd), 124.3 (dd), 122.9, 116.1, 114.2, 112.7, 111.6 (dd), 104.9 (dd), 55.6, 19.7

### Example 155:

### N-(5-{3-[2-Chloro-4-(2,4-difluoro-phenylamino)-benzoyl]-4-methyl-phenylsulfamoyl}-4-methyl-thiazol-2-yl)-acetamide (compound 255)

The compound was prepared as described in the preparation of compound 253 using compound 494 (0.042g, 0.11 mmol) and 2-acetamido-4-methyl-5-thiazolesulphonyl chloride (0.043 g, 0.17 mmol) in pyridine (0.3 mL). The crude product was purified by flash chromatography using a gradient of EtOAc/petroleum ether (40-60) 25:75 -> 0:100 as the eluent. This afforded the title compound as yellow oil. ¹³C NMR (CDCl₃) δ 195,9, 169.1, 159.7, 159.2 (dd), 155.6 (dd), 153.3, 148.4, 140.2, 135.5, 135.1, 133.7, 133.6, 132.3, 128.0, 125.5, 124.6 (dd), 124.2 (dd), 123.6, 122.0, 116.2, 112.7, 111.6 (dd), 104.9 (dd), 22.9, 19.8, 16.3

### Example 156:

### 5-Acetyl-2-chloro-N-{3-[2-chloro-4-(2,4-difluoro-phenylamino)-benzoyl]-4-methyl-phenyl}-benzenesulfonamide (compound 256)

The compound was prepared as described in the preparation of compound 253 using compound 494 (0.043g, 0.12 mmol) and 2-chloro-5-acetylbenzenesulphonyl chloride (0.044 g, 0.17 mmol) in pyridine (0.3 mL). The crude product was purified by flash chromatography using a gradient of EtOAc/petroleum ether (40-60) 10:90 -> 50:50 as the eluent. This afforded the title compound as yellow oil. ¹³C NMR (CDCl₃) δ 195.6, 195.3, 159.3 (dd), 155.7 (dd), 148,3, 7.40.2, 136.5, 136.2, 135.8, 135.6, 135.1, 133.5, 133.3, 132.8, 132.4, 132.1, 131.9, 128.4, 124.7 (dd), 124.6, 124.1 (dd), 122.7, 116.0, 112.7, 111.7 (dd), 105.0 (dd), 26.6, 19.7

### Example 157:

### Naphthalene-2-sulfonic acid {3-[2-chloro-4-(2,4-difluoro-phenylamino)-benzoy]-4-methyl-phenyl}-amide (compound 257)

The compound was prepared as described in the preparation of compound 253 using compound 494 (0.041g, 0.11 mmol) and naphthalene-2-sulphonyl chloride (0.037 g, 0.16 mmol) in pyridine (0.3 mL). The crude product was purified by flash chromatography using a gradient of EtOAc/petroleum ether (40-60) 10:90-> 50:50 as the eluent, This afforded the title compound as yellow oil. ¹³C NMR (CDCl₃) δ 195.4, 159.1 (dd), 155.5 (dd), 147.7, 139.8, 135.8, 135.4, 135.0, 135.0, 133.8, 133.3, 132.3, 132.0, 129.4, 129.4, 128.9, 128.8, 127.9, 127.5, 125.0, 124.4 (dd), 124.2 (dd), 123.4, 122.3, 116.1, 112.7, 111.6 (dd), 105.0 (dd), 19.7

### Example 158:

### N-{3-[2-Chloro-4-(2,4-difluoro-phenylamino)-benzoyl]-4-methyl-phenyl}-C-phenyl-methanesulfonamide (compound 258)

The compound was prepared as described in the preparation of compound 253 using compound 494 (0.041g, 0.11 mmol) and α-toluenesulphonyl chloride (0.031 g, 0.16 mmol) in pyridine (0.3 mL). The crude product was purified by flash chromatography using a gradient of EtOAc/petroleum ether (40-60) 10:90-> 50:50 as the eluent. This afforded the title compound as yellow oil. ¹³C NMR (CDCl₃) δ 195.5, 159.3 (dd), 155.7 (dd), 148.2, 140.2, 135.1, 134.4, 134.4, 133.7, 132.5, 130.8, 128.9, 128.9, 128.7, 128.4, 124.7 (dd), 124.2 (dd), 122.8, 121.2, 116.0, 112.8, 111.6 (dd), 105.0 (dd), 57.4, 19.7

### Example 159:

### 2-Methyl-acrylic acid 2-(3-{3-[2-chloro-4-(2,4-difluoro-phenylamino)-benzoyl]-4-methyl-phenyl}-ureido)-ethyl ester (compound 259)

Compound 494 (0.055 g, 0,15 mmol) was dissolved in dry pyridine (0.3 mL) and isocyanatoethyl methylacrylate (0.031 mL, 0.22 mmol) was added. The solution was stirred at room temperature for 1 h. H₂O was added and the water phase was extracted with EtOAc (x 2). The combined organic phases were washed with brine, dried (MgSO₄), filtered and concentrated *in vacuo.* The crude product was purified by flash chromatography using EtOAc/petroleum ether (40-60) 2:1 as the eluent. This afforded the title compound as oil. ¹³C NMR (CDCl₃) δ 196.5, 167.5, 159.2 (dd), 155.8, 155.6 (dd), 148.2, 139,7, 136.3, 1.35.9, 135.1, 133.9, 132.4, 132.0, 128.6, 126.1, 124.5 (dd), 124.2 (dd), 123.3, 121.2, 116.3, 112.7, 111.6 (dd), 104.9 (dd), 63.9, 39.3, 19.6, 18.2

### Example 160:

### 1-{3-[2-Chloro-4-(2,4-difluoro-phenylamino)-benzoyl]-4-methyl-phenyl}-3-(2-hydroxy-ethyl)-urea (compound 260)

Compound 259 (0.05 g, 0.095 mmol) was dissolved in EtOH (2,5 mL). 2 N NaOH (0.25 mL) was added and the solution was heated to reflux for 3 h. The reaction mixture was cooled to room temperature and saturated aqueous NaHCO₃ (2 mL) was added. H₂O and EtOAc were added and the phases were separated. The water phase was extracted with EtOAc. The combined organic phases were washed with brine, dried (MgSO₄), filtered and concentrated *in vacuo.* The crude product was purified by flash chromatography using EtOAc as the eluent. This afforded the title compound as a slightly coloured solid. ¹³C NMR (CD₃OD) δ 198.5, 161.1 (dd), 158.4, 157.9 (dd), 151.2, 141.0, 138.8, 136,1, 135.0, 132.7, 132.2, 128.9, 127.4 (dd), 126.0 (dd), 122.6, 120.9, 116.6, 112.9, 112.7 (dd), 105.8 (dd), 62.3, 43.3, 19.7

### Example 161:

### (3-{3-[2-Chloro-4-(2,4-difluoro-phenylamino)-benzoyl]-4-methyl-phenyl}-ureido)-acetic acid ethyl ester (compound 261)

Compound 494 (0.047 g, 0.13 mmol) was dissolved in pyridine (0.3 mL) and ethyl isocyanatoacetate (0.022 mL, 0.19 mmol) was added. The solution was stirred at room temperature for 2 h. Work up as described in the preparation of compound 259. The crude product was purified by flash chromatography using EtOAc/petroleum ether (40-60) 1:1 as the eluent. This afforded the title compound as a slightly coloured solid. ¹³C NMR (DMSO-d₆) δ 195.2, 170.7, 158.7 (dd), 155.7 (dd), 155.0, 149.0, 139.0, 137.8, 133.3, 131.4, 129.1, 127.0, 126.3 (dd), 124.3 (dd), 120.0, 118.1, 114.8, 111.9 (dd), 111.8, 105.0 (dd), 60.2, 41.3, 19.0, 14.0

### Example 162:

### 1-{3-[2-Chloro-4-(2-difluoro-phenylamino)-benzoyl]-4-methyl-phenyl}-3-(3-methoxy-phenyl)-urea(compound 262)

Compound 494 (0.03 g, 0.08 mmol) was dissolved in pyridine (0.2 mL) and 3-methoxyphenyl isocyanate (0,016 mL, 0.12 mmol) was added, The solution was stirred at room temperature for 1.5 h. Work up as described in the preparation of compound 259, The crude product was purified by flash chromatography using EtOAc/petroleum ether (40-60) 2:3 as the eluent. This afforded the title compound as a slightly coloured solid. ¹³C NMR (CD₃OD) δ 198.3, 161.7, 161.0 (dd), 157.9 (dd), 155.3, 151.2, 141.6, 141.2, 138.3, 136.2, 135.0, 132.7, 132.6, 130.6, 128.7, 127.4 (dd), 126.0 (dd), 122.8, 121.1, 116.7, 113.0, 112.2, 112.6 (dd), 109.5, 106.3, 105.8 (dd), 55.7, 19.7

### Examples 163:

### 1-{3-[2-Chloro-4-(2,4-difluoro-phenylamino)-benzoyl]-4-methyl-phenyl}-3-(3-trifluoromethyl-phenyl)-urea-(compound 263)

Compound 494 (0.03 g, 0.08 mmol) was dissolved in pyridine (0,2 mL) and 3-(trifluoromethyl)phenyl isocyanate (0.017 mL, 0.12 mmol) was added. The solution was stirred at room temperature for 1.5 h. Work up as described in the preparation of compound 259. The crude product was purified by flash chromatography using EtOAc/petroleum ether (40-60) 2:3 as the eluent. This afforded the title compound as a slightly coloured solid, ¹³C NMR (CD₃OD) 8 198.3, 161.1 (dd), 157,9 (dd), 155.0, 151.3, 141.5, 141.2, 138.1, 136.2, 135.0, 132.9, 132,8, 132.2 (q), 130.7, 128.8, 127.5 (dd), 126.0 (dd), 125.6 (q), 123.4, 123.0, 121.2, 120.0 (q), 116.6, 1.15.5 (q), 113.0, 112.6 (dd), 105.8 (dd), 19.7

### Example 164:

### 1-{3-[2-Chloro-4-(2,4-difluoro-phenylamino)-benzoyl]-4-methyl-phenyl}-3-propyl-urea (compound 264)

Compound 494 (0.03 g, 0,08 mmol) was dissolved in pyridine (0.2 mL) and *n-*propyl isocyanate (0,011 mL, 0.12 mmol) was added. The solution was stirred at room temperature for 18 h, Work up as described in the preparation of compound 259. The crude product was purified by flash chromatography using EtOAc/petroleum ether (40-60) 1:1 as the eluent. This afforded the title compound as a slightly coloured solid. ¹³C NMR (CD₃OD) δ 198.5, 161.0 (dd), 158.3, 157.9 (dd), 151.1, 141.0, 138.9, 136.1, 135.0, 132.7, 132.1, 128.9, 127.4 (dd), 126.0 (dd), 122.5, 120.9, 116.6, 112.9, 112.6 (dd), 105.8 (dd), 42.6, 24.4, 19.7, 11.6

### Example 165:

### 3-(3-{3-[2-Chloro-4-[2,4-difluoro-phenylamino)-benzoyl]-4-methyl-phenyl}-ureido)-propionic acid ethyl ester (compound 265)

Compound 494 (0.03 g, 0.08 mmol) was dissolved in pyridine (0.2 mL) and ethyl 3-isocyanatopropionate (0.016 mL, 0.12 mmol) was added. The solution was stirred at room temperature for 1,5 h. Work up as described in the preparation of compound 259. The crude product was purified by flash chromatography using EtOAc/petroleum ether (40-60) 1;1 as the eluent. This afforded the title compound as a slightly coloured solid. ¹³C NMR (CD₃OD) δ 198.4, 173.8, 161,0 (dd), 158.0, 157.8 (dd), 151.1, 141.0, 138,7, 136.1, 135.0, 132.7, 132.2, 128.8, 127.4 (dd), 126.0 (dd), 122.6, 120.9, 116.6, 112.9, 112.6 (dd), 105.8 (dd), 61.7, 36.7, 35.8, 19.7, 14.5

### Example 166:

### 1-{3-[2-Chloro-4-(2,4-difluoro-phenylamino)-benzoyl]-4-methyl-phenyl}-3-cyclohexyl-urea (compound 266)

Compound 494 (0.07 g, 0.18 mmol) was dissolved in pyridine (0,5 mL) and cyclohexyl isocyanate (0.036 mL, 0.28 mmol) was added. The solution was stirred at room temperature for 18 h. Work up as described in the preparation of compound 259. The crude product was purified by flash chromatography using a gradient of EtOAc/petroleum ether (40-60) 15:85 -> 60:40 as the eluent. This afforded the title compound as a slightly coloured solid. ¹³C NMR (CD₃OD) δ 198.5, 161.0 (dd), 157.9 (dd), 157.4, 151.1, 141.0, 138.9, 136.1, 135.0, 132.7, 132.0, 128.9, 127.4 (dd), 126.0 (dd), 122.5, 120.8, 116.6, 112.9, 112.6 (dd), 105.8 (dd), 49.8, 34.5, 26.7, 26.0, 19.7

### Example 167:

### 1-Allyl-3-{3-[2-chloro-4-(2,4-difluoro-phenylamino)-benzoyl]-4-methyl-phenyl}-urea (compound 267)

Compound 494 (0.07 g, 0.18 mmol) was dissolved in pyridine (0.5 mL) and allyl isocyanate (0.025 mL, 0,28 mmol) was added. The solution was stirred at room temperature for 18 h. Work up as described in the preparation of compound 259. The crude product was purified by flash chromatography using a gradient of EtOAc/petroleum ether (40-60) 15:85 -> 60:40 as the eluent. This afforded the title compound as a slightly coloured solid. ¹³C NMR (CD₃OD) δ 198.5, 161.1 (dd), 158.0, 157.9 (dd), 151.2, 141.0, 138.8, 136.6, 136.1, 135.0, 132.7, 132.2, 128.9, 127.5 (dd), 126.0 (dd), 122.6, 121.0, 116.6, 115.7, 112.9, 112.7 (dd), 105.8 (dd), 43.2, 19.7

### Example 168:

### 1-Benzyl-3-{3-[2-chloro-4-(2,4-difluoro-phenylamino)-benzyl]-4-methylphenyl}-urea (compound 268)

Compound 494 (0.07 g, 0.18 mmol) was dissolved in pyridine (0.5 mL) and benzyl isocyanate (0.035 mL, 0.28 mmol) was added. The solution was stirred at room temperature for 18 h. Work up as described in the preparation of compound 259. The crude product was purified by flash chromatography using a MeOH/CH₂Cl₂ 1:100 as the eluent. This afforded the title compound as slightly coloured solid. ¹³C NMR (DMSO-d₆) δ 195.3, 158.8 (dd), 155.8 (dd), 155.2, 149.1, 140.3, 139.1, 138.2, 3.33.4, 131.4, 129.0, 128.3, 127.1, 126.7, 126.4 (dd), 124,4 (dd), 120.1, 118.2, 114.9, 112.0 (dd), 111.9, 105.1 (dd), 42.7, 19.1

### Example 69:

### 1-{3-[2-Chloro-4-(2,4-difluoro-phenylamino)-benzoyl]-4-methyl-phenyl}-3-ethyl-urea (compound 269)

Compound 494 (0.04 g, 0.11 mmol) was dissolved in 1,4-dioxan (0.5 mL) and ethyl isocyanate (0.013 mL, 0.16 mmol) was added. The solution was stirred at room temperature for 18 h. Work up as described in the preparation of compound 259. The crude product was purified by flash chromatography using a gradient of EtOAc/petroleum ether (40-60) 30:70 -> 100:0 as the eluent. This afforded the title compound as an amorphous compound. ¹³C NMR (DMSO-d₆) δ 195.6, 159.0 (dd), 156.0 (dd), 155.3, 149.2, 139.3, 138.3, 133.7, 133.5, 131.5, 129.2, 127.5, 126.4 (dd), 124.5 (dd), 120.2, 118.4, 115.1, 11.2.1 (dd), 112.0, 105.1 (dd), 34.0, 19.1, 15.4

### Example 170:

### 1-{3-[2-Chloro-4-(2,4-difluoro-phenylamino)-benzoyl]-4-methyl-phenyl}-3-phenyl-urea (compound 270)

Compound 494 (0.03 g, 0.08 mmol) was dissolved in 1,4-dioxan (0,5 mL) and phenyl isocyanate (0.013 mL, 0.12 mmol) was added. The solution was stirred at 50°C for 18 h. Work up as described in the preparation of compound 259, The crude product was purified by flash chromatography using a gradient of EtOAc/petroleum ether (40-60) 15:85 -> 60:40 as the eluent. This afforded the title compound as an amorphous compound. ¹³C NMR (DMSO-d₆) δ 195.1, 158.7 (dd), 155.7 (dd), 152.3, 148.9, 139.3, 139.1, 137.2, 133.4, 131.5, 129.6, 128.7, 127.0, 126.2 (dd), 124.2 (dd), 121.8, 120.3, 118.4, 118.2, 118.1, 114.8, 111.9 (dd), 111.8, 105.0 (dd), 19.0

### Example 171:

### 1-Butyl-3-{3-[2-chloro-4-(2,4-difluoro-phenylamino)-benzoyl]-4-methyl-phenyl}-urea (compound 271)

Compound 494 (0.03 g, 0.08 mmol) was dissolved in 1,4-dioxan (0.5 mL) and n-butyl isocyanate (0.014 mL, 0.12 mmol) was added. The solution was stirred at 50°C for 18 h. Work up as described in the preparation of compound 259. The crude product was purified by flash chromatography using a gradient of EtOAc/petroleum ether (40-60) 15:85 -> 60:40 as the eluent, This afforded the title compound as a slightly coloured solid. ¹³C NMR (CD₃OD) δ 198.5, 161.0 (dd), 158.3, 157.9 (dd), 151.1, 141.0, 138.9, 136.1, 135.0, 132.7, 132.1, 128.9, 127.4 (dd), 126.0 (dd), 122.5, 120.9, 116.6, 112.9, 112.6 (dd), 105,8 (dd), 40.6, 33.3, 21.0, 19.7, 14.1

### Example 172:

### 1-{3-[2-Chloro-4-(2,4-difluoro-phenylamino)-benzoyl]-4-methyl-phenyl}-3-phenethyl-urea (compound 272)

Compound 494 (0.03 g, 0.08 mmol) was dissolved in 1,4-dioxan (0.5 mL) and 2-phenylethyl isocyanate (0.016 mL, 0.12 mmol) was added. The solution was stirred at 50 °C for 18 h. Work up as described in the preparation of compound 259. The crude product was purified by flash chromatography using a gradient of EtOAc/petroleum ether (40-60) 15:85 -> 60:40 as the eluent. This afforded the title compound as a slightly coloured solid. ¹³C NMR (CDCl₃) δ 196.5, 159.2 (dd), 156.0, 155.6 (dd), 148.1, 139.6, 138.9, 136.5, 135.1, 133.9, 132.2, 131.9, 128.7, 128,5, 126.4, 124.6 (dd), 124.2 (dd), 123.1, 121.1, 116.2, 112.7, 111.6 (dd), 104.9 (dd), 41.5, 36.3, 19.6

### Example 173:

### 2-(3-{3-[2-Chloro-4-(2,4-difluoro-phenylamino)-benzoyl]-4-methyl-phenyl}-ureido)-benzoic acid methyl ester (compound 273)

Compound 494 (0.03 g, 0.08 mmol) was dissolved in 1,4-dioxan (0.5 mL) and methyl 2-isocyanatobenzoate (0.021 mL, 0.12 mmol) was added. The solution was stirred at 50 °C for 24 h. Methyl 2-isocyanatobenzoate (0.01 mL, 0.06 mmol) was added. The solution was stirred at 50 °C for 24 h. Work up as described in the preparation of compound 259. The crude product was purified by flash chromatography using a gradient of EtOAc/petroleum ether (40-60) 10:90 -> 40:60 as the eluent. This afforded the title compound as yellow oil. ¹³C NMR (CDCl₃) δ 195.8, 169.0, 159.0 (dd), 155.4 (dd), 152.4, 147.6, 142.5, 139.5, 135.6, 135.1, 134.6, 133.6, 133.4, 132.1, 130.8, 129.4, 124.5 (dd), 124.1 (dd), 123.3, 121.7, 121.3, 119.8, 116.3, 114.3, 112.8, 111.5 (dd), 104.9 (dd), 52.2, 19.8

### Examples 174:

### 1-{3-[2-Chloro-4-(2,4-difluoro-phenylamino)-benzoyl-4-methyl-phenyl}-3-(3-cyano-phenyl)-urea (compound 274)

Compound 494 (0.03 g, 0.08 mmol) was dissolved in 1,4-dioxan (0.5 mL) and 3-cyanophenyl isocyanate (0.017g, 0.12 mmol) was added. The solution was stirred at 50 °C for 24 h. 3-Cyanophenyl isocyanate (0.09 g, 0.06 mmol) was added. The solution was stirred at 50 °C for 24 h. Work up as described in the preparation of compound 259. The crude product was purified by flash chromatography using MeOH/CH₂Cl₂ 1:100 as the eluent. This afforded the title compound as yellow oil. ¹³C NMR (DMSO-d₆) δ 195.1, 158.8 (dd), 155.8 (dd), 152.4, 149.2, 140.5, 139.3, 137.0, 133.5, 131.6, 130.2, 130.1, 126.9, 126.5 (dd), 125.4, 124.3 (dd), 123.0, 121.1, 120.8, 118.9, 118.8, 114.9, 112.0 (dd), 111.9, 111.6, 105.1 (dd), 19.1

### Example 175:

### 1-{3-[2-Chloro-4-(2,4-difluoro-phenylamino)-benzoyl]-4-methyl-phenyl}-3-isopropyl-urea (compound 275)

Compound 494 (0.03 g, 0.08 mmol) was dissolved in 1,4-dioxan (0.5 mL) and isopropyl isocyanate (0.012 mL, 0.12 mmol) was added. The solution was stirred at 50°C for 24 h. Isopropyl isocyanate (0,006 mL, 0.06 mmol) was added. The solution was stirred at 50°C for 24 h. Work up as described in the preparation of compound 259. The crude product was purified by flash chromatography using a gradient of EtOAc/petroleum ether (40-60) 15:85 -> 60:40 as the eluent. This afforded the title compound as a slightly coloured solid, ¹³C NMR (CD₃OD) δ 198.4, 160.9 (dd), 157.8 (dd), 157.4, 151,1, 140.9, 138.7, 136.1, 134.9, 132.6, 131.9, 128.7, 127.3 (dd), 125.9 (dd), 122.4, 120.7, 116.6, 112.9, 112.6 (dd), 105.7 (dd), 42.8, 23.4, 19.6

### Example 176:

### 1-{3-[2-Chloro-4-(2,4-difluoro-phenylamino)-benzoyl]-4-methyl-phenyl}-3-(4-methoxy-phenyl)-urea (compound 276)

Compound 494 (0.03 g, 0.08 mmol) was dissolved in 1,4-dioxan (0,5 mL) and 4-methoxyphenyl isocyanate (0.016 mL, 0.12 mmol) was added. The solution was stirred at 50 °C for 18 h. Work up as described in the preparation of compound 259. The crude product was purified by flash chromatography using MeON/CH₂Cl₂ 1:100 the eluent. This afforded the title compound as oil. ⁻¹³C NMR (DMSO-d₆) δ 195.2, 158.8 (dd), 155.8 (dd), 154.6, 152.7, 149.1, 139.2, 137.6, 133.5, 132.5, 131.5, 129.5, 127.0, 126.4 (dd), 124.4 (dd), 120.4, 120.2, 119.9, 118.5, 114.9, 114.0, 112.0 (dd), 111.9, 105.1 (dd), 55.2, 19.1

### Example 177:

### {3-[2-Chloro-4-(2,4-difluoro-phenylamino)-benzoyl]-4-methyl-phenyl}-carbamic acid benzyl ester (compound 277)

Compound 494 (0.06 g, 0.16 mmol) was suspended in dry CH₂Cl₂ (1.5 mL) under an argon atmosphere in a screw cap vessel. K₂CO₃ (0.044 g, 0.32 mmol) was added followed by benzyl chloroformate (0.046 mL, 0.032 mmol), The suspension was stirred for 18 h at room temperature. H₂O and CH₂Cl₂ were added and the phases were separated. The water phase was washed with CH₂Cl₂ (x 2) and the combined organic phases were washed with brine, dried (MgSO₄), filtered and concentrated *in vacuo.* The crude product was purified by flash chromatography using EtOAc/petroleum ether (40-60) 1:4 as the eluent. This afforded the title compound as yellow oil. ¹³C NMR (CD₃OD) δ 198.3, 161.0 (dd), 157.8 (dd), 155.8, 151.1, 141.1, 138.0, 136.2, 135.0, 132.8, 132.7, 129.5, 129.1, 129.0, 128.7, 127.3 (dd), 126.0 (dd), 122.3, 120.6, 116.7, 112.9, 112.6 (dd), 105.7 (dd), 67.6, 19.7

### Example 178:

### {3-[2-Chloro-4-(2,4-difluoro-phenylamino)-benzoyl]-4-methyl-phenyl}-carbamic acid allyl ester (compound 278)

Compound 494 (0.06 g, 0.16 mmol) was suspended in dry CH₂Cl₂ (1.5 mL) under an argon atmosphere in a screw cap vessel. K₂CO₃ (0.044 g, 0.32 mmol) was added followed by allyl chloroformate (0.034 mL, 0.032 mmol). The suspension was stirred for 48 h at room temperature. Work up as described in the preparation of compound 277. The crude product was purified by flash chromatography using a gradient of EtOAc/petroleum ether (40-60) 5:95 -> 30:70 as the eluent. This afforded the title compound as yellow oil. ¹³C NMR (CDCl₃) δ 195.8, 159.1 (dd), 155.5 (dd), 153.2, 147.7, 139.6, 135.4, 135.2, 133.6, 132.9, 132.3, 132.0, 129.3, 124.4 (dd), 124.2 (dd), 121.3, 119.7, 118.3, 116.3, 112.8, 111.6 (dd), 105.0 (dd), 65.9, 19.7

### Example 179:

### {3-[2-Chloro-4-(2,4-difluoro-phenylamino)-benzoyl]-4-methyl-phenyl}-carbamic acid ethyl ester (compound 279)

Compound 494 (0.039 g, 0.1 mmol) was suspended in dry CH₂Cl₂ (1 mL) under an argon atmosphere, K₂CO₃ (0,029 g, 0.21 mmol) was added followed by ethyl chloroformate (0.02 mL, 0.021 mmol). The suspension was stirred for 48 h at room temperature. Work up as described in the preparation of compound 277. The crude product was purified by flash chromatography using EtOAc/petroleum ether (40-60) 1:2 as the eluent. This afforded the title compound as oil. ¹³C NMR (CDCl₃) δ 196.0, 159.1 (dd), 155.6 (dd), 153.7, 147.9, 139.5, 135.6, 135.1, 133.6, 132.6, 131.9, 129.1, 124,4 (dd),124.3 (dd), 121.3, 119.8. 116.2, 112.7, 111.6 (dd), 104.9 (dd), 61.3, 19.7, 14.5

### Example 181:

### [2-Chloro-4-(2,4-difluoro-phenylamino)-phenyl]-[5-(3-hydroxy-butylamino)-2-methyl-phenyl]-methanone(compound 281)

Compound 494 (0.033 g, 0.09 mmol) was suspended in MeOH (1 mL). 3-Hydroxy-butyraldehyde (0.023 g, 0.27 mmol) and NaCN(BH₃) (0.055 g, 0.88 mmol) were added. The suspension was stirred at room temperature for 18 h. 3-Hydroxy-butyraldehyde (0,023 g, 0.27 mmol) and NaCN(BH₃) (0,055 g, 0,88 mmol) were added again and the suspension was stirred at room temperature for 24 h. The reaction mixture was concentrated *in vacuo.* The residue was dissolved in EtOAc and the organic phase was washed with brine. The water phase was extracted with EtOAc and the combined organic phases were dried (MgSO₄), filtered and concentrated *in vacuo.* The crude product was purified by flash chromatography using MeOH/CH₂Cl₂ 1:50 as the eluent. This afforded the title compound as oil. ¹³C NMR (CDCl₃) δ 196.7, 159,0 (dd), 155.4 (dd), 147.3, 146.0, 139.6, 135.0, 133.5, 132.0, 129.9, 126.7, 124.6 (dd), 124.0 (dd), 116.3, 115.9, 114.6, 112.8, 111.6 (dd), 104.9 (dd), 67.5, 42.1, 38.0, 24.0, 19.4

### Example 182:

### [2-Chloro-4-(2,4-difluoro-phenylamino)-phenyl]-(3'-hydroxymethyl-4-methyl-biphenyl-3-yl)-methanone (compound 282)

Compound 495 (0.039 g, 0.081 mmol) was dissolved in 1,2-dimethoxyethane (0.8 mL) in a screw cap vessel, 3-(Hydroxymethyl)phenylboronic acid (0.015 g, 0.097 mmol) and saturated aqueous NaHCO₃ (0.4 mL) were added. Argon was blown over the mixture and Pd(PPh₃)₄ (0.005 g, 0.004 mmol) was added. The reaction mixture was stirred at reflux temperature under an argon atmosphere for 2 h. H₂O and EtOAc were added and the water phase was extracted, with EtOAc (x 2). The combined organic phases were dried (MgSO₄) filtered and concentrated *In vacuo.* The crude product was purified by flash chromatography using EtOAc/petroleum ether (40-60) 1:1 as the eluent. This afforded the title compound as oil. ¹³C NMR (CDCl₃) δ 196.3, 159.1 (dd), 155.5 (dd), 147.8, 141.5, 140.6, 139.6, 138.3, 137.0, 135.3, 133.7, 131.8, 129.4, 129.1, 128.0, 126.3, 126.0, 125.6, 124.4 (dd), 124.3 (dd), 116.3, 112.8, 111.6 (dd), 104.9 (dd), 65.3, 20.1

### Example 183:

### [2-Chloro-4-(2,4-difluoro-phenylamino)-phenyl]-(3'-hydroxy-4-methyl-biophenyl-3-yl)-methanone (compound 283)

The compound was prepared as described in the preparation of compound 282. Starting materials were compound 495 (0.039 g, 0.081 mmol), 3-hydroxyphenylboronic acid (0.013 g, 0.097 mmol) in 1,2-dimethoxyethane (0.8 mL), saturated aqueous NaHCO₃ (0.4 mL) and Pd(PPh₃)₄ (0.005 g, 0.004 mmol). The crude product was purified by flash chromatography using EtOAc/petroleum ether (40-60) 1:2 as the eluent. This afforded the title compound as yellow oil. ¹³C NMR (CDCl₃) δ 196.7, 159.2 (dd), 156.1, 155.5 (dd), 147.9, 141.8, 139.4, 138.1, 137.1, 135.3, 133.7, 131.8, 130.0, 129.5, 129.3, 128.1, 124.3 (m), 119.4, 17.6.3, 114.5, 114.0, 112.8, 111.6 (dd), 104.9 (dd), 20.1

### Example 184:

### [2-Chloro-4-(2,4-difluoro-phenylamino)-phenyl]-(4'-methoxy-4-methyl-biphenyl-3-yl)-methanone (compound 284)

The compound was prepared as described in the preparation of compound 282. Starting materials were compound 495 (0.042 g, 0.087 mmol), 4-methoxyphenylboronic acid (0.016 g, 0.11 mmol) in 1,2-dimethoxyethane (1 mL), saturated aqueous NaHCO₃ (0.5 mL) and Pd(PPh₃)₄ (0.005 g, 0.004 mmol). The crude product was purified by flash chromatography using EtOAc/petroleum ether (40-60) 1:5 as the eluent. This afforded the title compound as yellow oil. ¹³C NMR (CDCl₃) δ 196.4, 159.3, 159.1 (dd), 155.5 (dd), 147.7, 139.4, 138.1, 136.3, 135.2, 133.6, 132.7, 131.8, 129.6, 129.0, 128.0, 127.8, 124.4 (dd), 124.2 (dd), 116.3, 114.3, 112.8, 111.6 (dd), 104.9 (dd), 55.4, 20.1

### Example 185:

### N-{3'-[2-Chloro-4-(2,4-difluoro-phenylamino)-benzoyl]-4'-methyl-biphenyl-3-yl}-acetamide (compound 285)

The compound was prepared as described in the preparation of compound 282. Starting materials were compound 495 (0.048 g, 0.1 mmol), 3-acetamidophenylboronic acid (0.021 g, 0.1.2 mmol) in 1,2-dimethoxyethane (1 mL), saturated aqueous NaHCO₃ (0.5 mL) and Pd(PPh₃)₄ (0.006 g, 0.005 mmol). The crude product was purified by flash chromatography using EtOAc/petroleum ether (40-60) 1:1 as the eluent. This afforded the title compound as a slightly coloured solid. ¹³C NMR (DMSO-d₆) δ 195.0, 168.4, 158.8 (dd), 155.8 (dd), 149.4, 140.0, 139.9, 139.6, 137.5, 135.8, 133.9, 133.8, 131.9, 129.4, 128.7, 126.6, 126.5, 126.4 (dd), 124.3 (dd), 121.2, 118.2, 116.9, 115.0, 112.0 (dd), 111.9, 105.1 (dd), 24.0, 19.4

### Examples 186:

### [2-Chloro-4-(2,4-difluoro-phenylamino)-phenyl]-(4-methyl-3'-trifluoromethoxybiphenyl-3-yl)-methanone (compound 286)

The compound was prepared as described in the preparation of compound 282. Starting materials were compound 495 (0.048 g, 0.1 mmol), 3-(trifluoromethoxy)benzeneboronic acid (0.025 g, 0.12 mmol) in 1,2-dimethoxyethane (1.2 mL), saturated aqueous NaHCO₃ (0.6 mL) and Pd(PPh₃)₄ (0.006 g, 0.005 mmol). The crude product was purified by flash chromatography using a gradient of EtOAc/petroleum ether (40-60) 2:98 -> 10:90 as the eluent. This afforded the title compound as yellow oil.

### Example 188:

### [2-Chloro-4-(2,4-difluoro-phenylamino)-phenyl]-(3',4',5'-trifluoro-4-methyl-biphenyl-3-yl)-methanone (compound 288)

The compound was prepared as described in the preparation of compound 282. Starting materials were compound 495 (0.055 g, 0.11 mmol), 3,4,5-trifluorobenzeneboronic add (0.024 g, 0.14 mmol) in 1,2-dimethoxyethane (1.2 mL), saturated aqueous NaHCO₃ (0.6 mL) and Pd(PPh₃)₄ (0.007 g. 0.006 mmol), The crude product was purified by flash chromatography using a gradient of EtOAc/petroleum ether (40-60) 2:98 -> 10:90 as the eluent. This afforded the title compound as yellow oil. ¹³C NMR (CDCl₃) δ 195.8, 159.3 (dd), 155.6 (dd), 151.4 (m), 148.1, 140.1, 139.3 (dt), 138.0, 136.3 (m), 135.5, 135.3, 133.8, 132.1, 128.9, 127.5, 124.6 (dd), 124.2 (dd), 116.2, 112.7, 111.6 (dd), 110.9 (m), 105.0 (dd), 20.1

### Example 189:

### [2-Chloro-4-(2,4-difluoro-phenylamino)-phenyl]-(3',4'-dimethoxy-4-methyl-biphenyl-3-yl)-methanone (289)

The compound was prepared as described in the preparation of compound 282. Starting materials were compound 495 (0.055 g, 0.11 mmol), 3,4,-dimethoxybenzeneboronic acid (0.025 g, 0.15 mmol) in 1,2-dimethoxyethane (1.2 mL), saturated aqueous NaHCO₃ (0.6 mL) and Pd(PPh₃)₄ (0.007 g, 0.006 mmol). The crude product was purified by flash chromatography using a gradient of EtOAc/petroleum ether (40-60) 12:88 → 50:50 as the eluent. This afforded the title compound as yellow oil, ¹³C NMR (CDCl₃) δ 196.4, 159.1 (dd), 155.5 (dd), 149.2, 148.7, 147.8, 139.5, 138.4, 136.3, 135.2, 133.7, 133.2, 131.7, 129.3, 129.1, 127.8, 124.3 (m), 119.3, 116.3, 112.7, 111.6 (dd), 111.5, 110.4, 105.0 (dd), 56.0, 20.0

### Example, 190:

### 3'-[2-Chloro-4-(2,4-difluoro-phenylamino)-benzoyl]-4'-methyl-biphenyl-3-carbonitrile (compound 290)

The compound was prepared as described in the preparation of compound 282. Starting materials were compound 495 (0.057 g, 0.12 mmol), 3-cyanobenzeneboronic acid (0.021 g, 0.14 mmol) in 1,2-dimethoxyethane (1.2 mL), saturated aqueous NaHCO₃ (0.6 mL) and Pd(PPh₃)₄ (0.007 g, 0.006 mmol), The crude product was purified by flash chromatography using a gradient of EtOAc/petroleum ether (40-60) 2:3 -> 4:1 as the eluent. This afforded the title compound as yellow oil. ¹³C NMR (CDCl₃) δ 195.9, 159.2 (dd), 155.6 (dd), 148.1, 141.4, 140.1, 138.1, 136.2, 135.3, 133.7, 132.2, 131.4, 130.8, 130.5, 129.7, 129.2, 128.9, 127.7, 124.5 (dd), 124.2 (dd), 118.8, 116.2, 113.0, 112.8, 111.6 (dd), 105.0 (dd), 20.1

### Preparation 96:

### 3-[2-Chloro-4-(2,4-difluoro-phenylamino)-benzoyl]-4-methyl-benzenesulfonyl chloride (compound 496)

Compound 494 (1.03 g, 2.76 mmol) was dissolved in CH₃CN (65 mL) by heating. The solution was cooled to room temperature and concentrated HCl (37%, 1.2 mL, ca. 14 mmol) and AcOH (99%, 2.3 mL) were added. The solution was cooled on an ice bath and the internal temperature was monitored. NaNO₂ (0.23 g, 3.31 mmol) dissolved in H₂O (0.6 mL) was added over 15 minutes under stirring. The internal temperature did not exceed 2 °C, The mixture was stirred on an ice bath for 20 minutes after which SO₂ gas was bubbled through the mixture for 45 minutes under stirring on ice bath. CuCl (0.37 g, 3.8 mmol) was added followed by Cucl₂·2H₂O (0.59 g, 3.46 mmol) dissolved in H₂O (0.6 mL). The mixture was stirred at 0 °C for 10 minutes followed by stirring at room temperature for 1 h. The mixture was concentrated *in vacuo* and dissolved in EtOAc and H₂O. The phases were separated and the water phase was extracted with EtOAc, The combined organic phases were washed with brine, dried (MgSO₄) filtered and concentrated *in vacuo.* The crude product was purified by flash chromatography using EtOAc/petroleum ether (40-60) 1:4 as the eluent to afford the title compound as a pale brown crystalline compound.

### Example 191:

### 3-[2-Chloro-4-(2,4-difluoro-phenylamino)-benzoyl]-N-(2-hydroxy-ethyl)-4-methyl-benzenesulfonamide (compound 291)

Compound 496 (0.07 g, 0.15 mmol) was dissolved in pyridine (0.4 mL) and ethanolamine (0.011 mL, 0.18 mmol) was added. The solution was kept at room temperature for 1, h after which it was concentrated *in vacuo.* The residue was dissolved in EtOAc and H₂O and the phases were separated. The water phase was extracted with EtOAc (x 2) and the combined organic phases were washed with brine, dried (MgSO₄), filtered and concentrated *in vacuo.* The crude product was purified by flash chromatography using a gradient of EtOAc/petroleum ether (40-60) 3:7 -> 1:0 as the eluent. This afforded the title compound as a solid. ¹³C NMR (CD₃OD) δ 196.5, 161.2 (dd), 158.0 (dd), 151.9, 143.3, 142.0, 139.5, 136.5, 135.2, 133.2, 129.9, 128.2, 127.8, 127.8 (dd), 125.7 (dd), 116.5, 113.1, 112.7 (dd), 105.8 (dd), 61.8, 46.3, 20.3

### Example 192;

### 3-[2-Chloro-4-(2,4-difluoro-phenylamino)-benzoyl]-4-methyl-N-(2-morpholin-4-yl-ethyl)-benzensulfonamide (compound 292)

The compound was prepared and worked up as described in the preparation of compound 291. Starting materials were compound 496 (0.081 g, 0.18 mmol) and 4-(2-aminoethyl)morpholine (0.028 mL, 0.21 mmol) in pyridine (0.5 mL). The crude product was purified by flash chromatography using a gradient of EtOAc/petroleum ether (40-60) 1:1 -> 1:0 as the eluent. This afforded the title compound as an amorphous compound. ¹³C NMR (CDCl₃) δ 194.5, 159.5 (dd), 155.9 (dd), 148.8, 142.8, 140.5, 137.0, 135.4, 134.0, 132.1, 128.7, 127.9, 127.3, 125.0 (dd), 123,9 (dd), 116.0, 112.9, 111.7 (dd), 105.1 (dd), 66.8, 56.2, 53.0, 38.9, 20.4

### Example 193:

### N-Allyl-3-[2-chloro-4-(2,4-difluoro-phenylamino)-benzoyl]-4-methyl-benzenesulfonamide (compound 293)

The compound was prepared and worked up as described in the preparation of compound 291. Starting materials were compound 496 (0.071 g, 0.16 mmol) and allylamine (0.014 mL, 0.19 mmol) in pyridine (0.4 mL). The crude product was purified by flash chromatography using a gradient of EtOAc/petroleum ether (40-60) 1:4 -> 2:3 as the eluent. This afforded the title compound as oil. ¹³C NMR (CDCl₃) δ 194.6, 159.4 (dd), 155.8 (dd), 148.7, 143.0, 140.4, 137.4, 135.5, 133.9, 132.8, 132.1, 128.8, 128.0, 127.5, 124.9 (dd), 124.0 (dd), 118.0, 116.1, 112.9, 111.7 (dd), 105.0 (dd), 45.8, 20.4

### Example 194:

### N-(2-{3-[2-Chloro-4-(2,4-difluoro-phenylamino)-benzoyl]-4-methyl-benzenesulfonylamino}-ethyl)-acetamide (compound 294)

The compound was prepared and worked up as described in the preparation of compound 291. Starting materials were compound 496 (0.075 g, 0.17 mmol) and *N*-acetylethylenediamine (0.019 mL, 0.2 mmol) in pyridine (0.4 mL). The crude product was purified by flash chromatography using a gradient of DCM/MeOH 95:5 -> 80:20 as the eluent. This afforded the title compound as oil. ¹³C NMR (CDCl₃) δ 194.6, 171.5, 159.5 (dd), 155.9 (dd), 149.0, 142.7, 140.5, 137.1, 135.6, 134.2, 132.2, 128.6, 127.4, 127.2, 125.2 (dd), 123.9 (dd), 116.1, 112.7, 111.7 (dd), 105.0 (dd), 43.2, 39.3, 23.1, 20.3

### Example 195:

### 3-[2-Chloro-4-(2,4-difluoro-phenylamino)-benzoyl]-4-methyl-N-propyl-benzenesulfonamide (compound 295)

The compound was prepared and worked up as described in the preparation of compound 291. Starting materials were compound 496 (0.074 g, 0.16 mmol) and n-propylamine (0,016 mL, 0.2 mmol) in pyridine (0.4 mL). The crude product was purified by flash chromatography using a gradient of EtOAc/petroleum ether (40-60) 1:4 -> 3:2 as the eluent. This afforded the title compound as oil. ¹³C NMR (CDCl₃) δ 194.7, 159.4 (dd), 155.8 (dd), 148.6, 142.8, 140.3, 137.4, 135.5, 133.9, 132.1, 128.8, 128.1, 127.5, 124.9 (dd), 124.0 (dd), 116.1, 112.9, 111.7 (dd), 105.0 (dd), 45.0, 22.9, 20.4, 11.1

### Example 196:

### 3-[2-Chloro-4-(2,4-difluoro-phenylamino)-benzoyl]-N-(2,3-dihydroxy-propyl)-4-methyl-benzenesulfonamide (compound 296)

The compound was prepared and worked up as described in the preparation of compound 291. Starting materials were compound 496 (0.073 g, 0.16 mmol) and 3-amino-1,2-propanediol (0.017 g, 0.19 mmol) in pyridine (0.4 mL). The crude product was purified by flash chromatography using a gradient of EtOAc/petroleum ether (40-60) 1:1 -> 1:0 as the eluent. This afforded the title compound as oil, ¹³C NMR (CDCl₃) δ 194.8, 159.5 (dd), 155.8 (dd), 149.0, 142.7, 140.5, 136.9, 135.6, 134.2, 132.2, 128.7, 127.4, 127.2, 125.1 (bd), 123.9 (dd), 116.1, 112.7, 111.7 (dd), 105.0 (dd), 70.4, 64.0, 45.4, 20.3

### Example 197:

### 3-[2-Chloro-4-(2,4-difluoro-phenylamino)-benzoyl]-N-(2-methoxy-ethyl)-4-methyl-benzenesulfonamide (compound 297)

The compound was prepared and worked up as described in the preparation of compound 291, Starting materials were compound 496 (0.03 g, 0.066 mmol) and 2-methoxy-ethylamine (0.007 mL, 0.08 mmol) in pyridine (0.15 mL). The crude product was purified by flash chromatography using EtOAc/petroleum ether (40-60) 3:2 as the eluent. This afforded the title compound as oil. ¹³C NMR (CDCl₃) δ 194.5, 159.4 (dd), 155.8 (dd), 148.6, 142.9, 140.3, 137.3, 135.5, 134.0, 132.1, 128.7, 128.1, 127.5, 124.9 (dd), 124.0 (dd), 116.1, 112.9, 111.7 (dd), 105.0 (dd), 70.4, 58.8, 42.9, 20.4

### Preparation 97:

### 2-Methyl-5-nitro-benzoic acid methyl ester (compound 497)

Acetyl chloride (15 ml) was added to MeOH (500 ml) at room temperature. After 10 min, 2-methyl-5-nitro-benzoic acid (25.00 g, 138.00 mmol) was added. The reaction solution was heated for reflux for 18 h. The solution was then concentrated *in vacuo,* The residue was dissolved in diethyl ether and washed with H₂O and saturated aqueous solution of NaHCO₃ respectively. The organic phase was dried over MgSO₄ and concentrated *in vacuo* to provide the title compound as a white solid.

### Preparation 98:

### 5-Amino-2-methyl-benzoic acid methyl ester (compound 498)

Compound 497 (26.5 g, 135.78 mmol) in ethanol (200 ml) was hydrogenated under 1 atmosphere at room temperature in the presence of Pd/C 5% (2.00 g) for 3 h. After reaction, the catalyst was filtered off. The filtrate was concentrated *in vacuo,* providing the title compound as a brownish oil,

### Preparation 99:

### 5-Hydroxy-2-methyl-benzoic acid methyl ester (compound 499)

To 2 N H₂SO₄ (200 ml) was dropwise added NaNO₂ (11.50 g, 167.00 mmol) in H₂O (100 ml) at 0°C. After being stirred at the same temperature for 20 min, the reaction mixture was heated for reflux for 2 h. Then the solution was cooled to room temperature and stirred at the same temperature overnight. The mixture was extracted three times with CHCl₃. The combined organic phases were dried over MgSO₄ and concentrated *in vacuo.* The residue was purified by chromatography (CH₂Cl₂/ethyl acetate 50:1), furnishing the title compound as a red solid.

### Preparation 100:

### 5-(4-Methoxy-benzyloxy)-2-methyl-benzoic acid methyl ester (compound 500)

A mixture of compound 499 (4.4 g, 26.48 mmol), 4-methoxybenzyl chloride (4.4 g, 28.09 mmol), K₂CO₃ (4.4 g, 31.83 mmol) and NaI (20 mg) was heated for reflux for 3 h. After reaction, the solid was filtered off. The filtrate was concentrated *in vacuo.* The residue was purified by chromatography (petroleum ether/CH₂Cl₂ 2:1, then CH₂Cl₂), giving the title compound as a yellow solid.

### Preparation 101:

### [5-(4-Methoxy-benzyloxy)-2-methyl-phenyl]-methanol (compound 501)

To a solution of compound 500 (6.0 g, 21.00 mmol) in CH₂Cl₂ (100 ml) at -78 °C was added DIBAL-H (1 M in n-hexane, 55 ml, 55 mmol). The solution was warmed to room temperature and stirred for 1 h. Then the reaction solution was quenched with saturated aqueous solution of NH₄Cl. The mixture was filtered and washed with acetone. The combined liquids were concentrated *in vacuo* to remove acetone and CH₂Cl₂. The aqueous mixture was extracted three times with CH₂Cl₂. The combined organic phases were dried over MgSO₄ and concentrated *in vacuo,* furnishing the title compound as greyish solid.

### Preparation 102:

### 5-(4-Methoxy-benzyloxy)-2-methyl-benzaldehyde (compound 502)

To a solution of compound 501 (5.06 g, 19.59 mmol) in CH₂Cl₂ (100 ml) was added Dess-Martin periodinane (8.36 g, 19.71 mmol) at room temperature over a 20 min period. The mixture was then stirred for 1 h. After total conversion, the reaction mixture was concentrated *in vacuo* together with silica gel. The residue was purified by chromatography (CH₂Cl₂), furnishing the title compound as a yellow solid.

### Preparation 103:

### (4-Bromo-2-nitro-phenyl)-[5-(4-methoxy-benzyloxy)-2-methyl-phenyl]-methanol (compound 503)

To a solution of 1,4-dibromo-2-nitro-benzene (5.89 g, 21.00 mmol) in THF (300 ml) was added a solution of PhLi (1.8 M in cyclohexane/diethyl ether 7:3, 12.8 ml, 23.1 mmol) at -110°C. The mixture was stirred at the same temperature for 1 h. Compound 502 (4.45 g, 17.4 mmol) in THF (100 ml) was dropwise added to the mixture. The reaction mixture was then allowed to warm to -78°C. and stirred at the same temperature for 4 h. Afterwards, the reaction mixture was quenched with saturated aqueous solution of NH₄Cl, the aqueous phase was extracted once with diethyl ether. The combined organic phases were dried over MgSO₄ and concentrated *in vacuo.* The residue was purified by chromatography (petroleum ether/ethyl acetate 5:1), furnishing a reddish foam.

### Preparation 104:

### (4-Bromo-2-nitro-phenyl)-[5-(4-methoxy-benzyloxy)-2-methyl-phenyl]-methanone (compound 504)

To a solution of compound 503 (6.47 g, 14.12 mmol) was added Dess-Martin periodinane (8.00 g, 18.86 mmol) in one portion at room temperature. After stirring at room temperature for 3 h, the reaction mixture was purified by chromatography (petroleum ether/ethyl acetate 10:1) to provide the title compound as a brownish oil.

### Preparation 106:

### Toluene-4-sulfonic acid 2,2-dimethyl-[1,3]dioxolan-4-ylmethyl ester (compound 506)

2,2-Dimethyl-4-hydroxymethyl-1,3-dioxolan (3.96 g, 30 mmol) and triethyl amine (5 mL) were dissolved in CH₂Cl₂ (50 mL). *p*-Toluenesulphonyl chloride (3.8 g, 20 mmol) was added. The solution was stirred for 3 h after which 4-dimethylaminopyridine (0.05 g, 0.04 mmol) was added. The solution was stirred for 0.5 h after which the solution was concentrated *in vacuo.* The residue was taken up in CH₂Cl₂ and the organic phase was washed with H₂O, dried (MgSO₄), filtered and concentrated *in vacuo.* The crude product was purified by flash chromatography to afford the title compound.

### Examples 206:

### [4-(2,4-Difluoro-phenylamino)-2-nitro-phenyl]-[5-(4-methoxy-benzyloxy)-2-methyl-phenyl]-methanone (Compound 306)

2,4-Difluoro-phenylamine (26 µL, 0.25 mmol) was dissolved in dry 1,4-dioxane (3 mL) in a vial under an argon atmosphere. Compound 504 (114 mg, 0.25 mmol) was added and dissolved in the solvent. Rac-BINAP (7.0 mg, 0.012 mmol), Pd₂(dba)₃ (7.0 mg, 0.008 mmol) and Cs₂CO₃ (114 mg, 0.35 mmol) were added, and the reaction mixture was stirred under an argon atmosphere at 100°C for 16 h. The reaction mixture was filtered through Celite and then purified by continuous gradient flash chromatography using EtOAc/petroleum ether (40-60) (v:v = 1:9 to 1:3) as the eluent to afford the title compound as curry yellow solid. ¹³C NMR (CDCl₃) δ 194.5, 159.6 (dd), 159.5, 156.1, 155.9 (dd), 149.6, 147.1, 137.2, 132.8, 131.8, 131.7, 129.2, 128.5, 126.8, 124.8 (dd), 123.8 (dd), 117.9, 117.1, 114.1, 111.8 (dd), 109.5, 105.2 (dd), 70.1, 55.3, 19.9

### Preparation 107:

### [4-(2,4-Difluoro-phenylamino)-2-nitro-phenyl]-(5-hydroxy-2-methyl-phenyl)-methanone (Compound 507)

Compound 306 (90 mg, 0.178 mmol) was dissolved in dry DCM (5 mL). TFA (5 mL) was added, and the reaction mixture was stirred at RT for 1h and then concentrated *in vacuo,* affording the crude product as beige/off-white solid.

### Example 207:

### [4-(2,4-Difluoro-phenylamino)-2-nitro-phenyl]-[5-(3-hydroxy-propoxy)-2-methyl-phenyl]-methanone (Compound 307)

Compound 507 (66 mg, 0.17 mmol) was dissolved in acetonitrile (3 mL) in a reaction vial. 3-chloro-propan-1-ol (22 µL, 0.26 mmol), K₂CO₃ (36 mg, 0.26 mmol) and NaI (cat. amount) were added. The reaction vial was flushed with argon, closed and then stirred at 170°C for 15 min, in a microwave oven. The reaction mixture was allowed to cool to RT and then poured into a mixture of EtOAc/water. The layers were separated and the organic phase was dried (MgSO₄), filtered and concentrated *in vacuo* affording the crude product. Purified by continuously gradient flash chromatography using EtOAc/petroleum ether (40-60) (v:v= 1:9 to 1:1) as the eluent, affording the title compound as a yellow oil. ¹³C NMR (CDCl₃) δ 194.4, 159.7 (dd), 156.2, 155.9 (dd), 149.9, 147.4, 137.4, 132.8, 131.9, 131.6, 126.5, 125.0 (dd), 123.8 (dd), 117.8, 117.5, 116.4, 111.9 (dd), 109.4, 105.2 (dd), 65.8, 60.2, 31.9, 19.8

### Example 208:

### [2-Amino-4-(2,4-difluoro-phenylamino)-phenyl]-[5-(3-hydroxy-propoxy)-2-methyl-phenyl]-methanone (compound 308)

Compound 307 (46 mg, 0.1 mmol) was dissolved in EtOH (5 mL), flushed with argon and added Pd/C (cat. amount). The flask was purged with H₂ for 2 min. and stirred under an H₂-atmosphere at RT for 1 h. The reaction mixture was filtered through Celite and concentrated *in vacuo.* Purified by continuously gradient flash chromatography using DCM/MeOH (v:v= 100:0 to 95:5) as the eluent, affording the title compound as a yellow oil. ¹³C NMR (DMSO-d₆) δ 196.3, 158.9 (dd), 156.2, 155.6 (dd), 154.2, 150.8, 142.2, 135.6, 131.1, 127.0 (dd), 125.2, 124.8 (dd), 114.5, 112.2, 111.6 (dd), 109.7, 104.8 (dd), 104.0, 96.9, 64,6, 57.2, 32.1, 17.9

### Example 209:

### [4-(2,4-Difluoro-phenylamino)-2-nitro-phenyl]-[2-methyl-5-(2-morpholin-4-yl-ethoxy)-phenyl]-methanone (compound 309)

Compound 507 (50 mg, 0.13 mmol) was dissolved in acetonitrile (3 mL) in a reaction vial. 4-(2-Chloro-ethyl)-morpholine hydrochloride (48 mg, 0.26 mmol), K₂CO₃ (72 mg, 0.52 mmol) and NaI (cat. amount) were added. The reaction vial was flushed with argon, closed and then stirred at 170 °C for 10 min. in a microwave oven, followed by reflux in a oil bath for 40 h. The reaction mixture was added EtOAc (30 mL) and washed with M₂O (2 x 20 mL), brine (2 x 20 mL). The organic phase was dried (Na₂SO₄), filtered, concentrated *in vacuo* and purified by continuously gradient flash chromatography using EtOAc/petroleum ether (40-60)(v:v= 2:1 to 100:0) as the eluent, affording the title compound as a yellow oil. ¹³C NMR (CDCl₃) δ 194,3, 159.7 (dd), 156.1, 155.9 (dd), 149.9, 147.3, 137.4, 132.8, 131.9, 131.8, 126.7, 124.9 (dd), 123.8 (dd), 117.9, 117.6, 116.6, 111.9 (dd), 109.4, 105.2 (dd), 66.8, 65.7, 57.5, 54.0, 19.8

### Example 210:

### [2-Amino-4-(2,4-difluoro-phenylamino)-phenyl]-[2-methyl-5-(2-morpholin-4-yl-ethoxy)-phenyl]-methanone (compound 310)

The reaction was carried out as described in the preparation of compound 308, using compound 309 (18 mg, 0.036 mmol) as the nitro-compound. Stirred under an H₂-atmosphere for 16 h. Purified by flash chromatography using EtOAc as the eluent, affording the title compound as oil. ¹³C NMR (CDCl₃) δ 198.4, 1.56.4, 153.5, 149.7, 141.9, 137.1, 131.4, 126.9, 124.8 (dd), 115.5, 112.8, 112.2, 111.3 (dd), 105.1, 104.8, 104.7 (dd), 98.9, 66.9, 66.1, 57.7, 54.1, 18.4

### Example 211:

### [4-(2,4-Difluoro-phenylamino)-2-nitro-phenyl]-[5-(2,2-dimethyl-[1,3]dioxolan-4-ylmethoxy)-2-methyl-phenyl]-methanone (compound 311)

Compound 507 (50 mg, 0.13 mmol) was dissolved in acetonitrile (3 mL) in a reaction vial. Compound 506 (75 mg, 0.26 mmol), K₂CO₃ (36 mg, 0.26 mmol) and NaI (cat. amount) was added. The reaction vial was flushed with argon, closed and then stirred at 170 °C for 10 min. in a microwave oven, followed by reflux in a oil bath for 40 h. The reaction mixture was added EtOAc (30 mL) and washed with H₂O (2 x 20 mL), brine (2 x 20 mL). The organic phase was dried (Na₂SO₄), filtered, concentrated *in vacuo* and purified by continuously gradient flash chromatography using EtOAc/petroleum ether (40-60)(v:v= 1:9 to 1:3) as the eluent, affording the title compound as a yellow oil, ¹³C NMR (CDCl₃) δ 194.3, 159.7 (dd), 156.0, 155.9 (dd), 149.9, 147.3, 137.5, 132.8, 132.0, 131.8, 126.6, 124.9 (dd), 123.8 (dd), 117.9, 117.3, 116.6, 111.9 (dd), 109.9, 109.5, 105.2 (dd), 73.9, 69.2, 66.7, 26.8, 25.3, 19.8

### Example 212:

### [4-(2,4-Difluoro-phenylamino)-2-nitro-phenyl]-[5-(2,3-dihydroxy-propoxy)-2-methyl-phenyl]methanone(compound 312)

Compound 311 (48 mg, 0.01 mmol) was dissolved in TFA:H₂O (3:1, 8 mL) and stirred at RT for 2 h. The reaction mixture was concentrated *in vacuo* and purified by flash chromatography using EtOAc/petroleum ether (40-60)(v:v= 1:1) as the eluent, affording the title compound as a yellow oil. ¹³C NMR (DMSO-d₆) δ 193.5, 156.3, 150.1, 148.8, 137.5, 132.5, 132.3, 129.3, 126.6 (dd), 123.9 (dd), 122.9, 117.3, 116.1, 115.6, 112.1 (dd), 108.6, 105.1 (dd), 69.8, 62.5,119.0

### Example 213

### [2-Amino-4-(2,4-difluoro-phenylamino)-phenyl]-[5-(2,3-dihydroxy-propoxy)-2-methy-phenyl]-methanone (compound 313)

The reaction was carried out as described in the preparation of compound 308, using compound 312 (48 mg, 0.096 mmol) as the nitro-compound. Stirred under an H₂-atmosphere for 16 h. Purified by flash chromatography using EtOAc/petroleum ether (40-60) (1:1) as the eluent, affording the title compound as colourless oil. ¹H NMR (DMSO-d₆) δ 8.32 (s,1H), 7.42 - 7.29 (m,2H), 7.15 (d,1H), 7.09 (m,1H), 6.89 (dd,1H), 6.82 (d,1H), 6,66 (d,1H), 6.02 - 5.92 (m,2H), 3.97 (dd,1H), 3.87 - 3.30 (m,4H), 2.05 (s,3H)

### Preparation 108:

### 2-Fluoro-5-hydroxy-benzaldehyde(compound 508)

2-Fluoro-5-methoxy-benzaldehyde (4 g, 26 mmol) was dissolved in dry DCM (25 mL) under an argon atmosphere, cooled to 0 °C and slowly added boron tribromide (26 mL, 1.0 M in DCM, 26 mmol). On completion of the addition the reaction mixture was allowed to warm to RT and stirred for 16 h under an argon atmosphere. The reaction was carefully quenched with water (10 mL), added sat. NaHCO₃ (30 mL), then shaken and the layers were separated. The aqueous phase was extracted with two more portions of DCM. The organic phases were combined, extracted with 2N NaOH (2 x 100 mL). The combined NaOH-phases were acidified with HCl (konc.) and extracted with DCM (4 x 150 mL). The organic phases were combined, dried (MgSO₄) filtered, and concentrated *in vacuo* to afford the crude product. Purified by chromatography using EtOAc/petroleum ether (40-60)(v:v= 1:5) as the eluent, affording the title compound as a white solid.

### Preparation 109:

### 5-(tert-Butyl-dimethyl-silanyloxy)-2-fluoro-benzaldehyde (compound 509)

Compound 508 (1.5 g, 10.7 mmol) was dissolved in dry DMF (40 mL) under an argon atmosphere. *tert*-Butyl-chloro-dimethyl-silane (2.43 g, 16.1 mmol) and imidazole (1.1 g, 16.1 mmol) was added. Stirred at RT for 2 h, added EtOAc (250 mL) and then washed with water (2 x 100 mL), 4% MgSO₄ (2 x 75 mL), dried (MgSO₄) filtered, and concentrated *in vacuo* to afford the crude product as yellow oil, Purified by chromatography using EtOAc/petroleum ether (40-60)(1:20) as the eluent, affording the title compound as an colourless, clear oil.

### Preparation 110:

### (4-Bromo-2-chloro-phenyl)-[5-(tert-butyl-dimethyl-silanyloxy)-2-fluoro-phenyl]-methanol (compound 510)

A dry flask was charged with 4-bromo-2-chloro-1-iodo-benzene (10.9 g, 34.5 mmol) and the flask was evaporated and then filled with argon, this process repeated twice. Dry THF (100 mL) was added, and the solution cooled to -50 °C; then isopropylmagnesium chloride (17.3 mL, 2.0 M in diethyl ether, 34.5 mmol) was added slowly over 15 min. keeping the temperature below -40 °C. On completion of the addition the reaction mixture was stirred at -40 °C for 45 min.

The temperature was raised to -25 °C for 5 min. and then lowered to -40 °C, followed by slowly addition of a solution of compound 509 in dry THF (15 mL). On completion of the addition the reaction mixture was stirred at -40 °C for 10 min. and was then allowed to warm to 5 °C over a period of 2.5 h. The reaction mixture was slowly poured into ice-cold 2N H₂SO₄ (200 mL) and extracted with EtOAc. The aqueous phase was extracted with four more portions of EtOAc. The organic phases were combined, dried (MgSO₄), filtered, and concentrated *in vacuo* to afford the crude product as brown oil. Used without further purification.

### Preparation 111:

### (4-Bromo-2-chloro-phenyl)-(2-fluoro-5-hydroxy-phenyl)-methanone (compound 511)

Compound 510 (15.9 g, 35.6 mmol) was dissolved in dry DCM (80 mL) and dry acetonitrile (10 mL) under an argon atmosphere. The mixture was cooled to 0°C and added 4-methyl-morpholine 4-oxide (6.27 g, 53.5 mmol), grinded molecular sieve (4Å, 17.8 g). Tetra-*N*-propylammonium perruthenate(VII) (250 mg, 0.71 mmol) was added in 5 portions (5 x 50 mg) and the reaction mixture was stirred for 10 min. at 0 °C, evaporated to 1/3 volume and filtered through silica. The silica was washed with EtOAc (300 mL). The organic phase was concentrated *in vacuo* affording the crude product. Purified by continuously gradient flash chromatography using EtOAc/petroleum ether (40-60)(v:v= 1:80 to 1:10) as the eluent, affording the title compound as a yellow solid.

### Preparation 112:

### [2-Chloro-4-(2,4-difluoro-phenylamino)-phenyl]-(2-fluoro-5-hydroxy-phenyl)-methanone (compound 512)

Compound 511 (200 mg, 0,6 mmol) was dissolved in dry 1,4-dioxane (5 mL) in a reaction vial under an argon atmosphere. 2,4-Difluoro-phenylamine (78 mg, 62 µL, 0.6 mmol) was added and dissolved in the solvent. Rac-BINAP (17 mg, 0.028 mmol), Pd₂(dba)₃ (17 mg, 0.018 mmol) and Cs₂CO₃ (277 mg, 0.85 mmol) were added. The reaction vial was flushed with argon, closed and then stirred at 130 °C for 40 min, in a microwave oven. The reaction mixture was allowed to cool to RT, filtered through Celite and then purified by continuous gradient flash chromatography using EtOAc/petroleum ether (40-60)(v:v= 1:7 to 1:3) as the eluent to afford the title compound as a yellow solid,

### Example 214:

### [2-Chloro-4-(2,4-difluoro-phenylamino)-phenyl]-[2-fluoro-5-(3-hydroxypropoxy)-phenyl]-methanone (compound 314)

Compound 512 (38 mg, 0.1 mmol) was dissolved in acetonitrile (2 mL) in a reaction vial, 3-Chloro-propan-1-ol (11 µL, 0.12 mmol), K₂CO₃ (17 mg, 0.12 mmol) and NaI (cat. amount) were added. The reaction vial was flushed with argon, closed and then stirred at 130 °C for 20 min. in a microwave oven. The reaction mixture was allowed to cool to RT and then poured into a mixture of EtOAc/water. The aqueous phase was acidified with HCl (4N) and the layers were separated. The aqueous phase was extracted with more EtOAc. The organic phases were combined, dried (MgSO₄), filtered and concentrated *in vacuo* affording the crude product. Purified by continuously gradient flash chromatography using EtOAc/petroleum ether (40-60) (v:v= 1:3 to 1:1) as the eluent, affording the title compound as a yellow solid. ¹³C NMR (CDCl₃) δ 190.8, 159.1 (dd), 155.6 (dd), 155.3 (d), 155.0 (d), 148.0, 134.7, 133.0, 129.4, 127.9 (d), 124.4 (m), 120.4 (d), 117.1 (d), 116.0, 115.1 (d), 112.8, 111.6 (dd), 104.9 (dd), 66.3, 60.0, 32.0

### Example 215:

### [2-Chloro-4-(2,4-difluoro-phenylamino)-phenyl]-[5-(2.2-dimethyl-[1,3]dioxolan-4-ylmethoxy)-2-fluoro-phenyl]-methanone(compound 315)

Compound 512 (54 mg, 0.14 mmol) was dissolved in acetonitrile (4 mL) in a reaction vial. Compound 506 (61 mg, 0.21 mmol) and K₂CO₃ (30 mg, 0.21 mmol) were added. The reaction vial was flushed with argon, closed and then stirred at 110 °C for 50 min. in a microwave oven, followed by reflux in a oil bath for 24 h. The reaction mixture was concentrated *in vacuo* and purified by continuously gradient flash chromatography using EtOAc/petroleum ether (40-60)(v:v= 1:7 to 1:3) as the eluent, affording the title compound as a yellow oil. ¹³C NMR (CDCl₃) δ 190.6, 159.1 (dd), 154.7, 154.5 (d), 154.4 (dd), 147.8, 134.7, 132.9, 129.6, 127.9 (d), 124.4 (dd), 124.2 (dd), 120.6 (d), 117.1 (d), 116.0, 115.1 (d), 112.9, 111.6 (dd), 109.9, 104.9 (dd), 73.9, 69.6, 66.6, 26.8, 25.4

### Example 216:

### [2-Chloro-4-(2,4-difluoro-phenylamino)-phenyl]-[5-(2,3-dihydroxy-propoxy)-2-fluoro-phenyl]-methanone (Compound 316)

Compound 315 was dissolved in TFA:water (3:1, 8 mL) and stirred at RT for 1 h. The reaction mixture was concentrated *in vacuo* and purified by continuously gradient flash chromatography using EtOAc/petroteum ether (40-60)(v:v= 1:2 to 100:0) as the eluent, affording the title compound as a yellow foam. ¹³C NMR (DMSO-d₆) δ 189.3, 158.8 (dd), 155.7 (dd), 154.9 (d), 153.8 (d), 149.5, 133.5, 133.4, 127,8 (d), 126.5 (dd), 126.3, 124.1 (dd), 119.7 (d), 117.1 (d), 114.9 (d), 114.7, 112.0 (dd), 111.7, 105.0 (dd), 70.4, 69.8, 62.5

### Preparation 121:

### (4-Bromo-2-chloro-phenyl)-(2-chloro-5-methoxy-phenyl)-methanol (compound 521)

To a solution of 4-bromo-2-chloro-1-iodo-benzene (10.0 g, 31.5 mmol) in THF (120 ml) was added 2 M solution of isopropylmagnesium chloride in THF (17.3 ml, 34.60 mmol) at - 65°C. The reaction mixture was stirred at -40°C for 20 min. After 2-chloro-5-methoxy- benzaldehyde (5.5 g, 32.20 mmol) was added, the reaction mixture was warmed to room temperature and stirred overnight. Then the solution was quenched with saturated aqueous solution of NH₄Cl, The aqueous phase was extracted twice with diethyl ether. The combined organic phases were dried over MgSO₄ and concentrated *in vacuo.* The crude material was purified by chromatography (petroleum ether/CH₂Cl₂ 1:1) to provide title compound as a yellowish oil.

### Preparation 122:

### (4-Bromo-2-chloro-phenyl)-(2-chloro-5-methoxy-phenyl)-methanone (compound 522)

To a solution of compound 521 (5.18 g, 14.3 mmol) in CH₂Cl₂ (100 ml) was portionswise added Dess-Martin periodinane (6.11 g, 14.40 mmol) at room temperature. After being stirred at room temperature for 1 h, the reaction mixture was concentrated together with silica gel *in vacuo.* The residue was purified by chromatography (petroleum ether/CH₂Cl₂ 1:1) to give the title compound as colourless oil,

### Preparation 123:

### (4-Bromo-2-chloro-phenyl)-(2-chloro-5-hydroxy-phenyl)-methanone (compound 523)

To a solution of compound 522 (4.63 g, 12.86 mmol) in CH₂Cl₂ (50 ml) was dropwise added a 1 M solution of BBr₃ in CH₂Cl₂ at -40°C. The reaction solution was warmed to room temperature and stirred overnight. After reaction, the solution was poured into brine. The aqueous phase was extracted twice with CH₂Cl₂. The combined organic phases were dried over MgSO₄ and concentrated *in vacuo* to give a solid, which was washed with petroleum ether/CH₂Cl₂ 1:1, giving the title compound as a greyish solid,

### Preparation 124:

### [2-Chloro-4-(2,6-difluoro-phenylamino)-phenyl]-(2-chloro-5-hydroxy-phenyl)-methanone (compound 524)

A mixture of compound 523 (1.00 g, 3.00 mmol), 2,6-difluoroaniline (0.47 g, 3.60 mmol), rac-BINAP (74 mg, 0.12 mmol), Pd₂(dba)₃ and Cs₂CO₃ (1.95 g, 6.00 mmol) in 1,4-dioxane (30 ml) was stirred at 120°C for 2 days. The mixture was filtered and washed with 1,4-dioxane. The filtrate was concentrated together with silica gel *in vacuo* and purified by chromatography (petroleum ether/ethyl acetate 3:1) to provide the title compound as a solid.

### Example 228:

### [2-Chloro-4-(2,6-difluoro-phenylamino)-phenyl]-[2-chloro-5-(2-morpholin-4-yl-ethoxy)-phenyl]-methanone (compound 328)

A mixture of compound 524 (42 mg, 0.11 mmol), 4-(2-chloro-ethyl)-morpholine (50 mg, 0.27 mmol) and K₂CO₃ (100 mg, 0.72 mmol) in CH₃CN (2 ml) in a sealed glass was stirred at 90°C for 18 h. The mixture was then filtered. The filtrate was concentrated *in vacuo*. The residue was purified by chromatography (ethyl acetate), furnishing the title compound as a brownish oil. ¹³C NMR (CDCl₃) δ 192.5, 157.5 (dd), 157.2, 148.2, 140.2, 135.6, 134.0, 131.0, 127.8, 126.1 (t), 123.5, 118.2, 116.7 (t), 116.4, 115.4, 112.7, 112.2 (m), 66.6, 66.1, 57.4, 54.0

### Example 229:

### (±)-[2-Chloro-4-(2,6-difluoro-phenylamino)-phenyl]-[2-chloro-5-(2,3-dihydroxy-propoxy)-phenyl]-methanone (compound 329)

A mixture of compound 524 (42 mg, 0.11 mmol), (±)-toluene-4-sulfonic acid 2,2-dimethyl-[1,3]dioxolan-4-ylmethyl ester (50 mg, 0.17 mmol) and K₂CO₃ (100 mg, 0.72 mmol) was treated as described for compound 328. Chromatography (petroleum other/ethyl acetate3:1) furnished an acetonide.
The acetonide was taken up in TFA/H₂O 3:1 (1 ml) at room temperature and the obtained solution was stirred at the same temperature for 0.5 h. The mixture was then concentrated *in vacuo.* The residue was purified by chromatography (ethyl acetate), giving the title compound as colourless foam. ¹³C NMR (CDCl₃) δ 192.6, 157.5 (dd), 157.1, 148.4, 140.3, 135.6, 134.1, 131.1, 127.5, 126.1 (t), 123.7, 118.0, 116.7 (t), 116.4, 115.4, 112.7, 112.2 (m), 70.2, 69.6, 63.4

### Example 230:

### [5-(3-Bromo-propoxy)-2-chloro-phenyl]-[2-chloro-4-(2,6-difluoro-phenylamino)-phenyl]-methanone (compound 330)

A mixture of compound 524 (0.20 g, 0.51 mmol), 1,3-dibromopropane (0.62 g, 3.06 mmol) and K₂CO₃ (0.21 g, 1.53 mmol) was treated as described for compound 328. Chromatography (petroleum ether/ethyl acetate 4:1) furnished the title compound. ¹³C NMR (CDCl₃) δ 192.6, 157.5 (dd), 157.3, 148.2, 140.2, 135.6, 134.1, 131.0, 127.8, 126.0 (t), 123.4, 118.0, 116.7 (t), 116.4, 115.3, 112.7, 112.2 (m), 65.8, 32.2, 29.7

### Preparation 125:

### 3-(4-Bromo-2-chloro-benzoyl)-4-methyl-benzoic acid methyl ester (compound 525)

To a solution of 3-iodo-4-methyl-benzoic acid methyl ester (13.50 g, 48.92 mmol) in THF (260 mL) was added 2 M solution of isopropylmagnesium chloride in THF (24.5 mL, 49.00 mmol) at - 50°C. After the reaction mixture was stirred at the same temperature for 30 min, compound 440 (13.39 g, 40.70 mmol) was added. The solution was warmed up to room temperature and stirred at the same temperature for 2 h. The reaction was then quenched with saturated aqueous solution of NH₄Cl. The aqueous phase was extracted twice with diethyl ether. The combined organic phases were dried over MgSO₄ and concentrated *in vacuo.* The crude material was taken up in ethanol and heated for reflux. The obtained solution was cooled down to room temperature, The crystals were filtered off and dried.

### Preparation 126:

### (4-Bromo-2-chloro-phenyl)-(5-hydroxymethyl-2-methyl-phenyl)-methanol (compound 526).

A mixture of compound 525 (4.01 g, 10.90 mmol) and LiAlH₄ (0.83 g, 21.82 mmol) in THF (100 mL) was heated for reflux for 1.5 h. After being cooled down to room temperature, the reaction mixture was poured into H₂O and aqueous solution of H₂SO₄ (2M, 50 mL) was added. This mixture was then extracted twice with diethyl ether. The combined organic phases were dried over Na₂SO₄ and concentrated *in vacuo,* giving the title compound.

### Preparation 127:

### (4-Bromo-2-chloro-phenyl)-(2-methyl-5-triisopropylsilanyloxymethyl-phenyl)-methanol (compound 527)

To a solution of compound 526 (3.68 g, 10.80 mmol) and imidazol (1.47g, 21.60 mmol) in DMF (40 mL) was added TIPS CI (2.30 mL, 10.80 mmol) at room temperature. The reaction solution was stirred at the same temperature for 3 h and then poured into H₂O. The aqueous mixture was extracted three times with diethyl ether. The combined organic phases were washed with brine, dried over Na₂SO₄, and concentrated *in vacuo*. The residue was purified by chromatography (ethyl acetate/petroleum ether 1:10), furnishing the title compound.

### Preparation 128:

### (4-Bromo-2-chloro-phenyl)-(2-methyl-5-triisopropylsilanyloxymethyl-phenyl)-methanone (compound 528)

Compound 527 (4.12 g, 8.27 mmol) was treated as described for compound 522. Flash chromatography (petroleum ether/ethyl acetate 3:97) to provide the title compound.

### Preparation 129:

### [2-Chloro-4-(2,4-difluoro-phenylamino)-phenyl]-(2-methyl-5-triisopropylsilanyloxymethyl-phenyl)-methanone (compound 529)

A mixture of compound 528 (505 mg, 1.02 mmol), 2,4-difluoroaniline (0.14 mL, 1.32 mmol), Cs₂CO₃ (995 mg, 3.65 mmol), BINAP (26 mg, 0.041 mg), and Pd(OAc)₂ (9 mg, 0.041 mmol) in 1,4-dioxane (15 mL) was stirred in a sealed bottle at 100°C for 18 h. The mixture was filtered. The filtrate was concentrated *in vacuo* together with silica gel. The residue was purified by flash chromatography (ethyl acetate/ petroleum ether: graduated from 0/100 to 40/60), furnishing the title compound.

### Example 231:

### [2-Chloro-4-(2,4-difluoro-phenylamino)-phenyl]-(5-hydroxymethyl-2-methyl-phenyl)-methanone (compound 331)

To a solution of compound 529 (1.85 g, 3.40 mmol) in THF (40 mL) was added 1 M solution of TBAF in THF (4.1 mL, 4.10 mmol) at room temperature. The reaction solution was stirred at room temperature for 30 min and then poured into H₂O and extracted with diethyl ether. The combined organic phases were dried over Na₂SO₄ and concentrated *in vacuo.* The residue was purified by flash chromatography (ethyl acetate/petroleum ether: graduated from 20/80 to 50/50), giving the title compound. ¹³C NMR (DMSO-d₆) δ 195.4, 158.7 (dd), 155.7 (dd), 149.0, 139.9, 138.8, 134.8, 133.3, 130.8, 128.7, 127.1, 126.7, 126.3 (dd), 124.3 (dd), 114.8, 111.9 (dd), 111.7, 105.0 (dd), 62.1, 19.4

### Example 232:

### [2-chloro-4-(2,4-difluoro-phenylamino)-phenyl]-(5-chloromethyl-2-methyl-phenyl)-methanone (compound 332)

To a solution of compound 331 (0.79 g, 2.09 mmol), Et₃N (0.58 mL, 4.18 mmol), and DMAP (10 mg) in CH₂Cl₂ (50 mL) was added p-toluenesulfonyl chloride (0.60 g, 3.14 mmol) at room temperature. The reaction mixture was stirred at the same temperature for 18 h and then poured into H₂O. The aqueous phase was extracted three times with CH₂Cl₂. The combined organic phases were dried over Na₂SO₄ and concentrated *in vacuo.* The residue was purified by chromatography (petroleum ether/ethyl acetate: graduated from 90/10 to 75/25), giving the title compound. ¹³C NMR (CDl₃) δ 195.7, 159,2 (dd), 155.5 (dd), 147.8, 139.4, 138.3, 135.2, 134.8, 133.5, 131.8, 131.0, 129.6, 129.4, 124.4 (dd), 124.3 (dd), 116.2, 112.8, 111.6 (dd), 105.0 (dd), 45.6, 20.2

### Example 233:

### (5-Azidomethyl-2-methyl-phenyl)-[2-chloro-4-(2,4-difluoro-phenylamino)-phenyl]-methanone (compound 333)

A mixture of compound 332 (56 mg, 0.14 mmol) and NaN₃ (18 mg, 0.28 mmol) in DMF (4 mL) was stirred at 80°C for 3 h. The reaction mixture was poured into H₂O and extracted three times with CH₂Cl₂. The combined organic phases were washed with brine, dried over Na₂SO₄, and concentrated *in vacuo,* furnishing the title compound. ¹³C NMR (CDCl₃) δ 195.9, 159.2 (dd), 155.6 (dd), 147.9, 139.6, 138.1, 135.2, 133.6, 132.6, 131.9, 130.6, 129.3, 124.4 (dd), 116.2, 112.8, 111.6 (dd), 105,0 (dd), 54.1, 20.2

### Example 234:

### (5-Aminomethyl-2-methyl-phenyl)-[2-chloro-4-(2,4-difluoro-phenylamino)-phenyl]-methanone (compound 334)

To a solution of compound 333 (46 mg, 0.11 mmol) was added triphenylphosphine (67 mg, 0.26 mmol) at room temperature. The solution was stirred at the same temperature for 18 h. H₂O (0.5 mL) was added. The mixture was then heated for reflux for 4 h and concentrated together with silica gel *in vacuo.* The residue was purified by chromatography (MeOH/ethyl acetate: graduated from 13/87 to 30/70), providing the title compound. ¹³C NMR (CDCl₃) δ 196.4, 159.1 (dd), 155.5 (dd), 147.8, 139.6, 139.3, 136.5, 135.1, 133.5, 131.6, 129.8, 129.5, 128.3, 124.5 (dd), 124.3 (dd), 116.2, 112.8, 111.6 (dd), 104.9 (dd), 45.6, 20.1

### Preparation 130 :

### (4-Bromo-2-chloro-phenyl)-(5-hydroxymethyl-2-methoxy-pheny)-methanol (compound 530)

Compound 441 (1.05 g, 2.70 mmol) was treated as described for compound 526. Flash chromatography (ethyl acetate/petroleum ether: graduated from 25/75 to 45/55) provided the title compound.

### Preparation 131:

### (4-Bromo-2-chloro-phenyl)-(2-methoxy-5-triisopropylsilanyloxymethyl-phenyl)-methanol (compound 531)

Compound 530 (766 mg, 2.14 mmol) was treated as described for compound 527. Flash chromatography (ethyl acetate/petroleum ether: graduated from 4/96 to 30/70) furnished the title compound.

### Preparation 132:

### (4-Bromo-2-chloro-phenyl)-(2-methoxy-5-triisopropylsilanyloxymethyl-phenyl)-methanone (compound 532)

Compound 531 (710 mg, 1.38 mmol) was treated as described for compound 504. Flash chromatography (petroleum ether/ethyl acetate 3:97) to provide the title compound.

### Preparation 133:

### [2-Chloro-4-(2,4-difluoro-phenylamino)-phenyl]-(2-methoxy-5-triisopropyl-silanyloxymethyl-phenyl)-methanone (533)

Compound 532 (449 mg, 0.87 mmol) and 2,4-difluoroaniline (0.12 mL, 1.14 mmol) were treated as described for compound 529. Flash chromatography (ethyl acetate/ petroleum ether: graduated from 10/90 to 25/75) furnished the title compound.

### Example 235:

### [2-Chloro-4-(2,4-difluoro-phenylamino)-phenyl]-(5-hydroxymethyl-2-methoxy-phenyl)-methanone (compound 335)

Compound 533 (163 mg, 0.29 mmol) was treated as described for compound 331. Flash chromatography (ethyl acetate/petroleum ether: graduated from 50/50 to 75/25) gave the title compound, ¹³C NMR (CD₃OD) δ 196.0, 161.0 (dd), 158.9, 157.8 (dd), 150.8, 135.8, 135.1, 134.7, 133.0, 130.7, 130.1, 129.7, 127.3 (dd), 126.2 (dd), 116.4, 112.9, 112.9, 112.6 (dd), 105.7 (dd), 64.5, 56.4

### Example 236:

### Acetic acid 3-[2-chloro-4-(2,4-difluoro-phenylamino)-benzoyl]-4-methoxy-benzyl ester (compound 336)

To a solution of compound 335 (45 mg, 0.11 mmol), Et₃N (46 µL, 0.34 mmol), and DMAP (3 mg) in CH₂Cl₂ (5 mL) was added Ac₂O (13 µL, 0.13 mmol) at room temperature. The solution was stirred at room temperature for 0.5 h. The reaction solution was then washed with H₂O and saturated aqueous solution of NaHCO₃, dried over MgSO₄, and concentrated *in vacuo.* The residue was purified by chromatography (ethyl acetate/petroleum ether 1:4), giving the title compound. ¹³C NMR (CDCl₃) δ 193.1, 170.9, 158.9 (dd), 158.2, 155.3 (dd), 147.2, 134.7, 133.2, 133.2, 130.8, 130.4, 129.5, 128.3, 124.7 (dd), 123.8 (dd), 116.1, 112.9, 111.7, 111.5 (dd), 104.9 (dd), 65.6, 56.0, 21.0

### Example 237:

### N-tert-Butoxy-3-[2-chloro-4-(2,4-difluoro-phenylamino)-benzoyl]-4-methoxybenzamide (compound 337)

To a mixture of compound 437 (51 mg, 0.12 mmol), *O*-*tert*-butylhydroxylamine (31 mg, 0.24 mmol), *N*-methylmorpholine (26 mg, 0.24 mmol) and 1-hydroxybenzotriazol (16 mg, 0.12 mmol) in CH₂Cl₂ (5 mL) was added EDCl (30 mg, 0.16 mmol) at room temperature. The reaction mixture was stirred at the same temperature for 6 h and then quenched with H₂O. The aqueous phase was extracted five times with CH₂Cl₂. The combined organic phases were dried over MgSO₄ and concentrated *in vacuo.* The residue was purified by flash chromatography (ethyl acetate/petroleum ether 2: 1), furnishing the title compound, ¹³C NMR (DMSO-d₆) δ 191.6, 164.8, 159.4, 158.7 (dd), 155.7 (dd), 149.2, 133.8, 133.6, 131.7, 129.1, 128.4, 126.5, 126.4 (dd), 124.7, 124.2 (dd), 114.8, 111,9 (dd), 111.8, 111.7, 105.0 (dd), 80.8, 56.0, 26.4

### Example 238:

### 3-[2-Chloro-4-(2,4-difluoro-phenylamino)-benzoyl]-N-methoxy-4-methyl-benzamide (compound 338)

Compound 424 (50 mg, 0.12 mmol) and *N*-methylhydroxylamine hydrochloride (21 mg, 0.25 mmol) were treated as described for compound 337. Flash chromatography (ethyl acetate/petroleum ether: graduated from 67/33 to 100/0) provided the title compound. ¹³C NMR (DMSO-d₆) δ 194.5, 158.8 (dd), 155.8 (dd), 149.5, 140.1, 139.5, 133.8, 133.7, 131.2, 129.4, 128.8, 126.9, 126.5 (dd), 126.2, 124.1 (dd), 114.8, 111.9 (dd), 111.8, 105.0 (dd), 63.2, 19.6

### Example 239:

### N-Butoxy-3-[2-chloro-4-(2,4-difluoro-phenylamino)-benzoyl]-4-methyl-benzamide (compound 339)

Compound 424 (100 mg, 0.25 mmol) and *O*-butyl-hydroxylamine (63 mg, 0.50 mmol) were treated as described for compound 337. Flash chromatography (ethyl acetate/petroleum ether: graduated from 20/80 to 60/40) provided the title compound. ¹³C NMR (CDCl₃) δ 195.4, 165.9, 159.3 (dd), 155.7 (dd), 148.3, 142.0, 139.8, 135.4, 133.8, 131.7, 129.5, 129.1, 128.6, 127.7, 124.6 (dd), 124.1 (dd), 116.2, 112.8, 111.7 (dd), 105.0 (dd), 76.9, 30.1, 20.4, 19.1, 13.8

### Example 240:

### 3-[2-Chloro-4-(2,4-difluoro-phenylamino)-benzoyl]-N-cyclohexylmethoxy-4-methyl-benzamide (compound 340)

Compound 424 (50 mg, 0.12 mmol) and *O*-cyclohexyl-hydroxylamine (42 mg, 0.25 mmol) were treated as described for compound 337. Flash chromatography (ethyl acetate/petroleum ether: graduated from 67/33 to 100/0) provided the title compound. ¹H NMR (CDCl₃) δ 8.68 (bs,1H), 7.72 (bd,1H), 7.66 (bs,1H), 7.43 - 7.28 (m,3H), 7.00 - 6.82 (m,3H), 6.75 (dd,1H), 5.94 (bs,1H), 3.80 (d,2H), 2.44 (s,3H), 1.89 - 1.56 (m,6H), 1.35 - 1.09 (m,3H), 1.07 - 0.84 (m,2H)

### Example 241:

### 3-[2-Chloro-4-(2,4-difluoro-phenylamino)-benzoyl]-4-methyl-N-(2-methyl-thiazol-4-ylmethoxy)-benzamide (compound 341)

Compound 424 (50 mg, 0.12 mmol) and *O*-(2-Methyl-thiazol-4-ylmethyl)-hydroxylamine (36 mg, 0.25 mmol) were treated as described for compound 337 Flash chromatography (ethyl acetate/petroleum ether: graduated from 67/33 to 100/0) provided the title compound. ¹H NMR (CDCl₃) δ 10.0 - 9.0 (bs,1H), 7.76 (dd,1H), 7.67 (d,1H), 7.42 - 7.30 (m,3H), 7.15 (s,1H), 7.00 - 6.84 (m,3H), 6.75 (dd,1H), 5.98 (bs,1H), 5.04 (s,2H), 2.64 (s,3H), 2.45 (s,3H)

### Example 242:

### N-benzyloxy-3-[2-chloro-4-(2,4-difluoro-phenylamino)-benzoyl]-4-methyl-benzamide (compound 342)

Compound 424 (100 mg, 0.25 mmol) and *O*-benzyl-hydroxylamine (80 mg, 0.50 mmol) were treated as described for compound 337. Flash chromatography (ethyl acetate/petroleum ether: graduated from 80/20 to 60/40) provided the title compound. ¹³C NMR (CDCl₃) δ 195.3, 165.8, 159.3 (dd), 155.7 (dd), 148.2, 142.2, 139.8, 135.4, 135.1, 133.8, 131.7, 129.4, 129.1, 128.9, 128.7, 127.8, 124.6 (dd), 124.1 (dd), 116.2, 112.8, 111.7 (dd), 105.0 (dd), 78.5, 20.4

### Example 243:

### 3-[2-Chloro-4-(2,4-difluoro-phenylamino)-benzoyl]-N-(4-methoxy-benzyloxyl-4-methyl-benzamide compound 343)

Compound 424 (100 mg, 0.25 mmol) and *O*-(4-methoxy-benzyl)-hydroxylamine (65 mg, 0.34 mmol) were treated as described for compound 337. Flash chromatography (ethyl acetate/petroleum ether: graduated from 80/20 to 60/40) provided the title compound. ¹³C NMR (CDCl₃) δ 195.3, 165.6, 160.1, 159.3 (dd), 155.7 (dd), 148.2, 142.1, 139.7, 135.3, 133.8, 131.7, 131.1, 129.4, 129.1, 128.6, 127.8, 127.2, 124.6 (dd), 124.2 (dd), 116.2, 114.1, 112.8, 111.6 (dd), 105.0 (dd), 78.0, 55.3, 20.4

### Example 244:

### 3-[2-Chloro-4-(2,4-difluoro-phenylamino)-benzoyl]-4-methyl-benzoic acid N',N'-dimethyl-hydrazide (compound 344)

Compound 424 (50 mg, 0.12 mmol) and *N*,*N*-dimethyl-hydrazine (19 µL, 0.25 mmol) were treated as described for compound 337. Flash chromatography (ethyl acetate/petroleum ether: graduated from 67/33 to 80/20) provided the title compound. ¹³C NMR (DMSO-d₆) δ 194.7, 163.2, 158.9 (dd), 155.9 (dd), 149.6, 139.6, 139.5, 133.9, 133.8, 131.3, 131.0, 129.1, 127.2, 126.7 (dd), 126.3, 124.2 (dd), 114.9, 112.0 (dd), 111.9, 105.1 (dd), 46.1, 19.6

### Example 245:

### 3-[2-Chloro-4-(2,4-difluoro-phenylamino)-benzoyl]-4-methyl-N-morpholin-4-yl-benzamide (compound 345)

Compound 424 (50 mg, 0.12 mmol) and *N*-amino-morpholine hydrochloride (35 mg, 0.25 mmol) were treated as described for compound 337. Flash chromatography (ethyl acetate/petroleum ether: graduated from 67/33 to 100/0) provided the title compound. ¹³C NMR (DMSO-d₆) δ 194.5, 168.2, 158.9 (dd), 155.8 (dd), 149.5, 139.1, 138.2, 133.7, 133.7, 132.9, 131.3, 129.3, 127.5, 126.5 (dd), 126.5, 124.3 (dd), 114.8, 112.0 (dd), 111.9, 105.1 (dd), 66.0, 19.7

### Example 246:

### 3-[2-Chloro-4-(2,4-difluoro-phenylamino)-benzoyl]-N-hydroxy-4-methyl-benzamide (compound 346)

Compound 424 (202 mg, 0.50 mmol) and O-[(dimethyl-t-butyl)silyl]-hydroxylamine (148 mg, 1.01 mmol) were treated as described for compound 337. Flash chromatography (ethyl acetate/petroleum ether: graduated from 67/33 to 100/0) provided the title compound. ¹³C NMR (DMSO-d₆) δ 194.6, 162.9, 158.8 (dd), 155.8 (dd), 149.4, 139.5, 139.1, 133.6, 131.1, 130.3, 128.7, 127.0, 126.5 (m), 126.4, 124.1 (dd), 114.8, 111.9 (dd), 111.8, 105.0 (dd), 19.6

### Preparation 134:

### 4-(4-Bromo-2-chloro-benzoy)-3-methyl-benzoic acid meth ester (compound 534)

4-Iodo-3-methyl-benzoic acid methyl ester (0.83 g, 3.00 mmol) and compound 440 (0.75 g, 3.00 mmol) were treated as described for compound 525. Flash chromatography (ethyl acetate/petroleum ether 1:15) provided the title compound as a white solid.

### Preparation 135:

### 4-[2-Chloro-4-(2,4-difluoro-phenylamino)-benzoyl]-3-methyl-benzoic acid methyl ester (compound 535)

Compound 534 (184 mg, 0.50 mmol) and 2,4-difluoroaniline (0.066 mL, 0.65 mmol) were treated as described for compound 529. Flash chromatography (ethyl acetate/petroleum ether: graduated from 0/100 to 20/80) gave the title compound.

### Example 247:

### 4-[2-Chloro-4-(2,4-difluoro-phenylamino)-benzoyl]-N-(2-hydroxy-ethyl)-3-methyl-benzamide (compound 347)

A mixture of compound 535 (127 mg, 0.30 mmol) and K₂CO₃ (63 mg, 0.45 mmol) in ethanolamine/CH₃CN 1:1 (4 mL) was stirred at room temperature for 18 h. The reaction mixture was then poured into H₂O and extracted three times with ethyl acetate. The combined organic phases were washed with brine, dried over MgSO₄, and concentrated *in vacuo.* The residue was purified by chromatography (ethyl acetate/petroleum ether 5:1), furnishing the title compound as a yellow solid. ¹³C NMR (CD₃OD) δ 197.7, 169.8, 161.2 (dd), 158.0 (dd), 151.7, 144.0, 138.5, 137.5, 136.4, 135.1, 131.0, 130.0, 128.2, 127.7 (dd), 125.8 (dd), 125.7, 116.5, 113.0, 112.7 (dd), 105.8 (dd), 61.6, 43.7, 20.3

### Example 248:

### [2-Chloro-4-(2,4-difluoro-phenylamino)-phenyl]-[5-(3-hydroxy-propenyl)-2-methyl-phenyl]-methanone (compound 348)

Compound 495 (0.132 g, 0.27 mmol) was dissolved in dry 1,4-dioxan (1.5 mL) in a screw cap vessel under an argon atmosphere. Tri(2-furyl)phosphine (0.013 g, 0.05 mmol) and tris(dibenzylideneacetone)dipalladium(O) (0.007 g, 0.008 mmol) were added and shaken, (E)-3-(tri-*n*-butyltin)prop-2-en-1-ol (0.104 g, 0.3 mmol) was added and the reaction mixture was heated under an argon atmosphere at 100 °C for 16 h on a shaking table, CH₃CN was added to the reaction mixture and the solution was washed three times with petroleum ether. The acetonitrile phase was concentrated *in vacuo* and purified by flash chromatography using a gradient of EtOAC/petroleum ether (40-60) 1:3 -> 3:1 as the eluent. This afforded the title compound as yellow oil.

### Example 249:

### 4-{3-[2-Chloro-4-(2,4-difluoro-phenylamino)-benzoyl]-4-methyl-benzoylamino}-thiophene-3-carboxylic acid methyl ester (compound 349)

The reaction was carried out similarly as described in the preparation of compound 120, using 4-amino-thiophene-3-carboxylic acid methyl ester (0.5 mmol) and compound 439 (0.5 mmol), Purification was done by flash chromatography to afford the title compound as foam. ¹³C NMR (CDCl₃) δ 195.6, 164.8, 163.9, 159.1 (dd), 155.5 (dd), 147.9, 142.4, 139.7, 136.5, 135.2, 133.6, 132.6, 131.9, 131.3, 129.1, 128.9, 128.6, 124.4 (dd), 124.2 (dd), 121.6, 116.3, 113.0, 111.6 (dd), 110.8, 104.9 (dd); 52.1, 20.5

### Example 250:

### 3-[2-Chloro-4-(2,4-difluoro-phenylamino)-benzoyl]-N-furan-2-ylmethyl-4-methyl-benzamide (compound 350)

The reaction was carried out similarly as described in the preparation of compound 120, using furan-2-yl-methylamine (0.5 mmol) and compound 439 (0.5 mmol). Purification was done by flash chromatography to afford the title compound as foam. ¹³C NMR (CDCl₃) δ 195.6, 166.4, 159.2 (dd), 155.6 (dd), 151.0, 148.2, 142.4, 141.6, 139.8, 135.3, 133.7, 131.6, 128.9, 128.8, 127.8, 124.5 (dd), 124.2 (dd), 116.2, 112.8, 111.6 (dd), 110.5, 107.8, 105.0 (dd), 37.0, 20.4

### Example 251:

### 3-[2-Chloro-4-(2,4-difluoro-phenylamino)-benzoyl]-N-(3-methoxy-phenyl)-4-methyl-benzamide (compound 351)

The reaction was carried out similarly as described in the preparation of compound 120, using 3-methoxy-phenylamine (0.5 mmol) and compound 439 (0.5 mmol). Purification was done by flash chromatography to afford the title compound as foam. ¹³C NMR (CDCl₃) δ 195.5, 165.0, 160.2, 159.3 (dd), 155.7 (dd), 148.3, 141.8, 139.9, 139.1, 135.4, 133.8, 132.3, 131.8, 129.7, 129.0, 128.5, 127.7, 124.6 (dd), 124.1 (dd), 116.2, 112.8, 112.3, 111.6 (dd), 110.6, 105.9, 105.0 (dd), 55.3, 20.4

### Example 252:

### 2-{3-[2-Chloro-4-(2,4-difluoro-phenylamino)-benzoyl]-4-methyl-benzoylamino}-benzoic acid methyl-ester (compound 352)

The reaction was carried out similarly as described in the preparation of compound 120, using 2-amino-benzoic acid methyl ester (0.5 mmol) and compound 439 (0.5 mmol). Purification was done by flash chromatography to afford the title compound as foam. ¹³C NMR (CDCl₃) S 195.6, 169.0, 164.9, 159.1 (dd), 155.5 (dd), 147.8, 142.4, 141.7, 139.6, 135.2, 134.8, 133.6, 132.3, 131.9, 130.9, 129.3, 129.1, 129.0, 124.4 (dd), 124.2 (dd), 122.7, 120.4, 116.3, 115.2, 113.0, 111.6 (dd), 104.9 (dd), 52.4, 20.6

### Example 253:

### 3-{3-[2-Chloro-4-(2,4-difluoro-phenylamino)-benzoyl]-4-methyl-benzoylamino}-thiophene-2-carboxylic acid methyl ester (compound 353)

The reaction was carried out similarly as described in the preparation of compound 120, using 3-amino-thiophene-2-carboxylic acid methyl ester (1.0 mmol) and compound 439 (0.5 mmol). Purification was done by flash chromatography to afford the title compound as foam. ¹³C NMR (DMSO-d₆) 8 194.2, 163.9, 162.4, 158.8 (dd), 155.7 (dd), 149.6, 143.7, 141.4, 139.8, 133.8, 133.7, 133.3, 131.9, 130.6, 128.7, 127.5, 126.5 (dd), 126.0, 124.1 (dd), 121.9, 124.8, 112.0 (dd), 111.9, 105.0 (dd), 52.1, 19.7

### Example 254:

### 4-{3-[2-Chloro-4-(2,4-difluoro-phenylamino)-benzoyl]-4-methyl-benzoylamino}-thiophene-3-carboxylic acid (compound 354)

To a suspension of compound 349 (75 mg, 0.14 mmol) in methanol (5 mL) was added water (0.5 mL) followed by lithium hydroxide (17 mg, 0.7 mmol). The mixture was then stirred at reflux for 30 min. The reaction mixture was made acidic (pH = 5) by slowly addition of HCl (1N), and then poured into a mixture of EtOAc/water. The aqueous phase was extracted with more EtOAc. The organic phases were combined, dried (MgSO₄), filtered, and concentrated *in vacuo* to afford the title compound as solid, ¹³C NMR (DMSO-d₆) δ 194.3, 165.6, 162.6, 158.8 (dd), 155.8 (dd), 149.6, 140.7, 139.9, 135.8, 133.9, 133.8, 131.8, 131.0, 128.2, 127.0, 126.5 (dd), 126.0, 124.0 (dd), 122.6, 114.8, 112.1, 112.0 (dd), 111.9, 110.8, 105.0 (dd), 19.6

### Example 255:

### 2-{3-[2-Chloro-4-(2,4-difluoro-phenylamino)-benzoyl]-4-methyl-benzoylamino}-benzoic acid (compound 355)

The reaction was carried out similarly as described in the preparation of compound 354, using compound 352 (0.5 mmol). The title compound was obtained as solid. ¹³C NMR (DMSO-d₆) δ 194.3, 169.9, 163.7, 158.8 (dd), 155.8 (dd), 149.6, 140.8, 140.6, 140.0, 134.0, 133.9, 133.8, 131.9, 131.7, 131.1, 128.5, 127.2, 126.6 (dd), 125.9, 124.0 (dd), 122.9, 119.9, 117.0, 114.9, 112.0 (dd), 111.9, 105.0 (dd), 19.6

### Preparation 136:

### 2-{3-[2-Chloro-4-(2,4-difluoro-phenylamino)-benzoyl]-4-methyl-benzoylamino}-benzoyl chloride (compound 536)

A suspension of compound 355 (68 mg, 0.13 mmol) in toluene (2 mL) was added thionyl chloride (19 µL, 0.26 mmol) and then refluxed for 2 h. The reaction mixture was concentrated *in vacuo* to afford the title compound without any further purification.

### Example 256:

### 3-[2-Chloro-4-(2,4-difluoro-phenylamino)-benzoyl]-N-[2-(2-hydroxyethylcarbamoyl)-phenyl]-4-methyl-benzamide (compound 356)

The reaction was carried out similarly as described in the preparation of compound 120, using 2-amino-ethanol (0.26 mmol) and compound 536 (0.13 mmol). Purification was done by flash chromatography to afford the title compound as foam. ¹³C NMR (CDCl₃) δ 195.8, 169.9, 164.8, 159.0 (dd), 155.4 (dd), 147.9, 142.2, 139.8, 139.5, 135.2, 133.6, 132.8, 132.2, 131.9, 129.1, 128.9, 126.7, 124.5 (m), 124.1 (m), 123.0, 121.5, 120.2, 116.4, 113.0, 111.6 (dd), 104.9 (dd), 61.8, 42.5, 20.5

### Example 257:

### 3-{3-[2-Chloro-4-(2,4-difluoro-phenylamino)-benzoyl]-4-methyl-benzoylamino}-thiophene-2-carboxylic acid (2-hydroxy-ethyl)-amide (compound 357)

A solution of compound 353 (124 mg, 0,23 mmol) in acetonitrile (2.0 mL) and 2-amino-ethanol (0.50 mL) was added K₂CO₃ (50 mg, 0.36 mmol) and stirred for 18 h at RT. The reaction mixture was poured into a mixture of EtOAc/water. The aqueous phase was extracted with more EtOAc. The organic phases were combined and concentrated on silica gel in vacou. Purification was done by flash chromatography eluting with mixtures of MeOH/DCM to afford the title compound. ¹³C NMR (DMSO-d₆) δ 193.8, 163.5, 161.5, 158.2 (dd), 155.2 (dd), 149.0, 141.8, 140.5, 139.3, 133.2, 133.2, 131.3, 130.5, 128.5, 127.8, 127.1, 125.9 (dd), 125.6, 123.6 (dd), 121.1, 114.3, 113.3, 111.4 (dd), 111.4, 104.5 (dd), 58.8, 41.3, 19.2

### Preparation 137:

### 3-(2-Chloro-4-nitro-benzoyl)-4-methyl-benzonitrile (compound 537)

A dry flask was charged with 3-iodo-4-methyl-benzonitrile (5.15 g, 21.2 mmol) and the flask was evaporated and then filled with argon and this process repeated twice. Dry THF (15 mL) was added, and the solution cooled to -35°C; then isopropylmagnesium chloride (10.6 mL, 2.0 M in diethyl ether, 21 mmol) was added slowly over 20 min keeping the temperature below -35 °C. On completion of the addition the reaction mixture was stirred at -35 °C for 30 min. A THF solution of ZnCl₂ (3.61 g, 26.5 mmol, 1.0 M) was added dropwise over 20 min. The reaction mixture was stirred at 0 °C for 20 min; then 2-chloro-4-nitrobenzoyl chloride (4.9 g, 22.3 mmol) and Cu(OAC)₂ (85 mg, 0.42 mmol) were added and the reaction mixture was allowed to warm to room temperature. After 16 h the reaction mixture was poured into a mixture of EtOAc/water, then shaken and separated. The aqueous phase was extracted with more EtOAc. The organic phases were combined, dried (MgSO₄), filtered, and concentrated *in vacuo* to afford the crude product. The crude product was purified by flash chromatography using EtOAc/petroleum ether (40-60) 1:6 followed by 1:4 as the eluent to give the title compound as yellow solid.

### Preparation 138:

### 3-(4-Amino-2-chloro-benzoyl)-4-methyl-benzonitrile (compound 538)

A mixture of compound 537 (1.05 g, 3.49 mmol) and stannous chloride dihydrate (3.31 g, 17.46 mmol) in absolute ethanol (20 mL) was heated to reflux, After 90 min the solution was cooled to RT and then poured into a mixture of ice/aqueous NaOH (7N)/EtOAc. The aqueous phase was extracted with more EtOAc followed by DCM, The organic phases were combined, washed with brine, dried (MgSO₄), filtered and concentrated *in vacuo.* The crude product was purified by flash chromatography using EtOAc/petroleum ether 1:2 as the euent to give the title compound as yellow solid.

### Preparation 139:

### 3-[2-Chloro-4-(4-fluoro-2-methyl-phenylamino)-benzoyl]-4-methyl-benzonitrile (compound 539)

A screw-capped vial (8 mL) was charged with compound 538 (100 mg, 0.37 mmol) in 1,4-dioxane (2 mL), 2-bromo-5-fluoro-toluene (55 µL, 0.44 mmol), Cs₂CO₃ (169 mg, 0.52 mmol), Pd₂(dba)₃ (9 mg, 0.009 mmol), and rac-BINAP (9 mg, 0.014 mmol). The tube was capped with a rubber septum, flushed with argon for 5 min, and then stirred at 100 °C for 18 h. The reaction mixture was allowed to cool to room temperature, and then poured into a mixture of water and EtOAc. The aqueous phase was extracted twice with more EtOAc. The combined organic phases were washed with brine, dried (MgSO₄), filtered and concentrated *in vacuo.* The crude product was purified by chromatography eluting with EtOAc/petroleum ether 1:3 to afford the title compound as light brown oil.

### Example 258:

### [2-Chloro-4-(4-fluoro-2-methyl-phenylamino)-phenyl]-[2-methyl-5-(1H-tetrazol-5-yl)-phenyl]-methanone (compound 358)

To a screw-capped vial with a magnetic stirrer were added compound 539 (100 mg, 0.26 mmol), TBAF·H₂O (41 mg, 0.13 mmol) and TMSN₃ (105 µL, 0.80 mmol) and THF (0.3 mL), and the resulting mixture were heated under vigorous stirring at 85 °C for 18 h. The reaction mixture was allowed to cool to room temperature, and then poured into a mixture of HCl (1N) and EtOAc. The aqueous phase was extracted with more EtOAc, The combined organic phases were washed with brine, dried (MgSO₄), filtered and concentrated *in vacuo.* The crude product was purified by chromatography eluting with DCM/ MeOH/CH₃COOH 100:2.5:0.25 to afford the title compound as yellow foam. ¹³C NMR (DMSO-d₆) δ 193.9, 159.5 (d), 154.8, 151.1, 140.7, 139.4, 136.2 (d), 134.4, 134.3, 132.1, 128.5, 127.1 (d), 126.4, 124.7, 121.8, 117.4 (d), 114.1, 113.5 (d), 111.1, 19.5, 17.6

### Preparation 145:

### [2-Chloro-4-(2,4-difluoro-phenylamino)-phenyl-(2-methyl-5-trimethylsilanylethynyl-phenyl)-methanone (compound 545)

In a screw-capped vial (8 mL) was placed dry degassed triethylamine (4.0 mL) and a magnetic stirrer under argon. Compound 495 (100 mg, 0.21 mmol), Pd₂(dba)₃ (3.8 mg), triphenyl phosphine (5.4 mg, 0.02 mmol), CuI (1 mg) and ethynyl-trimethyl-silane (29 µL, 0.21 mmol) were added to the vial and the reaction mixture was heated under vigorously stirring at 90 °C for 20 h. The cooled reaction mixture was filtered through Decalite and concentrated *in vacuo.* The crude product was purified by continuous gradient flash chromatography using EtOAc/petroleum ether (40-60) (v:v= 0:100 to 10:90) as the eluent to afford the title compound as foam.

### Example 262:

### [2-Chloro-4-(2,4-difluoro-phenylamino)-phenyl]-(5-ethynyl-2-methyl-phenyl)-methanone (compound 362)

A mixture of compound 545 (36 mg, 0.08 mmol) and K₂CO₃ (17 mg, 0.13 mmol) in methanol (3.0 mL) was stirred at RT for 3 h. The reaction mixture was poured into a mixture of EtOAc/water. The aqueous phase was extracted with more EtOAc. The organic phases were combined, dried (MgSO₄), filtered, and concentrated *in vacuo.* The crude product was purified by continuous gradient flash chromatography using EtOAc/petroleum ether (40-60) (v:v= 0:100 to 20:80) as the eluent to afford the title compound as foam. ¹³C NMR (CDCl₃) δ 195.4, 159.2 (dd), 155.5 (dd), 147.8, 139.3, 138.8, 135.2, 134.2, 133.5, 132.9, 131.5, 129.2, 124.3 (dd), 119.4, 116.2, 112.8, 111.6 (dd), 105.0 (dd), 82.9, 20.4

### Example 264:

### 3-[2-Chloro-4-(2,4-difluoro-phenylamino)-benzoyl]-4-methyl-benzoic acid hydrazide (compound 364)

A mixture of compound 423 (1.00 g, 2,4 mmol) and hydrazine hydrate (1.17 mL, 24 mmol) in methanol (40 mL) was stirred at reflux for 48 h. The reaction mixture was poured into a mixture of EtOAc/water. The aqueous phase was extracted with more EtOAc. The organic phases were combined and concentrated *in vacuo.* The crude product was purified by continuous gradient flash chromatography using MeOH/DCM (v:v= 5:95 to 10:90) as the eluent to afford the title compound as yellow foam. ¹³C NMR (CDCl₃) δ 195.5, 167.8, 159.3 (dd), 155.7 (dd), 148.3, 142.0, 139.7, 135.3, 133.7, 131.8, 129.9, 129.0, 128.6, 127.7, 124.6 (dd), 124.2 (dd), 116.1, 112.8, 111.6 (dd), 105.0 (dd), 20.4

### Example 265:

### 1-{3-[2-Chloro-4-(2,4-difluoro-phenylamino)-benzoxyl]-4-methyl-benzoyl}-4-ethyl-3-thio semicarbazide (compound 365)

In a screw-capped vial (8 mL) was placed isothiocyanato-ethane (69 µL, 0.79 mmol), compound 364 (300 mg, 0.72 mmol), methanol (6 mL) and a magnetic stirrer. The reaction mixture was heated under stirring at 95°C for 3 h. The cooled reaction mixture was filtered through Decalite and concentrated *in vacuo.* The crude product was purified by flash chromatography using THF/petroleum ether 2:3 as the eluent to afford the title compound as yellow foam. ¹³C NMR (DMSO-d₆) δ 194.6, 181.3, 165.0, 158.8 (dd), 155.8 (dd), 149.5, 140.3, 139.4, 133,8, 133.8, 130.9, 129.9, 129.7, 127.7, 126.6 (dd), 126.2, 124.1 (dd), 114.8, 112.0 (dd), 111.7, 105.0 (dd), 38.4, 19.6, 14.4

### Example 266:

### [2-Chloro-4-(2,4-difluoro-phenylamino)-phenyl]-[5-(5-ethymamino-[1,3,4]thiadiazol-2-yl)-2-methyl-phenyl]-methanone (compound 366)

A mixture of compound 365 (122 mg, 0.24 mmol) and POCl₃ (29 µL, 0.32 mmol) in 1,4-dioxane (1.0 mL) was stirred at 95 °C for 20 h. The reaction mixture was poured into a mixture of EtOAc/sat. aq, NaHCO₃. The aqueous phase was extracted with more EtOAc. The organic phases were combined, dried (MgSO₄), filtered, and concentrated *in vacuo.* The crude product was purified by continuous gradient flash chromatography using EtOAc/petroleum ether (40-60) (v:v= 20:80 to 50:50) as the eluent to afford the title compound as yellow solid ¹³C NMR (DMSO-d₆) δ 194.4, 168.3, 158.8 (dd), 155.8 (dd), 154.7, 149.5, 139.9, 137.9, 133.8, 133.7, 131.9, 128.4, 126.5 (dd), 126.2, 125.7, 124.1 (dd), 114.8, 112.0 (dd), 111.8, 105.0 (dd), 39.6, 19.5, 14.1

### Preparation 146:

### 3-[2-Chloro-4-(2,4-difluoro-phenylamino)-benzoyl]-4-methyl-benzonitrile (compound 546)

A flask was charged with compound 538 (500 mg, 1.85 mmol) in toluene (10 mL), 1-bromo-2,4-difluorobenzene (0.25 mL, 2.22 mmol), Cs₂CO₃ (841 mg, 2.59 mmol), Pd(OAc)₂ (8 mg, 0.04 mmol), and 4,5-bis-diphenylphosphanyl-9,9-dimethyl-9H-xanthene (32 mg, 0.056 mmol). The flask was flushed with argon for 5 min, closed and then warmed slowly to 120 °C. The reaction vial was stirred at 120 °C for 24 h. The reaction mixture was allowed to cool to room temperature, and then filtered through Decalite. Concentration *in vacuo* gave the crude product. The crude product was purified by continuous gradient flash chromatography using EtOAc/petroleum ether (40-60) (v:v = 15:85 to 33:67) as the eluent to afford the title compound as brown foam.

### Example 267:

### [2-Chloro-4-(2,4-difluoro-phenylamino)-phenyl]-[2-methyl-5-(1H-tetrazol-5-yl)-phenyl]-methanone (compound 367)

The reaction was carried out similarly as described in the preparation of compound 358, using compound 546 (0.84 mmol) as the nitrile. Purification was done by flash chromatography to afford the title compound as yellow solid. ¹³C NMR (DMSO-d₆) δ 194.2, 171.9, 158.9 (dd), 155.8 (dd), 149.7, 140.3, 139.7, 134.0, 133.9, 132.2, 128.7, 126.6, 126.0, 124.0 (dd), 121.7, 114.8, 112.0 (dd), 111.8, 105.0 (dd), 19.6

### Example 268:

### 3-{3-[2-chloro-4-(2,4-difluoro-phenylamino)-benzoyl]-4-methyl-phenyl}-3-oxo-propionic acid ethyl ester (compound 368)

A solution of potassium ethoxycarbonyl-acetate (136 mg, 0.80 mmol) in EtOAc (1.25 mL) was cooled to 0 °C in an ice-bath. Triethyl amine (279 µL, 2.0 mmol) and anhydrous magnesium chloride (91 mg, 0.96 mmol) was added to the solution and the mixture was stirred at 35 °C for 7 h. The reaction mixture was cooled to 0 °C and a solution of compound 439 (230 mg, 0.57 mmol) in EtOAc (1 mL) was added. The temperature was raised to RT and stirring was continued for 18 h. The reaction mixture was cooled to 0 °C and aqueous HCl (1.5 mL, 12%) was added slowly. The aqueous phase was separated a nd washed with EtOAc (10 mL). The organic phases were combined, washed with aqueous HCl (5 mL, 12%), aqueous NaHCO₃ (5 mL, 50%), water (5 mL) and brine (5 mL) and then dried (MgSO₄), filtered, and concentrated *in vacuo,* The crude product was purified by flash chromatography using DCM/petroleum ether 2:3 folloed by diethyl ether/petroleum ether 1:2 as the eluent to afford the title compound as yellow solid ¹³C NMR (CDCl₃) δ 195.2, 191.6, 167.3, 159.3 (dd), 155.6 (dd), 148.2, 144.2, 139.9, 135.4, 133.7, 133.6, 131.9, 130.5, 129.2, 128.7, 124.6 (d), 124.2. (dd), 116.2, 112.9, 111.7 (dd), 105.0 (t), 61.5, 46.0, 20.7, 14.1

### Example 269:

### [2-Chloro-4-(2,4-difluoro-phenylamino)-phenyl]-[5-(4,5-dihydro-oxazol-2-yl)-2-methyl-phenyl]-methanone (compound 369)

To a suspension of compound 115 (400 mg, 0.90 mmol) in DCM (4.0 mL) was added thionyl chloride (229 µL, 3.15 mmol) at the resulting mixture was stirred at RT for 1 h. Ice-water was added to the reaction mixture followed by EtOAc. The organic phase was separated, washed with brine, dried (MgSO₄), filtered, and concentrated *in vacuo.* The crude product was purified by continuous gradient flash chromatography using EtOAc/petroleum ether (40-60) (v:v = 5:95 to 40:60) as the eluent to afford the title compound as foam. ¹³C NMR (CDCl₃) δ 195.6, 164.0, 159.2 (dd), 155.5 (dd), 147.9, 141.4, 139.5, 135.3, 133.7, 131.5, 130.2, 129.1, 128.9, 125.2, 124.3 (m), 116.2, 112.8, 111.6 (dd), 105.0 (dd), 67.7, 54.9, 20.5

## Claims

1. A compound of general formula I 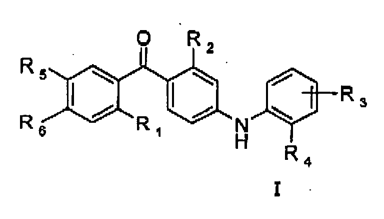 wherein
R₁ is halogen, hydroxy, mercapto, trifluoromethyl, amino, C₁₋₄alkyl, C₂₋₄alkenyl, C₂₋₄alkynyl, C₁₋₄alkoxy, C₁₋₄ alkylthio, C₁₋₆alkylamino, C₁₋₄alkoxycarbonyl, cyano,-CONH₂ or nitro;
R₂ is halogen, amino, C₁₋₄alkyl or nitro;
R₃ represents one or more same or different substituents selected from the group consisting of hydrogen, halogen, C₁₋₄alkyl or C₁₋₄alkoxy;
R₄ is fluoro;
one of R₅ and R₆ is -COOH, -C(O)NHOH, -C(O)NHNH₂, Y₂R₉, Y₂R₉Y₃R₁₀, C₁₋₆alkyl-Y₂R₉, C₁₋₆alkyl-Y₂R₉Y₃R₁₀, C₂₋₆alkenyl-Y₂R₉, C₂₋₆alkenyl-Y₂R₉Y₃R₁₀, Y₂R₉-C₁₋₆-alkyl-Y₃R₁₀, Y₂R₉-C₂₋₆-alkenyl-Y₃R₁₀, C₃₋₁₂carbocyclyl-Y₂R₉, C₃₋₁₂carbocyclyl-Y₂R₉Y₃R₁₀, C₁₋₁₂heterocyclyl-Y₂R₉, C₁₋₁₂-heterocyclyl-Y₂R₉Y₃R₁₀, C₃₋₁₂carbocycyl-C₁₋₆-alkyl-Y₂R₉, C₃₋₁₂carbocyclyl-C₁₋₆-alkyl-Y₂R₉Y₃R₁₀, C₁₋₁₂heterocyclyl-C₁₋₆-alkyl-Y₂R₉, C₁₋₁₂ heterocyclyl-C₁₋₆-alkyl-Y₂R₉Y₃R₁₀, C₃₋₁₂carbocyclyl-C₁₋₆-alkyl-Y₃R₁₀, C₁₋₁₂ heterocycyl-C₁₋₆-alkyl-Y₃R₁₀, C₁₋₁₂heterocyclyl-C₁₋₁₀alkyl, C₃₋₁₂carbocyclyl-C₁₋₁₀ alkyl, C₁₋₁₀alkyl-C₁₋₁₂heterocyclyl, C₁₋₁₀alkyl-C₃₋₁₂carbocyclyl, C₁₋₁₀alkyl, C₂₋₁₀ alkenyl, C₂₋₁₀ alkynyl, C₃₋₁₂carbocyclyl or C₁₋₁₂heterocyclyl, each of which being optionally substituted by one or more, same or different substituents represented by R₇, and the other is hydrogen, halogen, hydroxy, mercapto, trifluoromethyl, amino, C₁₋₄alkyl, C₂₋₄alkenyl, C₂₋₄alkynyl, C-₁₋₄alkoxy, C₁₋₄ alkylthio, C₁₋₆alkylamino, C₁₋₄alkoxycarbonyl, cyano, -CONH₂ or nitro;
with the proviso that when R₅ or R₆ is phenyl, C₁₋₅alkyl or C₂₋₃alkenyl, said R₅ or R₆ is substituted by one or more, same or different substituents represented by R₇ (except three fluorine when R₅ or R₆ is methyl),
R₇ is halogen, hydroxy, mercapto, trifluoromethyl, amino, C₁₋₄alkyl, C₁₋₆ hydroxyalkyl, C₁₋₄alkoxy, C₁₋₄alkylthio, C₁₋₆ alkylamino, C₁₋₄alkoxycarbonyl, C₁₋₉ trialkylammonium in association with an anion, cyano, azido, nitro, -S(O)₂NH₂,-S(O)₂NRₐR_{b}, -S(O)₂R, -COOH, -CONH₂, -NRₐC(O)R', -CONHR' or -CONRR', wherein R and R' are same or different, each representing hydrogen or C₁₋₃alkyl;
Rₐ, R_{b} and R_{c} are the same or different, each representing hydrogen, C₁₋₄alkyl, C_{2 4}alkenyl, C₂₋₄ alkynyl, C₃₋₈carbocyclyl, C₁₋₁₂heterocyclyl or aryl, each of C₁₋₄alkyl, C₂₋₄alkenyl, C₂₋₄ alkynyl, C₃₋₈carbocyclyl, C₁₋₁₂heterocyclyl or aryl being optionally substituted by one or more, same or different substituents represented by R₇;
Y₂ is -O-, -S-, -S(O)-, -S(O)₂-, -NRₐ-, -NRₐC(O)NR_{b}-, -NRₐC(O)-, -C(O)NRₐ-,-C(O)NRₐO-, -C(O)-, -NRₐC(O)O-, -NRₐS(O)₂-, -OC(O)-, -C(O)O-,-C(O)NRₐNR_{b}C(S)NR_{c}-, -C(O)NRₐNR_{b}-, or -S(O)₂NRₐ-;
R₉ is C₁₋₁₀alkyl-C₁₋₁₂heterocyclyl, C₁₋₁₀alkyl-C₃₋₁₂carbocyclyl, C₁₋₁₀alkyl, C₂₋₁₀ alkenyl, C₂₋₁₀ alkynyl, C₃₋₁₂carbocyclyl, C₁₋₁₂heterocyclyl, C₃₋₁₂carbocyclyl-C₁₋₁₀ alkyl, or C₁₋₁₂heterocyclyl-C₁₋₁₀alkyl, C₃₋₆carbocyclyl-C₁₋₆alkenyl, C₃₋₆carbocyclyl-C₂₋₆alkynyl, each being optionally substituted by one or more, same or different substituents represented by R₇,
with the proviso that when Y₂ is -O-, -NRₐ-, -S- or -C(O)O-, and R₉ is C₁₋₆alkyl, said C₁₋₆alkyl is substituted by one or more, same or different substituents represented by R₇ ;
Y₃ is -O-, -S-, -S(O)-, -S(O)₂-, -NRₐ-, -NRₐC(O)NR_{b}-, -NRₐC(O)-, -C(O)NRₐ-,-C(O)NRₐO-, -C(O)-, -NRₐC(O)O-, -NRₐS(O)₂-, -OC(O)- or -C(O)O-;
R₁₀ is C₁₋₁₀alkyl-C₁₋₁₂heterocyclyl, C₁₋₁₀alkyl-C₃₋₁₂carbocyclyl, C₁₋₄₀alkyl, C₂₋₁₀ alkenyl, C₂₋₁₀ alkynyl, C₃₋₁₂carbocyclyl or C₁₋₁₂heterocyclyl, each being optionally substituted by one or more, same or different substituents represented by R₇;
or, when one of R₅ or R₆ is the group -C(O)NRₐR₉, Rₐ and R₉ together with the nitrogen atom to which they are attached form a C₁₋₂₂heterocyclic ring optionally comprising one or more additional heteroatoms selected from the group consisting of O, 5 and N, optionally substituted with one or more substituents represented by R₇;
or a pharmaceutically acceptable salt, solvate, or ester thereof.

2. A compound according to claim 1, wherein R₁ is halogen, trifluoromethyl, C₁₋₄ alkyl, C₁₋₄alkoxy or nitro.

3. A compound according to claim 2, wherein R₁ is methyl, ethyl, methoxy, ethoxy, bromo, fluoro or chloro.

4. A compound according to any one of claims 1-3, wherein R₂ is Methyl, ethyl, nitro, bromo, fluoro or chloro.

5. A compound according to any one of claims 1-4, wherein R₃ is hydrogen, methyl, ethyl, methoxy, ethoxy, bromo, fluoro or chloro.

6. A compound according to any one of claims 1-5, wherein R₃ represents one substituent.

7. A compound according to claim 6, wherein R₃ is in the meta position with respect to R₄ and para with respect to -NH, or wherein R₃ is in the meta position with respect to R₄ and ortho with respect to -NH, or wherein R₃ is in the ortho position with respect to R₄ and meta with respect to -NH.

8. A compound according to any one of claims 1-7, wherein R₃ is fluorine.

9. A compound according to any one of claims 1-8, wherein R₇ is halogen, hydroxy, amino, -S(O)₂CH₃, trifluoromethyl, cyano, C₁₋₄hydroxyalkyl, C₁₋₄alkoxy, C₁₋₄alkyl, C₁₋₄alkylthio, C₁₋₄alkylamino, C₁₋₄alkoxycarbonyl, -COOH, -CONH₂, -S(O)₂NH₂, azido, -CONHR' or -CONRR', wherein R and R' are as indicated in claim 1.

10. A compound according to claim 9, wherein R₇ is methyl, ethyl, methoxy, ethoxy, hydroxy, methoxycarbonyl, ethoxycarbonyl, dimethylamino, ethylamino, amino, -COOH, fluoro, chloro, bromo, -CONH₂, -S(O)₂NH₂, azido, methylthio,-S(O)₂CH₃, trifluoromethyl, cyano or hydroxymethyl.

11. A compound according to any one of claims 1-10, wherein one of R₅ and R₆ is Y₂R₉, C₁₋₄alkyl-Y₂R₉, Y₂R₉Y₃R₁₀, C₁₋₄alkyl-Y₂R₉Y₃R₁₀, C₂₋₄alkenyl-Y₂R₉, C₂₋₄alkenyl-Y₂R₉Y₃R₁₀, Y₂R₉-C₁₋₄-alkyl-Y₃R₁₀, Y₂R₉-C₂₋₄-alkenyl-Y₃R₁₀, C₁₋₆heterocyclyl-C₁₋₄-alkyl-Y₂R₉, C₁₋₄alkyl-C₁₋₆heterocyclyl, C₁₋₄alkyl-C₃₋₆carbocyclyl, C₃₋₆carbocyclyl-C₁₋₄ alkyl, C₁₋₄alkyl substituted by R₇, C₂₋₄alkenyl, C₂₋₄alkynyl, C₃₋₆carbocycyl, C₁₋₆ heterocyclyl, -COOH, -C(O)NHOH, or C(O)NHNH₂, and the other is hydrogen, halogen, C₁₋₄alkyl or C₁₋₄alkoxy.

12. A compound according to claim 11, wherein R₅ is Y₂R₉, C₁₋₄alkyl-Y₂R₉, Y₂R₉Y₃R₁₀, C₁₋₄alkyl-Y₂R₉Y₃R₁₀, C₂₋₄alkenyl-Y₂R₉, C₂₋₄alkenyl-Y₂R₉Y₃R₁₀, Y₂R₉-C₁₋₄-alkyl-Y₃R₁₀, Y₂R₉-C₂₋₄-alkenyl-Y₃R₁₀, C₁₋₆heterocyclyl-C₁₋₄-alkyl-Y₂R₉, C₁₋₄alkyl-C₁. ₆heterocyclyl, C₁₋₄alkyl-C₃₋₆carbocyclyl, C₃₋₆carbocyclyl-C₁₋₄alkyl, C₁₋₄alkyl substituted by R₇, C₂₋₄alkenyl, C₂₋₄ alkynyl, C₃₋₆carbocyclyl, C₁₋₆heterocylcyl,-COOH, -C(O)NHOH, or C(O)NHNH₂, and R₆ is hydrogen, halogen, C₁₋₄alkyl or C₁₋₄ alkoxy.

13. A compound according to claim 11, wherein one of R₅ and R₆ is Y₂R₉, Y₂R₉Y₃R₁₀, phenyl, methylphenyl, propenyl, methyl-Y₂R₉, tetrazole, ethynyl, triazole, thiadiazole, dihydrooxazole, -COOH, -C(O)NHOH, or C(O)NHNH₂, and the other is hydrogen, fluoro, chloro, methyl or methoxy.

14. A compound according to any one of claims 1-13, wherein R₆ is hydrogen.

15. A compound according to any one of claims 1-11 or 13, wherein R₅ is hydrogen.

16. A compound according to any one of claims 1-15, wherein Y₂ is -O-, -NRₐ-,-NRₐC(O)NR_{b}-, -NRₐCCO)-, -C(O)NRₐ-, -C(O)NRₐO-, -C(O)-, -NRₐC(O)O-,-NRₐS(O)₂-, -C(O)NRₐNR_{b}- or -S(O)₂NRₐ-.

17. A compound according to any one of claims 1-16, wherein Y₃ is -O-,-NRₐC(O)-, -C(O)NRₐ-, -C(O)-, -C(O)O- or -NRₐC(O)O-.

18. A compound according to any one of claims 1-17, wherein R₉ is C₁₋₄alkyl-C₁₋₆ heterocyclyl, C₁₋₄alkyl-C₃₋₆carbocyclyl, C₁₋₆alkyl, C₂₋₄alkenyl, C₂₋₄ alkynyl, C₃₋₁₀ carbocyclyl, C₁₋₆heterocyclyl, C₃₋₆carbocyclyl-C₁₋₆alkyl, C₁₋₆heterocyclyl-C₁₋₆alkyl, C₃₋₆carbocyclyl-C₂₋₄alkenyl or C₃₋₆carbocyclyl-C₂₋₄alkynyl.

19. A compound according to claims 1-18, wherein R₉ is C₁₋₄heterocyclyl, C₁₋₆ alkyl, C₁₋₃alkyl-C₁₋₅heterocyclyl, C₆₋₁₀carbocyclyl, C₁₋₃alkyl-C₆carbocyclyl, C₃alkenyl, C₆carbocyclyl-C₁alkyl, C₆carbocyclyl-C₃alkenyl or C₆carbocyclyl-C₂alkynyl.

20. A compound according to claim 19, wherein R₉ is morpholinyl, propylmorpholinyl, piperazinyl, methyl, ethyl, n-propyl, n-butyl, *tert*-butyl, isobutyl, hexyl, isopropyl, dimethylpropyl, methyltetrahydrofuranyl, methylpyridinyl, ethylpiperazinyl, cyclohexyl, propyloxopyrrolidinyl, benzyl, methylcyclohexyl, propylphenyl, ethylmorpholinyl, allyl, ethylfuranyl, phenyl, methyldioxoimidazolidinyl, dioxohexahydropyrimidinyl, thiazolyl, methylphenyl, ethylphenyl, methyldioxolanyl, methylthiazolyl, propenylphenyl, methylfuranyl, thiophenyl, tetrahydropyranyl or ethynylphenyl.

21. A compound according to any one of claims 1-20, wherein R₁₀ is C₁₋₄alkyl, C₂₋₄ alkenyl, C₃₋₆carbocyclyl or C₁₋₆heterocycyl.

22. A compound according to claim 21, wherein R₁₀ is methyl, ethyl, methacryl, tert-butyl, tetrahydropyranyl or ethenyl.

23. A compound according to any one of claims 1-22 wherein said heterocycle or heterocyclyl contains one or two oxygen atoms or one sulphur atom, and/or up to two nitrogen atoms, or three or four nitrogen atoms, wherein optionally one or two CH₂ ring fragments is/are replaced by one or two -C(O)- fragments respectively.

24. A compound according to any one of claims 1-23, wherein Ra, Rb, or Rc independently represent hydrogen, methyl, ethyl, 2-hydroxyethyl or 2-methoxyethyl.

25. A compound according to any one of claims 1-24 selected from the group consisting of
3-[2-Chloro-4-(2,4-difluorophenylamino)benzoyl]-*N*-(2-hydroxyethyl)-4-methylbenzamide (Compound 115),
3-[2-Chloro-4-(4-chloro-2-fluoro-phenylamino)-benzoyl]-*N*-(2-hydroxy-ethyl)-4-methyl-benzamide (Compound 120),
3-[2-Chloro-4-(4-chloro-2-fluoro-phenylamino)-benzoyl]-4,*N*-dimethyl-benzamide (Compound 121),
(2-{3-[2-Chloro-4-(4-chloro-2-fluoro-phenylamino)-benzoyl]-4-methyl-benzoylamino}-acetylamino)-acetic acid ethyl ester (Compound 122),
{3-[2-Chloro-4-(4-chloro-2-fluoro-phenylamino)-benzoyl]-4-methyl-benzoylamino}-acetic acid ethyl ester (Compound 123),
3-[2-Chloro-4-(4-chloro-2-fluoro-phenylamino)-benzoyl]-*N*-(2-methoxy-ethyl)-4-methyl-benzamide (Compound 124),
3-[2-Chloro-4-(4-chloro-2-fluoro-phenylamino)-benzoyl]-*N*-cyclohexyl-4-methyl-benzamide (Compound 125),
3-[2-Chloro-4-(4-chloro-2-fluoro-phenylamino)-benzoyl]-*N*-ethyl-4-methyl-benzamide (Compound 126),
3-[2-Chloro-4-(4-chloro-2-fluoro-phenylamino)-benzoyl]-*N*-(6-hydroxy-hexyl)-4-methyl-benzamide (Compound 127),
3-[2-Chloro-4-(4-chloro-2-fluoro-phenylamino)-benzoyl]-*N*-isopropyl-4-methyl-benzamide (Compound 128),
3-[2-Chloro-4-(4-chloro-2-fluoro-phenylamino)-benzoyl]-*N*-isobutyl-4-methyl-benzamide (Compound 129),
3-[2-Chloro-4-(4-chloro-2-fluoro-phenylamino)-benzoyl]-*N*-(2,2-dimethyl-propyl)-4-methyl-benzamide (Compound 130),
3-[2-Chloro-4-(4-chloro-2-fluoro-phenylamino)-benzoyl]-*N*-(3-methoxy-propyl)-4-methyl-benzamide (Compound 131),
3-[2-Chloro-4-(4-chloro-2-fluoro-phenylamino)-benzoyl]-4-methyl-*N*-[3-(2-oxo-pyrrolidin-1-yl)-propyl]-benzamide (Compound 132),
3-[2-Chloro-4-(4-chloro-2-fluoro-phenylamino)-benzoyl]-*N*-(2-dimethylamino-ethyl)-4-methyl-benzamide (Compound 133),
2-Methyl-acrylic acid 2-{3-[2-chloro-4-(4-chloro-2-fluoro-phenylamino)-benzoyl]-4-methyl-benzoylamino}-ethyl ester (Compound 134),
3-[2-Chloro-4-(4-chloro-2-fluoro-phenylamino)-benzoyl]-*N*-cis-(4-hydroxy-cyclohexyl)-4-methyl-benzamide (Compound 135),
3-[2-Chloro-4-(4-chloro-2-fluoro-phenylamino)-benzoyl]-*N*-trans-(4-hydroxy-cyclohexyl)-4-methyl-benzamide (Compound 136),
(2-{3-[2-Chloro-4-(4-chloro-2-fluoro-phenylamino)-benzoyl]-4-methyl-benzoylamino}-ethyl)-carbamic acid tert-butyl ester (Compound 137),
*N*-(2-Amino-ethyl)-3-[2-chloro-4-(4-chloro-2-fluoro-phenylamino)-benzoyl]-4-methyl-benzamide (Compound 138),
(2-{3-[2-Chloro-4-(4-chloro-2-fluoro-phenylamino)-benzoyl]-4-methyl-benzoylamino}-acetylamino)-acetic acid (Compound 139),
3-[2-Chloro-4-(2,4-difluoro-phenylamino)-benzoyl]-*N*-(2-hydroxy-ethyl)-4-methoxy-benzamide (compound 140),
3-[2-Chloro-4-(2,4-difluoro-phenylamino)-benzoyl]-*N*-(2,2-difluoro-ethyl)-4-methoxy-benzamide (compound 141),
3-[2-Chloro-4-(2,4-difluoro-phenylamino)-benzoyl]-*N*-(2-fluoro-ethyl)-4-methoxy-benzamide (compound 142),
3-[2-Chloro-4-(2,4-difluoro-phenylamino)-benzoyl]-*N*-(2,3-dihydroxy-propyl)-4-methoxy-benzamide (compound 143),
*N*-Carbamoylmethyl-3-[2-chlorb-4-(2,4-difluoro-phenylamino).benzoyl].4-methoxy-benzamide (compound 144),
*N*-Carbamoylmethyl-3-[2-chloro-4-(2,4-difluoro-phenylamino)-benzoyl]-4-methyl-benzamide (Compound 145),
*N*-Benzyl-3-[2-chloro-4-(2,4-difluoro-phenylamino)-benzoyl]-4-methyl-benzamide (compound 146),
3-[2-Chloro-4-(2,4-difluoro-phenylamino)-benzoyl]-*N*-(2-fluoro-ethyl)-4-methyl-benzamide (compound 147),
3-[2-Chloro-4-(2,4-difluoro-phenylamino)-benzoyl]-4-methyl-*N*-(2,2,2.trifluoro-ethyl)-benzamide (compound 148),
3-[2-Chloro-4-(2,4-difluoro-phenylamino)-benzoyl]-*N*-ethyl-4-methyl-benzamide (compound 149),
3-[2-Chloro-4-(2,4-difluoro-phenylamino)-benzoyl]-*N*-cyoohexylmethyl-4-methyl-benzamide (compound 150),
3-[2-Chloro-4-(2,4-difluoro-phenylamino)-benzoyl]-*N*-(2-hydroxy-propyl)-4-methyl-benzamide (compound 151),
3-[2-Chloro-4-(2,4-difluoro-phenylamino)-benzoyl]-*N*-(2,3-dihydroxy-propyl)-4-methyl-benzamide (compound 152),
3-[2-Chloro-4-(2,4-difluoro-phenylamino)-benzoyl]-*N*-(1-hydroxymethyl-propyl)-4-methyl-benzamide (compound 153),
3-[2-Chloro-4-(2,4-difluoro-phenylamino)-benzoyl]-4-methyl-*N*-(2,2,3,3,3-pentafluoro-propyl)-benzamide (compound 154),
3-[2-Chloro-4-(2,4-difluoro-phenylamino)-benzoyl]-*N*-(3-hydroxy-propyl)-4-methyl-benzamide (compound 155),
3-[2-Chloro-4-(2,4-difluoro-phenylamino)-benzoyl]-*N*-(2-hydroXy-I,I-dimethyl-ethyl)-4-methyl-benzamide (compound 156),
3-[2-Chloro-4-(2,4-difluoro-phenylamino)-benzoyl]-*N*-(2-hydroxy-1-hydroxymethyl-1-methyl-ethyl)-4-methyl-benzamide (compound 157),
{3-[2-Chloro-4-(2,4-difluoro-phenylamino)-benzoyl]-4-methyl-benzoylamino}-acetic acid ethyl ester (compound 158),
3-[2-Chloro-4-(2,4-difluoro-phenylamino)-benzoyl]-*N*-(4-hydroxy-butyl)-4-methyl-benzamide (compound 159),
3-[2-Chloro-4-(2,4-difluoro-phenylamino)-benzoyl]-*N*-(3-hydroxy-1,1-dimethyl-butyl)-4-methyl-benzamide (compound 160),
3-[2-Chloro-4-(2,4-difluoro-phenylamino)-banzoyl]-4-methyl-*N*-(3-phenyl-propyl)-benzamide (compound 161),
(R)-3-[2-Chloro-4-(2,4-difluoro-phenylamino)-benzoyl]-*N*-(1-hydroxymethyl-3-methyl-butyl)-4-methyl-benzamide (compound 162),
3-[2-Chloro-4-(2,4-difluoro-phenytamino)-benzoyl]-*N*-isopropyl-4-methyl-benzamide (compound 164),
3-[2-Chloro-4-(2,4-difluoro-phenylamino)-benzoyl]-*N*-cyclohexyl-4-methyl-benzamide (compound 165),
3-[2-Chloro-4-(2,4-difluoro-phenylamino)-benzoyl]-*N*-(2,2-difluoro-ethyl)-4-methyl-benzamide (compound 166),
5-{3-[2-Chloro-4-(2,4-difluoro-phenylamino)-benzoyl]-4-methyl-benzoylamino}-4-oxo-pentanoic acid methyl ester (compound 167),
*N*-[(2-Carbamoyl-ethylcarbamoyl)-methyl]-3-[2-chloro-4-(2,4-difluoro-phenylamino)-benzoyl]-4-methyl-benzamide (compound 168),
(2-{3-[2-Chloro-4-(2,4-difluoro-phenylamino)-benzoyl]-4-methyl-benzoylamino}-acetylamino)-acetic acid ethyl ester (compound 169),
*N*-Allyl-3-[2-chloro-4-(2,4-difluoro-phenylamino)-benzoyl]-4-methyl-benzamide (compound 170),
3-[2-Chloro-4-(2,4-difluoro-phenylamino)-benzoyl]-4-methyl-*N*-(2-sulfamoyl-ethyl)-benzamide (compound 171),
*N*-(2-Acatylamino-ethyl)-3-[2-chloro-4-(2,4-difluoro-phenylamino)-benzoyl]-4-methyl-benzamide (compound 172),
3-[2-Chloro-4-(2,6-difluoro-phenylamino)-benzoyl]-*N*-(2-hydroxy-ethyl)-4-methoxy-benzamide (compound 173),
3-[2-Chloro-4-(2,6-difluoro-phenylamino)-benzoyl]-*N*-(2-fluoro-ethyl)-4-methoxy-benzamide (compound 174),
3-[2.Chloro-4-(2,6-difluoro-phenylamino)-benzoyl]-*N*-(2,3-dihydroxy-propyl)-4-methoxy-benzamide (compound 175),
3-[2-Chloro-4-(2,6-difluoro-phenylamino)-benzoyl]-*N*-(3-hydroxy-propyl)-4-methoxy-benzamide (compound 176),
3-[2-Chloro-4-(2,6-difluoro-phenylamino)-benzoyl]-4-methoxy-*N*-phenethyl-benzamide (compound 177),
3-[2-Chloro-4-(2,6-difluoro-phenylamino)-benzoyl]-*N*-(2-hydroxy-1,1-dimethyl-ethyl)-4-methoxy-benzamide (compound 178),
3-[2-Chloro-4-(2,6-difluoro-phenylamino)-benzoyl]-4-methoxy-*N*-(2-morpholin-4-yl-ethyl)-benzamide (compound 179),
3-[2-Chloro-4-(2,6-difluoro-phenylamino)-benzoyl]-*N*-(2-hydroxy-1-hydroxymethyl-1-methyl-ethyl)-4-methoxy-benzamide (compound 180),
3-[2-Chloro-4-(2,6-difluoro-phenylamino)-benzoyl]-*N*-(2-hydroxy-ethyl)-4-methoxy-*N*-methyl-benzamide (compound 181),
{3-[2-Chloro-4-(2,6-difluoro-phenylamino)-benzoyl]-4-methoxy-benzoylamino}-acetic acid ethyl ester (compound 182),
(2-{3-[2-Chloro-4-(2,6-difluoro-phenylamino)-benzoyl]-4-methoxy-benzoylamino}-acetylamino)-acetic acid ethyl ester (compound 183),
3-[2-Chloro-4-(2,6-difluoro-phenylamino)-benzoyl]-*N*,*N*-bis-(2-hydroxy-ethyl)-4-methoxy-benzamide-(compound 184),
3-[2-Chloro-4-(2,6-difluoro-phenylamino)-benzoyl]-4-methoxy-*N*,*N*-bis-(2-methoxy-ethyl)-benzamide (compound 185);
4-Chloro-3-[2-chloro-4-(2,4-difluoro-phenylamino)-benzoyl]-*N*-(2-hydroxy-ethyl)-benzamide (compound 204),
*N*-{3-[2-Chloro-4-(2,4-difluoro-phenylamino)-benzoyl]-4-methyl-phenyl}-3-methoxy-propionamide (compound 241),
*N*-{3-[2-Chloro-4-(2,4-difluoro-phenylamino)-benzoyl]-4-methyl-phenyl}-propionamide (compound 242),
*N*-{3-[2-Chloro-4-(2,4-difluoro-phenylamino)-benzoyl]-4-methyl-phenyl}-2-(2-methoxy-ethoxy)-acetamide (compound 243),
*N*-{3-[2-Chloro-4-(2,4-difluoro-phenylamino)-benzoyl]-4-methyl-phenyl}-3-morpholin-4-yl-propionamide (compound 244),
*N*-{3-[2-Chloro-4-(2,4-difluoro-phenylamino)-benzoyl]-4-methyl-phenyl}-3-hydroxy-propionamide (compound 245),
*N*-{3-[2-Chloro-4-(2,4-difluoro-phenylamino)-benzoyl]-4-methyl-phenyl}-3-furan-2-yl-propionamide (compound 246),
*N*-{3-[2-Chloro-4-(2,4-difluoro-phenylamino)-benzoyl]-4-methyl-phenyl}-2-hydroxy-benzamide (compound 247),
*N*-{3-[2-Chloro-4-(2,4-difluoro-phenylamino)-benzoyl]-4-methyl-phenyl}-2-(2,5-dioxo-imidazolidin-4-yl)-acetamide (compound 248),
2,6-Dioxo-hexahydro-pyrimidine-4-carboxylic acid {3-[2-chloro-4-(2,4-difluoro-phenylamino)-benzoyl]-4-methyl-phenyl}-amide (compound 249),
Acrylic acid 2-{3-[2-chloro-4-(2,4-difluoro-phenylamino)-benzoyl]-4-methyl-phenylcarbamoyl}-ethyl ester (compound 250),
*N*-{3-[2-Chloro-4-(2,4-difluoro-phenylamino)-benzoyl]-4-methyl-phenyl}-3-methylsulfanyl-propionamide (compound 251),
*N*-{3-[2-Chloro-4-(2,4-difluoro-phenylamino)-benzoyl]-4-methyl-phenyl}-3-methanesulfonyl-propionamide (compound 252),
Ethanesulfonic acid {3-[2-chloro-4-(2,4-difluoro-phenylamino)-benzoyl]-4-methyl-phenyl}-amide (compound 253),
*N*-{3-[2-Chloro-4-(2,4-difluoro-phenylamino)-benzoyl]-4-methyl-phenyl}-4-methoxy-benzenesulfonamide (compound 254),
*N*-(5-{3-[2-Chloro-4-(2,4-difluoro-phenylamino)-benzoyl]-4-methyl-phenylsulfamoyl}-4-methyl-thiazol-2-yl)-acetamide (compound 255),
5-Acetyl-2-chloro-*N*-{3-[2-chloro-4-(2,4-difluoro-phenylamino)-benzoyl]-4-methyl-phenyl}-benzenesulfonamide (compound 256),
Naphthalene-2-sulfonic acid {3-[2-chloro-4-(2,4-difluoro-phenylamino)-benzoyl]-4-methyl-phenyl}-amide (compound 257),
*N*-{3-[2-Chloro-4-(2,4-difluoro-phenylamino)-benzoyl]-4-methyl-phenyl}-C-phenyl-methanesulfonamide (compound 258),
2-Methyl-acryilc acid 2-(3-{3-[2-chloro-4-(2,4-difluoro-phenylamino)-benzoyl]-4-methyl-phenyl}-ureido)-ethyl ester (compound 259),
1-{3-[2-Chloro-4-(2,4-difluoro-phenylamino)-benzoyl]-4-methyl-phenyl}-3-(2-hydroxy-ethyl)-urea (compound 260),
(3-{3-[2-Chloro-4-(2,4-difluoro-phenylamino)-benzoyl]-4-methyl-phenyl}-ureido)-acetic acid ethyl ester (compound 261),
1-{3-[2-Chloro-4-(2,4-difluoro-phenylamino)-benzoyl]-4-methyl-phenyl}-3-(3-methoxy-phenyl)-urea (compound 262),
1-{3-[2-Chloro-4-(2,4-difluoro-phenylamino)-benzoyl]-4-methyl-phenyl}-3-(3-trifluoromethyl-phenyl)-urea (compound 263),
1-{3-[2-Chloro-4-(2,4-difluoro-phenylamino)-benzoyl]-4-methyl-phenyl}-3-propyl-urea (compound 264),
3-(3-{3-[2-Chloro-4-(2,4-difluoro-phenylamino)-benzoyl]-4-methyl-phenyl}-ureido)-propionic acid ethyl ester (compound 265),
1-{3-[2-Chloro-4-(2,4-difluoro-phenylamino)-benzoyl]-4-methyl-phenyl}-3-cyclohexyl-urea (compound 266),
1-Allyl-3-{3-[2-chloro-4-(2,4-difluoro-phenylamino)-benzoyl]-4-methyl-phenyl}-urea (compound 267),
1-Benzyl-3-{3-[2-chloro-4-(2,4-difluoro-phenylamino)-benzoyl]-4-methyl-phenyl}-urea (compound 268),
1-{3-[2-Chloro-4-(2,4-difluoro-phenylamino)-benzoyl]-4-methyl-phenyl}-3-ethyl-urea (compound 269),
1-{3-[2-Chloro-4-(2,4-difiuoro-phenylamino)-benzoyl]-4-methyl-phenyl}-3-phenyl-urea (compound 270),
1-Butyl-3-{3-[2-chloro-4-(2,4-difluoro-phenylamino)-benzoyl]-4-methyl-phenyl}-urea (compound 271),
1-{3-[2-Chloro-4-(2,4-difluoro-phenylamino)-benzoyl]-4-methyl-phenyl}-3-phenethyl-urea (compound 272),
2-(3-{3-[2-chloro-4-(2,4-difluoro-phenylamino)-benzoyl]-4-methyl-phenyl}-ureido)-benzoic acid methyl ester (compound 273),
1-{3-[2-Chloro-4-(2,4-difluoro-phenylamino)-benzoyl]-4-methyl-phenyl}-3-(3-cyano-phenyl)-urea (compound 274),
1-{3-[2-chloro-4-(2,4-difluoro-phenylamino)-benzoyl]-4-methyl-phenyl}-3-isopropyl-urea (compound 275),
1-{3-[2-Chloro-4-(2,4-difluoro-phenylamino)-benzoyl]-4-methyl-phenyl}-3-(4-methoxy-phenyl)-urea (compound 276),
{3-[2-Chloro-4-(2,4-difluoro-phenylamino)-benzoyl]-4-methyl-phenyl}-carbamic acid benzyl ester (compound 277),
{3-[2-Chloro-4-(2,4-difluoro-phenylamino)-benzoyl]-4-methyl-phenyl}-carbamic acid allyl ester (compound 278),
{3-[2-Chloro-4-(2,4-difluoro-phenylamino)-benzoyl]-4-methyl-phenyl}-carbamic acid ethyl ester (compound 279),
[2-Chloro-4-(2,4-difluoro-phenylamino)-phenyl]-[5-(3-hydroxy-butylamino)-2-methyl-phenyl]-methanone (compound 281),
[2-Chloro-4-(2,4-difluoro-phenylamino)-phenyl]-(3'-hydroxymethyl-4-methyl-biphenyl-3-yl)-methanone (compound 282),
[2-Chloro-4-(2,4-difluoro-phenylamino)-phenyl]-(3'-hydroxy-4-methyl-biphenyl-3-yl)-methanone (compound 283),
[2-Chloro-4-(2,4-difluoro-phenylamino)-phenyl]-(4'-methoxy-4-methyl-biphenyl-3-yl)-methanone (compound 284),
*N*-{3'-[2-Chloro-4-(2,4-difluoro-phenylamino)-benzoyl]-4'-methyl-biphenyl-3-yl}-acetamide (compound 285),
[2-Chloro-4-(2,4-difluoro-phenylamino)-phenyl]-(4-methyl-3'-trifluoromethoxy-biphenyl-3-yl)-methanone (compound 286),
[2-Chloro-4-(2,4-difluoro-phenylamino)-phenyl]-(3',4',5'-trifluoro-4-methyl-biphenyl-3-yl)-methanone (compound 288),
[2-Chloro-4-(2,4-difluoro-phenylamino)-phenyl]-(3',4'-dimethoxy-4-methyl-biphenyl-3-yl)-methanone (289),
3'-[2-Chloro-4-(2,4-difluoro-phenylamino)-benzoyl]-4'-methyl-biphenyl-3-carbonitrile (compound 290),
3-[2-Chloro-4-(2,4-difluoro-phenylamino)-benzoyl]-*N*-(2-hydroxy-ethyl)-4-methyl-benzenesulfonamide (compound 291),
3-[2-Chloro-4-(2,4-difluoro-phenylamino)-benzoyl]-4-methyl-*N*-(2-morpholin-4-yl-ethyl)-benzenesulfonamide (compound 292),
*N*-Allyl-3-[2-chloro-4-(2,4-difluoro-phenylamino)-benzoyl]-4-methyl-benzenesulfonamide (compound 293),
*N*-(2-{3-[2-Chloro-4-(2,4-difluoro-phenylamino)-benzoyl]-4-methyl-benzenesulfonylamino}-ethyl)-acetamide (compound 294),
3-[2-Chloro-4-(2,4-difluoro-phenylamino)-benzoyl]-4-methyl-*N*-propyl-benzenesulfonamide (compound 295),
3-[2-Chloro-4-(2,4-difluoro-phenylamino)-benzoyl]-*N*-(2,3-dihydroxy-propyl)-4-methyl-benzenesulfonamide (compound 296),
3-[2-Chloro-4-(2,4-difluoro-phenylamino)-benzoyl]-*N*-(2-methoxy-ethyl)-4-methyl-benzenesulfonamide (compound 297),
[4-(2,4-Difluoro-phenylamino)-2-nitro-phenyl]-[5-(4-methoxy-benzyloxy)-2-methyl-phenyl]-methanone (Compound 306),
[4-(2,4-Difluoro-phenylamino)-2-nitro-phenyl]-[5-(3-hydroxy-propoxy)-2-methyl-phenyl]-methanone (Compound 307),
[2-Amino-4-(2,4-difluoro-phenylamino)-phenyl]-[5-(3-hydroxy-propoxy)-2-methyl-phenyl]-methanone-(compound-308),
[4-(2,4-Difluoro-phenylamino)-2-nitro-phenyl]-[2-methyl-5-(2-morpholin-4-yl-ethoxy)-phenyl]-methanone (compound 309),
[2-Amino-4-(2,4-difluoro-phenylamino)-phenyl]-[2-methyl-5-(2-morpholin-4-yl-ethoxy)-phenyl]-methanone (compound 310),
[4-(2,4-Difluoro-phenylamino)-2-nitro-phenyl]-[5-(2,2-dimethyl-[1,3]dioxolan-4-ylmethoxy)-2-methyl-phenyl]-methanone (compound 311),
[4-(2,4-Difluoro-phenylamino)-2-nitro-phenyl]-[5-(2,3-dihydroxy-propoxy)-2-methyl-phenyl]-methanone (compound 312),
[2-Amino-4-(2,4-difluoro-phenylamino)-phenyl]-[5-(2,3-dihydroxy-propoxy)-2-methyl-phenyl]-methanone (compound 313),
[2-Chloro-4-(2,4-difluoro-phenylamino)-phenyl]-[2-fluoro-5-(3-hydroxy-propoxy)-phenyl]-methanone (compound 314),
[2-Chloro-4-(2,4-difluoro-phenylamino)-phenyl]-[5-(2,2-dimethyl-[1,3]dioxolan-4-ylmethoxy)-2-fluoro-phenyl]-methanone (compound 315),
[2-Chloro-4-(2,4-difluoro-phenylamino)-phenyl]-[5-(2,3-dihydroxy-propoxy)-2-fluoro-phenyl]-methanone (Compound 316),
[2-Chloro-4-(2,6-difluoro-phenylamino)-phenyl]-[2-chloro-5-(2-morpholin-4-yl-ethoxy)-phenyl]-methanone (compound 328),
(±)-[2-Chloro-4-(2,6-difluoro-phenylamino)-phenyl]-[2-chloro-5-(2,3-dihydroxy-propoxy)-phenyl]-methanone (compound 329),
[5-(3-Bromo-propoxy)-2-chloro-phenyl]-[2-chloro-4-(2,6-difluoro-phenylamino)-phenyl]-methanone (compound 330),
[2-Chloro-4-(2,4-difluoro-phenylamino)-phenyl]-(5-hydroxymethyl-2-methyl-phenyl)-methanone (compound 331),
[2-chloro-4-(2,4-difluoro-phenylamino)-phenyl]-(5-chloromethyl-2-methyl-phenyl)-methanone (compound 332),
(5-Azidomethyl-2-methyl-phenyl)-[2-chloro-4-(2,4-difluoro-phenylamino)-phenyl]-methanone (compound 333),
(5-Aminomethyl-2-methyl-phenyl)-[2-chloro-4-(2,4-difluoro-phenylamino)-phenyl]-methanone (compound 334),
[2-Chloro-4-(2,4-difluoro-phenylamino)-phenyl]-(5-hydroxymethyl-2-methoxy-phenyl)-methanone (compound 335),
Acetic acid 3-[2-chloro-4-(2,4-difluoro-phenylamino)-benzoyl]-4-methoxy-benzyl ester (compound 336),
*N*-*tert*-Butoxy-3-[2-chloro-4-(2,4-difluoro-phenylamino)-benzoyl]-4-methoxy-benzamide (compound 337),
3-[2-Chloro-4-(2,4-difluoro-phenylamino)-benzoyl]-*N*-methoxy-4-methyl-benzamide (compound 338),
N-Butoxy-3-[2-chloro-4-(2,4-difluoro-phenylamino)-benzoyl]-4-methyl-benzamide (compound 339),
3-[2-Chloro-4-(2,4-difluoro-phenylamino)-benzoyl]-*N*-cyclohexylmethoxy-4-methyl-benzamide (compound 340),
3-[2-Chloro-4-(2,4-difluoro-phenylamino)-benzoyl]-4-methyl-*N*-(2-methyl-thiazol-4-ylmethoxy)-benzamide (compound 341),
N-benzyloxy-3-[2-chloro-4-(2,4-difluoro-phenylamino)-benzoyl]-4-methyl-benzamide (compound 342),
3-[2-Chloro-4-(2,4-difluoro-phenylamino)-benzoyl]-*N*-(4-methoxy-benzyloxy)-4-methyl-benzamide (compound 343),
3-[2-Chloro-4-(2,4-difluoro-phenylamino)-benzoyl]-4-methyl-benzoic acid *N',N'-*dimethyl-hydrazide (compound 344),
3-[2-Chloro-4-(2,4-difluoro-phenylamino)-benzoyl]-4-methyl-*N*-morpholin-4-yl-benzamide (compound 345),
3-[2-Chloro-4-(2,4-difluoro-phenylamino)-benzoyl]-*N*-hydroxy-4-methyl-benzamide (compound 346),
4-[2-Chloro-4-(2,4-difluoro-phenylamino)-benzoyl]-*N*-(2-hydroxy-ethyl)-3-methyl-benzamide (compound 347),
[2-Chloro-4-(2,4-difluoro-phenylamino)-phenyl]-[5-(3-hydroxy-propenyl)-2-methyl-phenyl]-methanone (compound 348),
4-{3-[2-Chloro-4-(2,4-difluoro-phenylamino)-benzoyl]-4-methyl-benzoylamino}-thiophene-3-carboxylic acid methyl ester (compound 349),
3-[2-Chloro-4-(2,4-difluoro-phenylamino)-benzoyl]-*N*-furan-2-ylmethyl-4-methyl-benzamide (compound 350),
3-[2-Chloro-4-(2,4-difluoro-phenylamino)-benzoyl]-*N*-(3-methoxy-phenyl)-4-methyl-benzamide (compound 351),
2-{3-[2-Chloro-4-(2,4-difluoro-phenylamino)-benzoyl]-4-methyl-benzoylamino}-benzoic acid methyl ester (compound 352),
3-{3-[2-Chloro-4-(2,4-difluoro-phenylamino)-benzoyl]-4-methyl-benzoylamino}-thiophene-2-carboxylic acid methyl ester (compound 353),
4-{3-[2-Chloro-4-(2,4-difluoro-phenylamino)-benzoyl]-4-methyl-benzoylamino}-thiophene-3-carboxylic acid (compound 354),
2-{3-[2-Chloro-4-(2,4-difluoro-phenylamino)-benzoyl]-4-methyl-benzoylamino}-benzoic acid (compound 355),
3-[2-Chloro-4-(2,4-difluoro-phenylamino)-benzoyl]-*N*-[2-(2-hydroxy-ethylcarbamoyl)-phenyl]-4-methyl-benzamide (compound 356),
3-{3-[2-Chloro-4-(2,4-difluoro-phenylamino)-benzoyl]-4-methyl-benzoylamino}-thiophene-2-carboxylic acid (2-hydroxy-ethyl)-amide (compound 357),
[2-Chloro-4-(2,4-difluoro-phenylamino)-phenyl]-(5-ethynyl-2-methyl-phenyl)-methanone (compound 362),
3-[2-Chloro-4-(2,4-difluoro-phenylamino)-benzoyl]-4-methyl-benzoic acid hydrazide (compound 364),
1-{3-[2-Chloro-4-(2,4-difluoro-phenylamino)-benzoyl]-4-methyl-benzoyl}-4-ethyl-3-thio semicarbazide (compound 365),
[2-Chloro-4-(2,4-difluoro-phenylamino)-phenyl]-[5-(5-ethylamino-[1,3,4]thiadiazol-2-yl)-2-methyl-phenyl]-methanone (compound 366),
[2-Chloro-4-(2,4-difluoro-phenylamino)-phenyl]-[2-methyl-5-(1H-tetrazol-5-yl)-phenyl]-methanone (compound 367),
3-{3-[2-Chloro-4-(2,4-difluoro-phenylamino)-benzoyl]-4-methyl-phenyl}-3-oxo-propionic acid ethyl ester (compound 368),
[2-chloro-4-(2,4-difluoro-phenylamino)-phenyl]-[5-(4,5-dihydro-oxazol-2-yl)-2-methyl-phenyl]-methanone (compound 369),
3-[2-Chloro-4-(2,4-difluorophenylamino)benzoyl]-4-methylbenzoic acid (Compound 424),
2-Methylacrylic acid 2-{3-[2-chloro-4-(2,4-difluorophenylamino)benzoyl]-4-methylbenzoylamino}ethyl ester (Compound 425),
3-[2-Chloro-4-(4-chloro-2-fluoro-phenylamino)-benzoyl]-4-methyl-benzoic acid (Compound 432),
3-[2-Chloro-4-(2,4-difluoro-phenylamino)-benzoyl]-4-methoxy-benzoic acid (compound 437),
3-[2-Chloro-4-(2,6-difluoro-phenylamino)-benzoyl]-4-methoxy-benzoic acid (compound 443),

26. A pharmaceutical composition comprising a compound according to any one of claims 1-25 or a pharmaceutically acceptable salt or ester thereof together with a pharmaceutically acceptable vehicle or excipient.

27. A composition according to claim 26 further comprising another active component selected from the group consisting of glucocorticoids, vitamin D analogues, anti-histamines, platelet activating factor (PAF) antagonists, anticolergenic agents, methyl xanthines, β-adregenic agents, COX-2 inhibitors, salicylates, indomethacin, flufenamate, naproxen, timegadine, gold salts, peniciliamine, serum cholesterol reducing agents, retinoids, zinc salts and salicylazosulfapyridin.

28. A compound according to any one of claims 1-25 for use as a medicament.

29. A compound according to any one of claims 1-25 for use as an anti-inflammatory agent or anticancer agent.

30. A compound according to any one of claims 1-25 for use in the prevention, treatment or amelioration of ophthalmic diseases or conditions.

31. Use of a compound according to any one of claims 1-25 for the manufacture of a medicament for the prophylaxis, treatment or amelioration of inflammatory diseases or conditions, or ophthalmic diseases or conditions.

32. Use of a compound according to any one of claims 1-25 for the manufacture of a medicament for the treatment or amelioration of cancer.

33. The use of claim 31, wherein the medicament is intended for administration together with another active component selected from the group consisting of glucocorticoids, vitamin D analogues, anti-histamines, platelet activating factor (PAF) antagonists, anticholinergenic agents, methyl xanthines, β-adregenic agents, COX-2 inhibitors, salicylates, indomethacin, flufenamate, naproxen, timegadine, gold salts, peniciliamine, serum cholesterol reducing agents, retinoids, zinc salts and salicylazosulfapyridin.

34. The use of claim 31 or 33, wherein the inflammatory disease or condition is asthma, allergy, arthritis, rheumatoid arthritis, spondyloarthritis, gout, atherosclerosis, chronic inflammatory bowel disease, Crohn's disease, neurological inflammations, inflammatory eye diseases, proliferative and inflammatory skin disorders, psoriasis, atopic dermatitis, acne, uveitis, sepsis, septic shock or acne, osteoporosis,

35. The use of claim 31, wherein the ophthalmic disease is acute macular degeneration or age-related macular degeneration.

36. A method for producing a compound of general structure I, 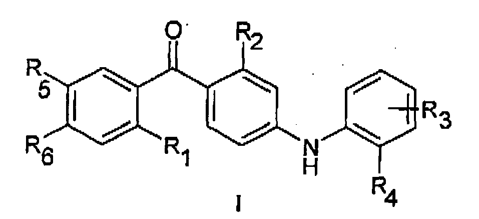 wherein R₁, R₂, R₃, R₄, R₅, and R₆ are defined as in claim 1, comprising the steps of
a) transforming a compound general structure VI, 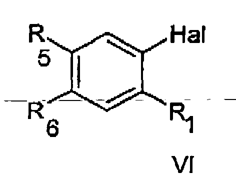 wherein Hal is a halogen, and R₁, R₅ and R₆ are defined as in claim 1, each of which are independently protected or unprotected, into an organometallic intermediate;
b) transmetalating said organometallic intermediate to an organozinc intermediate;
c) coupling said organozinc intermediate with an acid halide of general structure V, 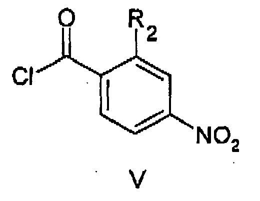 wherein R₂ is defined as in claim 1, protected or unprotected, in the presence of a catalyst to give a compound of general structure IV, 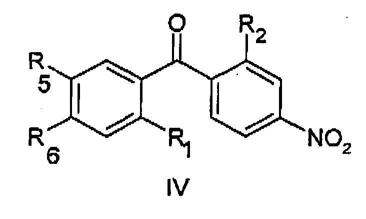 wherein R₁, R₂, R₅, and R₆ are defined as above, each of which are independently protected or unprotected;
d) optionally transforming, protecting or deprotecting one or more substituents or functional groups of R₁, R₂, R₅, and R₆ of the compound of general structure IV to give another compound of general structure IV;
e) reducing the compound of general structure IV from step c) or d) to an amine of general structure III, 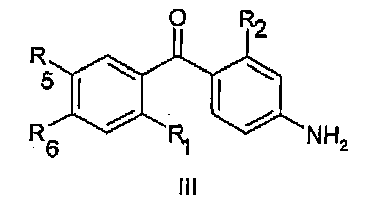 wherein R₁, R₂, R₅, and R₆ are defined as above, each of which are independently protected or unprotected;
f) optionally transforming, protecting or deprotecting one or more substituents or functional groups of R₁, R₂, R₅, and R₆ of the compound of general structure III to give another compound of general structure III;
g) coupling of the amine of general structure III from step e) or f) with a compound of general structure II, 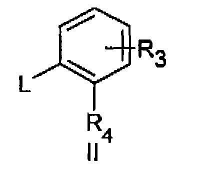 wherein L is triflate or halogen, R₃ and R₄ are defined in claim 1, each of which are independently protected or unprotected, to give a compound of general structure I, wherein R₁, R₂, R₃, R₄, R₅, and R₆ are defined as above, each of which are independently protected or unprotected;
h) optionally transforming, protecting or deprotecting one or more substituents or functional groups of R₁, R₂, R₃, R₄, R₅, or R₆ of the compound of general structure I from step g) to give a another compound of general structure I.

37. A method for producing a compound of general structure I, 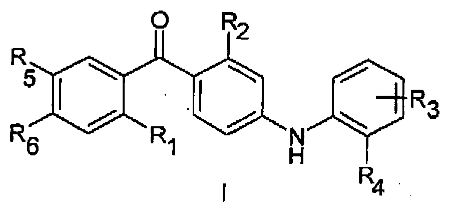 wherein R₁, R₂, R₃, R₄, R₅, and R₆ are defined as in claim 1, comprising the steps of
a) transforming a compound general structure VIIa, 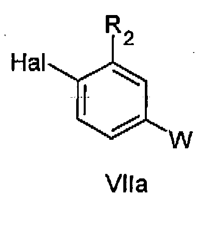 wherein Hal is halogen, W is halogen or triflate, and R₂ is as defined in claim 1, protected or unprotected, into an organometallic intermediate;
b) transmetalating said organometallic intermediate to an organozinc intermediate;
c) coupling said organozinc intermediate with an acid halide of general structure VIII, 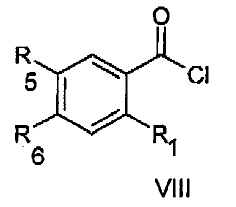 wherein R₁, R₅, and R₆ are as defined in claim 1, each of which are independently protected or unprotected, in the presence of a catalyst to give a compound of general structure IIIa, 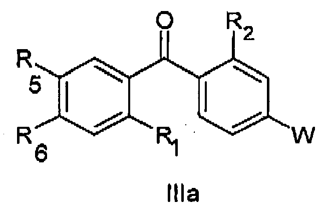 wherein W, R₁, R₂, R₅, and R₆ are defined as above, each of which are independently protected or unprotected;
d) optionally transforming, protecting or deprotecting one or more substituents or functional groups of W, R₁, R₂, R₅, and R₆ of the compound of general structure IIIa to give another compound of general structure IIIa;
e) coupling of the compound of general structure IIIa from step c) or d) with an amine of general structure IIa, 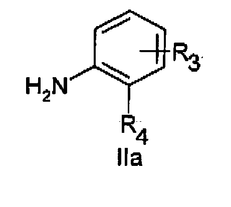 wherein R₃ and R₄ are defined as in claim 1, each of which are independently protected or unprotected, to give a compound of general structure I,
wherein R₁, R₂, R₃, R₄, R₅, and R₆ are defined as above, each of which are independently protected or unprotected;
f) optionally transforming, protecting or deprotecting one or more substituents or functional groups of R₁, R₂, R₃, R₄, R₅, or R₆ of the compound of general structure I from step e) to give another compound of general structure I.

38. The method according to claim 40 or 41, wherein the coupling in step c) is in the presence of a copper salt.

## Patentansprüche

1. Verbindung der allgemeinen Formel I 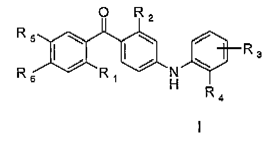 wobei
R₁ für Halogen, Hydroxy, Mercapto, Trifluormethyl, Amino, C₁₋₄Alkyl, C₂₋₄Alkenyl, C₂₋₄Alkinyl, C₁₋₄Alkoxy, C₁₋₄Alkylthio, C₁₋₆Alkylamino, C₁₋₄Alkoxycarbonyl, Cyano, -CONH₂ oder Nitro steht;
R₂ für Halogen, Amino, C₁₋₄Alkyl oder Nitro steht;
R₃ für einen oder mehrere, gleiche oder verschiedene Substituenten steht, die unter Wasserstoff, Halogen, C₁₋₄Alkyl und C₁₋₄Alkoxy ausgewählt sind;
R₄ für Fluor steht;
einer der Reste R₅ und R₆ für -COOH, -C(O)NHOH, -C(O)NHNH₂, Y₂R₉, Y₂R₉Y₃R₁₀, C₁₋₆Alkyl-Y₂R₉, C₁₋₆Alkyl-Y₂R₉Y₃R₁₀, C₂₋₆Alkenyl-Y₂R₉, C₂₋₆Alkenyl-Y₂R₉Y₃R₁₀, Y₂R₉-C₁₋₆Alkyl-Y₃R₁₀, Y₂R₉-C₂₋₆Alkenyl-Y₃R₁₀, C₃₋₁₂Carbocyclyl-Y₂R₉, C₃₋₁₂Carbocyclyl-Y₂R₉Y₃R₁₀, C₁₋₁₂Heterocyclyl-Y₂R₉, C₁₋₁₂Heterocyclyl-Y₂R₉Y₃R₁₀, C₃₋₁₂Carbocyclyl-C₁₋₆alkyl-Y₂R₉, C₃₋₁₂Carbocyclyl-C₁₋₆alkyl-Y₂R₉Y₃R₁₀, C₁₋₁₂Heterocyclyl-C₁₋₆alkyl-Y₂R₉, C₁₋₁₂Heterocyclyl-C₁₋₆alkyl-Y₂R₉Y₃R₁₀, C₃₋₁₂Carbocyclyl-C₁₋₆alkyl-Y₃R₁₀, C₁₋₁₂Heterocyclyl-C₁₋₆alkyl-Y₃R₁₀, C₁₋₁₂Heterocyclyl-C₁₋₁₀alkyl, C₃₋₁₂Carbocyclyl-C₁₋₁₀alkyl, C₁₋₁₀Alkyl-C₁₋₁₂heterocyclyl, C₁₋₁₀Alkyl-C₃₋₁₂carbocyclyl, C₁₋₁₀Alkyl, C₂₋₁₀Alkenyl, C₂₋₁₀Alkinyl, C₃₋₁₂Carbocyclyl oder C₁₋₁₂Heterocyclyl, jeweils gegebenenfalls mit einem oder mehreren, gleichen oder verschiedenen Substituenten R₇ substituiert, steht und der Rest für Wasserstoff, Halogen, Hydroxy, Mercapto, Trifluormethyl, Amino, C₁₋₄Alkyl, C₂₋₄Alkenyl, C₂₋₄Alkinyl, C₁₋₄Alkoxy, C₁₋₄Alkylthio, C₁₋₆Alkylamino, C₁₋₄Alkoxycarbonyl, Cyano, -CONH₂ oder Nitro steht;
mit der Maßgabe, dass R₅ oder R₆ mit einem oder mehreren, gleichen oder verschiedenen Substituenten R₇ (ausgenommen drei Fluor, wenn R₅ oder R₆ Methyl ist) substituiert ist, wenn R₅ oder R₆ für Phenyl, C₁₋₅Alkyl oder C₂₋₃Alkenyl steht,
R₇ für Halogen, Hydroxy, Mercapto, Trifluormethyl, Amino, C₁₋₄Alkyl, C₁₋₆Hydroxyalkyl, C₁₋₄Alkoxy, C₁₋₄Alkylthio, C₁₋₆Alkylamino, C₁₋₄Alkoxycarbonyl, C₁₋₉Trialkylammonium in Verbindung mit einem Anion, Cyano, Azido, Nitro, -S(O)₂NH₂, -S(O)₂NRₐR_{b}, -S(O)₂R, -COOH, -CONH₂, -NRₐC(O)R', -CONHR' oder -CONRR' steht, wobei R und R' gleich oder verschieden sind und jeweils für Wasserstoff oder C₁₋₃Alkyl stehen;
Rₐ, R_{b} und R_{c} gleich oder verschieden sind und jeweils für Wasserstoff, C₁₋₄Alkyl, C₂₋₄Alkenyl, C₂₋₄Alkinyl, C₃₋₈Carbocyclyl, C₁₋₁₂Heterocyclyl oder Aryl stehen, wobei C₁₋₄Alkyl, C₂₋₄Alkenyl, C₂₋₄Alkinyl, C₃₋₈Carbocyclyl, C₁₋₁₂Heterocyclyl oder Aryl jeweils gegebenenfalls mit einem oder mehreren, gleichen oder verschiedenen Substituenten R₇ substituiert ist;
Y₂ für -O-, -S-, -S(O)-, -S(O)₂-, -NRₐ-, -NRₐC(O)NR_{b}-, -NRₐC(O)-, -C(O)NRₐ-, -C(O)NRₐO-, -C(O)-, -NRₐC(O)O-, -NRₐS(O)₂-, -OC(O)-, -C(O)O-, -C(O)NRₐNR_{b}C(S)NR_{c}-, -C(O)NRₐNR_{b}- oder -S(O)₂NRₐ- steht;
R₉ für C₁₋₁₀Alkyl-C₁₋₁₂heterocyclyl, C₁₋₁₀Alkyl-C₃₋₁₂carbocyclyl, C₁₋₁₀Alkyl, C₂₋₁₀Alkenyl, C₂₋₁₀Alkinyl, C₃₋₁₂Carbocyclyl, C₁₋₁₂Heterocyclyl, C₃₋₁₂Carbocyclyl-C₁₋₁₀alkyl oder C₁₋₁₂Heterocyclyl-C₁₋₁₀alkyl, C₃₋₆Carbocyclyl-C₁₋₆alkenyl, C₃₋₆Carbocyclyl-C₂₋₆alkinyl, jeweils gegebenenfalls mit einem oder mehreren, gleichen oder verschiedenen Substituenten R₇ substituiert, steht,
mit der Maßgabe, dass C₁₋₆Alkyl mit einem oder mehreren, gleichen oder verschiedenen Substituenten R₇ substituiert ist, wenn Y₂ für -O-, -NRₐ-, -S- oder-C(O)O- steht und R₉ für C₁₋₆Alkyl steht,
Y₃ für -O-, -S-, -S(O)-, -S(O)₂-, -NRₐ-, -NRₐC(O)NR_{b}-, -NRₐC(O)-, -C(O)NRₐ-, -C(O)NRₐO-, -C(O)-, -NRₐC(O)O-, -NRₐS(O)₂-, -OC(O)- oder -C(O)O- steht;
R₁₀ für C₁₋₁₀Alkyl-C₁₋₁₂heterocyclyl, C₁₋₁₀Alkyl-C₃₋₁₂carbocyclyl, C₁₋₁₀Alkyl, C₂₋₁₀Alkenyl, C₂₋₁₀Alkinyl, C₃₋₁₂Carbocyclyl oder C₁₋₁₂Heterocyclyl, jeweils gegebenenfalls mit einem oder mehreren, gleichen oder verschiedenen Substituenten R₇ substituiert, steht,
oder Rₐ und R₉ zusammen mit dem Stickstoffatom, an das sie gebunden sind, einen C₁₋₁₂heterocyclischen Ring bilden, der gegebenenfalls ein oder mehrere zusätzliche unter O, S und N ausgewählte Heteroatome umfasst und gegebenenfalls mit einem oder mehreren Substituenten R₇ substituiert ist, wenn einer der Reste R₅ oder R₆ die Gruppe -C(O)NRₐR₉ ist,
oder ein pharmazeutisch verträgliches Salz, Solvat oder Ester davon.

2. Verbindung gemäß Anspruch 1, wobei R₁ für Halogen, Trifluormethyl, C₁₋₄Alkyl, C₁₋₄Alkoxy oder Nitro steht.

3. Verbindung gemäß Anspruch 2, wobei R₁ für Methyl, Ethyl, Methoxy, Ethoxy, Brom, Fluor oder Chlor steht.

4. Verbindung gemäß einem der Ansprüche 1-3, wobei R₂ für Methyl, Ethyl, Nitro, Brom, Fluor oder Chlor steht.

5. Verbindung gemäß einem der Ansprüche 1-4, wobei R₃ für Wasserstoff, Methyl, Ethyl, Methoxy, Ethoxy, Brom, Fluor oder Chlor steht.

6. Verbindung gemäß einem der Ansprüche 1-5, wobei R₃ für 1 Substituenten steht.

7. Verbindung gemäß Anspruch 6, wobei R₃ bezogen auf R₄ in meta- und bezogen auf -NH in para-Position steht oder wobei R₃ bezogen auf R₄ in meta- und bezogen auf -NH in ortho-Position steht oder wobei R₃ bezogen auf R₄ in ortho- und bezogen auf -NH in meta-Position steht.

8. Verbindung gemäß einem der Ansprüche 1-7, wobei R₃ Fluor ist.

9. Verbindung gemäß einem der Ansprüche 1-8, wobei R₇ für Halogen, Hydroxy, Amino, -S(O)₂CH₃, Trifluormethyl, Cyano, C₁₋₄Hydroxyalkyl, C₁₋₄Alkoxy, C₁₋₄Alkyl, C₁₋₄Alkylthio, C₁₋₄Alkylamino, C₁₋₄Alkoxycarbonyl, -COOH, -CONH₂, -S(O)₂NH₂, Azido, -CONHR' oder -CONRR' steht, wobei R und R' wie in Anspruch 1 definiert sind.

10. Verbindung gemäß Anspruch 9, wobei R₇ für Methyl, Ethyl, Methoxy, Ethoxy, Hydroxy, Methoxycarbonyl, Ethoxycarbonyl, Dimethylamino, Ethylamino, Amino, -COOH, Fluor, Chlor, Brom, -CONH₂, -S(O)₂NH₂, Azido, Methylthio, -S(O)₂CH₃, Trifluormethyl, Cyano oder Hydroxymethyl steht.

11. Verbindung gemäß einem der Ansprüche 1-10, wobei einer der Reste R₅ und R₆ für Y₂R₉, C₁₋₄Alkyl-Y₂R₉, Y₂R₉Y₃R₁₀, C₁₋₄Alkyl-Y₂R₉Y₃R₁₀, C₂₋₄Alkenyl-Y₂R₉, C₂. ₄Alkenyl-Y₂R₉Y₃R₁₀, Y₂R₉-C₁₋₄Alkyl-Y₃R₁₀, Y₂R₉-C₂₋₄Alkenyl-Y₃R₁₀, C₁₋₆Heterocyclyl-C₁₋₄alkyl-Y₂R₉, C₁₋₄Alkyl-C₁₋₆heterocyclyl, C₁₋₄Alkyl-C₃₋₆carbocyclyl, C₃₋₆Carbocyclyl-C₁₋₄alkyl, mit R₇ substituiertes C₁₋₄Alkyl, C₂₋₄Alkenyl, C₂₋₄Alkinyl, C₃₋₆Carbocyclyl, C₁₋₆Heterocyclyl, -COOH, -C(O)NHOH oder -C(O)NHNH₂ steht und der andere Rest für Wasserstoff, Halogen, C₁₋₄Alkyl oder C₁₋₄Alkoxy steht.

12. Verbindung gemäß Anspruch 11, wobei R₅ für Y₂R₉, C₁₋₄Alkyl-Y₂R₉, Y₂R₉Y₃R₁₀, C₁₋₄Alkyl-Y₂R₉Y₃R₁₀, C₂₋₄Alkenyl-Y₂R₉, C₂₋₄Alkenyl-Y₂R₉Y₃R₁₀, Y₂R₉-C₁₋₄Alkyl-Y₃R₁₀, Y₂R₉-C₂₋₄Alkenyl-Y₃R₁₀, C₁₋₆Heterocyclyl-C₁₋₄alkyl-Y₂R₉, C₁₋₄Alkyl-C₁₋₆heterocyclyl, C₁₋₄Alkyl-C₃₋₆carbocyclyl, C₃₋₆Carbocyclyl-C₁₋₄alkyl, mit R₇ substituiertes C₁₋₄Alkyl, C₂₋₄Alkenyl, C₂₋₄Alkinyl, C₃₋₆Carbocyclyl, C₁₋₆Heterocyclyl, -COOH, -C(O)NHOH oder -C(O)NHNH₂ steht und R₆ für Wasserstoff, Halogen, C₁₋₄Alkyl oder C₁₋₄Alkoxy steht.

13. Verbindung gemäß Anspruch 11, wobei einer der Reste R₅ und R₆ für Y₂R₉, Y₂R₉Y₃R₁₀, Phenyl, Methylphenyl, Propenyl, Methyl-Y₂R₉, Tetrazol, Ethinyl, Triazol, Thiadiazol, Dihydrooxazol, -COOH, -C(O)NHOH oder -C(O)NHNH₂ steht und der andere Rest für Wasserstoff, Fluor, Chlor, Methyl oder Methoxy steht.

14. Verbindung gemäß einem der Ansprüche 1-13, wobei R₆ Wasserstoff ist.

15. Verbindung gemäß einem der Ansprüche 1-11 oder 13, wobei R₅ Wasserstoff ist.

16. Verbindung gemäß einem der Ansprüche 1-15, wobei Y₂ für -O-, -NRₐ-, -NRₐC(O)NR_{b}-, -NRₐC(O)-, -C(O)NRₐ-, -C(O)NRₐO-, -C(O)-, -NRₐC(O)O-, -NRₐS(O)₂-, -C(O)NRₐNR_{b}- oder -S(O)₂NRₐ- steht.

17. Verbindung gemäß einem der Ansprüche 1-16, wobei Y₃ für -O-, -NRₐC(O)-, -C(O)NRₐ-, -C(O)-, -C(O)O- oder -NRₐC(O)O- steht.

18. Verbindung gemäß einem der Ansprüche 1-17, wobei R₉ für C₁₋₄Alkyl-C₁₋₆heterocyclyl, C₁₋₄Alkyl-C₃₋₆carbocyclyl, C₁₋₈Alkyl, C₂₋₄Alkenyl, C₂₋₄Alkinyl, C₃₋₁₀Carbocyclyl, C₁₋₆Heterocyclyl, C₃₋₆Carbocyclyl-C₁₋₆alkyl, C₁₋₆Heterocyclyl-C₁₋₆alkyl, C₃₋₈Carbocyclyl-C₂₋₄alkenyl oder C₃₋₆Carbocyclyl-C₂₋₄alkinyl steht.

19. Verbindung gemäß einem der Ansprüche 1-18, wobei R₉ für C₁₋₄Heterocyclyl, C₁₋₆Alkyl, C₁₋₃Alkyl-C₁₋₅heterocyclyl, C₆₋₁₀Carbocyclyl, C₁₋₃Alkyl-C₆carbocyclyl, C₃Alkenyl, C₆Carbocyclyl-C₁alkyl, C₆Carbocyclyl-C₃alkenyl oder C₆Carbocyclyl-C₂alkinyl steht.

20. Verbindung gemäß Anspruch 19, wobei R₉ für Morpholinyl, Propylmorpholinyl, Piperazinyl, Methyl, Ethyl, n-Propyl, n-Butyl, *tert*-Butyl, Isobutyl, Hexyl, Isopropyl, Dimethylpropyl, Methyltetrahydrofuranyl, Methylpyridinyl, Ethylpiperazinyl, Cyclohexyl, Propyloxopyrrolidinyl, Benzyl, Methylcyclohexyl, Propylphenyl, Ethylmorpholinyl, Allyl, Ethylfuranyl, Phenyl, Methyldioxoimidazolidinyl, Dioxohexahydropyrimidinyl, Thiazolyl, Methylphenyl, Ethylphenyl, Methyldioxolanyl, Methylthiazolyl, Propenylphenyl, Methylfuranyl, Thiophenyl, Tetrahydropyranyl oder Ethinylphenyl steht.

21. Verbindung gemäß einem der Ansprüche 1-20, wobei R₁₀ für C₁₋₄Alkyl, C₂₋₄Alkenyl, C₃₋₆Carbocyclyl oder C₁₋₆Heterocyclyl steht.

22. Verbindung gemäß Anspruch 21, wobei R₁₀ für Methyl, Ethyl, Methacryl, *tert-*Butyl, Tetrahydropyranyl oder Ethenyl steht.

23. Verbindung gemäß einem der Ansprüche 1-22, wobei der Heterocyclus oder das Heterocyclyl ein oder zwei Sauerstoffatome oder ein Schwefelatom und/oder bis zu zwei Stickstoffatome oder drei oder vier Stickstoffatome enthält, wobei gegebenenfalls ein oder zwei CH₂-Ringfragmente durch ein beziehungsweise zwei -C(O)-Fragmente ersetzt ist/sind.

24. Verbindung gemäß einem der Ansprüche 1-23, wobei Rₐ, R_{b} oder R_{c} jeweils unabhängig für Wasserstoff, Methyl, Ethyl, 2-Hydroxyethyl oder 2-Methoxyethyl stehen.

25. Verbindung gemäß einem der Ansprüche 1-24, die ausgewählt ist unter:
3-[2-Chlor-4-(2,4-difluorphenylamino)benzoyl]-*N*-(2-hydroxyethyl)-4-methylbenzamid (Verbindung 115),
3-[2-Chlor-4-(4-chlor-2-fluorphenylamino)-benzoyl]-*N*-(2-hydroxyethyl)-4-methyl-benzamid (Verbindung 120),
3-[2-Chlor-4-(4-chlor-2-fluorphenylamino)-benzoyl]-4,*N*-dimethylbenzamid (Verbindung 121),
(2-{3-[2-Chlor-4-(4-chlor-2-fluorphenylamino)-benzoyl]-4-methylbenzoylamino}-acetylamino)-essigsäureethylester(Verbindung 122),
{3-[2-Chlor-4-(4-chlor-2-fluorphenylamino)-benzoyl]-4-methylbenzoylamino}-essigsäureethylester(Verbindung 123),
3-[2-Chlor-4-(4-chlor-2-fluorphenylamino)-benzoyl]-*N*-(2-methoxyethyl)-4-methyl-benzamid (Verbindung 124),
3-[2-Chlor-4-(4-chlor-2-fluorphenylamino)-benzoyl]-*N*-cyclohexyl-4-methylbenzamid (Verbindung 125),
3-[2-Chlor-4-(4-chlor-2-fluorphenylamino)-benzoyl]-*N*-ethyl-4-methylbenzamid (Verbindung 126),
3-[2-Chlor-4-(4-chlor-2-fluorphenylamino)-benzoyl]-*N*-(6-hydroxyhexyl)-4-methyl-benzamid (Verbindung 127),
3-[2-Chlor-4-(4-chlor-2-fluorphenylamino)-benzoyl]-*N*-isopropyl-4-methylbenzamid (Verbindung 128),
3-[2-Chlor-4-(4-chlor-2-fluorphenylamino)-benzoyl]-*N*-isobutyl-4-methylbenzamid (Verbindung 129),
3-[2-Chlor-4-(4-chlor-2-fluorphenylamino)-benzoyl]-*N*-(2,2-dimethylpropyl)-4-methyl-benzamid (Verbindung 130),
3-[2-Chlor-4-(4-chlor-2-fluorphenylamino)-benzoyl]-*N*-(3-methoxypropyl)-4-methyl-benzamid (Verbindung 131),
3-[2-Chlor-4-(4-chlor-2-fluorphenylamino)-benzoyl]-4-methyl-*N*-[3-(2-oxopyrrolidin-1-yl)-propyl]-benzamid (Verbindung 132),
3-[2-Chlor-4-(4-chlor-2-fluorphenylamino)-benzoyl]-*N*-(2-dimethylaminoethyl)-4-methylbenzamid (Verbindung 133),
2-Methacrylsäure-2-{3-[2-chlor-4-(4-chlor-2-fluorphenylamino)-benzoyl]-4-methyl-benzoylamino}-ethylester (Verbindung 134),
3-[2-Chlor-4-(4-chlor-2-fluorphenylamino)-benzoyl]-*N*-cis-(4-hydroxycyclohexyl)-4-methylbenzamid (Verbindung 135),
3-[2-Chlor-4-(4-chlor-2-fluorphenylamino)-benzoyl]-*N*-trans-(4-hydroxycyclohexyl)-4-methylbenzamid (Verbindung 136),
(2-{3-[2-Chlor-4-(4-chlor-2-fluorphenylamino)-benzoyl]-4-methylbenzoylamino}-ethyl)-carbaminsäure-*tert*-butylester (Verbindung 137),
*N*-(2-Aminoethyl)-3-[2-chlor-4-(4-chlor-2-fluorphenylamino)-benzoyl]-4-methyl-benzamid (Verbindung 138),
(2-{3-[2-Chlor-4-(4-chlor-2-fluorphenylamino)-benzoyl]-4-methylbenzoylamino}-acetylamino)-essigsäure(Verbindung 139),
3-[2-Chlor-4-(2,4-difluorphenylamino)-benzoyl]-*N*-(2-hydroxyethyl)-4-methoxy-benzamid (Verbindung 140),
3-[2-Chlor-4-(2,4-difluorphenylamino)-benzoyl]-*N*-(2,2-difluorethyl)-4-methoxy-benzamid (Verbindung 141),
3-[2-Chlor-4-(2,4-difluorphenylamino)-benzoyl]-*N*-(2-fluorethyl)-4-methoxybenzamid (Verbindung 142),
3-[2-Chlor-4-(2,4-difluorphenylamino)-benzoyl]-*N*-(2,3-dihydroxypropyl)-4-Methoxy-benzamid (Verbindung 143),
*N*-Carbamoylmethyl-3-[2-chlor-4-(2,4-difluorphenylamino)-benzoyl]-4-methoxy-benzamid (Verbindung 144),
*N*-Carbamoylmethyl-3-[2-chlor-4-(2,4-difluorphenylamino)-benzoyl]-4-methyl-benzamid (Verbindung 145),
*N*-Benzyl-3-[2-chlor-A-(2,4-difluorphenylamino)-benzoyl]-4-methylbenzamid (Verbindung 146),
3-[2-Chlor-4-(2,4-difluorphenylamino)-benzoyl]-*N*-(2-fluorethyl)-4-methylbenzamid (Verbindung 147).
3-[2-Chlor-4-(2,4-difluorphenylamino)-benzoyl]-4-methyl-*N*-(2,2,2-trifluorethyl)-benzamid (Verbindung 148),
3-[2-Chlor-4-(2,4-difluorphenylamino)-benzoyl]-*N*-ethyl-4-methylbenzamid (Verbindung 149),
3-[2-Chlor-4-(2,4-difluorphenylamino)-benzoyl]-*N*-cyclohexylmethyl-4-methyl-benzamid (Verbindung 150),
3-[2-Chlor-4-(2,4-difluorphenylamino)-benzoyl]-*N*-(2-hydroxypropyl)-4-methyl-benzamid (Verbindung 151),
3-[2-Chlor-4-(2,4-difluorphenylamino)-benzoyl]-*N*-(2,3-dihydroxypropyl)-4-methyl-benzamid (Verbindung 152),
3-[2-Chlor-4-(2,4-difluorphenylamino)-benzoyl]-*N*-(1-hydroxymethylpropyl)-4-methyl-benzamid (Verbindung 153),
3-[2-Chlor-4-(2,4-difluorphenylamino)-benzoyl]-4-methyl-*N*-(2,2,3,3,3-pentafluorpropyl)-benzamid (Verbindung 154),
3-[2-Chlor-4-(2,4-difluorphenylamino)-benzoyl]-*N*-(3-hydroxypropyl)-4-methyl-benzamid (Verbindung 155),
3-[2-Chlor-4-(2,4-difluorphenylamino)-benzoyl]-*N*-(2-hydroxy-1,1-dimethylethyl)-4-methylbenzamid (Verbindung 156),
3-[2-Chlor-4-(2,4-difluorphenylamino)-benzoyl]-*N*-(2-hydroxy-1-hydroxymethyl-1-methylethyl)-4-methylbenzamid (Verbindung 157),
{3-[2-Chlor-4-(2,4-difluorphenylamino)-benzoyl]-4-methylbenzoylamino}-essigsäureethylester(Verbindung 158),
3-[2-Chlor-4-(2,4-difluorphenylamino)-benzoyl]-*N*-(4-hydroxybutyl)-4-methylbenzamid (Verbindung 159),
3-[2-Chlor-4-(2,4-difluorphenylamino)-benzoyl]-*N*-(3-hydroxy-1,1-dimethylbutyl)-4-methylbenzamid (Verbindung 160),
3-[2-Chlor-4-(2,4-difluorphenylamino)-benzoyl]-4-methyl-*N*-(3-phenylpropyl)-benzamid (Verbindung 161),
(R)-3-[2-Chlor-4-(2,4-difluorphenylamino)-benzoyl]-*N*-(1-hydroxymethyl-3-methylbutyl)-4-methylbenzamid (Verbindung 162),
3-[2-Chlor-4-(2,4-difluorphenylamino)-benzoyl]-*N*-isopropyl-4-methylbenzamid (Verbindung 164),
3-[2-Chlor-4-(2,4-difluorphenylamino)-benzoyl]-*N*-cyclohexyl-4-methylbenzamid (Verbindung 165),
3-[2-Chlor-4-(2,4-difluorphenylamino)-benzoyl]-*N*-(2,2-difluorethyl)-4-methylbenzamid (Verbindung 166),
5-{3-[2-Chlor-4-(2,4-difluorphenylamino)-benzoyl]-4-methylbenzoylamino}-4-oxo-pentansäuremethylester (Verbindung 167).
*N*-[(2-Carbamoylethylcarbamoyl)-methyl]-3-[2-chlor-4-(2,4-difluorphenylamino)-benzoyl]-4-methylbenzamid (Verbindung 168),
(2-{3-[2-Chlor-4-(2,4-difluorphenylamino)-benzoyl]-4-methylbenzoylamino}-acetylamino)-essigsäureethylester(Verbindung 169),
*N*-Allyl-3-[2-chlor-4-(2,4-difluorphenylamino)-benzoyl]-4-methylbenzamid (Verbindung 170),
3-[2-Chlor-4-(2,4-difluorphenylamino)-benzoyl]-4-methyl-*N*-(2-sulfamoylethyl)-benzamid (Verbindung 171),
*N*-(2-Acetylaminoethyl)-3-[2-chlor-4-(2,4-difluorphenylamino)-benzoyl]-4-methylbenzamid (Verbindung 172),
3-[2-Chlor-4-(2,6-difluorphenylamino)-benzoyl]-*N*-(2-hydroxyethyl)-4-methoxybenzamid (Verbindung 173),
3-[2-Chlor-4-(2,6-difluorphenylamino)-benzoyl]-*N*-(2-fluorethyl)-4-methoxybenzamid (Verbindung 174),
3-[2-Chlor-4-(2,6-difluorphenylamino)-benzoyl]-*N*-(2,3-dihydroxypropyl)-4-methoxy-benzamid (Verbindung 175),
3-[2-Chlor-4-(2,6-difluorphenylamino)-benzoyl]-*N*-(3-hydroxypropyl)-4-methoxy-benzamid (Verbindung 176),
3-[2-Chlor-4-(2,6-difluorphenylamino)-benzoyl]-4-methoxy-*N*-phenethylbenzamid (Verbindung 177),
3-[2-Chlor-4-(2,6-difluorphenylamino)-benzoyl]-*N*-(2-hydroxy-1,1-dimethylethyl)-4-methoxybenzamid (Verbindung 178),
3-[2-Chlor-4-(2,6-difluorphenylamino)-benzoyl]-4-methoxy-*N*-(2-morpholin-4-ylethyl)-benzamid (Verbindung 179),
3-[2-Chlor-4-(2,6-difluorphenylamino)-benzoyl]-*N*-(2-hydroxy-1-hydroxymethyl-1-methylethyl)-4-methoxybenzamid (Verbindung 180),
3-[2-Chlor-4-(2,6-difluorphenylamino)-benzoyl]-*N*-(2-hydroxy-ethyl)-4-methoxy-*N-*methylbenzamid (Verbindung 181),
{3-[2-Chlor-4-(2,6-difluorphenylamino)-benzoyl]-4-methoxybenzoylamino}-essigsäureethylester(Verbindung 182),
(2-{3-[2-Chlor-4-(2,6-difluorphenylamino)-benzoyl]-4-methoxybenzoylamino}-acetylamino)-essigsäureethylester(Verbindung 183),
3-[2-Chlor-4-(2,6-difluorphenylamino)-benzoyl]-*N*,*N*-bis-(2-hydroxyethyl)-4-methoxy-benzamid (Verbindung 184),
3-[2-Chlor-4-(2,6-difluorphenylamino)-benzoyl]-4-methoxy-*N*,*N*-bis-(2-methoxyethyl)-benzamid (Verbindung 185),
4-Chlor-3-[2-chlor-4-(2,4-difluorphenylamino)-benzoyl]-*N*-(2-hydroxyethyl)-benzamid (Verbindung 204),
*N*-{3-[2-Chlor-4-(2,4-difluorphenylamino)-benzoyl]-4-methylphenyl}-3-methoxypropionamid (Verbindung 241),
*N*-{3-[2-Chlor-4-(2,4-difluorphenylamino)-benzoyl]-4-methylphenyl}-propionamid (Verbindung 242),
*N*-{3-[2-Chlor-4-(2,4-difluorphenylamino)-benzoyl]-4-methylphenyl}-2-(2-methoxy-ethoxy)-acetamid (Verbindung 243),
*N*-{3-[2-Chlor-4-(2,4-difluorphenylamino)-benzoyl]-4-methylphenyl}-3-morpholin-4-yl-propionamid (Verbindung 244).
*N*-{3-[2-Chlor-4-(2,4-difluorphenylamino)-benzoyl]-4-methylphenyl}-3-hydroxypropion-amid (Verbindung 245),
*N*-{3-[2-Chlor-4-(2,4-difluorphenylamino)-benzoyl]-4-methylphenyl}-3-furan-2-yl-propionamid (Verbindung 246),
*N*-{3-[2-Chlor-4-(2,4-difluorphenylamino)-benzoyl]-4-methylphenyl}-2-hydroxybenzamid (Verbindung 247),
*N*-{3-[2-Chlor-4-(2,4-difluorphenylamino)-benzoyl]-4-methylphenyl}-2-(2,5-dioxo-imidazolidin-4-yl)-acetamid (Verbindung 248),
2,6-Dioxohexahydropyrimidin-4-carbonsäure-{3-[2-chlor-4-(2,4-difluorphenylamino)-benzoyl]-4-methylphenyl}-amid (Verbindung 249),
Acrylsäure-2-{3-[2-chlor-4-(2,4-difluorphenylamino)-benzoyl]-4-methylphenyl-carbamoyl}-ethylester (Verbindung 250),
*N*-{3-[2-Chlor-4-(2,4-difluorphenylamino)-benzoyl]-4-methylphenyl}-3-methylsulfanyl-propionamid (Verbindung 251),
*N*-{3-[2-Chlor-4-(2,4-difluorphenylamino)-benzoyl]-4-methylphenyl}-3-methansulfonyl-propionamid (Verbindung 252),
Ethansulfonsäure-{3-[2-chlor-4-(2,4-difluorphenylamino)-benzoyl]-4-methylphenyl}-amid (Verbindung 253),
*N*-{3-[2-Chlor-4-(2,4-difluorphenylamino)-benzoyl]-4-methylphenyl}-4-methoxy-benzolsulfonamid (Verbindung 254),
*N*-(5-{3-[2-Chlor-4-(2,4-difluorphenylamino)-benzoyl]-4-methylphenylsulfamoyl}-4-methylthiazol-2-yl)-acetamid (Verbindung 255),
5-Acetyl-2-chlor-*N*-{3-[2-chlor-4-(2,4-difluorphenylamino)-benzoyl]-4-methylphenyl}-benzolsulfonamid (Verbindung 256),
Naphthalen-2-sulfonsäure-{3-[2-chlor-4-(2,4-difluorphenylamino)-benzoyl]-4-methyl-phenyl}-amid (Verbindung 257),
*N*-{3-[2-Chlor-4-(2,4-difluorphenylamino)-benzoyl]-4-methylphenyl}-*C*-phenyl-methansulfonamid (Verbindung 258),
2-Methacrylsäure-2-(3-{3-[2-chlor-4-(2,4-difluorphenylamino)-benzoyl]-4-methyl-phenyl}-ureido)-ethylester (Verbindung 259),
1-{3-[2-Chlor-4-(2,4-difluorphenylamino)-benzoyl]-4-methylphenyl}-3-(2-hydroxyethyl)-harnstoff (Verbindung 260),
(3-{3-[2-Chlor-4-(2,4-difluorphenylamino)-benzoyl]-4-methylphenyl}-ureido)-essigsäureethylester(Verbindung 261),
1-{3-[2-Chlor-4-(2,4-difluorphenylamino)-benzoyl]-4-methylphenyl}-3-(3-methoxy-phenyl)-harnstoff (Verbindung 262),
1-{3-[2-Chlor-4-(2,4-difluorphenylamino)-benzoyl]-4-methylphenyl}-3-(3-trifluormethylphenyl)-harnstoff (Verbindung 263),
1-{3-[2-Chlor-4-(2,4-difluorphenylamino)-benzoyl]-4-methylphenyl}-3-propylharnstoff (Verbindung 264),
3-(3-{3-[2-Chlor-4-(2,4-difluorphenylamino)-benzoyl]-4-methylphenyl}-ureido)-propionsäureethylester (Verbindung 265),
1-{3-[2-Chlor-4-(2,4-difluorphenylamino)-benzoyl]-4-methylphenyl}-3-cyclohexyl-harnstoff (Verbindung 266),
1-Allyl-3-{3-[2-chlor-4-(2,4-difluorphenylamino)-benzoyl]-4-methylphenyl}-harnstoff (Verbindung 267),
1-Benzyl-3-{3-[2-chlor-4-(2,4-difluorphenylamino)-benzoyl]-4-methylphenyl}-harnstoff (Verbindung 268),
1-{3-[2-Chlor-4-(2,4-difluorphenylamino)-benzoyl]-4-methylphenyl}-3-ethylharnstoff (Verbindung 269),
1-{3-[2-Chlor-4-(2,4-difluorphenylamino)-benzoyl]-4-methylphenyl}-3-phenylharnstoff (Verbindung 270),
1-Butyl-3-{3-[2-chlor-4-(2,4-difluorphenylamino)-benzoyl]-4-methylphenyl}-harnstoff (Verbindung 271),
1-{3-[2-Chlor-4-(2,4-difluorphenylamino)-benzoyl]-4-methylphenyl}-3-phenethyl-harnstoff (Verbindung 272),
2-(3-{3-[2-Chlor-4-(2,4-difluorphenylamino)-benzoyl]-4-methylphenyl}-ureido)-benzoesäuremethylester (Verbindung 273),
1-{3-[2-Chlor-4-(2,4-difluorphenylamino)-benzoyl]-4-methylphenyl}-3-(3-cyanophenyl)-harnstoff (Verbindung 274),
1-{3-[2-Chlor-4-(2,4-difluorphenylamino)-benzoyl]-4-methylphenyl}-3-isopropyl-harnstoff (Verbindung 275),
1-{3-[2-Chlor-4-(2,4-difluorphenylamino)-benzoyl]-4-methylphenyl}-3-(4-methoxy-phenyl)-harnstoff (Verbindung 276),
{3-[2-Chlor-4-(2,4-difluorphenylamino)-benzoyl]-4-methylphenyl}-carbaminsäure-benzylester (Verbindung 277),
{3-[2-Chlor-4-(2,4-difluorphenylamino)-benzoyl]-4-methylphenyl}-carbaminsäureallyl-ester (Verbindung 278),
{3-[2-Chlor-4-(2,4-difluorphenylamino)-benzoyl]-4-methylphenyl}-carbaminsäureethyl-ester (Verbindung 279),
[2-Chlor-4-(2,4-difluorphenylamino)-phenyl]-[5-(3-hydroxybutylamino)-2-methyl-phenyl]-methanon (Verbindung 281),
[2-Chlor-4-(2,4-difluorphenylamino)-phenyl]-(3'-hydroxymethyl-4-methylbiphenyl-3-yl)-methanon (Verbindung 282),
[2-Chlor-4-(2,4-difluorphenylamino)-phenyl]-(3'-hydroxy-4-methylbiphenyl-3-yl)-methanon (Verbindung 283),
[2-Chlor-4-(2,4-difluorphenylamino)-phenyl]-(4'-methoxy-4-methylbiphenyl-3-yl)-methanon (Verbindung 284),
*N*-{3'-[2-Chlor-4-(2,4-difluorphenylamino)-benzoyl]-4'-methylbiphenyl-3-yl}-acetamid (Verbindung 285),
[2-Chlor-4-(2,4-difluorphenylamino)-phenyl]-(4-methyl-3'-trifluormethoxybiphenyl-3-yl)-methanon (Verbindung 286),
[2-Chlor-4-(2,4-difluorphenylamino)-phenyl]-(3',4',5'-trifluor-4-methylbiphenyl-3-yl)-methanon (Verbindung 288),
[2-Chlor-4-(2,4-difluor-phenylamino)-phenyl]-(3',4'-dimethoxy-4-methyl-biphenyl-3-yl)-methanon (289),
3'-[2-Chlor-4-(2,4-difluorphenylamino)-benzoyl]-4'-methylbiphenyl-3-carbonitril (Verbindung 290),
3-[2-Chlor-4-(2,4-difluorphenylamino)-benzoyl]-*N*-(2-hydroxy-ethyl)-4-methyl-benzolsulfonamid (Verbindung 291),
3-[2-Chlor-4-(2,4-difluorphenylamino)-benzoyl]-4-methyl-*N*-(2-morpholin-4-yl-ethyl)-benzolsulfonamid (Verbindung 292),
*N*-Allyl-3-[2-chlor-4-(2,4-difluorphenylamino)-benzoyl]-4-methylbenzolsulfonamid (Verbindung 293),
*N*-(2-{3-[2-Chlor-4-(2,4-difluorphenylamino)-benzoyl]-4-methylbenzolsulfonylamino}-ethyl)-acetamid (Verbindung 294),
3-[2-Chlor-4-(2,4-difluorphenylamino)-benzoyl]-4-methyl-*N*-propylbenzolsulfonamid (Verbindung 295),
3-[2-Chlor-4-(2,4-difluorphenylamino)-benzoyl]-*N*-(2,3-dihydroxypropyl)-4-methyl-benzolsulfonamid (Verbindung 296),
3-[2-Chlor-4-(2,4-difluorphenylamino)-benzoyl]-*N*-(2-methoxyethyl)-4-methyl-benzolsulfonamid (Verbindung 297),
[4-(2,4-Difluorphenylamino)-2-nitrophenyl]-[5-(4-methoxybenzyloxy)-2-methylphenyl]-methanon (Verbindung 306),
[4-(2,4-Difluorphenylamino)-2-nitrophenyl]-[5-(3-hydroxypropoxy)-2-methylphenyl]-methanon (Verbindung 307),
[2-Amino-4-(2,4-difluorphenylamino)-phenyl]-[5-(3-hydroxypropoxy)-2-methylphenyl]-methanon (Verbindung 308),
[4-(2,4-Difluorphenylamino)-2-nitrophenyl]-[2-methyl-5-(2-morpholin-4-yl-ethoxy)-phenyl]-methanon (Verbindung 309),
[2-Amino-4-(2,4-difluorphenylamino)-phenyl]-[2-methyl-5-(2-morpholin-4-yl-ethoxy)-phenyl]-methanon (Verbindung 310),
[4-(2,4-Difluorphenylamino)-2-nitrophenyl]-[5-(2,2-dimethyl-[1,3]dioxolan-4-yl-methoxy)-2-methylphenyl]-methanon (Verbindung 311),
[4-(2,4-Difluorphenylamino)-2-nitrophenyl]-[5-(2,3-dihydroxypropoxy)-2-methyl-phenyl]-methanon (Verbindung 312),
[2-Amino-4-(2,4-difluorphenylamino)-phenyl]-[5-(2,3-dihydroxypropoxy)-2-methyl-phenyl]-methanon (Verbindung 313),
[2-Chlor-4-(2,4-difluorphenylamino)-phenyl]-[2-fluor-5-(3-hydroxypropoxy)-phenyl]-methanon (Verbindung 314),
[2-Chlor-4-(2,4-difluorphenylamino)-phenyl]-[5-(2,2-dimethyl-[1,3]dioxolan-4-yl-methoxy)-2-fluorphenyl]-methanon (Verbindung 315),
[2-Chlor-4-(2,4-difluorphenylamino)-phenyl]-[5-(2,3-dihydroxypropoxy)-2-fluorphenyl]-methanon (Verbindung 316),
[2-Chlor-4-(2,6-difluorphenylamino)-phenyl]-[2-chlor-5-(2-morpholin-4-ylethoxy)-phenyl]-methanon (Verbindung 328),
(±)-[2-Chlor-4-(2,6-difluorphenylamino)-phenyl]-[2-chlor-5-(2,3-dihydroxypropoxy)-phenyl]-methanon (Verbindung 329),
[5-(3-Brompropoxy)-2-chlorphenyl]-[2-chlor-4-(2,6-difluorphenylamino)-phenyl]-methanon (Verbindung 330),
[2-Chlor-4-(2,4-difluorphenylamino)-phenyl]-(5-hydroxymethyl-2-methylphenyl)-methanon (Verbindung 331),
[2-Chlor-4-(2,4-difluorphenylamino)-phenyl]-(5-chlormethyl-2-methylphenyl)-methanon (Verbindung 332),
(5-Azidomethyl-2-methylphenyl)-[2-chlor-4-(2,4-difluorphenylamino)-phenyl]-methanon (Verbindung 333),
(5-Aminomethyl-2-methylphenyl)-[2-chlor-4-(2,4-difluorphenylamino)-phenyl]-methanon (Verbindung 334),
[2-Chlor-4-(2,4-difluorphenylamino)-phenyl]-(5-hydroxymethyl-2-methoxyphenyl)-methanon (Verbindung 335),
Essigsäure-3-[2-chlor-4-(2,4-difluorphenylamino)-benzoyl]-4-methoxybenzylester (Verbindung 336),
*N*-*tert*-Butoxy-3-[2-chlor-4-(2,4-difluorphenylamino)-benzoyl]-4-methoxybenzamid (Verbindung 337),
3-[2-Chlor-4-(2,4-difluorphenylamino)-benzoyl]-*N*-methoxy-4-methylbenzamid (Verbindung 338),
N-Butoxy-3-[2-chlor-4-(2,4-difluorphenylamino)-benzoyl]-4-methylbenzamid (Verbindung 339),
3-[2-Chlor-4-(2,4-difluorphenylamino)-benzoyl]-*N*-cyclohexylmethoxy-4-methyl-benzamid (Verbindung 340),
3-[2-Chlor-4-(2,4-difluorphenylamino)-benzoyl]-4-methyl-*N*-(2-methylthiazol-4-yl-methoxy)-benzamid (Verbindung 341),
N-Benzyloxy-3-[2-chlor-4-(2,4-difluorphenylamino)-benzoyl]-4-methylbenzamid (Verbindung 342),
3-[2-Chlor-4-(2,4-difluorphenylamino)-benzoyl]-*N*-(4-methoxybenzyloxy)-4-methyl-benzamid (Verbindung 343),
3-[2-Chlor-4-(2,4-difluorphenylamino)-benzoyl]-4-methylbenzoesäure-*N*',*N*'-dimethyl-hydrazid (Verbindung 344),
3-[2-Chlor-4-(2,4-difluorphenylamino)-benzoyl]-4-methyl-*N*-morpholin-4-yl-benzamid (Verbindung 345),
3-[2-Chlor-4-(2,4-difluorphenylamino)-benzoyl]-*N*-hydroxy-4-methylbenzamid (Verbindung 346),
4-[2-Chlor-4-(2,4-difluorphenylamino)-benzoyl]-*N*-(2-hydroxyethyl)-3-methylbenzamid (Verbindung 347),
[2-Chlor-4-(2,4-difluorphenylamino)-phenyl]-[5-(3-hydroxypropenyl)-2-methylphenyl]-methanon (Verbindung 348),
4-{3-[2-Chlor-4-(2,4-difluorphenylamino)-benzoyl]-4-methylbenzoylamino}-thiophen-3-carbonsäuremethylester (Verbindung 349),
3-[2-Chlor-4-(2,4-difluorphenylamino)-benzoyl]-*N*-furan-2-ylmethyl-4-methylbenzamid (Verbindung 350),
3-[2-Chlor-4-(2,4-difluorphenylamino)-benzoyl]-*N*-(3-methoxyphenyl)-4-methyl-benzamid (Verbindung 351),
2-{3-[2-Chlor-4-(2,4-difluorphenylamino)-benzoyl]-4-methylbenzoylamino}-benzoesäuremethylester (Verbindung 352),
3-{3-[2-Chlor-4-(2,4-difluorphenylamino)-benzoyl]-4-methylbenzoylamino}-thiophen-2-carbonsäuremethylester (Verbindung 353),
4-{3-[2-Chlor-4-(2,4-difluorphenylamino)-benzoyl]-4-methylbenzoylamino}-thiophen-3-carbonsäure (Verbindung 354),
2-{3-[2-Chlor-4-(2,4-difluorphenylamino)-benzoyl]-4-methylbenzoylamino}-benzoesäure (Verbindung 355),
3-[2-Chlor-4-(2,4-difluorphenylamino)-benzoyl]-*N*-[2-(2-hydroxyethylcarbamoyl)-phenyl]-4-methylbenzamid (Verbindung 356),
3-{3-[2-Chlor-4-(2,4-difluorphenylamino)-benzoyl]-4-methylbenzoylamino}-thiophen-2-carbonsäure-(2-hydroxyethyl)-amid (Verbindung 357),
[2-Chlor-4-(2,4-difluorphenylamino)-phenyl]-(5-ethinyl-2-methylphenyl)-methanon (Verbindung 362),
3-[2-Chlor-4-(2,4-difluorphenylamino)-benzoyl]-4-methylbenzoesäurehydrazid (Verbindung 364),
1-{3-[2-Chlor-4-(2,4-difluorphenylamino)-benzoyl]-4-methylbenzoyl}-4-ethyl-3-thiosemicarbazid (Verbindung 365),
[2-Chlor-4-(2,4-difluorphenylamino)-phenyl]-[5-(5-ethylamino-[1,3,4]thiadiazol-2-yl)-2-methylphenyl]-methanon (Verbindung 366),
[2-Chlor-4-(2,4-difluorphenylamino)-phenyl]-[2-methyl-5-(1H-tetrazol-5-yl)-phenyl]-methanon (Verbindung 367),
3-{3-[2-Chlor-4-(2,4-difluorphenylamino)-benzoyl]-4-methylphenyl}-3-oxopropionsäureethylester (Verbindung 368),
[2-Chlor-4-(2,4-difluorphenylamino)-phenyl]-[5-(4,5-dihydrooxazol-2-yl)-2-methyl-phenyl]-methanon (Verbindung 369),
3-[2-Chlor-4-(2,4-difluorphenylamino)benzoyl]-4-methylbenzoesäure (Verbindung 424),
2-Methacrylsäure-2-{3-[2-chlor-4-(2,4-difluorphenylamino)benzoyl]-4-methylbenzoylamino}-ethylester (Verbindung 425),
3-[2-Chlor-4-(4-chlor-2-fluorphenylamino)-benzoyl]-4-methylbenzoesäure (Verbindung 432),
3-[2-Chlor-4-(2,4-difluorphenylamino)-benzoyl]-4-methoxybenzoesäure (Verbindung 437),
3-[2-Chlor-4-(2,6-difluorphenylamino)-benzoyl]-4-methoxybenzoesäure (Verbindung 443).

26. Pharmazeutische Zusammensetzung, umfassend eine Verbindung gemäß einem der Ansprüche 1-25 oder ein pharmazeutisch verträgliches Salz oder einen Ester davon zusammen mit einem pharmazeutisch verträglichen Träger oder Hilfstoff.

27. Zusammensetzung gemäß Anspruch 26, ferner umfassend eine weitere Wirkkomponente, die ausgewählt ist unter Glucocorticoiden, Vitamin D-Analoga, Antihistaminika, Antagonisten des Plättchenaktivierenden Faktors (PAF), anticholinergen Mitteln, Methylxanthinen, β-adrenergischen Mitteln, COX-2-Inhibitoren, Salicylaten, Indomethacin, Flufenamat, Naproxen, Timegadin, Goldsalzen, Penicillamin, serumcholesterinsenkenden Mitteln, Retinoiden, Zinksalzen und Salicylazosulfapyridin.

28. Verbindung gemäß einem der Ansprüche 1-25 zur Verwendung als Medikament.

29. Verbindung gemäß einem der Ansprüche 1-25 zur Verwendung als antiinflammatorisches Mittel oder Antikrebsmittel.

30. Verbindung gemäß einem der Ansprüche 1-25 zur Verwendung bei der Prävention, Behandlung oder Verbesserung von Augenerkrankungen oder -leiden.

31. Verwendung einer Verbindung gemäß einem der Ansprüche 1-25 zur Herstellung eines Medikaments zur Prophylaxe, Behandlung oder Verbesserung von entzündlichen Erkrankungen oder Leiden oder Augenerkrankungen oder -leiden.

32. Verwendung einer Verbindung gemäß einem der Ansprüche 1-25 zur Herstellung eines Medikaments zur Behandlung oder Verbesserung von Krebs.

33. Verwendung nach Anspruch 31, wobei das Medikament zur gemeinsamen Verabreichung mit einer weiteren Wirkkomponente bestimmt ist, die ausgewählt ist unter Glucocorticoiden, Vitamin D-Analoga, Antihistaminika, Antagonisten des Plättchenaktivierenden Faktors (PAF), anticholinergen Mitteln, Methylxanthinen, βadrenergischen Mitteln, COX-2-Inhibitoren, Salicylaten, Indomethacin, Flufenamat, Naproxen, Timegadin, Goldsalzen, Penicillamin, serumcholesterinsenkenden Mitteln, Retinoiden, Zinksalzen und Salicylazosulfapyridin.

34. Verwendung nach Anspruch 31 oder 33, wobei die entzündliche Erkrankung oder das Leiden Asthma, Allergie, Arthritis, rheumatoide Arthritis, Spondyloarthritis, Gicht. Atherosclerose, eine chronische entzündliche Darmerkrankung, Morbus Crohn, neurologische Entzündungen, entzündliche Augenerkrankungen, proliferative und entzündliche Hauterkrankungen, Psoriasis, atopische Dermatitis, Akne, Uveitis, Sepsis, septischer Schock oder Osteoporose ist.

35. Verwendung nach Anspruch 31, wobei die Augenerkrankung akute Maculadegeneration oder altersbedingte Maculadegeneration ist.

36. Verfahren zur Herstellung einer Verbindung der allgemeinen Struktur I, 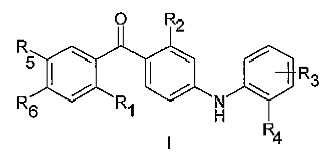 wobei R₁, R₂, R₃, R₄, R₅ und R₆ wie in Anspruch 1 definiert sind, umfassend die Schritte:
a) Überführen einer Verbindung der allgemeinen Struktur VI, 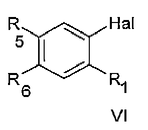 wobei Hal ein Halogen ist und R₁, R₅ und R₆ wie in Anspruch 1 definiert sind und jeweils unabhängig voneinander geschützt oder ungeschützt sind, in ein organometallisches Zwischenprodukt:
b) Transmetallieren des organometallischen Zwischenprodukts zu einem Organozink-Zwischenprodukt:
c) Kuppeln des Organozink-Zwischenprodukts mit einem Säurehalogenid der allgemeinen Struktur V, 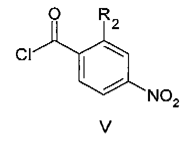 wobei R₂ wie in Anspruch 1 definiert und geschützt oder ungeschützt ist, in Gegenwart eines Katalysators unter Bildung einer Verbindung der allgemeinen Struktur IV, 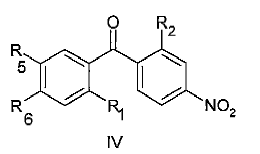 wobei R₁, R₂, R₅ und R₆ wie in Anspruch 1 definiert und jeweils unabhängig voneinander geschützt oder ungeschützt sind;
d) gegebenenfalls Überführen, Schützen oder Entschützen eines oder mehrerer Substituenten oder funktioneller Gruppen von R₁, R₂, R₅ und R₆ der Verbindung der allgemeinen Struktur IV unter Bildung einer weiteren Verbindung der allgemeinen Struktur IV;
e) Reduzieren der Verbindung der allgemeinen Struktur IV aus Schritt c) oder d) zu einem Amin der allgemeinen Struktur III, 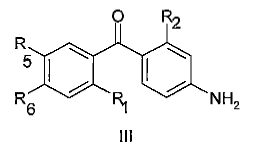 wobei R₁, R₂, R₅ und R₆ wie oben angegeben definiert und jeweils unabhängig voneinander geschützt oder ungeschützt sind;
f) gegebenenfalls Überführen, Schützen oder Entschützen eines oder mehrerer Substituenten oder funktioneller Gruppen von R₁, R₂, R₅ und R₆ der Verbindung der allgemeinen Struktur III unter Bildung einer weiteren Verbindung der allgemeinen Struktur III;
g) Kuppeln des Amins der allgemeinen Struktur III aus Schritt e) oder f) mit einer Verbindung der allgemeinen Struktur II, 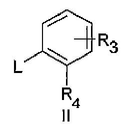 wobei L Triflat oder Halogen ist, R₃ und R₄ wie in Anspruch 1 definiert sind und jeweils unabhängig voneinander geschützt oder ungeschützt sind, unter Bildung einer Verbindung der allgemeinen Struktur I, wobei R₁, R₂, R₃, R₄, R₅ und R₆ wie oben angegeben definiert und jeweils unabhängig geschützt oder ungeschützt sind;
h) gegebenenfalls Überführen, Schützen oder Entschützen eines oder mehrerer Substituenten oder funktioneller Gruppen von R₁, R₂, R₃, R₄, R₅ oder R₆ der Verbindung der allgemeinen Struktur I aus Schritt g) zu einer weiteren Verbindung der allgemeinen Struktur I.

37. Verfahren zur Herstellung einer Verbindung der allgemeinen Struktur I, 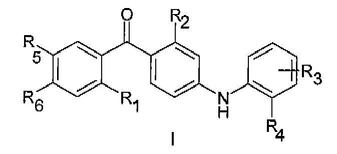 wobei R₁, R₂, R₃, R₄, R₅ und R₆ wie in Anspruch 1 definiert sind, umfassend die Schritte:
a) Überführen einer Verbindung der allgemeinen Struktur VIIa, 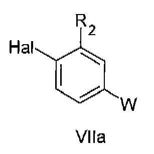 wobei Hal ein Halogen ist, W ein Halogen oder Triflat ist und R₂ wie in Anspruch 1 definiert ist und geschützt oder ungeschützt ist, in ein organometallisches Zwischenprodukt;
b) Transmetallieren des organometallischen Zwischenprodukts zu einem Organozink-Zwischenprodukt;
c) Kuppeln des Organozink-Zwischenprodukts mit einem Säurehalogenid der allgemeinen Struktur VIII, 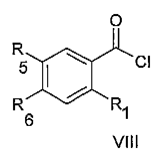 wobei R₁, R₅ und R₆ wie in Anspruch 1 definiert und jeweils unabhängig voneinander geschützt oder ungeschützt sind, in Gegenwart eines Katalysators unter Bildung einer Verbindung der allgemeinen Struktur IIIa, 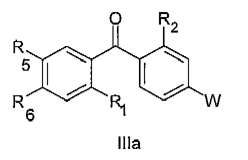 wobei W, R₁, R₂, R₅ und R₆ wie oben angegeben definiert und jeweils unabhängig voneinander geschützt oder ungeschützt sind;
d) gegebenenfalls Überführen, Schützen oder Entschützen eines oder mehrerer Substituenten oder funktioneller Gruppen von W, R₁, R₂, R₅ und R₆ der Verbindung der allgemeinen Struktur IIIa unter Bildung einer weiteren Verbindung der allgemeinen Struktur IIIa;
e) Kuppeln der Verbindung der allgemeinen Struktur IIIa aus Schritt c) oder d) mit einem Amin der allgemeinen Struktur IIa, 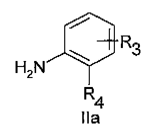 wobei R₃ und R₄ wie in Anspruch 1 definiert und jeweils unabhängig voneinander geschützt oder ungeschützt sind, zu einer Verbindung der allgemeinen Struktur I, wobei R₁, R₂, R₃, R₄, R₅ und R₆ wie oben angegeben definiert und jeweils unabhängig voneinander geschützt oder ungeschützt sind;
f) gegebenenfalls Überführen, Schützen oder Entschützen eines oder mehrerer Substituenten oder funktioneller Gruppen von R₁, R₂, R₃, R₄, R₅ oder R₆ der Verbindung der allgemeinen Struktur I aus Schritt e) unter Bildung einer weiteren Verbindung der allgemeinen Struktur I.

38. Verfahren gemäß Anspruch 36 oder 37, wobei das Kuppeln in Schritt c) in Gegenwart eines Kupfersalzes erfolgt.

## Revendications

1. Composé de formule générale I 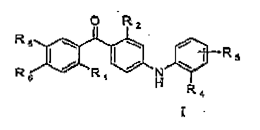 dans laquelle
R₁ est un atome d'halogène, un groupe hydroxy, mercapto, trifluorométhyle, amino, alkyle en C₁-C₄, alcényle en C₂-C₄, alcynyle en C₂-C₄, alcoxy en C₁-C₄, (alkyle en C₁-C₄)thio, (alkyle en C₁-C₆)amino, (alcoxy en C₁-C₄)carbonyle, cyano,-CONH₂ ou nitro ;
R₂ est un atome d'halogène, un groupe amino, alkyle en C₁-C₄ ou nitro ;
R₃ représente un ou plusieurs substituants identiques ou différents choisis dans le groupe constitué par un atome d'hydrogène, un atome d'halogène, un groupe alkyle en C₁-C₄ ou un groupe alcoxy en C₁-C₄ ;
R₄ est un atome de fluor ;
un de R₅ et R₆ est un groupe -COOH, -C(O)NHOH, -C(O)NHNH₂, Y₂R₉, Y₂R₉Y₃R₁₀, (alkyle en C₁-C₆)Y₂R₉, (alkyle en C₁-C₆)Y₂R₉Y₃R₁₀, (alcényle en C₂-C₆)Y₂R₉, (alcényle en C₂-C₆)Y₂R₉Y₃R₁₀, Y₂R₉(alkyle en C₁-C₆)Y₃R₁₀, Y₂R₉(alcényle en C₂-C₆)Y₃R₁₀, (carbocyclyle en C₃-C₁₂)Y₂R₉, (carbocyclyle en C₃-C₁₂)Y₂R₉Y₃R₁₀, (hétérocyclyle en C₁-C₁₂)Y₂R₉, (hétérocyclyle en C₁-C₁₂)Y₂R₉Y₃R₁₀, (carbocyclyle en C₃-C₁₂) (alkyle en C₁-C₆)Y₂R₉, (carbocyclyle en C₃-C₁₂) (alkyle en C₁-C₆)Y₂R₉Y₃P₁₀, (hétérocyclyle en C₁-C₁₂) (alkyle en C₁-C₆)Y₂R₉, (hétérocyclyle en C₁-C₁₂) (alkyle en C₁-C₆)Y₂R₉Y₃R₁₀, (carbocyclyle en C₃-C₁₂)(alkyle en C₁-C₆)Y₃R₁₀, (hétérocyclyle en C₁-C₁₂) (alkyle en C₁-C₆)Y₃R₁₀, (hétérocyclyle en C₁-C₁₂)alkyle en C₁-C₁₀, (carbocyclyle en C₃-C₁₂)alkyle en C₁-C₁₀, (alkyle en C₁-C₁₀)hétérocyclyle en C₁-C₁₂, (alkyle en C₁-C₁₀)carbocyclyle en C₃-C₁₂, alkyle en C₁-C₁₀, alcényle en C₂-C₁₀, alcynyle en C₂-C₁₀, carbocyclyle en C₃-C₁₂ ou hétérocyclyle en C₁-C₁₂, chacun étant facultativement substitué par un ou plusieurs substituants identiques ou différents représentés par R₇, et l'autre est un atome d'hydrogène, d'halogène, un groupe hydroxy, mercapto, trifluorométhyle, amino, alkyle en C₁-C₄, alcényle en C₂-C₄, alcynyle en C₂-C₄, alcoxy en C₁-C₄, (alkyle en C₁-C₄)thio, (alkyle en C₁-C₆)amino, (alcoxy en C₁-C₄)carbonyle, cyano,-CONH₂ ou nitro ;
à condition que lorsque R₅ ou R₆ est un groupe phényle, alkyle en C₁-C₅ ou alcényle en C₂-C₃, ledit R₅ ou R₆ est substitué par un ou plusieurs substituants identiques ou différents représentés par R₇ (à l'exception de trois atomes de fluor lorsque R₅ ou R₆ est un groupe méthyle),
R₇ est un atome d'halogène, un groupe hydroxy, mercapto, trifluorométhyle, amino, alkyle en C₁-C₄, hydroxyalkyle en C₁-C₄, alcoxy en C₁-C₄, (alkyle en C₁-C₄)thio, (alkyle en C₁-C₆)amino, (alcoxy en C₁-C₄)carbonyle, (trialkyle en C₁-C₉)ammonium en association avec un anion, cyano, azido, nitro, -S(O)₂NH₂, -S(O)₂NRₐR_{b}, -S(O)₂R, -COOH, -CONH₂, -NR_{b}C(O)R',-CONHR' ou -CONRR', R et R' étant identiques ou différents et chacun représentant un atome d'hydrogène ou un groupe alkyle en C₁-C₃ ;
Rₐ, R_{b} et R_{c} sont identiques ou différents, chacun représentant un atome d'hydrogène, un groupe alkyle en C₁-C₄, alcényle en C₂-C₄, alcynyle en C₂-C₄, carbocyclyle en C₃-C₈, hétérocyclyle en C₁-C₁₂ ou aryle, chaque groupe alkyle en C₁-C₄, alcényle en C₂-C₄, alcynyle en C₂-C₄, carbocyclyle en C₃-C₈, hétérocyclyle en C₁-C₁₂ ou aryle étant facultativement substitué par un ou plusieurs substituants identiques ou différents représentés par R₇ ;
Y₂ est -O-, -S-, -S(O)-, -S(O)₂-, -NRₐ-, -NRₐC(O)NR_{b}-,-NRₐC(O)-, -C(O)NRₐ-, -C(O)NRₐO- , -C(O)-, -NRₐC(O)O-, -NRₐS(O)₂--OC(O)-, -C(O)O-, -C(O)NRₐNR_{b}C(S)NR_{c}-, -C(O)NRₐNR_{b},- ou-S(O)₂NRₐ- ;
R₉ est un groupe (alkyle en C₁-C₁₀)hétérocyclyle en C₁-C₁₂, (alkyle en C₁-C₁₀) carbocyclyle en C₃-C₁₂, alkyle en C₁-C₁₀, alcényle, en C₂-C₁₀, alcynyle en C₂-C₁₀, carbocyclyle en C₃-C₁₂, hétérocyclyle en C₁-C₁₂, (carbocyclyle en C₃-C₁₂)alkyle en C₁-C₁₀, (hétérocyclyle en C₁-C₁₂) alkyle en C₁-C₁₀, (carbocyclyle en C₃-C₆)alcényle en C₁-C₆, (carbocyclyle en C₃-C₆)alcynyle en C₂-C₆, chacun étant facultativement substitué par un ou plusieurs substituants identiques ou différents représentés par R₇;
à condition que lorsque Y₂ est -O-, -NRₐ-, -S- ou -C(O)O-, R₉ est un groupe alkyle en C₁-C₆, ledit groupe alkyle en C₁-C₆ étant substitué par un ou plusieurs substituants identiques ou différents représentés par R₇ ;
Y₃ est -O-, -S-, -S(O)-, -S(O)₂-, -NRₐ-, -NRₐC(O)NR_{b}-,-NRₐC(O)-, -C(O)NRₐ-, -C(O)NRₐO- , -C(O)-, -NRₐC(O)O-, -NRₐS(O)₂-, -OC(O)- ou -C(O)O- ;
R₁₀ est un groupe (alkyle en C₁-C₁₀)hérérocyclyle en C₁-C₁₂, (alkyle en C₁-C₁₀) carbocyclyle en C₃-C₁₂, alkyle en C₁-C₁₀, alcényle en C₂-C₁₀, alcynyle en C₂-C₁₀, carbocyclyle en C₃-C₁₂ ou hétérocyclyle en C₁-C₁₂, chacun étant facultativement substitué par un ou plusieurs substituants identiques ou différents représentés par R₇ ;
ou lorsqu'un de R₅ ou R₆ est le groupe -C(O)NR₈R₉, R₈ et R₉ ensemble avec l'atome d'azote auquel ils sont liés forment un cycle hétérocyclique en C₁-C₁₂ comprenant facultativement un ou plusieurs hétéroatomes supplémentaires choisis dans le groupe constitué par O, S et N, facultativement substitué par un ou plusieurs substituants représentés par R₇ ;
ou un sel, solvate ou ester pharmaceutiquement acceptable de celui-ci.

2. Composé selon la revendication 1, dans lequel R₁ est un atome d'halogène, un groupe trifluorométhyle, alkyle en C₁-C₄, alcoxy en C₁-C₄ ou nitro.

3. Composé selon la revendication 2, dans lequel R₁ est un groupe méthyle, éthyle, méthoxy, éthoxy, bromo, fluoro ou chloro.

4. Composé selon l'une quelconque des revendications 1 à 3, dans lequel R₂ est un groupe méthyle, éthyle, nitro, bromo, fluoro ou chloro.

5. Composé selon l'une quelconque des revendications 1 à 4, dans lequel R₃ est un atome d'hydrogène, un groupe méthyle, éthyle, méthoxy, éthoxy, bromo, fluoro ou chloro.

6. Composé selon l'une quelconque des revendications 1 à 5, dans lequel R₃ représente un substituant.

7. Composé selon la revendication 6, dans lequel R₃ est en position méta par rapport à R₄ et en position para par rapport à -NH, ou dans lequel R₃ est en position méta par rapport à R₄ et en position ortho par rapport à -NH, ou dans lequel R₃ est en position ortho par rapport à R₄ et en position méta par rapport à -NH.

8. Composé selon l'une quelconque des revendications 1 à 7, dans lequel R₃ est un atome de fluor.

9. Composé selon l'une quelconque des revendications 1 à 8, dans lequel R₇ est un atome d'halogène, un groupe hydroxy, amino, -S(O)₂CH_{3,} trifluorométhyle, cyano, hydroxyalkyle en C₁-C₄, alcoxy en C₁-C₄, alkyle en C₁-C₄, (alkyle en C₁-C₄)thio, (alkyle en C₁-C₄)amino, (alcoxy en C₁-C₄)carbonyle, -COOH, -CONH₂, -S(O)₂NH₂, azido, -CONHR' ou CONRR', R et R' étant tels qu'indiqués selon la revendication 1.

10. Composé selon la revendication 9, dans lequel R₇ est un groupe méthyle, éthyle, méthoxy, éthoxy, hydroxy, méthoxycarbonyle, éthoxycarbonyle, diméthylamino, éthylamino, amino, -COOH, fluoro, chloro, bromo, -CONH₂, -S(O)₂NH₂, azido, méthylthio, -S(O)₂CH₃, trifluorométhyle, cyano ou hydroxyméthyle.

11. Composé selon l'une quelconque des revendications 1 à 10, dans lequel l'un de R₅ et R₆ est un groupe Y₂R₉, (alkyle en C₁-C₄)Y₂R₉, Y₂R₉Y₃R₁₀, (alkyle en C₁-C₄)Y₂R₉Y₃R₁₀, (alcényle en C₂-C₄)Y₂R₉, (alcényle C₂-C₄)Y₂R₉Y₃R₁₀, Y₂R₉(alkyle en C₁-C₄)Y₃R₁₀, Y₂R₉(alcényle en C₂-C₄)Y₃R₁₀, (hétérocyclyle en C₁-C₆) (alkyle en C₁-C₄)Y₂R₉, (alkyle en C₁-C₄)hétérocyclyle en C₂-C₆, (alkyle en C₁-C₄)carbocyclyle en C₃-C₆, (carbocyclyle en C₃-C₆)alkyle en C₁-C₄, alkyle en C₁-C₄ substitué par R⁷, alcényle en C₂-C₄, alcynyle en C₂-C₄, carbocyclyle en C₃-C₆, hétérocyclyle en C₁-C₆, -COOH, -C(O)NHOH ou -C(O)NHNH₂, et l'autre est un atome d'hydrogène, un atome d'halogène, un groupe alkyle en C₁-C₄ ou un groupe alcoxy en C₁-C₄.

12. Composé selon la revendication 11, dans lequel R₅ est un groupe Y₂R₉, (alkyle en C₁-C₄)Y₂R₉, Y₂R₉Y₃R₁₀, (alkyle en C₁-C₄)Y₂R₉Y₃R₁₀, (alcényle en C₂-C₄)Y₂R₉, (alcényle en C₂-C₄)Y₂R₉Y₃R₁₀, Y₂R₉ (alkyle en C₁-C₄)Y₃R₁₀, Y₂R₉(alcényle en C₂-C₄)Y₃R₁₀, (hétérocyclyle en C₁-C₆) (alkyle en C₁-C₄)Y₂R₉, (alkyle en C₁-C₄)hétérocyclyle en C₁-C₆, (alkyle en C₁-C₄)carbocyclyle en C₃-C₆, (carbocyclyle en C₃-C₆)alkyle en C₁-C₄, alkyle en C₁-C₄ substitué par R₇, alcényle en C₂-C₄, alcynyle en C₂-C₄, carbocyclyle en C₃-C₆, hétérocyclyle en C₁-C₆, -COOH, -C(O)NHOH ou -C(O)NHNH₂, et R₆ est un atome d'hydrogène, un atome d'halogène, un groupe alkyle en C₁-C₄ ou un groupe alcoxy en C₁-C₄.

13. Composé selon la revendication 11, dans lequel l'un de R₅ et R₆ est un groupe Y₂R₉, Y₂R₉Y₃R₁₀, phényle, méthylphényle, propényle, méthyle-Y₂R₉, tétrazole, éthynyle, triazole, thiadiazole, dihydrooxazole, -COOH, -C(O)NHOH ou-C(O)NHNH₂, et l'autre est un atome d'hydrogène, de fluor, de chlore, un groupe méthyle ou méthoxy.

14. Composé selon l'une quelconque des revendications 1 à 13, dans lequel R₆ est un atome d'hydrogène.

15. Composé selon l'une quelconque des revendications 1 à 11 ou 13, dans lequel P₅ est un atome d'hydrogène.

16. Composé selon l'une quelconque des revendications 1 à 15, dans lequel Y₂ est -O-, -NRₐ-, -NRₐC(O)NR_{b}-, -NRₐC(O)-,-C(O)NRₐ-, -C(O)NRₐO-, -C(O)-, -NRₐC(O)O-, -NRₐS(O)₂₋,-C (O)NRₐNR_{b}- ou -S(O)₂NRₐ-.

17. Composé selon l'une quelconque des revendications 1 à 16, dans lequel Y₃ est -O-, -NRₐC(O)-, -C(O)NRₐ-, -C(O)-,-C(O)O- ou -NRₐC(O)O-.

18. Composé selon l'une quelconque des revendications 1 à 17, dans lequel R₉ est un groupe (alkyle en C₁-C₄)hétérocyclyle en C₁-C₆, (alkyle en C₁-C₄)carbocyclyle en C₃-C₆, alkyle en C₁-C₆, alcényle en C₂-C₄, alcynyle en C₂-C₄, carbocyclyle en C₃-C₁₀, hétérocyclyle en C₁-C₆, (carbocyclyle en C₃-C₆)alkyle en C₁-C₆, (hétérocyclyle en C₁-C₆)alkyle en C₁-C₆, (carbocyclyle en C₃-C₆)alcényle en C₂-C₄ ou (carbocyclyle en C₃-C₆)alcynyle en C₂-C₄.

19. Composé selon l'une quelconque des revendications 1 à 18, dans lequel R₉ est un groupe hétérocyclyle en C₁-C₄, alkyle en C₁-C₆, (alkyle en C₁-C₃)hérérocyclyle en C₁-C₅, carbocyclyle en C₆-C₁₀, (alkyle en C₁-C₃) carbocyclyle en C₆, alcényle en C₃, (carbocyclyle en C₆)alkyle en C₁, carbocyclyle en C₆)alcényle en C₃ ou (carbocyclyle en C₆)alcynyle en C₂.

20. Composé selon la revendication 19, dans lequel R₉ est un groupe morpholinyle, propylmorpholinyle, pipérazinyle, méthyle, éthyle, n-propyle, n-butyle, tert-butyle, isobutyle, hexyle, isopropyle, diméthylpropyle, méthyltétrahydrofuranyle, méthylpyridinyle, éthylpipérazinyle, cyclohexyle, propyloxopyrrolidinyle, benzyle, méthylcyclohexyle, propylphényle, éthylmorpholinyle, allyle, éthylfuranyle, phényle, méthyldioxoimidazolidinyle, dioxohexahydropyrimidinyle, thiazolyle, méthylphényle, éthylphényle, méthyldioxolanyle, méthylthiazolyle, propénylphényle, méthylfuranyle, thiophényle, tétrahydropyranyle ou éthynylphényle.

21. Composé selon l'une quelconque des revendications 1 à 20, dans lequel R₁₀ est un groupe alkyle en C₁-C₄, alcényle en C₂-C₄, carbocyclyle en C₃-C₆ ou hétérocyclyle en C₁-C₆.

22. Composé selon la revendication 21, dans lequel R₁₀ est un groupe méthyle, éthyle, méthacryle, tert-butyle, tétrahydropyranyle ou éthényle.

23. Composé selon l'une quelconque des revendications 1 à 22, dans lequel ledit hétérocycle ou groupe hétérocyclyle comprend un ou deux atomes d'oxygène ou un atome de soufre, et/ou jusqu'à deux atomes d'azote, ou trois ou quatre atomes d'azote, un ou deux fragments CH₂ du cycle étant facultativement remplacé(s) respectivement par un ou deux fragments -C(O)-.

24. Composé selon l'une quelconque des revendications 1 à 23, dans lequel Rₐ, R_{b} ou R_{c} représentent indépendamment un atome d'hydrogène, un groupe méthyle, éthyle, 2-hydroxyéthyle ou 2-méthoxyéthyle.

25. Composé selon l'une quelconque des revendications 1 à 24, choisi dans le groupe constitué par
3-[2-chloro-4-(2,4-difluorophénylamino)benzoyl]-N-(2-hydroxyéthyl)-4-méthylbenzamide (Composé 115),
3-[2-chloro-4-(4-chloro-2-fluoro-phénylamino)-benzoyl]-N-(2-hydroxyéthyl)-4-méthylbenzamide (Composé 120),
3-[2-chloro-4-(4-chloro-2-fluorophénylamino)-benzoyl]-4,N-diméthylbenzamide (Composé 121),
ester éthylique de l'acide (2-{3-[2-chloro-4-(4-chloro-2-fluorophénylamino)-benzoyl]-4-méthyl-benzoylamino}-acétylamino)-acétique (Composé 122),
ester éthylique de l'acide {3-[2-chloro-4-(4-chloro-2-fluorophénylamino)-benzoyl]-4-méthyl-benzoylamino}-acétique (Composé 123),
3-[2-chloro-4-(4-chloro-2-fluorophénylamino)-benzoyl]-N-(2-méthoxy-éthyl)-4-méthylbenzamide (Composé 124),
3-[2-chloro-4-(4-chloro-2-fluorophénylamino)-benzoyl]-N-cyclohexyl-4-méthylbenzamide (Composé 125),
3-[2-chloro-4-(4-chloro-2-fluorophénylamino)-benzoyl]-N-éthyl-4-méthylbenzamide (Composé 126),
3-[2-chloro-4-(4-chloro-2-fluorophénylamino)-benzoyl]-N-(6-hydroxy-hexyl)-4-méthylbenzamide (Composé 127),
3-[2-chloro-4-(4-chloro-2-fluorophénylamino)-benzoyl]-N-isopropyl-4-méthylbenzamide (Composé 128),
3-[2-chloro-4-(4-chloro-2-fluorophénylamino)-benzoyl]-N-isobutyl-4-méthylbenzamide (Composé 129),
3-[2-chloro-4-(4-chloro-2-fluorophénylamino)-benzoyl]-N-(2,2-diméthylpropyl)-4-méthylbenzamide (Composé 130),
3-[2-chloro-4-(4-chloro-2-fluorophénylamino)-benzoyl]-N-(3-méthoxypropyl)-4-méthylbenzamide (Composé 131),
3-[2-chloro-4-(4-chloro-2-fluorophénylamino)-benzoyl]-4-méthyl-N-[3-(2-oxo-pyrrolidin-1-yl)-propyl]-benzamide (Composé 132),
3-[2-chloro-4-(4-chloro-2-fluorophénylamino)-benzoyl]-N-(2-diméthylamino-éthyl)-4-méthylbenzamide (Composé 133),
ester 2-{3-[2-chloro-4-(4-chloro-2-fluorophénylamino)-benzoyl]-4-méthylbenzoylamino}-éthylique de l'acide 2-méthylacrylique (Composé 134),
3-[2-chloro-4-(4-chloro-2-fluorophénylamino)-benzoyl]-N-cis-(4-hydroxycyclohexyl)-4-méthylbenzamide (Composé 135),
3-[2-chloro-4-(4-chloro-2-fluorophénylamino)-benzoyl]-N-trans-(4-hydroxycyclohexyl)-4-méthylbenzamide (Composé 136),
ester tert-butylique de l'acide (2-{3-[2-chloro-4-(4-chloro-2-fluorophénylamino)-benzoyl]-4-méthylbenzoylamino}-éthyl)-carbamique (Composé 137),
N-(2-amino-éthyl)-3-[2-chloro-4-(4-chloro-2-fluorophénylamino)-benzoyl]-4-méthylbenzamide (Composé 138),
acide (2-{3-[2-chloro-4-(4-chloro-2-fluorophénylamino)-benzoyl]-4-méthylbenzoylamino}-acétylamino)-acétique (Composé 139),
3-[2-chloro-4-(2,4-difluorophénylamino)-benzoyl]-N-(2-hydroxyéthyl)-4-méthoxybenzamide (Composé 140),
3-[2-chloro-4-(2,4-difluorophénylamino)-benzoyl]-N-(2,2-difluoroéthyl)-4-méthoxybenzamide (Composé 141),
3-[2-chloro-4-(2,4-difluorophénylamino)-benzoyl]-N-(2-fluoroéthyl)-4-méthoxybenzamide (Composé 142),
3-[2-chloro-4-(2,4-difluorophénylamino)-benzoyl]-N-(2,3-dihydroxy-propyl)-4-méthoxybenzamide (Composé-143),
N-carbamoylméthyl-3-[2-chloro-4-(2,4-difluorophénylamino)-benzoyl]-4-méthoxybenzamide (Composé 144),
N-carbamoylméthyl-3-[2-chloro-4-(2,4-difluorophénylamino)-benzoyl]-4-méthylbenzamide (Composé 145),
N-benzyl-3-[2-chloro-4-(2,4-difluorophénylamino)-benzoyl]-4-méthylbenzamide (Composé 146),
3-[2-chloro-4-(2,4-difluorophénylamino)-benzoyl]-N-(2-fluoroéthyl)-4-méthylbenzamide (Composé 147),
3-[2-chloro-4-(2,4-difluorophénylamino)-benzoyl]-4-méthyl-N-(2,2,2-trifluoroéthyl)-benzamide (Composé 148),
3-[2-chloro-4-(2,4-difluorophénylamino)-benzoyl]-N-éthyl-4-méthylbenzamide (Composé 149),
3-[2-chloro-4-(2,4-difluorophénylamino)-benzoyl]-N-cyclohexylméthyl-4-méthylbenzamide (Composé 150),
3-[2-chloro-4-(2,4-difluorophénylamino)-benzoyl]-N-(2-hydroxypropyl)-4-méthylbenzamide (Composé 151),
3-[2-chloro-4-(2,4-difluorophénylamino)-benzoyl]-N-(2,3-dihydroxypropyl)-4-méthylbenzamide (Composé 152),
3-[2-chloro-4-(2,4-difluorophénylamino)-benzoyl]-N-(1-hydroxyméthyl-propyl)-4-méthylbenzamide (Composé 153),
3-[2-Chloro-4-(2,4-difluorophénylamino)-benzoyl]-4-méthyl-N-(2,2,3,3,3-pentafluoropropyl)benzamide (Composé 154),
3-[2-chloro-4-(2,4-difluorophénylamino)-benzoyl]-N-(3-hydroxypropyl)-4-méthylbenzamide (Composé 155),
3-[2-chloro-4-(2,4-difluorophénylamino)-benzoyl]-N-(2-hydroxy-1,1-diméthyléthyl)-4-méthylbenzamide (Composé 156),
3-[2-chloro-4-(2,4-difluorophénylamino)-benzoyl]-N-(2-hydroxy-1-hydroxyméthyl-1-méthyléthyl)-4-méthylbenzamide (Composé 157),
ester éthylique de l'acide {3-[2-chloro-4-(2,4-difluorophénylamino)-benzoyl]-4-méthylbenzoylamino}-acétique (Composé 158),
3-[2-chloro-4-(2,4-difluorophénylamino)-benzoyl]-N-(4-hydroxybutyl)-4-méthylbenzamide (Composé 159),
3-[2-chloro-4-(2,4-difluorophénylamino)-benzoyl]-N-(3-hydroxy-1,1-diméthylbutyl)-4-méthylbenzamide (Composé 160),
3-[2-chloro-4-(2,4-difluorophénylamino)-benzoyl]-4-méthyl-N-(3-phénylpropyl)-benzamide (Composé 161),
(R)-3-[2-chloro-4-(2,4-difluorophénylamino)-benzoyl]-N-(1-hydroxyméthyl-3-méthylbutyl)-4-méthylbenzamide (Composé 162),
3-[2-chloro-4-(2,4-difluorophénylamino)-benzoyl]-N-isopropyl-4-méthylbenzamide (Composé 164),
3-[2-chloro-4-(2,4-difluorophénylamino)-benzoyl]-N-cyclohexyl-4-méthylbenzamide (Composé 165),
3-[2-chloro-4-(2,4-difluorophénylamino)-benzoyl]-N-(2,2-difluoroéthyl)-4-méthylbenzamide (Composé 166),
ester méthylique de l'acide 5-{3-[2-chloro-4-(2,4-difluorophénylamino)-benzoyl]-4-méthyl-benzoylamino}-4-oxo-pentanoïque (Composé 167),
N-[(2-carbamoyléthylcarbamoyl)-méthyl]-3-[2-chloro-4-(2,4-difluorophànylamino)-benzoyl]-4-méthylbenzamide (Composé 168),
ester éthylique de l'acide (2-{3-[2-chloro-4-(2,4-difluorophénylamino)-benzoyl]-4-méthyl-benzoylamino}-acétylamino)-acétique (Composé 169),
N-allyl-3-[2-chloro-4-(2,4-difluorophénylamino)-benzoyl]-4-méthylbenzamide (Composé 170),
3-[2-chloro-4-(2,4-difluorophénylamino)-benzoyl]-4-méthyl-N-(2-sulfamoyléthyl)-benzamide (Composé 171),
N-(2-acétylaminoéthyl)-3-[2-chloro-4-(2,4-difluorophénylamino)-benzoyl]-4-méthylbenzamide (Composé 172),
3-[2-chloro-4-(2,6-difluorophénylamino)-benzoyl]-N-(2-hydroxyéthyl)-4-méthoxybenzamide (Composé 173),
3-[2-chloro-4-(2,6-difluorophénylamino)-benzoyl]-N-(2-fluoroéthyl)-4-méthoxybenzamide (Composé 174),
3-[2-chloro-4-(2,6-difluorophénylamino)-benzoyl]-N-(2,3-dihydroxypropyl)-4-méthoxybenzamide (Composé 175),
3-[2-chloro-4-(2,6-difluorophénylamino)-benzoyl]-N-(3-hydroxypropyl)-4-méthoxybenzamide (Composé 176),
3-[2-chloro-4-(2,6-difluorophénylamino)-benzoyl]-4-méthoxy-N-phénéthylbenzamide (Composé 177),
3-[2-chloro-4-(2,6-difluorophénylamino)-benzoyl]-N-(2-hydroxy-1,1-diméthyléthyl)-4-méthoxybenzamide (Composé 178),
3-[2-chloro-4-(2,6-difluorophénylamino)-benzoyl]-4-méthoxy-N-(2-morpholin-4-yl-éthyl)-benzamide (Composé 179),
3-[2-chloro-4-(2,6-difluorophénylamino)-benzoyl]-N-(2-hydroxy-1-hydroxyméthyl-1-méthyléthyl)-4-méthoxybenzamide (Composé 180),
3-[2-chloro-4-(2,6-difluorophénylamino)-benzoyl]-N-(2-hydroxyéthyl)-4-méthoxy-N-méthylbenzamide (Composé 181),
ester éthylique de l'acide {3-[2-chloro-4-(2,6-difluorophénylamino)-benzoyl]-4-méthoxybenzoylamino}-acétique (Composé 182),
ester éthylique de l'acide (2-{3-[2-chloro-4-(2,6-difluorophénylamino)-benzoyl]-4-méthoxybenzoylamino}-acétylamino)-acétique (Composé 183),
3-[2-chloro-4-(2,6-difluorophénylamino)-benzoyl]-N,N-bis-(2-hydroxyéthyl)-4-méthoxybenzamide (Composé 184),
3-[2-chloro-4-(2,6-difluorophénylamino)-benzoyl]-4-méthoxy-N,N-bis-(2-méthoxy-éthyl)-benzamide (Composé 185),
4-chloro-3-[2-chloro-4-(2,4-difluorophénylamino)-benzoyl]-N-(2-hydroxyéthyl)-benzamide (Composé 204),
N-{3-[2-chloro-4-(2,4-difluorophénylamino)-benzoyl]-4-méthylphényl}-3-méthoxypropionamide (Composé 241),
N-{3-[2-chloro-4-(2,4-difluorophénylamino)-benzoyl]-4-méthylphényl}-propionamide (Composé 242),
N-{3-[2-chloro-4-(2,4-difluorophénylamino)-benzoyl]-4-méthylphényl}-2-(2-méthoxyéthoxy)-acétamide (Composé 243),
N-{3-[2-chloro-4-(2,4-difluorophénylamino)-benzoyl]-4-méthylphényl}-3-morpholin-4-yl-propionamide (Composé 244),
N-{3-[2-chloro-4-(2,4-difluorophénylamino)-benzoyl]-4-méthylphényl}-3-hydroxypropionamide (Composé 245),
N-{3-[2-chloro-4-(2,4-difluorophénylamino)-benzoyl]-4-méthylphényl}-3-furan-2-yl-propionamide (Composé 246),
N-{3-[2-chloro-4-(2,4-difluorophénylamino)-benzoyl]-4-méthylphényl}-2-hydroxybenzamide (Composé 247),
N-{3-[2-chloro-4-(2,4-difluorophénylamino)-benzoyl]-4-méthylphényl}-2-(2,5-dioxo-imidazolidin-4-yl)-acétamide (Composé 248),
{3-[2-chloro-4-(2,4-difluorophénylamino)-benzoyl]-4-méthylphényl}-amide de l'acide 2,6-dioxo-hexahydropyrimidine-4-carboxylique (Composé 249),
ester 2-{3-[2-chloro-4-(2,4-difluorophénylamino)-benzoyl]-4-méthylphénylcarbamoyl}-éthylique de l'acide acrylique (Composé 250),
N-{3-[2-chloro-4-(2,4-difluorophénylamino)-benzoyl]-4-méthylphényl}-3-méthylsulfanylpropionamide (Composé 251),
N-{3-[2-chloro-4-(2,4-difluorophénylamino)-benzoyl]-4-méthylphényl}-3-méthanesulfonylpropionamide (Composé 252),
{3-[2-chloro-4-(2,4-difluorophénylamino)-benzoyl]-4-méthylphényl}-amide de l'acide éthanesulfonique (Composé 253),
N-{3-[2-chloro-4-(2,4-difluorophénylamino)-benzoyl]-4-méthylphényl}-4-méthoxybenzènesulfonamide (Composé 254),
N-(5-{3-[2-chloro-4-(2,4-difluorophénylamino)-benzoyl]-4-méthylphénylsulfamoyl}-4-méthyl-thiazol-2-yl)-acétamide. (Composé 255),
5-acétyl-2-chloro-N-{3-[2-chloro-4-(2,4-difluorophénylamino)-benzoyl]-4-méthylphényl}-benzènesulfonamide (Composé 256),
{3-[2-chloro-4-(2,4-difluorophénylamino)-benzoyl]-4-méthylphényl}-amide de l'acide naphtaléne-2-sulfonique (Composé 257),
N-{3-[2-chloro-4-(2,4-difluorophénylamino)-benzoyl]-4-méthylphényl}-C-phényl-méthanesulfonamide (Composé 258),
ester 2-(3-{3-[2-chloro-4-(2,4-difluorophénylamino)-benzoyl]-4-méthylphényl}-uréido)-éthylique de l'acide 2-méthylacrylique (Composé 259),
1-{3-[2-chloro-4-(2,4-difluorophénylamino)-benzoyl]-4-méthylphényl}-3-(2-hydroxyéthyl)-urée (Composé 260),
ester éthylique de l'acide (3-{3-[2-chloro-4-(2,4-difluorophénylamino)-benzoyl]-4-méthylphényl}-uréido)-acétique (Composé 261),
1-{3-[2-chloro-4-(2,4-difluorophénylamino)-benzoyl]-4-méthylphényl}-3-(3-méthoxyphényl)-urée (Composé 262),
1-{3-[2-chloro-4-(2,4-difluorophénylamino)-benzoyl]-4-méthylphényl}-3-(3-trifluorométhylphényl)-urée (Composé 263),
1-{3-[2-chloro-4-(2,4-difluorophénylamino)-benzoyl]-4-méthylphényl}-3-propylurée (Composé 264),
ester éthylique de l'acide 3-(3-{3-[2-chloro-4-(2,4-difluorophénylamino)-benzoyl]-4-méthylphényl}-uréido)-propionique (Composé 265),
1-{3-[2-chloro-4-(2,4-difluorophénylamino)-benzoyl]-4-méthylphényl}-3-cyclohexylurée (Composé 266),
1-allyl-3-{3-[2-chloro-4-(2,4-difluorophénylamino)-benzoyl]-4-méthylphényl}-urée (Composé 267),
1-benzyl-3-{3-[2-chloro-4-(2,4-difluorophénylamino)-benzoyl]-4-méthylphényl}-urée (Composé 268),
1-{3-[2-chloro-4-(2,4-difluorophénylamino)-benzoyl]-4-méthylphényl}-3-éthylurée (Composé 269),
1-{3-[2-chloro-4-(2,4-difluorophénylamino)-benzoyl]-A-méthylphényl}-3-phénylurée (Composé 270),
1-butyl-3-{3-[2-chloro-4-(2,4-difluorophénylamino)-benzoyl]-4-méthylphényl}-urée (Composé 271),
1-{3-[2-chloro-4-(2,4-difluorophénylamino)-benzoyl]-4-méthylphényll-3-phénéthylurée (Composé 272),
ester méthylique de l'acide 2-(3-{3-[2-chloro-4-(2,4-difluorophénylamino)-benzoyl]-4-méthylphényl}-uréido)-benzoïque (Composé 273),
1-{3-[2-ohloro-4-(2,4-difluorophénylamino)-benzoyl]-4-méthylphényl}-3-(3-cyanophényl)-urée (Composé 274),
1-{3-[2-chloro-4-(2,4-difluorophénylamino)-benzoyl]-4-méthylphényl}-3-isopropylurée (Composé 275),
1-{3-[2-chloro-4-(2,4-difluorophénylamino)-benzoyl]-4-méthylphényl}-3-(4-méthoxyphényl)-urée (Composé 276),
ester benzylique de l'acide {3-[2-chloro-4-(2,4-difluorophénylamino)-benzoyl]-4-méthylphényl}-carbamique (Composé 277),
ester allylique de l'acide {3-[2-chloro-4-(2,4-difluorophénylamino)-benzoyl]-4-méthylphényl}-carbamique (Composé 278),
ester éthylique de l'acide {3-[2-chloro-4-(2,4-difluorophénylamino)-benzoyl]-4-méthylphényl}-carbamique (Composé 279),
[2-chloro-4-(2,4-difluorophénylamino)-phényl]-[5-(3-hydroxybutylamino)-2-méthylphényl]-méthanane (Composé 281),
[2-chloro-4-(2,4-difluorophénylamino)-phényl]-(3'-hydroxyméthyl-4-méthy)biphényl-3-yl)-méthanone (Composé 282),
[2-chloro-4-(2,4-difluorophénylamino)-phényl]-(3'-hydroxy-4-méthylbiphényl-3-yl)-méthanone (Composé 283),
[2-chloro-4-(2,4-difluorophénylamino)-phényl]-(4'-méthoxy-4-méthylbiphényl-3-yl)-méthanone (Composé 284),
N-{3'-[2-chloro-4-(2,4-difluorophénylamino)-benzoyl]-4'-méthylbiphényl-3-yl}-acétamide (Composé 285),
[2-chloro-4-(2,4-difluorophénylamino)-phényl]-(4-méthyl-3'-trifluorométhoxybiphényl-3-yl)-méthanone (Composé 286),
[2-chloro-4-(2,4-difluorophénylamino)-phényl]-(3',4',5'-trifluoro-4-méthylbiphényl-3-yl)-méthanone (Composé 288),
[2-cloro-4-(2,4-difluorophénylamino)-phényl]-(3',4'-diméthoxy-4-méthylbiphényl-3-yl)-méthanane (289),
3'-[2-chloro-4-(2,4-difluorophénylamino)-benzoyl]-4'-méthylbiphényl-3-carbonitrile (Composé 290),
3-[2-chloro-4-(2,4-difluorophénylamino)-benzoyl]-N-(2-hydroxyéthyl)-4-méthyl-benzénesulfonamide (Composé 291),
3-[2-chloro-4-(2,4-difluorophénylamino)-benzoyl]-4-méthyl-N-(2-morpholin-4-yl-éthyl)-benzènesulfonamide (Composé 292),
N-allyl-3-[2-chloro-4-(2,4-difluorophénylamino)-benzoyl]-4-méthyl-benzènesulfonamide (Composé 293),
N-(2-{3-[2-chloro-4-(2,4-difluorophénylamino)-benzoyl]-4-méthylbenzènesulfonylamino}-éthyl)-acétamide (Composé 294),
3-[2-chloro-4-(2,4-difluorophénylamino)-benzoyl]-4-méthyl-N-propyl-benzènesulfonamide (Composé 295),
3-[2-chloro-4-(2,4-difluorophénylamino)-benzoyl]-N-(2,3-dihydroxypropyl)-4-méthyl-benzènesulfonamide (Composé 296),
3-[2-chloro-4-(2,4-difluorophénylamino)-benzoyl]-N-(2-méthoxyéthyl)-4-méthyl-benzènesulfonamide (Composé 297),
[4-(2,4-difluorophénylamino)-2-nitrophényl]-[5-(4-méthoxybenzyloxy)-2-méthylphényl]-méthanone (Composé 306),
[4-(2,4-difluorophénylamino)-2-nitrophényl]-[5-(3-hydroxypropoxy)-2-méthylphényl]-méthanone (Composé 307),
[2-amino-4-(2,4-difluorophénylamino)-phényl]-[5-(3-hydroxypropoxy)-2-méthylphényl]-méthanone (Composé 308),
[4-(2,4-difluorophénylamine)-2-nitrophényl]-[2-méthyl-5-(2-morpholin-4-yl-éthoxy)-phényl]-méthanone (Composé 309),
[2-amino-4-(2,4-difluorophénylamino)-phényl]-[2-méthyl-5-(2-morpholin-4-yl-éthoxy)-phényl]-méthanone (Composé 310),
[4-(2,4-difluorophénylamino)-2-nitrophényl]-[5-(2,2-diméthyl-[1,3]dioxolan-4-ylméthoxy)-2-méthylphényl]-méthanone (Composé 311),
[4-(2,4-difluorophénylamino)-2-nitrophényl]-[5-(2,3-dihydroxypropoxy)-2-méthylphényl]-méthanone (Composé 312),
[2-amino-4-(2,4-difluorophénylamino)-phényl]-[5-(2,3-dihydroxypropoxy)-2-méthylphényl]-méthanone (Composé 313),
[2-chloro-4-(2,4-difluorophénylamino)-phényl]-[2-fluoro-5-(3-hydroxypropoxy)-phényl]-méthanone (Composé 314),
[2-chloro-4-(2,4-difluorophénylamino)-phényl]-[5-(2,2-diméthyl-[1,3]dioxolan-4-ylméthoxy)-2-fluorophényl]-méthanone (Composé 315),
[2-chloro-4-(2,4-difluorophénylamino)-phényl]-[5-(2,3-dihydroxypropoxy)-2-fluorophényl]-méthanone (Composé 316),
[2-chloro-4-(2,6-difluorophénylamino)-phényl]-[2-chloro-5-(2-morpholin-4-yl-éthoxy)-phényl]-méthanone (Composé 328),
(±)-[2-chloro-4-(2,6-difluorophénylamino)-phényl]-[2-chloro-5-(2,3-dihydroxypropoxy)-phényl]-méthanone (Composé 329),
[5-(3-bromopropoxy)-2-chlorophényl]-[2-chloro-4-(2,6-difluorophénylamino)-phényl]-méthanone (Composé 330),
[2-chloro-4-(2,4-difluorophénylamino)-phényl]-(5-hydroxyméthyl-2-méthylphényl)-méthanone (Composé 331),
[2-chloro-4-(2,4-difluorophénylamino)-phényl]-(5-chlorométhyl-2-méthylphényl)-méthanone (Composé 332),
(5-azidométhyl-2-méthylphényl)-[2-chloro-4-(2,4-difluorophénylamino)-phényl]-méthanone (Composé 333),
(5-aminométhyl-2-méthylphényl)-[2-chloro-4-(2,4-difluorophénylamino)-phényl]-méthanone (Composé 334),
[2-chloro-4-(2,4-difluorophénylamino)-phényl]-(5-hydroxyméthyl-2-méthoxyphényl)-méthanone (Composé 335),
ester 3-[2-chloro-4-(2,4-difluorophénylamino)-benzoyl]-4-méthoxybenzylique de l'acide acétique (Composé 336),
N-tert-butoxy-3-[2-chloro-4-(2,4-difluorophénylamino)-benzoyl]-4-méthoxybenzamide (Composé 337),
3-[2-chloro-4-(2,4-difluorophénylamino)-benzoyl]-N-méthoxy-4-méthylbenzamide (Composé 338),
N-butoxy-3-[2-chloro-4-(2,4-difluoxophénylamino)-benzoyl]-4-méthylbenzamide (Composé 339),
3-[2-chloro-4-(2,4-difluorophénylamino)-benzoyl]-N-cyclohexylméthoxy-4-méthylbenzamide (Composé 340),
3-[2-chloro-4-(2,4-difluorophénylamino)-benzoyl]-4-méthyl-N-(2-méthyl-thiazol-4-ylméthoxy)-benzamide (Composé 341),
N-benzyloxy-3-[2-chloro-4-(2,4-difluorophénylamino)-benzoyl]-4-méthylbenzamide (Composé 342),
3-[2-chloro-4-(2,4-difluorophénylamino)-benzoyl]-N-(4-méthoxybenzyloxy)-4-méthylbenzamide (Composé 343),
N,N'-diméthylhydrazide de l'acide 3-[2-chloro-4-(2,4-difluorophénylamino)-benzoyl]-4-méthyl-benzoique (Composé 344),
3-[2-chloro-4-(2,4-difluorophénylamino)-benzoyl]-4-méthyl-N-morpholin-4-yl-benzamide (Composé 345),
3-[2-chloro-4-(2,4-difluorophénylamino)-benzoyl]-N-hydroxy-4-méthylbenzamide (Composé 346),
4-[2-chloro-4-(2,4-difluorophénylamino)-benzoyl]-N-(2-hydroxyéthyl)-3-méthylbenzamide (Composé 347),
[2-chloro-4-(2,4-difluorophénylamino)-phényl]-[5-(3-hydroxypropényl)-2-méthylphényl]-méthanone (Composé 348),
ester méthylique de l'acide 4-{3-[2-chloro-4-(2,4-difluorophénylamino)-benzoyl]-4-méthyl-benzoylamino}-thiophène-3-carboxylique (Composé 349),
3-[2-chloro-4-(2,4-difluorophénylamino)-benzoyl]-N-furan-2-ylméthyl-4-méthylbenzamide (Composé 350),
3-[2-chloro-4-(2,4-difluorophénylamino)-benzoyl]-N-(3-méthoxyphényl)-4-méthylbenzamide (Composé 351),
ester méthylique de l'acide 2-{3-[2-chloro-4-(2,4-difluorophénylamino)-benzoyl]-4-méthyl-benzoylamino}-benzoïque (Composé 352),
ester méthylique de l'acide 3-{3-[2-chloro-4-(2,4-difluorophénylamino)-benzoyl]-4-méthylbenzoylamino}-thiophène-2-carboxylique (Composé 353),
acide 4-{3-[2-chloro-4-(2,4-difluorophénylamino)-benzoyl]-4-méthylbenzoylamino}-thiophène-3-carboxylique (Composé 354),
acide 2-{3-[2-chloro-4-(2,4-difluorophénylamino)-benzoyl]-4-méthylbenzoylamino}-benzoïque (Composé 355),
3-[2-chloro-4-(2,4-difluorophénylamino)-benzoyl]-N-[2-(2-hydroxyéthylcarbamoyl)-phényl]-4-méthylbenzamide (Composé 356),
(2-hydroxyéthyl)-amide de l'acide 3-{3-[2-chloro-4-(2,4-difluorophénylamino)-benzoyl]-4-méthylbenzoylamino}-thiophène-2-carboxylique (Composé 357),
[2-chloro-4-(2,4-difluorophénylamino)-phényl]-(5-éthynyl-2-méthylphényl)-méthanone (Composé 362),
hydrazide de l'acide 3-[2-chloro-4-(2,4-difluorophénylamino)-benzoyl]-4-méthyl-benzoïque (Composé 364),
1-{3-[2-chloro-4-(2,4-difluorophénylamino)-benzoyl]-4-méthylbenzoyl}-4-éthyl-3-thiosemicarbazide (Composé 365),
[2-chloro-4-(2,4-difluorophénylamino)-phényl]-[5-(5-éthylamino-[1,3,4]thiadiazol-2-yl)-2-méthylphényl]-méthanone (Composé 366),
[2-chloro-4-(2,4-difluorophénylamino)-phényl]-[2-méthyl-5-(1H-tétrazol-5-yl)-phényl]-méthanone (Composé 367),
ester éthylique de l'acide 3-{3-[2-chloro-4-(2,4-difluorophénylamino)-benzoyl]-4-méthylphényl}-3-oxo-propionique (Composé 368),
[2-chloro-4-(2,4-difluorophénylamino)-phényl]-[5-(4,5-dihydro-oxazol-2-yl)-2-méthylphényl]-méthanone (Composé 369),
acide 3-[2-chloro-4-(2,4-difluorophénylamino)benzoyl]-4-méthylbenzoïque (Composé 424),
ester 2-{3-[2-chloro-4-(2,4-difluorophénylamino)benzoyl]-4-méthylbenzoylamino}éthylique de l'acide 2-méthylacrylique (Composé 425),
acide 3-[2-chloro-4-(4-chloro-2-fluorophénylamino)-benzoyl]-4-méthyl-benzobenzoïque (Composé 432),
acide 3-[2-chloro-4-(2,4-difluorophénylamino)-benzoyl]-4-méthoxybenzoïque (Composé 437),
acide 3-[2-chloro-4-(2,6-difluorophénylamino)-benzoyl]-4-méthoxybenzoïque (Composé 443).

26. Composition pharmaceutique comprenant un composé selon l'une quelconque des revendications 1 à 25 ou un sel ou un ester pharmaceutiquement acceptable de celui-ci avec un véhicule ou un excipient pharmaceutiquement acceptable.

27. Composition selon la revendication 26, comprenant en outre un autre composant actif choisi dans le groupe constitué par les glucocorticoïdes, les analogues de la vitamine D, les antihistaminiques, les antagonistes du facteur d'activation des plaquettes (PAF), les agents anticholinergiques, les méthylxanthines, les agents β-adrénergiques, les inhibiteurs de la COX-2, les salicylates, l'indométhacine, le flufénamate, le naproxène, la timegadine, les sels d'or, la pénicillamine, les agents de réduction du cholestérol sérique, les rétinoïdés, les sels de zinc et la salicylazosulfapyridine.

28. Composé selon l'une quelconque des revendications 1 à 25, pour une utilisation en tant que médicament.

29. Composé selon l'une quelconque des revendications 1 à 25, pour une utilisation en tant qu'agent anti-inflammatoire ou agent anticancéreux.

30. Composé selon l'une quelconque des revendications 1 à 25, pour une utilisation pour la prévention, le traitement ou l'amélioration de maladies ou pathologies ophtalmiques.

31. Utilisation d'un composé selon l'une quelconque des revendications 1 à 25, pour la fabrication d'un médicament pour la prophylaxie, le traitement ou l'amélioration de maladies ou pathologies inflammatoires ou de maladies ou pathologies ophtalmiques.

32. Utilisation d'un composé selon l'une quelconque des revendications 1 à 25, pour la fabrication d'un médicament pour le traitement ou l'amélioration d'un cancer.

33. Utilisation selon la revendication 31, dans laquelle le médicament est destiné à une administration avec un autre composant actif choisi dans le groupe constitué par les glucocorticoïdes, les analogues de la vitamine D, les antihistaminiques, les antagonistes du facteur d'activation des plaquettes (PAF), les agents anticholinergiques, les méthylxanthines, les agents β-adrénergiques, les inhibiteurs de la COX-2, les salicylates, l'indométhacine, le flufénamate, le naproxène, la timegadine, les sels d'or, la pénicillamine, les agents de réduction du cholestérol sérique, les rétinoïdes, les sels de zinc et la salicylazosulfapyridine.

34. Utilisation selon la revendication 31 ou 33, dans laquelle la maladie ou la pathologie inflammatoire est l'asthme, une allergie, l'arthrite, la polyarthrite rhumatoïde, la spondylarthrite, la goutte, l'athérosclérose, une maladie inflammatoire chronique intestinale, la maladie de Crohn, les inflammations neurologiques, les maladies oculaires inflammatoires, les troubles cutanées prolifératifs et inflammatoires, le psoriasis, la dermite atopique, l'acné, l'uvéite, la septicémie, le choc septique, l'acné ou l'ostéoporose.

35. Utilisation selon la revendication 31, dans laquelle la maladie ophtalmique est une dégénérescence maculaire aiguë ou une dégénérescence maculaire liée à l'âge.

36. Procédé de production d'un composé de structure générale I 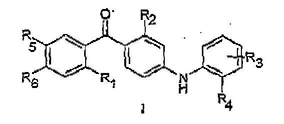 dans laquelle R₁, R₂, R₃, R₄, R₅ et R₆ sont tels que définis selon la revendication 1, comprenant les étapes consistant à
a) transformer un composé de structure générale VI 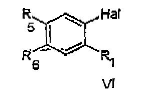 dans laquelle Hal est un atome d'halogène, et R₁, R₅ et R₆ sont tels que définis selon la revendication 1, chacun étant indépendamment protégé ou non protégé, en un intermédiaire organométallique ;
b) transmétaller ledit intermédiaire organométallique en un intermédiaire d'organozinc ;
c) coupler ledit intermédiaire d'organozinc avec un halogénure d'acide de structure générale V 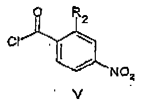 dans laquelle R₂ est tel que défini selon la revendication 1, protégé ou non protégé, en présence d'un catalyseur pour donner un composé de structure générale IV 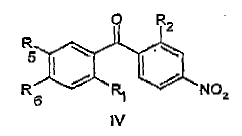 dans laquelle R₁, R₂, R₅ et R₆ sont tels que définis ci-dessus, chacun étant indépendamment protégé ou non protégé ;
d) transformer, protéger ou déprotéger facultativement un ou plusieurs substituants ou groupes fonctionnels de R₁, R₂, R₅ et R₆ du composé de structure générale IV pour donner un autre composé de structure générale IV ;
e) réduire le composé de structure générale IV obtenu dans l'étape c) ou d) en une amine de structure générale III 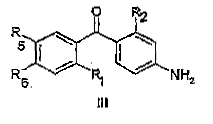 dans laquelle R₁, R₂, R₅ et R₆ sont tels que définis ci-dessus, chacun étant indépendamment protégé ou non protégé ;
f) transformer, protéger ou déprotéger facultativement un ou plusieurs substituants ou groupes fonctionnels de R₁, R₂, R₅ et R₆ du composé de structure générale III pour donner un autre composé de structure générale III ;
g) coupler l'amine de structure générale III obtenue dans l'étape e) ou f) avec un composé de structure générale II 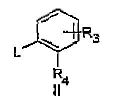 dans laquelle L est un groupe triflate ou un atome d'halogène, R₃ et R₄ sont tels que définis selon la revendication 1, chacun étant indépendamment protégé ou non protégé, pour donner un composé de structure générale 1, dans laquelle R₁, R₂, R₃, R₄, R₅ et R₆ sont tels que définis ci-dessus, chacun étant indépendamment protégé ou non protégé ;
h) transformer, protéger ou déprotéger facultativement un ou plusieurs substituants ou groupes fonctionnels de R₁, R₂, R₃, R₄, R₅ ou R₆ du composé de structure générale I obtenu dans l'étape g) pour donner un autre composé de structure générale I.

37. Procédé de préparation d'un composé de structure générale I 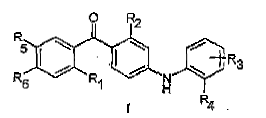 laquelle R₁, R₂, R₃, R₄, R₅ et R₆ sont tels que définis selon la revendication 1, comprenant les étapes consistant à
a) transformer un composé de structure générale VTIa 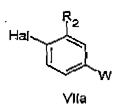 dans laquelle Hal est un atome d'halogène, W est un atome d'halogène ou un groupe triflate et R₂ est tel que défini selon la revendication 1, protégé ou non protégé; en un intermédiaire organométallique ;
b) transmétaller ledit intermédiaire organométallique en un intermédiaire d'organozinc ;
c) coupler ledit intermédiaire d'organozinc avec un halogénure d'acide de structure générale VIII 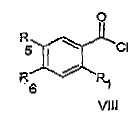 dans laquelle R₁, R₅ et R₆ sont tels que définis selon la revendication 1, chacun étant indépendamment protégé ou non protégé, en présence d'un catalyseur pour donner un composé de structure générale IIIa 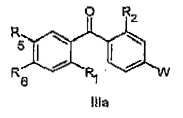 dans laquelle W, R₁, R₂, R₅ et R₆ sont tels que définis ci-dessus, chacun étant indépendamment protégé ou non protégé ;
d) transformer, protéger ou déprotéger facultativement un ou plusieurs substituants ou groupes fonctionnels de W, R₁, R₂, R₅ et R₆ du composé de structure générale IIIa pour donner un autre composé de structure générale IIIa ;
e) coupler le composé de structure générale IIIa obtenu dans l'étape c) ou d) avec une amine de structure générale IIa 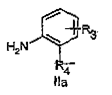 dans laquelle R₃ et R₄ sont tels que définis selon la revendication 1, chacun étant indépendamment protégé ou non protégé, pour donner un composé de structure générale I, dans laquelle R₁, R₂, R₃, R₄, R₅ et R₆ sont tels que définis ci-dessus, chacun étant indépendamment protégé ou non protégé ;
f) transformer, protéger ou déprotéger facultativement un ou plusieurs substituants ou groupes fonctionnels de R₁, R₂, R₃, R₄, R₅ ou R₆ du composé de structure générale I obtenu dans l'étape e) pour donner un autre composé de structure générale I.

38. Procédé selon la revendication 36 ou 37, dans lequel le couplage de l'étape c) est réalisé en présence d'un sel de cuivre.
